# EUROPEAN PATENT SPECIFICATION

(11) **EP 4 007 752 B1**
(45) Date of publication and mention of the grant of the patent: **24.09.2025**
(21) Application number: 20757196.9
(22) Date of filing: 03.08.2020
(51) Int. Cl.: C07D 221/20, C07D 401/12, C07D 405/12, C07D 409/12, C07D 413/12, A61P 35/00, A61K 31/438

(54) **KIF18A INHIBITORS**
KIF18A-INHIBITOREN
INHIBITEURS DE KIF18A

(30) Priority: 02.08.2019 US 201962882255 P
(43) Date of publication of application: 08.06.2022
(73) Proprietor: Amgen Inc., Thousand Oaks, California 91320-1799 (US)
(72) Inventor: TAMAYO, Nuria A., Thousand Oaks, California 91320-1799 (US); BANERJEE, Abhisek, Thousand Oaks, California 91320-1799 (US); CHEN, Jian Jeffrey, Thousand Oaks, California 91320-1799 (US); BOURBEAU, Matthew Paul, Thousand Oaks, California 91320-1799 (US); KALLER, Matthew Richard, Thousand Oaks, California 91320-1799 (US); LOW, Jonathan Dante, Thousand Oaks, California 91320-1799 (US); MINATTI, Ana Elena, Thousand Oaks, California 91320-1799 (US); NGUYEN, Thomas T., Thousand Oaks, California 91320-1799 (US); NISHIMURA, Nobuko, Thousand Oaks, California 91320-1799 (US); PETTUS, Liping H., Thousand Oaks, California 91320-1799 (US); WALTON, Mary Catherine, Thousand Oaks, California 91320-1799 (US); XUE, Qiufen May, Thousand Oaks, California 91320-1799 (US); ALLEN, John Gordon, Thousand Oaks, California 91320-1799 (US)
(74) Representative: Murgitroyd & Company
(86) International application number: PCT/US2020/044797
(87) International publication number: WO 2021/026098

(56) References cited:
- WO-A1-2020/132649
- WO-A1-2020/132653
- JOACHIM BRAUN ET AL: "Synthesis and Biological Evaluation of Optimized Inhibitors of the Mitotic Kinesin Kif18A", ACS CHEMICAL BIOLOGY, vol. 10, no. 2, 26 November 2014 (2014-11-26), pages 554 - 560, XP055671701, ISSN: 1554-8929, DOI: 10.1021/cb500789h

## Description

The invention relates to the field of pharmaceutical agents and, more specifically, is directed to compounds and compositions useful for modulating KIF18A, and to uses and methods for managing cell proliferation and for treating cancer. The references to methods of treatment in the subsequent paragraphs are to be interpreted as references to the compounds, pharmaceutical compositions and medicaments of the present invention for use in a method for treatment of the human (or animal) body by therapy.

### BACKGROUND OF THE INVENTION

Cancer is one of the most widespread diseases afflicting mankind and a major cause of death worldwide. In an effort to find an effective treatment or a cure for one or more of the many different cancers, over the last couple of decades, numerous groups have invested a tremendous amount of time, effort and financial resources. However, to date, of the available cancer treatments and therapies, only a few offer any considerable degree of success.

Cancer is often characterized by unregulated cell proliferation. Damage to one or more genes, responsible for the cellular pathways, which control progress of proliferation through the cell cycle and centrosome cycle, can cause the loss of normal regulation of cell proliferation. These deregulated genes can code for various tumor suppressor or oncogene proteins, which participate in a cascade of events, leading to unchecked cell-cycling progression and cell proliferation. Various kinase and kinesin proteins have been identified, which play key roles in cell cycle and mitotic regulation and progression of normal dividing cells and cancer cells.

Kinesins are molecular motors that play important roles in cell division and intracellular vesicle and organelle transport. Mitotic kinesin plays roles in several aspects of spindle assembly, chromosome segregation, centrosome separation and dynamics (reviewed in O. Rath and F. Kozielski, Nature Review Cancer, 12:527-39, 2012). Human kinesins are categorized into 14 subfamilies based on sequence homology within the so-called "motor domain", this domains ATPase activity drives unidirectional movement along microtubules (MTs). The non-motor domain of these proteins is responsible for cargo attachment; a "cargo" can include any one of a variety of different membranous organelles, signal transduction scaffolding systems, and chromosomes. Kinesins use the energy of ATP hydrolysis to move cargo along polarized microtubules. Thus, kinesins are often called "plus-end" or "minus-end" directed motors.

*KIF18A* gene belongs to Kinesin-8 subfamily and is a plus-end-directed motor. KIF18A is believed to influence dynamics at the plus end of kinetochore microtubules to control correct chromosome positioning and spindle tension. Depletion of human KIF18A leads to longer spindles, increased chromosome oscillation at metaphase, and activation of the mitotic spindle assembly checkpoint in HeLa cervical cancer cells (MI Mayr et al, Current Biology 17, 488-98, 2007). KIF18A appears to be viable target for the treatment of cancer. KIF18A is overexpressed in various types of cancers, including but not limited to colon, breast, lung, pancreas, prostate, bladder, head, neck, cervix, and ovarian cancers.

Further, genetic deletion or knockdown, or inhibition of KIF18A effects mitotic spindle apparatus in cancer cell lines. Particularly, inhibition of KIF18A has been found to induce mitotic cell arrest, a known vulnerability that can promote cell death in mitosis via apoptosis, mitotic catastrophe, or multipolarity driven lethality or death after mitotic slippage in interphase. Accordingly, there has been a strong interest in finding inhibitors of KIF18A proteins.

WO2020132649A1 relates to chemical compounds having a general formula (I), as defined herein, and synthetic intermediates thereof, which are capable of modulating KIF18A protein thereby influencing the process of cell cycle and cell proliferation to treat cancer and cancer-related diseases. WO2020132653A1 relates to chemical compounds having a general formula (I), as defined herein, and synthetic intermediates thereof, which are capable of modulating KIF18A protein thereby influencing the process of cell cycle and cell proliferation to treat cancer and cancer-related diseases

Thus, the inhibition of KIF18A ATPase activity is a promising approach for the development of novel anti-cancer agents.

### SUMMARY OF THE INVENTION

An aspect of the present invention is a new class of compounds useful for modulating KIF18A protein alone or in a bound complex with microtubules for treating KIF18A-mediated conditions and/or diseases, including cancer, inflammation, or ciliopathologies.

The compounds provided by the invention have MT-based KIF18A modulatory activity and, in particular, KIF18A inhibitory activity. To this end, the invention also provides the use of these compounds, as well as pharmaceutically acceptable salts thereof, in the preparation and manufacture of a pharmaceutical composition or medicament for therapeutic, prophylactic, acute or chronic treatment of KIF18A mediated diseases and disorders, including without limitation, cancer. Thus, the compounds of the invention are useful in the manufacture of anti-cancer medicaments. The invention also provides processes for making compounds of Formula I, as well as intermediates useful in such processes.

In embodiment 1, the present invention provides a compound of formula 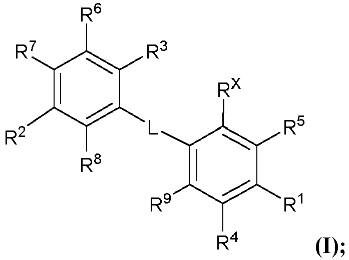 or any pharmaceutically-acceptable salt thereof, wherein:
R^{X} is 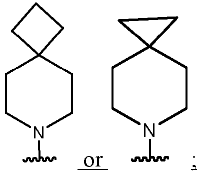
R¹ is a group -Z-R¹²; wherein Z is absent, -C₀₋₄alk-S-C₀₋₄alk-, C₀₋₄alk-S(=O)-C₀₋₄alk-, -C₀₋₄alk-SO₂-C₀₋₄alk-, -C₀₋₄alk-NR¹¹-C₀₋₄alk-, -C₀₋₄alk-NR¹¹SO₂-C₀₋₄alk-, -C₀₋₄alk-SO₂NR¹¹-C₀₋₄alk-, -C₀₋₄alk-NR¹¹SO₂NR¹¹-C₀₋₄alk-, -C₀₋₄alk-O-C₀₋₄alk-, -C₀₋₄alk-C(=O)-C₀₋₄alk-, -C₀₋₄alk-C(=O)-O-C₀₋₄alk-, -C₀₋₄alk-(C=O)NR¹¹-C₀₋₄alk-, -C₀₋₄alk-NR¹¹(C=O)-C₀₋₄alk-, -C₀₋₄alk-S(=O)(=NH)-, -N=S(=O)<, -(C=O)-, or -C(=N-OH)-;
R² is a group -Y-R¹³, wherein Y is -C₀₋₄alk-S-C₀₋₄alk-, C₀₋₄alk-S(=O)-C₀₋₄alk-, -C₀₋₄alk-SO₂-C₀₋₄alk-, -C₀₋₄alk-NR^{13c}-C₀₋₄alk-, -C₀₋₄alk-SO₂NR^{13c}-C₀₋₄alk-, -C₀₋₄alk-NR^{13c}SO₂-C₀₋₄alk-, -C₀₋₄alk-S(=O)(=NH)-, -C₀₋₄alk-O-C₀₋₄alk-, -C₀₋₄alk-C(=O)-C₀₋₄alk-, -C₀₋₄alk-C(=O)-O-C₀₋₄alk-, -C₀₋₄alk-(C=O)NR^{13c}-C₀₋₄alk-, -C₀₋₄alk-NR^{13c}(C=O)-C₀₋₄alk-, -or -N=S(=O)<;
is H, halo, C₁₋₄alk, or C₁₋₄haloalk;
is H, halo, C₁₋₄alk, or C₁₋₄haloalk;
R⁵ is H, halo, C₁₋₈alk, or C₁₋₄haloalk;
R⁶ is H, halo, C₁₋₈alk, or C₁₋₄haloalk;
R⁷ is H, halo, C₁₋₄alk, or C₁₋₄haloalk;
R⁸ is H, halo, C₁₋₈alk, or C₁₋₄haloalk;
or alternatively, R² and R⁸ can combine with the carbon atoms attached to each of them to form a saturated or partially-saturated 5- or 6-membered monocyclic ring fused to the phenyl ring; wherein said 5- or 6-membered monocyclic ring contains 0, 1, 2 or 3 N atoms and 0 or 1 atoms selected from O and S, and further wherein said 5- or 6-membered monocyclic ring is substituted by 0, 1, 2 or 3 group(s) selected from F, Cl, Br, C₁₋₆alk, C₁₋₄haloalk, -OR^{a}, -OC₁₋₄haloalk, CN, -NR^{a}R^{a}, or oxo;
R⁹ is H, halo, C₁₋₄alk, or C₁₋₄haloalk;
L is -(C=O)-NR¹⁰- or -NR¹⁰-(C=O)-;
R¹⁰ is H or C₁₋₄alk;
R¹¹ is H or C₁₋₄alk;
R¹² is R^{12a}, or R^{12b};
R¹³ is halo, R^{13a} or R^{13b} ;
R^{13c} is H or C₁₋₄alk;
R^{12a} and R^{13a} is independently, at each instance, selected from the group consisting of a saturated, partially-saturated or unsaturated 3-, 4-, 5-, 6-, or 7-membered monocyclic or 8-, 9-, 10-, 11-, or 12-membered bicyclic ring containing 0, 1, 2 or 3 N atoms and 0 or 1 atoms selected from O and S, which is substituted by 0, 1, 2 or 3 group(s) selected from the group consisting of F, Cl, Br, C₁₋₆alk, C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, a saturated, partially-saturated or unsaturated 3-, 4-, 5-, or 6-membered monocyclic ring, and oxo;
R^{12b} and R^{13b} is independently, at each instance, selected from the group consisting of C₁₋₆alk substituted by 0, 1, 2, 3, 4, or 5 group(s) selected from the group consisting of F, Cl, Br, -CH₂F, -CHF₂, -CF₃, -C(=O)OR^{a}, -OR^{a}, -OC₁₋₄haloalk, CN, NH₂, NH(CH₃), N(CH₃)₂, and a saturated, partially-saturated or unsaturated 3-, 4-, 5-, or 6-membered monocyclic ring;
R^{a} is independently, at each instance, H or R^{b}; and
R^{b} is independently, at each instance, C₁₋₆alk, phenyl, or benzyl, wherein the C₁₋₆alk is being substituted by 0, 1, 2 or 3 substituents selected from halo, -OH, -OC₁₋₄alk, -NH₂, -NHC₁₋₄alk, -OC(=O)C₁₋₄alk, or -N(C₁₋₄alk)C₁₋₄alk; and the phenyl or benzyl is being substituted by 0, 1, 2 or 3 substituents selected from halo, C₁₋₄alk, C₁₋₃haloalk, -OH, -OC₁₋₄alk, -NH₂, -NHC₁₋₄alk, -OC(=O)C₁₋₄alk, or -N(C₁₋₄alk)C₁₋₄alk.

In embodiment 2, the present invention provides compounds of embodiment 1 wherein L is -NR¹⁰-(C=O)-.

In embodiment 3, the present invention provides compounds of embodiment 1 wherein L is -(C=O)-NR¹⁰-.

In embodiment 4, the present invention provides compounds of embodiment 1 wherein R¹⁰ is H or methyl.

In embodiment 5, the present invention provides compounds of embodiment 1 wherein Z is absent, -SO₂-, -CH₂-SO₂, -NH-, -NHSO₂-, -SO₂NH-, -SO₂N(CH₃)-, -O-, -(C=O)O-, -(C=O)NH-, -(C=O)N(CH₃)-, -S(=O)(=NH)-, -CH₂-N(CH₃)-, or -C(=N-OH)-.

In embodiment 6, the present invention provides compounds of embodiment 1 wherein R¹² is selected from:
a) H, F, Br, OH, or CN;
b) R^{12a} selected from a saturated, partially-saturated or unsaturated 3-, 4-, 5-, 6-, or 7-membered monocyclic ring containing 0, 1, 2 or 3 N atoms and 0 or 1 atoms selected from O and S, which is substituted by 0, 1, 2 or 3 group(s) selected from F, Cl, Br, methyl, ethyl, -CF₃, -CH₂OH, -OH, -OCH₃, -NH₂, or oxo; or
c) R^{12b} selected from C₁₋₆alk substituted by 0, 1, 2 or 3 group(s) selected from F, Cl, Br, -CF₃, or -OH.

In embodiment 7, the present invention provides compounds of embodiment 1 wherein R¹² is R^{12a} selected from cyclopropyl, oxetanyl, imidazolyl, isothiazolidinyl, azetidinyl, oxazolyl, pyrazolyl, or diazirinyl; each of which is independently substituted by 0, 1, 2 or 3 group(s) selected from methyl, ethyl, - CF₃, or oxo; or R^{12b} selected from methyl, ethyl, isopropyl, tert-butyl,-ethenyl, substituted by 0, 1, 2 or 3 group(s) selected from F, Cl, Br, -CF₃, or -OH.

In embodiment 8, the present invention provides compounds of embodiment 1 wherein R¹ is a group -Z-R¹², wherein Z is absent, -SO₂-, -CH₂SO₂-, -(C=O)NH-, -NH-, -NHSO₂- or -SO₂NH-; and R¹² is cyclopropyl, oxetanyl, azetidinyl, or imidazolyl ring, each of which is independently substituted by 0, 1, or 2 group(s) selected from methyl, -CF₃, or oxo; or R¹² is methyl, ethyl, isopropyl, or *tert*-butyl, each of which is independently substituted by 0, 1, 2 or 3 F, -CF₃ or OH group(s).

In embodiment 9, the present invention provides compounds of embodiment 1 wherein R¹ is a group -Z-R¹², wherein Z is -NHSO₂- and R¹² is -CH₂-CH₂-OH or -CH(CH₃)CH₂OH.

In embodiment 10, the present invention provides compounds of embodiment 1 wherein Y is absent,
- SO₂NH-,
- NH-, - SO₂-, -S(=O)(=NH)-, or -O-.

In embodiment 11, the present invention provides compounds of embodiment 1 wherein R¹³ is H or F; R^{13a} selected from morpholinyl, piperidinyl, cyclopentyl, cyclopropyl, azetidinyl, or oxetanyl; wherein each said ring is substituted by 0, 1, 2 or 3 OH group(s) selected from methyl or -OH; or R^{13b} selected from methyl, ethyl, propyl, isopropyl, *tert*-butyl, or isopentyl; each of which is independently substituted by 0, 1, 2 or 3 OH group(s).

In embodiment 12, the present invention provides compounds of embodiment 1 wherein R² and R⁸ can combine with the carbon atoms attached to each of them to form a saturated or partially-saturated 6-membered monocyclic ring fused to the phenyl ring; wherein said 6-membered monocyclic ring contains 0, 1, 2 or 3 N atoms and 0 or 1 atoms selected from O and S, and further wherein said 6-membered monocyclic ring is substituted by 0, 1, 2 or 3 group(s) selected from F, Cl, Br, C₁₋₆alk, C₁₋₄haloalk, or oxo.

In embodiment 13, the present invention provides compounds of embodiment 1 wherein R⁴ is H.

In embodiment 14, the present invention provides compounds of embodiment 1 wherein R⁵ is H

In embodiment 15, the present invention provides compounds of embodiment 1 wherein R⁶ is H or F.

In embodiment 16, the present invention provides compounds of embodiment 1 wherein R⁷ is H or F.

In embodiment 17, the present invention provides compounds of embodiment 1 wherein R⁸ is H, F, or methyl; or alternatively, R² and R⁸ can combine with the carbon atoms attached to each of them to form a saturated 6-membered monocyclic ring fused to the phenyl ring; selected from the group:

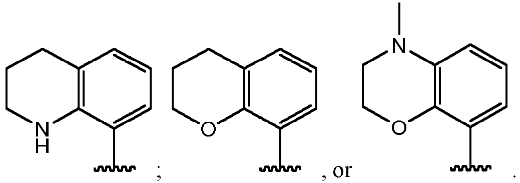

In embodiment 18, the present invention provides compounds of embodiment 1 wherein R² is:
a) a group -Y-R^{13a}, wherein Y is absent or -S(=O)(=NH)-; and R¹³ is piperidinyl or azetidinyl; wherein each said ring is independently substituted by 0, 1, 2 or 3 F group(s);
b) a group -Y-R¹³ , wherein Y is -SO₂NH-, -O-. NH-; and wherein R^{13b} is *tert-butyl* substituted by 0, 1, 2, or 3 OH group(s); or
c) alternatively, the carbon atoms attached to R² and R⁸ combine to form a saturated 6-membered monocyclic ring fused to the phenyl ring; which is 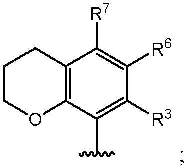 wherein said 6-membered monocyclic ring is unsubstituted.

In embodiment 19, the present invention provides compounds of embodiment 1 wherein R² is - SO₂NH-*tert*-butyl group or -NH- tert-butyl-OH group.

In embodiment 20, the present invention provides compounds of embodiment 1 wherein R⁸ is H.

In embodiment 21, the present invention provides compounds of embodiment 1 wherein R⁹ is H.

In embodiment 23, the present invention provides compounds of embodiment 1 wherein R¹⁰ is H.

In embodiment 22, the present invention provides a compound, or pharmaceutically acceptable salts thereof, selected from:

In embodiment 23, the present invention provides a compound, or the pharmaceutically acceptable salt thereof, selected from the group consisting of:
*N*-(3-(*N*-(*tert*-butyl)sulfamoyl)phenyl)-4-((3-methyloxetan-3-yl)sulfonyl)-2-(6-azaspiro[2.5]octan-6-yl)benzamide;
*N*-(3-(*N*-(*tert*-butyl)sulfamoyl)phenyl)-4-(methylsulfonyl)-2-(6-azaspiro[2.5]octan-6-yl)benzamide;
*N*-(3-isopropylphenyl)-4-(methylsulfonyl)-2-(6-azaspiro[2.5]octan-6-yl)benzamide;
*N*-(3-cyclopropylphenyl)-4-(methylsulfonyl)-2-(6-azaspiro[2.5]octan-6-yl)benzamide;
*N*-(3-(*tert*-butyl)phenyl)-4-(methylsulfonyl)-2-(6-azaspiro[2.5]octan-6-yl)benzamide;
4-(methylsulfonyl)-*N*-(quinolin-8-yl)-2-(6-azaspiro[2.5]octan-6-yl)benzamide;
*N*-(4-methylquinolin-8-yl)-4-(methylsulfonyl)-2-(6-azaspiro[2.5]octan-6-yl)benzamide;
*N*-(chroman-8-yl)-4-(methylsulfonyl)-2-(6-azaspiro[2.5]octan-6-yl)benzamide;
*N*-(benzofuran-7-yl)-4-(methylsulfonyl)-2-(6-azaspiro[2.5]octan-6-yl)benzamide;
*N*-(benzo[b]thiophen-7-yl)-4-(methylsulfonyl)-2-(6-azaspiro[2.5]octan-6-yl)benzamide;
4-(methylsulfonyl)-*N*-(3-morpholinophenyl)-2-(6-azaspiro[2.5]octan-6-yl)benzamide;
*N*-(3-(*N*-(*tert*-butyl)sulfamoyl)phenyl)-4-((methylsulfonyl)methyl)-2-(6-azaspiro[2.5]octan-6-yl)benzamide;
*N*-(3-(*N*-(*tert*-butyl)sulfamoyl)phenyl)-4-(((2-hydroxyethyl)sulfonyl)methyl)-2-(6-azaspiro[2.5]octan-6-yl)benzamide;
4-(*N*-(*tert*-butyl)sulfamoyl)-*N*-(3-(*N*-(*tert*-butyl)sulfamoyl)phenyl)-2-(6-azaspiro[2.5]octan-6-yl)benzamide;
*N*-(3-(*N*-(*tert*-butyl)sulfamoyl)phenyl)-4-(3-methyloxetan-3-yl)-2-(6-azaspiro[2.5]octan-6-yl)benzamide;
*N*-(3-(*N*-(*tert*-butyl)sulfamoyl)phenyl)-4-(3-hydroxyoxetan-3-yl)-2-(6-azaspiro[2.5]octan-6-yl)benzamide;
*N*-(3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide;
*N*-(2-fluoro-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide;
*N*-(2-fluoro-3-((1-hydroxy-2-methylpropan-2-yl)amino)phenyl)-4-((1-methylcyclopropane)-1-sulfonamido)-2-(6-azaspiro[2.5]octan-6-yl)benzamide;
*N*-(3-((1-hydroxy-2-methylpropan-2-yl)amino)phenyl)-4-((1-methylcyclopropane)-1-sulfonamido)-2-(6-azaspiro[2.5]octan-6-yl)benzamide;
*N*-(3-(*N*-(*tert*-Butyl)sulfamoyl)phenyl)-4-((1-methylcyclopropane)-1-sulfonamido)-2-(6-azaspiro[2.5]octan-6-yl)benzamide;
*N*-(3-(*N*-(*tert*-butyl)sulfamoyl)phenyl)-4-(methylsulfonamido)-2-(6-azaspiro[2.5]octan-6-yl)benzamide;
*N*-(3-(*N*-(*tert*-butyl)sulfamoyl)phenyl)-4-(ethylsulfonamido)-2-(6-azaspiro[2.5]octan-6-yl)benzamide;
*N*-(3-(*N*-(*tert*-butyl)sulfamoyl)phenyl)-4-((1-methylethyl)sulfonamido)-2-(6-azaspiro[2.5]octan-6-yl)benzamide;
*N*-(3-(*N*-(*tert*-butyl)sulfamoyl)phenyl)-4-(cyclopropanesulfonamido)-2-(6-azaspiro[2.5]octan-6-yl)benzamide;
*N*-(3-(*N*-(*tert*-butyl)sulfamoyl)phenyl)-4-((1,1-dimethylethyl)sulfonamido)-2-(6-azaspiro[2.5]octan-6-yl)benzamide;
*N*-(3-(*N*-(*tert-*butyl)sulfamoyl)phenyl)-4-(1,1-dioxidoisothiazolidin-2-yl)-2-(6-azaspiro[2.5]octan-6-yl)benzamide;
*N*-(3-(*N*-(*tert*-butyl)sulfamoyl)phenyl)-4-((2-hydroxyethyl)sulfonamido)-2-(6-azaspiro[2.5]octan-6-yl)benzamide;
(*R*)-4-((2-hydroxyethyl)sulfonamido)-*N*-(3-(2-methylmorpholino)phenyl)-2-(6-azaspiro[2.5]octan-6-yl)benzamide;
(*R*)-*N*-(2-fluoro-3-(2-methylmorpholino)phenyl)-4-((2-hydroxyethyl)sulfonamido)-2-(6-azaspiro[2.5]octan-6-yl)benzamide;
(*R*)-*N*-(3-fluoro-5-(2-methylmorpholino)phenyl)-4-((2-hydroxyethyl)sulfonamido)-2-(6-azaspiro[2.5]octan-6-yl)benzamide;
(*R*)-*N*-(4-fluoro-3-(2-methylmorpholino)phenyl)-4-((2-hydroxyethyl)sulfonamido)-2-(6-azaspiro[2.5]octan-6-yl)benzamide;
*N*-(3-(4,4-difluoropiperidin-1-yl)-5-methylphenyl)-4-((2-hydroxyethyl)sulfonamido)-2-(6-azaspiro[2.5]octan-6-yl)benzamide;
*N*-(3-(4,4-difluoropiperidin-1-yl)phenyl)-4-((2-hydroxyethyl)sulfonamido)-2-(6-azaspiro[2.5]octan-6-yl)benzamide;
*N*-(3-(4,4-difluoropiperidin-1-yl)-2-fluorophenyl)-4-((2-hydroxyethyl)sulfonamido)-2-(6-azaspiro[2.5]octan-6-yl)benzamide;
(*S*)-*N*-(3-(N-(tert-butyl)sulfamoyl)phenyl)-4-((2-hydroxy-1-methylethyl)sulfonamido)-2-(6-azaspiro[2.5]octan-6-yl)benzamide;
(*R*)*-N-*(3-(*N*-(*tert*-butyl)sulfamoyl)phenyl)-4-((2-hydroxy-1-methylethyl)sulfonamido)-2-(6-azaspiro[2.5]octan-6-yl)benzamide;
*N*-(3-(2-Hydroxy-2-methylpropoxy)phenyl)-4-((2-hydroxyethyl)sulfonamido)-2-(6-azaspiro[2.5]octan-6-yl)benzamide;
4-(Azetidin-1-ylsulfonyl)-*N*-(3-(*N*-(*tert*-butyl)sulfamoyl)phenyl)-2-(6-azaspiro[2.5]octan-6-yl)benzamide;
*N*-(3-(*N*-(*tert*-butyl)sulfamoyl)phenyl)-4-(*N*-(1-hydroxy-2-methylpropan-2-yl)sulfamoyl)-2-(6-azaspiro[2.5]octan-6-yl)benzamide;
*N*-(3-(*N*-(*tert*-butyl)sulfamoyl)phenyl)-4-(*N*-(2-hydroxyethyl)sulfamoyl)-2-(6-azaspiro[2.5]octan-6-yl)benzamide;
*N*-(3-(*N*-(*tert*-butyl)sulfamoyl)phenyl)-4-(*N*,*N*-dimethylsulfamoyl)-2-(6-azaspiro[2.5]octan-6-yl)benzamide;
*N*-(3-(*N*-(*tert*-butyl)sulfamoyl)phenyl)-4-(*N*-methylsulfamoyl)-2-(6-azaspiro[2.5]octan-6-yl)benzamide;
*N*-(3-(*N*-(*tert*-butyl)sulfamoyl)phenyl)-4-(*N*-(oxetan-3-yl)sulfamoyl)-2-(6-azaspiro[2.5]octan-6-yl)benzamide;
*N*-(3 -(*N*-(*tert*-butyl)sulfamoyl)phenyl)-4-(N-cyclopropylsulfamoyl)-2-(6-azaspiro[2.5]octan-6-yl)benzamide;
*N*-(3-(N-(tert-butyl)sulfamoyl)phenyl)-4-(N-(1-methylcyclopropyl)sulfamoyl)-2-(6-azaspiro[2.5]octan-6-yl)benzamide;
*N*-(3-(*N*-(*tert*-butyl)sulfamoyl)phenyl)-2-(6-azaspiro[2.5]octan-6-yl)-4-sulfamoylbenzamide;
(*R*)*-N*-(3-(*N*-(*tert*-butyl)sulfamoyl)phenyl)-4-(1,2-dihydroxypropan-2-yl)-2-(6-azaspiro[2.5]octan-6-yl)benzamide;
(*S*)-*N*-(3-(*N*-(*tert*-butyl)sulfamoyl)phenyl)-4-(1,2-dihydroxypropan-2-yl)-2-(6-azaspiro[2.5]octan-6-yl)benzamide;
*N*-(3-(*N*-(*tert*-butyl)sulfamoyl)phenyl)-4-(1-methyl-1H-imidazol-2-yl)-2-(6-azaspiro[2.5]octan-6-yl)benzamide;
*N*-(3-(*N*-(*tert*-butyl)sulfamoyl)phenyl)-4-(1-methyl-1H-pyrazol-5-yl)-2-(6-azaspiro[2.5]octan-6-yl)benzamide;
*N*-(3-(*N*-(*tert*-butyl)sulfamoyl)phenyl)-4-(1-methyl-1H-pyrazol-4-yl)-2-(6-azaspiro[2.5]octan-6-yl)benzamide;
*N*-(3-(*N*-(*tert*-butyl)sulfamoyl)phenyl)-4-(oxazol-2-yl)-2-(6-azaspiro[2.5]octan-6-yl)benzamide;
*N*-(3-(*N*-(*tert*-Butyl)sulfamoyl)phenyl)-4-((1-hydroxy-2-methylpropan-2-yl)amino)-2-(6-azaspiro[2.5]octan-6-yl)benzamide;
*N*-(3-(*N*-(*tert*-butyl)sulfamoyl)phenyl)-4-((2-hydroxyethyl)amino)-2-(6-azaspiro[2.5]octan-6-yl)benzamide;
*N*¹-(3-(*N*-(*tert*-Butyl)sulfamoyl)phenyl)-*N⁴*-(2-hydroxyethyl)-2-(6-azaspiro[2.5]octan-6-yl)terephthalamide;
*N¹*-(3-(*N*-(*tert*-butyl)sulfamoyl)phenyl)-*N⁴*-methyl-2-(6-azaspiro[2.5]octan-6-yl)terephthalamide;
*N*¹-(3-(*N-*(*tert*-butyl)sulfamoyl)phenyl)-*N⁴*-(2-hydroxyethyl)-*N⁴*-methyl-2-(6-azaspiro[2.5]octan-6-yl)terephthalamide;
*N¹* -(3-(*N-*(*tert*-butyl)sulfamoyl)phenyl)-*N⁴*-(1-hydroxy-2-methylpropan-2-yl)-2-(6-azaspiro[2.5]octan-6-yl)terephthalamide;
*N¹*-(3-(cyclopentylsulfonyl)phenyl)-*N⁴*-(2-hydroxyethyl)-2-(6-azaspiro[2.5]octan-6-yl)terephthalamide;
(*R*)-*N*-(3-(Azetidine-1-sulfonimidoyl)phenyl)-4-((2-hydroxyethyl)sulfonamido)-2-(6-azaspiro[2.5]octan-6-yl)benzamide;
(*S*)-*N*-(3-(Azetidine-1-sulfonimidoyl)phenyl)-4-((2-hydroxyethyl)sulfonamido)-2-(6-azaspiro[2.5]octan-6-yl)benzamide;
(*R)*-4-((2-hydroxyethyl)sulfonamido)-*N*-(3-(S-methylsulfonimidoyl)phenyl)-2-(6-azaspiro[2.5]octan-6-yl)benzamide;
(*S*)-4-((2-hydroxyethyl)sulfonamido)-*N*-(3-(S-methylsulfonimidoyl)phenyl)-2-(6-azaspiro[2.5]octan-6-yl)benzamide;
(*R*)-4-((2-hydroxyethyl)sulfonamido)-*N*-(3-(2-methylpropan-2-ylsulfonimidoyl)phenyl)-2-(6-azaspiro[2.5]octan-6-yl)benzamide;
(*S*)-4-((2-hydroxyethyl)sulfonamido)-*N*-(3-(2-methylpropan-2-ylsulfonimidoyl)phenyl)-2-(6-azaspiro[2.5]octan-6-yl)benzamide; or
*N*-(4-((2-hydroxyethyl)sulfonamido)-2-(6-azaspiro[2.5]octan-6-yl)phenyl)-3-(piperidin-1-yl)benzamide.

In embodiment 24, the present invention provides a compound, or the pharmaceutically acceptable salt thereof, selected from the group consisting of:

| Example # | Chemical Structure | Chemical Name |
|---|---|---|
| 100 | 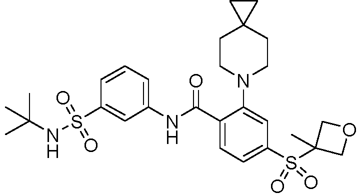 | *N*-(3-(*N*-(*tert*-Butyl)sulfamoyl)phenyl)-4-((3-methyloxetan-3-yl)sulfonyl)-2-(6-azaspiro[2.5]octan-6-yl)benzamide |
| 100-7 | 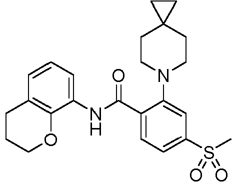 | **[0125]** *N*-(Chroman-8-yl)-4-(methylsulfonyl)-2-(6-azaspiro[2.5]octan-6-yl)benzamide |
| 100-11 | 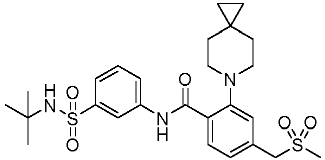 | *N*-(3-(*N*-(*tert*-Butyl)sulfamoyl)phenyl)-4-((methylsulfonyl)methyl)-2-(6-azaspiro[2.5]octan-6-yl)benzamide |
| 100-13 | 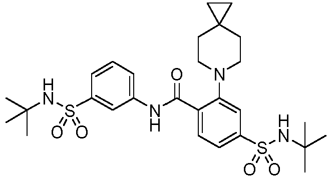 | 4-(*N*-(*tert*-Butyl)sulfamoyl)-N-(3-(*N-*(*tert*-butyl)sulfamoyl)phenyl)-2-(6-azaspiro[2.5]octan-6-yl)benzamide |
| 101 | 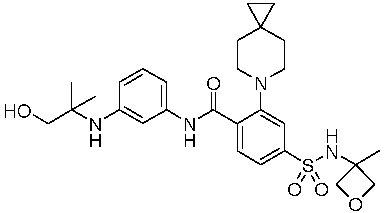 | *N*-(3-((1-Hydroxy-2-methylpropan-2-yl)amino)phenyl)-4-(*N*-(3-methyloxetan-3-yl)sulfamoyl)-2-(6-azaspiro[2.5]octan-6-yl)benzamide |
| 101-1 | 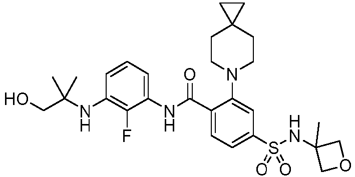 | *N*-(2-Fluoro-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide |
| 102 | 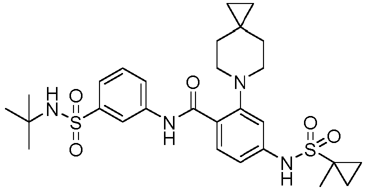 | **[0126]** *N*-(3-(*N*-(*tert-*Butyl)sulfamoyl)phenyl)-4-((1-methylcyclopropane)-1-sulfonamido)-2-(6-azaspiro[2.5]octan-6-yl)benzamide |
| 102-3 | 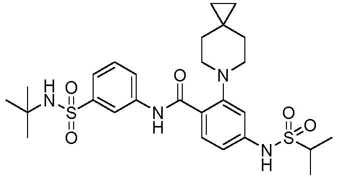 | *N*-(3-(*N*-(*tert*-Butyl)sulfamoyl)phenyl)-4-((1-methylethyl)sulfonamido)-2-(6-azaspiro[2.5]octan-6-yl)benzamide |
| 102-4 | 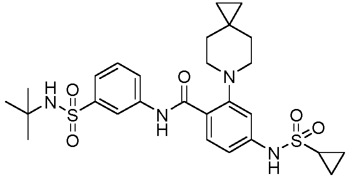 | *N*-(3-(*N*-(*tert*-Butyl)sulfamoyl)phenyl)-4-(cyclopropanesulfonamido)-2-(6-azaspiro[2.5]octan-6-yl)benzamide |
| 103 | 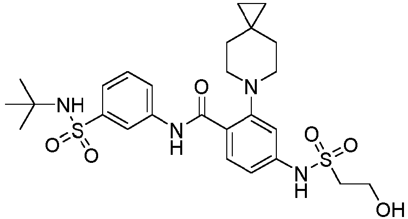 | *N*-(3-(*N*-(*tert*-Butyl)sulfamoyl)phenyl)-4-((2-hydroxyethyl)sulfonamido)-2-(6-azaspiro[2.5]octan-6-yl)benzamide |
| 104 | 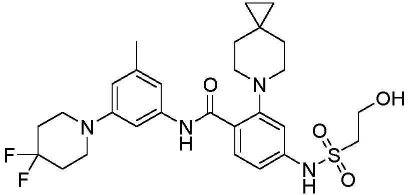 | *N*-(3-(4,4-Difluoropiperidin-1-yl)-5-methylphenyl)-4-((2-hydroxyethyl)sulfonamido)-2-(6-azaspiro[2.5]octan-6-yl)benzamide |
| 104-1 | 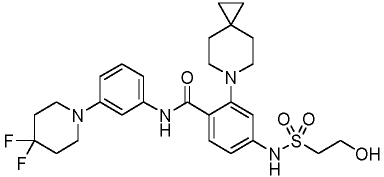 | *N*-(3-(4,4-Difluoropiperidin-1-yl)phenyl)-4-((2-hydroxyethyl)sulfonamido)-2-(6-azaspiro[2.5]octan-6-yl)benzamide |
| 104-2 | 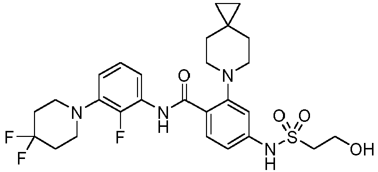 | *N*-(3-(4,4-Difluoropiperidin-1-yl)-2-fluorophenyl)-4-((2-hydroxyethyl)sulfonamido)-2-(6-azaspiro[2.5]octan-6-yl)benzamide |
| 105-1 | 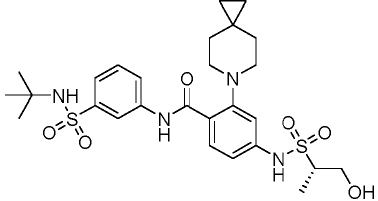 | (*S*)-*N*-(3-(*N*-(*tert-*Butyl)sulfamoyl)phenyl)-4-((2-hydroxy-1-methylethyl)sulfonamido)-2-(6-azaspiro[2.5]octan-6-yl)benzamide |
| 105-2 | 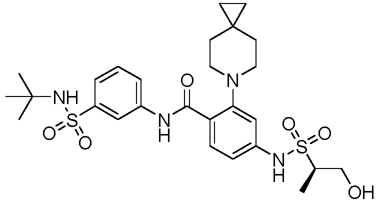 | (*R*)-*N*-(3-(*N*-(*tert-*Butyl)sulfamoyl)phenyl)-4-((2-hydroxy-1-methylethyl)sulfonamido)-2-(6-azaspiro[2.5]octan-6-yl)benzamide |
| 106 | 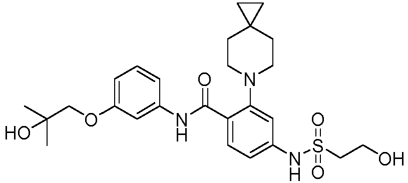 | *N*-(3-(2-Hydroxy-2-methylpropoxy)phenyl)-4-((2-hydroxyethyl)sulfonamido)-2-(6-azaspiro[2.5]octan-6-yl)benzamide |
| 107 | 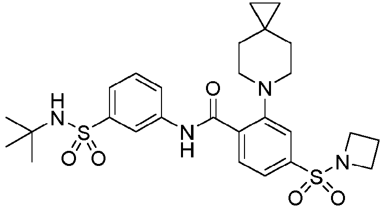 | 4-(Azetidin-1-ylsulfonyl)-*N*-(3-(*N*-(*tert-*butyl)sulfamoyl)phenyl)-2-(6-azaspiro[2.5]octan-6-yl)benzamide |
| 108-1 | 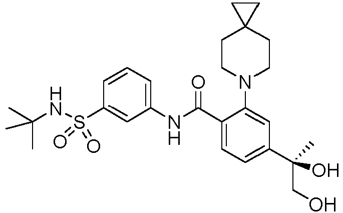 | (*R*)-*N*-(3-(*N*-(*tert-*Butyl)sulfamoyl)phenyl)-4-(1,2-dihydroxypropan-2-yl)-2-(6-azaspiro[2.5]octan-6-yl)benzamide |
| 108-2 | 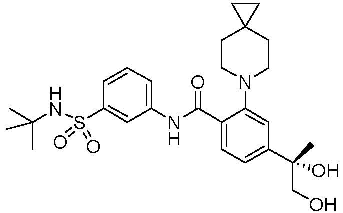 | (*S*)-*N*-(3-(*N*-(*tert-*Butyl)sulfamoyl)phenyl)-4-(1,2-dihydroxypropan-2-yl)-2-(6-azaspiro[2.5]octan-6-yl)benzamide |
| 109 | 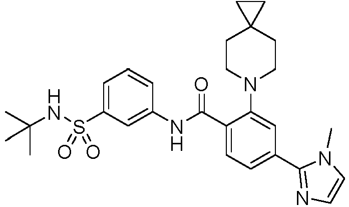 | *N*-(3-(*N*-(*tert*-Butyl)sulfamoyl)phenyl)-4-(1-methyl-1H-imidazol-2-yl)-2-(6-azaspiro[2.5]octan-6-yl)benzamide |
| 110 | 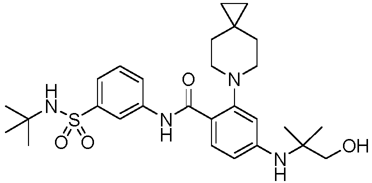 | *N*-(3-(*N*-(tert-Butyl)sulfamoyl)phenyl)-4-((1-hydroxy-2-methylpropan-2-yl)amino)-2-(6-azaspiro[2.5]octan-6-yl)benzamide |
| 111 | 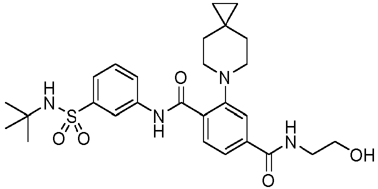 | *N*¹-(3-(*N*-(*tert*-Butyl)sulfamoyl)phenyl)-*N⁴*-(2-hydroxyethyl)-2-(6-azaspiro[2.5]octan-6-yl)terephthalamide |
| 112-1 | 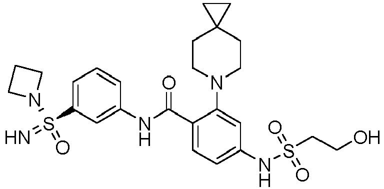 | (*R*)-*N*-(3-(Azetidine-1-sulfonimidoyl)phenyl)-4-((2-hydroxyethyl)sulfonamido)-2-(6-azaspiro[2.5]octan-6-yl)benzamide |
| 112-2 | 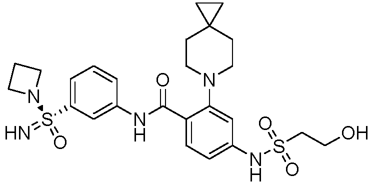 | (*S*)-*N*-(3-(Azetidine-1-sulfonimidoyl)phenyl)-4-((2-hydroxyethyl)sulfonamido)-2-(6-azaspiro[2.5]octan-6-yl)benzamide |
| 115 | 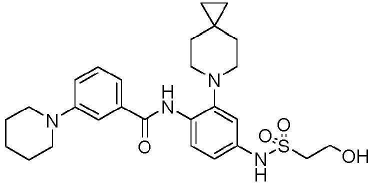 | *N*-(4-((2-Hydroxyethyl)sulfonamido)-2-(6-azaspiro[2.5]octan-6-yl)phenyl)-3-(piperidin-1-yl)benzamide |

or any pharmaceutically-acceptable salt thereof.

Another aspect of the present invention is a pharmaceutical composition comprising a new class of compounds useful for modulating KIF18A protein alone or in a bound complex with microtubules or pharmaceutically acceptable salts thereof.

In embodiment 25, the present invention provides pharmaceutical compositions comprising a compound, or pharmaceutically acceptable salts thereof, in accordance with any one of embodiments 1-24, and a pharmaceutically acceptable diluent or carrier.

Yet another aspect of the present invention is a method of treating a condition that may be treated with KIF18a inhibitors, the method comprising administering to a patient in need thereof a therapeutically effective amount of a new class of compounds useful for modulating KIF18A protein alone or in a bound complex with microtubules or pharmaceutically acceptable salts thereof.

In embodiment 27, the present invention provides a method of treating a condition that may be treated with KIF18a inhibitors, the method comprising administering to a patient in need thereof a therapeutically effective amount of the compound in accordance with embodiments 1-24, or the composition according to embodiment 25. The condition is a proliferative disorder selected from cancerpsoriasis, atopic dermatitis, an autoimmune disorder, or inflammatory bowel disease; wherein said cancer can be melanoma, prostate cancer, cervical cancer, breast cancer, colon cancer, sarcoma, or leukemia; wherein said autoimmune disorder can be rheumatoid arthritis, systemic lupus erythematosus, Sjögren's syndrome, Scleroderma, mixed connective tissue disease, dermatomyositis, polymyositis, Reiter's syndrome, autoimmune lymphoproliferative syndrome (ALPS), also known as Canale-Smith syndrome, or autoimmune disease of the central nervous system, such as multiple Sclerosis, myasthenia gravis, and encephalomyelitis; and wherein the. inflammatory bowel disease can be ulcerative colitis or Crohn's Disease. See: Zhang C. et. al., "Kif18A is involved in human breast carcinogenesis", Carcinogenesis, 2010 Sep;31(9):1676-84. doi: 10.1093/carcin/bgq134. Epub 2010 Jul 1. See also: (1) https://www.proteinatlas.org/ENSG00000121621-KIF18A/pathology; (2) Nagahara, M. et. al., "Kinesin 18A expression: clinical relevance to colorectal cancer progression", Int. J. Cancer: 129, 2543-2552 (2011) VC 2011 UIC; and (3) Yu, Y. et. al., "The Role of Kinesin Family Proteins in Tumorigenesis and Progression - Potential Biomarkers and Molecular Targets for Cancer Therapy", Cancer 2010;116:5150-60. VC 2010 American Cancer Society.

In embodiment 28, the present invention provides a method of reducing the size of a solid tumor in a subject, the method comprising administering to the subject in need thereof a therapeutically effective amount of the compound in accordance with embodiments 1-24, or the composition according to embodiment 25.

In embodiment 29, the present invention provides a method of treating a cell proliferation disorder in a subject, the method comprising administering to the subject in need thereof a therapeutically effective amount of the compound in accordance with embodiments 1-24, or the composition according to embodiment 25.

In embodiment 30, the present invention provides a *"ex vivo"* method of inhibiting KIF18A in a cell, comprising contacting the cell with a compound, or pharmaceutically acceptable salts thereof, in accordance with embodiments 1-24, or the composition according to embodiment 25.

Yet another aspect of the present invention is a method, not claimed, of preparing a new class of compounds useful for modulating KIF18A protein alone or in a bound complex with microtubules or pharmaceutically acceptable salts thereof.

### DETAILED DESCRIPTION OF THE INVENTION

The present invention includes all pharmaceutically acceptable isotopically-labelled compounds of the present invention wherein one or more atoms are replaced by atoms having the same atomic number, but an atomic mass or mass number different from the atomic mass or mass number which predominates in nature.

Examples of isotopes suitable for inclusion in the compounds of the invention include, but are not limited to, isotopes of hydrogen, such as ²H and ³H, carbon, such as ¹¹C, ¹³C and ¹⁴C, chlorine, such as ³⁸Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of the present invention, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the present invention can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

Specific embodiments of the present invention include the compounds exemplified in the Examples below and their pharmaceutically acceptable salts, complexes, solvates, polymorphs, stereoisomers, metabolites, prodrugs, and other derivatives thereof.

Unless otherwise specified, the following definitions apply to terms found in the specification and claims:

"C_{α-β}alk" means an alkyl group comprising a minimum of α and a maximum of β carbon atoms in a branched or linear relationship or any combination of the three, wherein α and β represent integers. The alkyl groups described in this section may also contain one or two double or triple bonds. A designation of C₀alk indicates a direct bond. Examples of C₁₋₆alkyl include, but are not limited to the following:

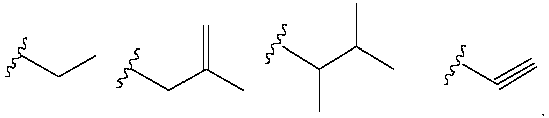

"Benzo group", alone or in combination, means the divalent radical C₄H₄=, one representation of which is -CH=CH-CH=CH-, that when vicinally attached to another ring forms a benzene-like ring--for example tetrahydronaphthylene, indole and the like.

The terms "oxo" and "thioxo" represent the groups =O (as in carbonyl) and =S (as in thiocarbonyl), respectively.

"Halo" or "halogen" means a halogen atom selected from F, Cl, Br and I.

"C_{α-β}haloalk" means an alk group, as described above, wherein any number --at least one-- of the hydrogen atoms attached to the alk chain are replaced by F, Cl, Br or I.

The group N(R^{a})R^{a} and the like include substituents where the two R^{a} groups together form a ring, optionally including a N, O or S atom, and include groups such as:

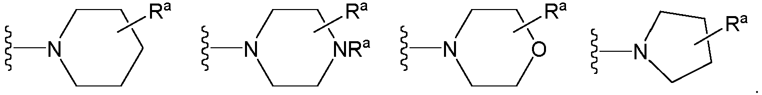

The group N(C_{α-β}alk) C_{α-β}alk, wherein α and β are as defined above, include substituents where the two C_{α-β}alk groups together form a ring, optionally including a N, O or S atom, and include groups such as:

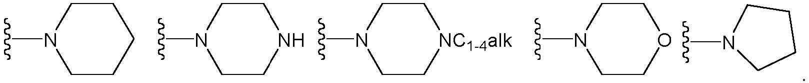

"Bicyclic ring" means a group that features two joined rings. A bicyclic ring can be carbocyclic (all of the ring atoms are carbons), or heterocyclic (the rings atoms consist, for example, 1, 2 or 3 heteroatoms, such as N, O, or S, in addition to carbon atoms). The two rings can both be aliphatic (*e.g.* decalin and norbornane), or can be aromatic (*e.g. naphthalene*), or a combination of aliphatic and aromatic (e.g. tetralin).

Bicyclic rings include:
(a) spirocyclic compounds, wherein the two rings share only one single atom, the spiro atom, which is usually a quaternary carbon. Examples of spirocyclic compound include, but are not limited to: 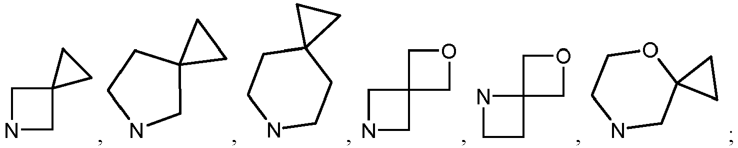
(b) fused bicyclic compounds, wherein two rings share two adjacent atoms. In other words, the rings share one covalent bond, *i.e.* the bridgehead atoms are directly connected (*e.g*. α-thujene and decalin). Examples of fused bicyclic rings include, but are not limited to: 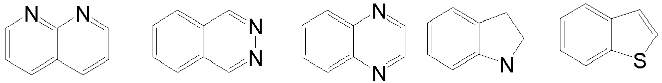 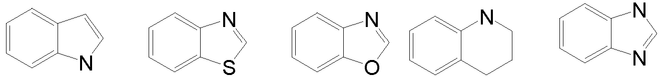 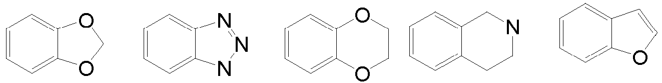 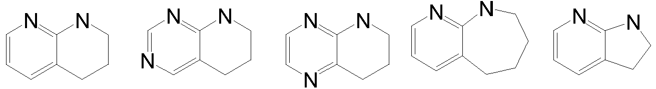 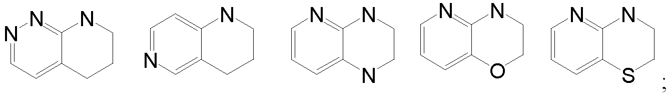 and
(c) bridged bicyclic compounds, wherein the two rings share three or more atoms, separating the two bridgehead atoms by a bridge containing at least one atom. For example, norbornane, also known as bicyclo[2.2.1]heptane, can be thought of as a pair of cyclopentane rings each sharing three of their five carbon atoms. Examples of bridged bicyclic rings include, but are not limited to: 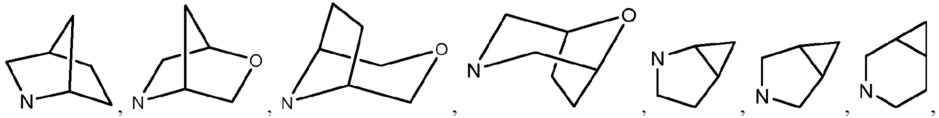 or

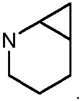

"Carbocycle" or "Carbocyclic" means a ring comprising by itself or in combination with other terms, represents, unless otherwise stated, cyclic version of "C_{α-β}alk". Examples of carbocycle include cyclopentyl, cyclohexyl, 1-cyclohexenyl, 3-cyclohexenyl, cycloheptyl, cyclobutylene, cyclohexylene and the like.

"Heterocycle" or "Heterocyclic" means a ring comprising at least one carbon atom and at least one other atom selected from N, O and S. Examples of heterocycles that may be found in the claims include, but are not limited to, the following: 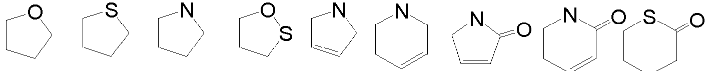 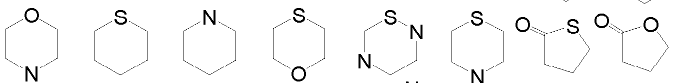 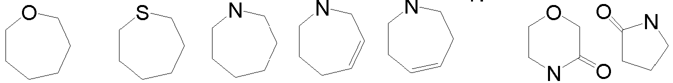 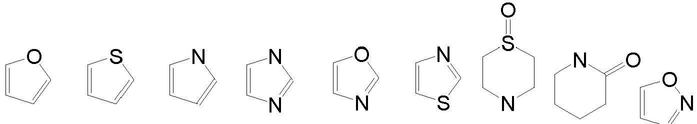 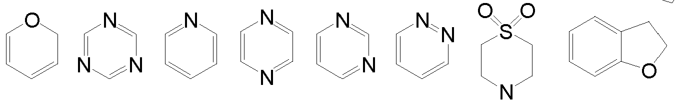 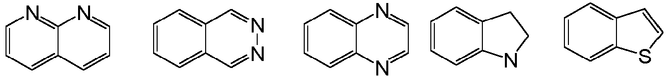 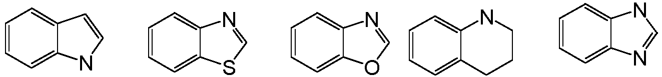 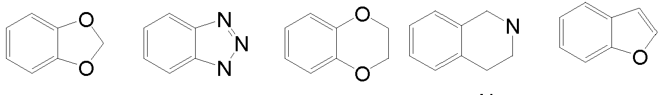 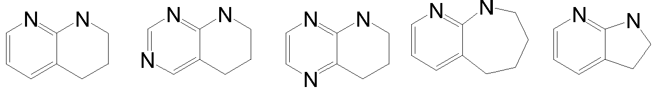 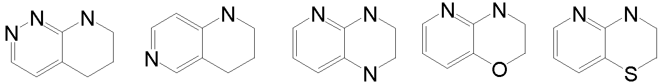 and

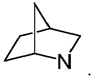

"Pharmaceutically-acceptable salt" means a salt prepared by conventional means, and are well known by those skilled in the art. The "pharmacologically acceptable salts" include basic salts of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. When compounds of the invention include an acidic function such as a carboxy group, then suitable pharmaceutically acceptable cation pairs for the carboxy group are well known to those skilled in the art and include alkaline, alkaline earth, ammonium, quaternary ammonium cations and the like. For additional examples of "pharmacologically acceptable salts," *see infra* and Berge et al., J. Pharm. Sci. 66:1 (1977).

"Saturated, partially-saturated or unsaturated" includes substituents saturated with hydrogens, substituents completely unsaturated with hydrogens and substituents partially saturated with hydrogens.

"Leaving group" generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.

"Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, ortho-methylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, trifluoroacetyl, trichloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluenesulfonic acid, trifluoroacetic acid and the like. Many of the amino protecting groups are also suitable for protecting carboxy, hydroxy and mercapto groups. For example, aralkyl groups. Alkyl groups are also suitable groups for protecting hydroxy and mercapto groups, such as tert-butyl.

Silyl protecting groups are silicon atoms optionally substituted by one or more alkyl, aryl and aralkyl groups. Suitable silyl protecting groups include, but are not limited to, trimethylsilyl, triethylsilyl, triisopropylsilyl, tert-butyldimethylsilyl, dimethylphenylsilyl, 1,2-bis(dimethylsilyl)benzene, 1,2-bis(dimethylsilyl)ethane and diphenylmethylsilyl. Silylation of an amino groups provide mono- or disilylamino groups. Silylation of aminoalcohol compounds can lead to a N,N,O-trisilyl derivative. Removal of the silyl function from a silyl ether function is readily accomplished by treatment with, for example, a metal hydroxide or ammonium fluoride reagent, either as a discrete reaction step or in situ during a reaction with the alcohol group. Suitable silylating agents are, for example, trimethylsilyl chloride, tert-butyl-dimethylsilyl chloride, phenyldimethylsilyl chloride, diphenylmethyl silyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A t-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, tert-butyl, 4-methoxyphenylmethyl and the like, can be removed under hydrolysis and hydrogenolysis conditions well known to those skilled in the art.

It should be noted that compounds of the invention may contain groups that may exist in tautomeric forms, such as cyclic and acyclic amidine and guanidine groups, heteroatom substituted heteroaryl groups (Y' = O, S, NR), and the like, which are illustrated in the following examples: 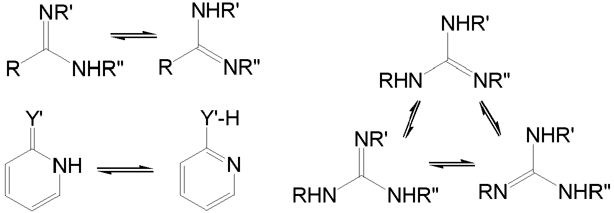 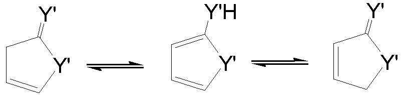 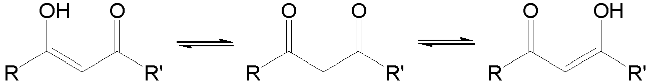 and though one form is named, described, displayed and/or claimed herein, all the tautomeric forms are intended to be inherently included in such name, description, display and/or claim.

Prodrugs of the compounds of this invention are also contemplated by this invention. A prodrug is an active or inactive compound that is modified chemically through in vivo physiological action, such as hydrolysis, metabolism and the like, into a compound of this invention following administration of the prodrug to a patient. The suitability and techniques involved in making and using prodrugs are well known by those skilled in the art. For a general discussion of prodrugs involving esters see Svensson and Tunek Drug Metabolism Reviews 165 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bungaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxy groups have been masked as esters and ethers. EP 039,051 (Sloan and Little, 4/11/81) discloses Mannich-base hydroxamic acid prodrugs, their preparation and use.

The specification and claims contain listing of species using the language "selected from . . . and . . ." and "is... or ..." (sometimes referred to as Markush groups). When this language is used in this application, unless otherwise stated it is meant to include the group as a whole, or any single members thereof, or any subgroups thereof. The use of this language is merely for shorthand purposes and is not meant in any way to limit the removal of individual elements or subgroups as needed.

### PHARMACEUTICAL COMPOSITIONS, DOSING, AND ROUTES OF ADMINISTRATION

Also provided herein are pharmaceutical compositions that includes a compound as disclosed herein, together with a pharmaceutically acceptable excipient, such as, for example, a diluent or carrier. Compounds and pharmaceutical compositions suitable for use in the present invention include those wherein the compound can be administered in an effective amount to achieve its intended purpose. Administration of the compound described in more detail below.

Suitable pharmaceutical formulations can be determined by the skilled artisan depending on the route of administration and the desired dosage. See, e.g., Remington's Pharmaceutical Sciences, 1435-712 (18th ed., Mack Publishing Co, Easton, Pennsylvania, 1990). Formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein as well as the pharmacokinetic data obtainable through animal or human clinical trials.

The phrases "pharmaceutically acceptable" or "pharmacologically acceptable" refer to molecular entities and compositions that do not produce adverse, allergic, or other untoward reactions when administered to an animal or a human. As used herein, "pharmaceutically acceptable e" includes any and all solvents, dispersion media, coatings, antibacterial and antifungal agents, isotonic and absorption delaying agents and the like. The use of such excipients for pharmaceutically active substances is well known in the art. Except insofar as any conventional media or agent is incompatible with the therapeutic compositions, its use in therapeutic compositions is contemplated. Supplementary active ingredients also can be incorporated into the compositions. In exemplary embodiments, the formulation may comprise corn syrup solids, high-oleic safflower oil, coconut oil, soy oil, L-leucine, calcium phosphate tribasic, L-tyrosine, L-proline, L-lysine acetate, DATEM (an emulsifier), L-glutamine, L-valine, potassium phosphate dibasic, L-isoleucine, L-arginine, L-alanine, glycine, L-asparagine monohydrate, L-serine, potassium citrate, L-threonine, sodium citrate, magnesium chloride, L-histidine, L-methionine, ascorbic acid, calcium carbonate, L-glutamic acid, L-cystine dihydrochloride, L-tryptophan, L-aspartic acid, choline chloride, taurine, m-inositol, ferrous sulfate, ascorbyl palmitate, zinc sulfate, L-carnitine, alphatocopheryl acetate, sodium chloride, niacinamide, mixed tocopherols, calcium pantothenate, cupric sulfate, thiamine chloride hydrochloride, vitamin A palmitate, manganese sulfate, riboflavin, pyridoxine hydrochloride, folic acid, beta-carotene, potassium iodide, phylloquinone, biotin, sodium selenate, chromium chloride, sodium molybdate, vitamin D3 and cyanocobalamin.

The compound can be present in a pharmaceutical composition as a pharmaceutically acceptable salt. As used herein, "pharmaceutically acceptable salts" include, for example base addition salts and acid addition salts.

Pharmaceutically acceptable base addition salts may be formed with metals or amines, such as alkali and alkaline earth metals or organic amines. Pharmaceutically acceptable salts of compounds may also be prepared with a pharmaceutically acceptable cation. Suitable pharmaceutically acceptable cations are well known to those skilled in the art and include alkaline, alkaline earth, ammonium and quaternary ammonium cations. Carbonates or hydrogen carbonates are also possible. Examples of metals used as cations are sodium, potassium, magnesium, ammonium, calcium, or ferric, and the like. Examples of suitable amines include isopropylamine, trimethylamine, histidine, N,N'-dibenzylethylenediamine, chloroprocaine, choline, diethanolamine, dicyclohexylamine, ethylenediamine, N-methylglucamine, and procaine.

Pharmaceutically acceptable acid addition salts include inorganic or organic acid salts. Examples of suitable acid salts include the hydrochlorides, formates, acetates, citrates, salicylates, nitrates, phosphates. Other suitable pharmaceutically acceptable salts are well known to those skilled in the art and include, for example, formic, acetic, citric, oxalic, tartaric, or mandelic acids, hydrochloric acid, hydrobromic acid, sulfuric acid or phosphoric acid; with organic carboxylic, sulfonic, sulfo or phospho acids or N-substituted sulfamic acids, for example acetic acid, trifluoroacetic acid (TFA), propionic acid, glycolic acid, succinic acid, maleic acid, hydroxymaleic acid, methylmaleic acid, fumaric acid, malic acid, tartaric acid, lactic acid, oxalic acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid; and with amino acids, such as the 20 alpha amino acids involved in the synthesis of proteins in nature, for example glutamic acid or aspartic acid, and also with phenylacetic acid, methanesulfonic acid, ethanesulfonic acid, 2-hydroxyethanesulfonic acid, ethane 1,2-disulfonic acid, benzenesulfonic acid, 4-methylbenzenesulfonic acid, naphthalene 2-sulfonic acid, naphthalene 1,5-disulfonic acid, 2- or 3-phosphoglycerate, glucose 6-phosphate, N-cyclohexylsulfamic acid (with the formation of cyclamates), or with other acid organic compounds, such as ascorbic acid.

Pharmaceutical compositions containing the compounds disclosed herein can be manufactured in a conventional manner, e.g., by conventional mixing, dissolving, granulating, dragee-making, levigating, emulsifying, encapsulating, entrapping, or lyophilizing processes. Proper formulation is dependent upon the route of administration chosen.

For oral administration, suitable compositions can be formulated readily by combining a compound disclosed herein with pharmaceutically acceptable excipients such as carriers well known in the art. Such excipients and carriers enable the present compounds to be formulated as tablets, pills, dragees, capsules, liquids, gels, syrups, slurries, suspensions and the like, for oral ingestion by a patient to be treated. Pharmaceutical preparations for oral use can be obtained by adding a compound as disclosed herein with a solid excipient, optionally grinding a resulting mixture, and processing the mixture of granules, after adding suitable auxiliaries, if desired, to obtain tablets or dragee cores. Suitable excipients include, for example, fillers and cellulose preparations. If desired, disintegrating agents can be added. Pharmaceutically acceptable ingredients are well known for the various types of formulation and may be for example binders (e.g., natural or synthetic polymers), lubricants, surfactants, sweetening and flavoring agents, coating materials, preservatives, dyes, thickeners, adjuvants, antimicrobial agents, antioxidants and carriers for the various formulation types.

When a therapeutically effective amount of a compound disclosed herein is administered orally, the composition typically is in the form of a solid (e.g., tablet, capsule, pill, powder, or troche) or a liquid formulation (e.g., aqueous suspension, solution, elixir, or syrup).

When administered in tablet form, the composition can additionally contain a functional solid and/or solid carrier, such as a gelatin or an adjuvant. The tablet, capsule, and powder can contain about 1 to about 95% compound, and preferably from about 15 to about 90% compound.

When administered in liquid or suspension form, a functional liquid and/or a liquid carrier such as water, petroleum, or oils of animal or plant origin can be added. The liquid form of the composition can further contain physiological saline solution, sugar alcohol solutions, dextrose or other saccharide solutions, or glycols. When administered in liquid or suspension form, the composition can contain about 0.5 to about 90% by weight of a compound disclosed herein, and preferably about 1 to about 50% of a compound disclosed herein. In one embodiment contemplated, the liquid carrier is non-aqueous or substantially non-aqueous. For administration in liquid form, the composition may be supplied as a rapidly-dissolving solid formulation for dissolution or suspension immediately prior to administration.

When a therapeutically effective amount of a compound disclosed herein is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, in addition to a compound disclosed herein, an isotonic vehicle. Such compositions may be prepared for administration as solutions of free base or pharmacologically acceptable salts in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations can optionally contain a preservative to prevent the growth of microorganisms.

Injectable compositions can include sterile aqueous solutions, suspensions, or dispersions and sterile powders for the extemporaneous preparation of sterile injectable solutions, suspensions, or dispersions. In all embodiments the form must be sterile and must be fluid to the extent that easy syringability exists. It must be stable under the conditions of manufacture and storage and must resist the contaminating action of microorganisms, such as bacteria and fungi, by optional inclusion of a preservative. The carrier can be a solvent or dispersion medium containing, for example, water, ethanol, polyol (e.g., glycerol, propylene glycol, and liquid polyethylene glycol, and the like), suitable mixtures thereof, and vegetable oils. In one embodiment contemplated, the carrier is non-aqueous or substantially non-aqueous. The proper fluidity can be maintained, for example, by the use of a coating, such as lecithin, by the maintenance of the required particle size of the compound in the embodiment of dispersion and by the use of surfactants. The prevention of the action of microorganisms can be brought about by various antibacterial and antifungal agents, for example, parabens, chlorobutanol, phenol, sorbic acid, thimerosal, and the like. In many embodiments, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the embodiment of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Slow release or sustained release formulations may also be prepared in order to achieve a controlled release of the active compound in contact with the body fluids in the GI tract, and to provide a substantially constant and effective level of the active compound in the blood plasma. For example, release can be controlled by one or more of dissolution, diffusion, and ion-exchange. In addition, the slow release approach may enhance absorption via saturable or limiting pathways within the GI tract. For example, the compound may be embedded for this purpose in a polymer matrix of a biological degradable polymer, a water-soluble polymer or a mixture of both, and optionally suitable surfactants. Embedding can mean in this context the incorporation of micro-particles in a matrix of polymers. Controlled release formulations are also obtained through encapsulation of dispersed micro-particles or emulsified microdroplets via known dispersion or emulsion coating technologies.

For administration by inhalation, compounds of the present invention are conveniently delivered in the form of an aerosol spray presentation from pressurized packs or a nebulizer, with the use of a suitable propellant. In the embodiment of a pressurized aerosol, the dosage unit can be determined by providing a valve to deliver a metered amount. Capsules and cartridges of, e.g., gelatin, for use in an inhaler or insufflator can be formulated containing a powder mix of the compound and a suitable powder base such as lactose or starch.

The compounds disclosed herein can be formulated for parenteral administration by injection (e.g., by bolus injection or continuous infusion). Formulations for injection can be presented in unit dosage form (e.g., in ampules or in multidose containers), with an added preservative. The compositions can take such forms as suspensions, solutions, or emulsions in oily or aqueous vehicles, and can contain formulatory agents such as suspending, stabilizing, and/or dispersing agents.

Pharmaceutical formulations for parenteral administration include aqueous solutions of the compounds in water-soluble form. Additionally, suspensions of the compounds can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle (e.g., sterile pyrogen-free water) before use.

Compounds disclosed herein also can be formulated in rectal compositions, such as suppositories or retention enemas (e.g., containing conventional suppository bases). In addition to the formulations described previously, the compounds also can be formulated as a depot preparation. Such long-acting formulations can be administered by implantation (e.g., subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

**In** particular, a compound disclosed herein can be administered orally, buccally, or sublingually in the form of tablets containing excipients, such as starch or lactose, or in capsules or ovules, either alone or in admixture with excipients, or in the form of elixirs or suspensions containing flavoring or coloring agents. Such liquid preparations can be prepared with pharmaceutically acceptable additives, such as suspending agents. A compound also can be injected parenterally, for example, intravenously, intramuscularly, subcutaneously, or intracoronarily. For parenteral administration, the compound is best used in the form of a sterile aqueous solution which can contain other substances, for example, salts, or sugar alcohols, such as mannitol, or glucose, to make the solution isotonic with blood.

For veterinary use, a compound disclosed herein is administered as a suitably acceptable formulation in accordance with normal veterinary practice. The veterinarian can readily determine the dosing regimen and route of administration that is most appropriate for a particular animal.

**In** some embodiments, all the necessary components for the treatment of KIF18A-related disorder using a compound as disclosed herein either alone or in combination with another agent or intervention traditionally used for the treatment of such disease may be packaged into a kit. Specifically, the present invention provides a kit for use in the therapeutic intervention of the disease comprising a packaged set of medicaments that include the compound disclosed herein as well as buffers and other components for preparing deliverable forms of said medicaments, and/or devices for delivering such medicaments, and/or any agents that are used in combination therapy with the compound disclosed herein, and/or instructions for the treatment of the disease packaged with the medicaments. The instructions may be fixed in any tangible medium, such as printed paper, or a computer readable magnetic or optical medium, or instructions to reference a remote computer data source such as a world wide web page accessible via the internet.

A "therapeutically effective amount" means an amount effective to treat or to prevent development of, or to alleviate the existing symptoms of, the subject being treated. Determination of the effective amounts is well within the capability of those skilled in the art, especially in light of the detailed disclosure provided herein. Generally, a "therapeutically effective dose" refers to that amount of the compound that results in achieving the desired effect. For example, in one preferred embodiment, a therapeutically effective amount of a compound disclosed herein decreases KIF18A activity by at least 5%, compared to control, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, or at least 90%.

The amount of compound administered can be dependent on the subject being treated, on the subject's age, health, sex, and weight, the kind of concurrent treatment (if any), severity of the affliction, the nature of the effect desired, the manner and frequency of treatment, and the judgment of the prescribing physician. The frequency of dosing also can be dependent on pharmacodynamic effects on arterial oxygen pressures. While individual needs vary, determination of optimal ranges of effective amounts of the compound is within the skill of the art. Such doses may be administered in a single dose or it may be divided into multiple doses.

The terms "cancer" and "cancerous" when used herein refer to or describe the physiological condition in mammals that is typically characterized by unregulated cell growth. Examples of cancer include, without limitation, carcinoma, lymphoma, sarcoma, blastoma and leukemia. More particular examples of such cancers include squamous cell carcinoma, lung cancer, pancreatic cancer, cervical cancer, bladder cancer, hepatoma, breast cancer, colon carcinoma, and head and neck cancer, ovarian cancer, and endometrial cancer. While the term "cancer" as used herein is not limited to any one specific form of the disease, it is believed that the methods of the invention will be particularly effective for cancers which are found to be accompanied by unregulated levels of KIF18A or dependent on KIF18A for proper chromosome segregation and survival in the mammal.

The terms "treat", "treating" and "treatment" as used herein refer to therapy, including without limitation, curative therapy, prophylactic therapy, and preventative therapy. Prophylactic treatment generally constitutes either preventing the onset of disorders altogether or delaying the onset of a preclinically evident stage of disorders in individuals.

The term "patient", "subject", or "mammal" as used herein refers to any "patient", "subject", or "mammal" including humans, cows, horses, dogs and cats. In one embodiment of the invention, the mammal is a human.

The term "comprising" is meant to be open ended, including the indicated component(s) but not excluding other elements.

The terms "Formula I" include any sub formulas.

### METHODS OF USING KIF18A INHIBITORS

The present disclosure provides compounds having MT-based KIF18A modulatory activity in general, and inhibitory activity in particular. In one embodiment of the invention, there is provided a method of modulating KIF18A protein in a subject, the method comprising administering to the subject an effective dosage amount of a compound of Formulas I. As such, the compounds of the invention may be used to treat cellular proliferation disorders, including uncontrolled cell growth, aberrant cell cycle regulation, centrosome abnormalities (structural and or numeric, fragmentation). Other diseases or disorders associated with the accumulation of extra centrosomes (>2) include human papillomavirus (HPV) infection, including HPV-associated neoplasias. The compounds are also useful for cilia-related diseases as well as ablating haploid germ cell population which could be used as a male contraceptive.

In addition, compounds of the invention are useful for, but not limited to, the prevention or treatment of cancer and other KIF18A-mediated diseases or disorders. For example, compounds of the invention would be useful for the treatment of various solid and hematologically derived tumors, such as carcinomas, including, without limitation, cancer of the bladder, breast, colon, kidney, liver, lung (including squamous cell and small cell lung cancer), esophagus, gall-bladder, ovary, pancreas, stomach, cervix, thyroid, prostate, and skin (including squamous cell carcinoma); hematopoietic tumors of lymphoid lineage (including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g. soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma).

The compounds of the invention are also useful in the treatment of cancer related indications such as solid tumors, sarcomas (especially Ewing's sarcoma and osteosarcoma), retinoblastoma, rhabdomyosarcomas, neuroblastoma, hematopoietic malignancies, including leukemia and lymphoma, tumor- induced pleural or pericardial effusions, and malignant ascites.

Based on the ability to modulate kinesin impacting angiogenesis, the compounds of the invention are also useful in treatment and therapy of proliferative diseases. Particularly, these compounds can be used for the treatment of an inflammatory disease, especially of manifestations at the locomotor apparatus, such as various inflammatory rheumatoid diseases, especially chronic polyarthritis including rheumatoid arthritis, juvenile arthritis or psoriasis arthropathy; paraneoplastic syndrome or tumor-induced inflammatory diseases, turbid effusions, collagenosis, such as systemic Lupus erythematosus, polymyositis, dermato-myositis, systemic sclerodermia or mixed collagenosis; postinfectious arthritis (where no living pathogenic organism can be found at or in the affected part of the body), seronegative spondylarthritis, such as spondylitis ankylosans; vasculitis, sarcoidosis, or arthrosis; or further any combinations thereof.

The compounds of the invention can also be used as active agents against such disease states as arthritis, atherosclerosis, psoriasis, hemangiomas, myocardial angiogenesis, coronary and cerebral collaterals, ischemic limb angiogenesis, wound healing, peptic ulcer Helicobacter related diseases, fractures, cat scratch fever, rubeosis, neovascular glaucoma and retinopathies such as those associated with diabetic retinopathy or macular degeneration. In addition, some of these compounds can be used as active agents against solid tumors, malignant ascites, hematopoietic cancers and hyperproliferative disorders such as thyroid hyperplasia (especially Grave's disease), and cysts (such as hypervascularity of ovarian stroma, characteristic of polycystic ovarian syndrome (Stein- Leventhal syndrome)) since such diseases require a proliferation of blood vessel cells for growth and/or metastasis.

Besides being useful for human treatment, these compounds are useful for veterinary treatment of companion animals, exotic animals and farm animals, including mammals, rodents, and the like. For example, animals including horses, dogs, and cats may be treated with compounds provided by the invention.

### COMBINATIONS

While the compounds of the invention can be dosed or administered as the sole active pharmaceutical agent, they can also be used in combination with one or more compounds of the invention or in conjunction with other agents. When administered as a combination, the therapeutic agents can be formulated as separate compositions that are administered simultaneously or sequentially at different times, or the therapeutic agents can be given as a single composition.

The phrase "co-therapy" (or "combination-therapy"), in defining use of a compound of the present invention and another pharmaceutical agent, not claimed, is intended to embrace administration of each agent in a sequential manner in a regimen that will provide beneficial effects of the drug combination, and is intended as well to embrace co-administration of these agents in a substantially simultaneous manner, such as in a single capsule having a fixed ratio of these active agents or in multiple, separate capsules for each agent.

Specifically, the administration of compounds of the present invention may be in conjunction with additional therapies known to those skilled in the art in the prevention or treatment of cancer, such as with radiation therapy, small molecule targeted agents (e.g. PARP inhibitors, kinase inhibitors), therapeutic antibodies (e.g. naked and drug-conjugate) immunotherapy antibodies (checkpoint inhibitors, bi-specific T-cell engagers) with neoplastic or cytotoxic agents.

If formulated as a fixed dose, such combination products employ the compounds of this invention within the accepted dosage ranges. Compounds of Formula I may also be administered sequentially with known anticancer or cytotoxic agents when a combination formulation is inappropriate. The invention is not limited in the sequence of administration; compounds of the invention may be administered either prior to, simultaneous with or after administration of the known anticancer or cytotoxic agent.

There are large numbers of anticancer agents available in commercial use, in clinical evaluation and in pre-clinical development, which would be selected for treatment of neoplasia by combination drug chemotherapy. Such agents fall into several major categories such as antibiotic-type agents, alkylating and alkylating-like agents, antimitotic agents, targeted small molecule agents, antimetabolite agents, hormonal agents, immunological agents, anti-angiogenic agents, interferon-type agents and a category of miscellaneous agents.

The present disclosure also provides methods for combination therapies in which an agent known to modulate other pathways, or other components of the same pathway, or even overlapping sets of target enzymes are used in combination with a compound of the present disclosure, or a pharmaceutically acceptable salt thereof. In one aspect, such therapy includes but is not limited to the combination of one or more compounds of the disclosure with chemotherapeutic agents, therapeutic antibodies, targeted small molecule agents, and radiation treatment, to provide a synergistic or additive therapeutic effect.

Many chemotherapeutics are presently known in the art and can be used in combination with the compounds of the disclosure. In some embodiments, the chemotherapeutic is selected from the group consisting of antimitotic agents, alkylating agents, anti-metabolites, intercalating antibiotics, growth factor inhibitors, cell cycle inhibitors, enzymes, topoisomerase inhibitors, biological response modifiers, antihormones, angiogenesis inhibitors, and anti-androgens. Non-limiting examples are chemotherapeutic agents, cytotoxic agents, and non-peptide small molecules such as Gleevec^{®} (Imatinib Mesylate), Kyprolis^{®} (carfilzomib), Velcade^{®} (bortezomib), Casodex (bicalutamide), Iressa^{®} (gefitinib), and Adriamycin as well as a host of chemotherapeutic agents. Non-limiting examples of chemotherapeutic agents include alkylating agents such as thiotepa and cyclosphosphamide (CYTOXANTM); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, CasodexTM, chromomycins, dactinomycin, daunorubicin, detorubicin, 6-diazo-5-oxo- L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel and docetaxel, Nab-paclitaxel; retinoic acid; esperamicins; capecitabine; and pharmaceutically acceptable salts, acids or derivatives of any of the above.

Also included as suitable chemotherapeutic cell conditioners are anti-hormonal agents that act to regulate or inhibit hormone action on tumors such as anti-estrogens including for example tamoxifen, (NolvadexTM), raloxifene, aromatase inhibiting 4(5)- imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; chlorambucil; gemcitabine; 6-thioguanine; mercaptopurine; methotrexate; platinum analogs such as cisplatin, oxaliplatin, carboplatin; etoposide (VP-16); ifosfamide; mitomycin C; mitoxantrone; vinblastine vincristine; vinorelbine; navelbine; novantrone; teniposide; daunomycin; aminopterin; xeloda; ibandronate; topotecan; camptothecin-11 (CPT-11); topoisomerase inhibitor RFS 2000; difluoromethylomithine (DMFO),

Where desired, the compounds or pharmaceutical composition of the present disclosure can be used in combination with commonly prescribed anti-cancer drugs such as Herceptin^{®}, Avastin^{®}, Erbitux^{®}, Rituxan^{®}, Taxol^{®}, Abraxane, Arimidex^{®}, Taxotere^{®}, ABVD, AVICINE, Abagovomab, Acridine carboxamide, Adecatumumab, 17-N-Allylamino-17-demethoxygeldanamycin, Alpharadin, Alvocidib, 3-Aminopyridine-2-carboxaldehyde thiosemicarbazone, Amonafide, Anthracenedione, Anti-CD22 immunotoxins, Antineoplastic, Antitumorigenic herbs, Apaziquone, Atiprimod, Azathioprine, Belotecan, Bendamustine, BIBW 2992, Biricodar, Brostallicin, Bryostatin, Buthionine sulfoximine, CBV (chemotherapy), Calyculin, cell-cycle nonspecific antineoplastic agents, Dichloroacetic acid, Discodermolide, Elsamitrucin, Enocitabine, Epothilone, Eribulin, Everolimus, Exatecan, Exisulind, Ferruginol, Forodesine, Fosfestrol, ICE chemotherapy regimen, IT-101, Imexon, Imiquimod, Indolocarbazole, Irofulven, Laniquidar, Larotaxel, Lenalidomide, Lucanthone, Lurtotecan, Mafosfamide, Mitozolomide, Nafoxidine, Nedaplatin, Olaparib, Talazoparib, Niraparib, Ortataxel, PAC-1, Pawpaw, Pixantrone, Proteasome inhibitor, Rebeccamycin, Resiquimod, Rubitecan, SN-38, Salinosporamide A, Sapacitabine, Stanford V, Swainsonine, Talaporfin, Tariquidar, Tegafur-uracil, Temodar, Tesetaxel, Triplatin tetranitrate, Tris(2-chloroethyl)amine, Troxacitabine, Uramustine, Vadimezan, Vinflunine, ZD6126 or Zosuquidar, CDK4/6 inhibitors (Palbociclib, Ibrance; Ribociclib, Kisqali; Abemaciclib, Verzenio).

This disclosure further relates to a method for using the compounds or pharmaceutical compositions provided herein, in combination with radiation therapy for inhibiting abnormal cell growth or treating the hyperproliferative disorder in the mammal. Techniques for administering radiation therapy are known in the art, and these techniques can be used in the combination therapy described herein. The administration of the compound of the disclosure in this combination therapy can be determined as described herein.

Radiation therapy can be administered through one of several methods, or a combination of methods, including without limitation external-beam therapy, internal radiation therapy, implant radiation, stereotactic radiosurgery, systemic radiation therapy, radiotherapy and permanent or temporary interstitial brachytherapy. The term "brachytherapy," as used herein, refers to radiation therapy delivered by a spatially confined radioactive material inserted into the body at or near a tumor or other proliferative tissue disease site. The term is intended without limitation to include exposure to radioactive isotopes (e.g. At-211, I-131, I-125, Y-90, Re-186, Re-188, Sm- 153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I-131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s), e.g., a solution of I-125 or I-131, or a radioactive fluid can be produced using a slurry of a suitable fluid containing small particles of solid radionuclides, such as Au-198, Y-90. Moreover, the radionuclide(s) can be embodied in a gel or radioactive micro spheres.

The compounds or pharmaceutical compositions of the disclosure can be used in combination with an amount of one or more substances selected from anti- angiogenesis agents, signal transduction inhibitors, antiproliferative agents, glycolysis inhibitors, or autophagy inhibitors.

Anti-angiogenesis agents, such as MMP-2 (matrix-metalloproteinase 2) inhibitors, MMP-9 (matrix-metalloprotienase 9) inhibitors, and COX-11 (cyclooxygenase 11) inhibitors, can be used in conjunction with a compound of the disclosure and pharmaceutical compositions described herein. Anti-angiogenesis agents include, for example, rapamycin, temsirolimus (CCI-779), everolimus (RAD001), sorafenib, sunitinib, and bevacizumab. Examples of useful COX-II inhibitors include alecoxib, valdecoxib, and rofecoxib. Examples of useful matrix metalloproteinase inhibitors are described in WO 96/33172 WO 96/27583 European Patent Publication EP0818442, European Patent Publication EP1004578 , WO 98/07697, WO 98/03516, WO 98/34918, WO 98/34915, WO 98/33768, WO 98/30566, European Patent Publication 606046, European Patent Publication 931 788, WO 90/05719, WO 99/52910, WO 99/52889, WO 99/29667, WO1999007675 , European Patent Publication EP1786785, European Patent Publication No. EP1181017, United States Publication No. US20090012085 , United States Publication US5863 949, United States Publication US5861 510, and European Patent Publication EP0780386. Preferred MMP-2 and MMP-9 inhibitors are those that have little or no activity inhibiting MMP-1. More preferred, are those that selectively inhibit MMP-2 and/or AMP-9 relative to the other matrix- metalloproteinases (i. e., MAP-1, MMP-3, MMP-4, MMP-5, MMP-6, MMP- 7, MMP- 8, MMP-10, MMP-11, MMP-12, andMMP-13). Some specific examples of MMP inhibitors useful in the disclosure are AG-3340, RO 32-3555, and RS 13-0830.

The present compounds may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflomithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-N1, interferon alfa-n3, interferon alfacon-1, interferon alpha, natural, interferon beta, interferon beta-1a, interferon beta-1b, interferon gamma, natural interferon gamma-1a, interferon gamma-1b, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid; abarelix; AE 941 (Aeterna), ambamustine, antisense oligonucleotide, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techniclone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

The compounds of the invention may further be used with VEGFR inhibitors. Other compounds described in the following patents and patent applications can be used in combination therapy: US 6,258,812, US 2003/0105091, WO 01/37820, US 6,235,764, WO 01/32651, US 6,630,500, US 6,515,004, US 6,713,485, US 5,521,184, US 5,770,599, US 5,747,498, WO 02/68406, WO 02/66470, WO 02/55501, WO 04/05279, WO 04/07481, WO 04/07458, WO 04/09784, WO 02/59110, WO 99/45009, WO 00/59509, WO 99/61422, US 5,990,141, WO 00/12089, and WO 00/02871.

In some embodiments, the combination comprises a composition of the present invention in combination with at least one anti-angiogenic agent. Agents are inclusive of, but not limited to, in vitro synthetically prepared chemical compositions, antibodies, antigen binding regions, radionuclides, and combinations and conjugates thereof. An agent can be an agonist, antagonist, allosteric modulator, toxin or, more generally, may act to inhibit or stimulate its target (e.g., receptor or enzyme activation or inhibition), and thereby promote cell death or arrest cell growth.

Exemplary anti-angiogenic agents include ERBITUX^{™} (IMC-C225), KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF, or soluble VEGF receptors or a ligand binding region thereof) such as AVASTIN^{™} or VEGF-TRAP^{™}, and anti-VEGF receptor agents (e.g., antibodies or antigen binding regions that specifically bind thereto), EGFR inhibitory agents (e.g., antibodies or antigen binding regions that specifically bind thereto) such as Vectibix (panitumumab), IRESSA^{™} (gefitinib), TARCEVA^{™} (erlotinib), anti-Ang1 and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). The pharmaceutical compositions of the present invention can also include one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met".

Other anti-angiogenic agents include Campath, IL-8, B-FGF, Tek antagonists (Ceretti et al., U.S. Publication No. 2003/0162712; U.S. Patent No. 6,413,932), anti-TWEAK agents (e.g., specifically binding antibodies or antigen binding regions, or soluble TWEAK receptor antagonists; see, Wiley, U.S. Patent No. 6,727,225), ADAM distintegrin domain to antagonize the binding of integrin to its ligands (Fanslow et al., U.S. Publication No. 2002/0042368), specifically binding anti-eph receptor and/or antiephrin antibodies or antigen binding regions (U.S. Patent Nos. 5,981,245; 5,728,813; 5,969,110; 6,596,852; 6,232,447; 6,057,124 and patent family members thereof), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto).

Additional anti-angiogenic/anti-tumor agents include: SD-7784 (Pfizer, USA); cilengitide.(Merck KGaA, Germany, EPO 770622); pegaptanib octasodium, (Gilead Sciences, USA); Alphastatin, (BioActa, UK); M-PGA, (Celgene, USA, US 5712291); ilomastat, (Arriva, USA, US 5892112); emaxanib, (Pfizer, USA, US 5792783); vatalanib, (Novartis, Switzerland); 2-methoxyestradiol, (EntreMed, USA); TLC ELL-12, (Elan, Ireland); anecortave acetate, (Alcon, USA); alpha-D148 Mab, (Amgen, USA); CEP-7055,(Cephalon, USA); anti-Vn Mab, (Crucell, Netherlands) DAC:antiangiogenic, (ConjuChem, Canada); Angiocidin, (InKine Pharmaceutical, USA); KM-2550, (Kyowa Hakko, Japan); SU-0879, (Pfizer, USA); CGP-79787, (Novartis, Switzerland, EP 970070); ARGENT technology, (Ariad, USA); YIGSR-Stealth, (Johnson & Johnson, USA); fibrinogen-E fragment, (BioActa, UK); angiogenesis inhibitor, (Trigen, UK); TBC-1635, (Encysive Pharmaceuticals, USA); SC-236, (Pfizer, USA); ABT-567, (Abbott, USA); Metastatin, (EntreMed, USA); angiogenesis inhibitor, (Tripep, Sweden); maspin, (Sosei, Japan); 2-methoxyestradiol, (Oncology Sciences Corporation, USA); ER-68203-00, (IVAX, USA); Benefin, (Lane Labs, USA); Tz-93, (Tsumura, Japan); TAN-1120, (Takeda, Japan); FR-111142, (Fujisawa, Japan, JP 02233610); platelet factor 4, (RepliGen, USA, EP 407122); vascular endothelial growth factor antagonist, (Borean, Denmark); bevacizumab (pINN), (Genentech, USA); angiogenesis inhibitors, (SUGEN, USA); XL 784, (Exelixis, USA); XL 647, (Exelixis, USA); MAb, alpha5beta3 integrin, second generation, (Applied Molecular Evolution, USA and MedImmune, USA); gene therapy, retinopathy, (Oxford BioMedica, UK); enzastaurin hydrochloride (USAN), (Lilly, USA); CEP 7055, (Cephalon, USA and Sanofi-Synthelabo, France); BC 1, (Genoa Institute of Cancer Research, Italy); angiogenesis inhibitor, (Alchemia, Australia); VEGF antagonist, (Regeneron, USA); rBPI 21 and BPI-derived antiangiogenic, (XOMA, USA); PI 88, (Progen, Australia); cilengitide (pINN), (Merck KGaA, German; Munich Technical University, Germany, Scripps Clinic and Research Foundation, USA); cetuximab (INN), (Aventis, France); AVE 8062, (Ajinomoto, Japan); AS 1404, (Cancer Research Laboratory, New Zealand); SG 292, (Telios, USA); Endostatin, (Boston Childrens Hospital, USA); ATN 161, (Attenuon, USA); ANGIOSTATIN, (Boston Childrens Hospital, USA); 2-methoxyestradiol, (Boston Childrens Hospital, USA); ZD 6474, (AstraZeneca, UK); ZD 6126, (Angiogene Pharmaceuticals, UK); PPI 2458, (Praecis, USA); AZD 9935, (AstraZeneca, UK); AZD 2171, (AstraZeneca, UK); vatalanib (pINN), (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); vaccine, gene-based, VEGF-2, (Scripps Clinic and Research Foundation, USA); SPV5.2, (Supratek, Canada); SDX 103, (University of California at San Diego, USA); PX 478, (ProlX, USA); METASTATIN, (EntreMed, USA); troponin I, (Harvard University, USA); SU 6668, (SUGEN, USA); OXI 4503, (OXiGENE, USA); o-guanidines, ( Dimensional Pharmaceuticals, USA); motuporamine C, (British Columbia University, Canada); CDP 791, (Celltech Group, UK); atiprimod (pINN), (GlaxoSmithKline, UK); E 7820, (Eisai, Japan); CYC 381, (Harvard University, USA); AE 941, (Aeterna, Canada); vaccine, angiogenesis, (EntreMed, USA); urokinase plasminogen activator inhibitor, (Dendreon, USA); oglufanide (pINN), (Melmotte, USA); HIF-1alfa inhibitors, (Xenova, UK); CEP 5214, (Cephalon, USA); BAY RES 2622, (Bayer, Germany); Angiocidin, (InKine, USA); A6, (Angstrom, USA); KR 31372, (Korea Research Institute of Chemical Technology, South Korea); GW 2286, (GlaxoSmithKline, UK); EHT 0101, (ExonHit, France); CP 868596, (Pfizer, USA); CP 564959, (OSI, USA); CP 547632, (Pfizer, USA); 786034, (GlaxoSmithKline, UK); KRN 633, (Kirin Brewery, Japan); drug delivery system, intraocular, 2-methoxyestradiol, (EntreMed, USA); anginex, (Maastricht University, Netherlands, and Minnesota University, USA); ABT 510, (Abbott, USA); AAL 993, (Novartis, Switzerland); VEGI, (ProteomTech, USA); tumor necrosis factor-alpha inhibitors, (National Institute on Aging, USA); SU 11248, (Pfizer, USA and SUGEN USA); ABT 518, (Abbott, USA); YH16, (Yantai Rongchang, China); S-3APG , (Boston Childrens Hospital, USA and EntreMed, USA); MAb, KDR, (ImClone Systems, USA); MAb, alpha5 beta1, (Protein Design, USA); KDR kinase inhibitor, (Celltech Group, UK, and Johnson & Johnson, USA); GFB 116, (South Florida University, USA and Yale University, USA); CS 706, (Sankyo, Japan); combretastatin A4 prodrug, (Arizona State University, USA); chondroitinase AC, (IBEX, Canada); BAY RES 2690, (Bayer, Germany); AGM 1470, (Harvard University, USA, Takeda, Japan, and TAP, USA); AG 13925, (Agouron, USA); Tetrathiomolybdate, (University of Michigan, USA); GCS 100, (Wayne State University, USA) CV 247, (Ivy Medical, UK); CKD 732, (Chong Kun Dang, South Korea); MAb, vascular endothelium growth factor, (Xenova, UK); irsogladine (INN), (Nippon Shinyaku, Japan); RG 13577, (Aventis, France); WX 360, (Wilex, Germany); squalamine (pINN), (Genaera, USA); RPI 4610, (Sirna, USA); cancer therapy, (Marinova, Australia); heparanase inhibitors, (InSight, Israel); KL 3106, (Kolon, South Korea); Honokiol, (Emory University, USA); ZK CDK, (Schering AG, Germany); ZK Angio, (Schering AG, Germany); ZK 229561, (Novartis, Switzerland, and Schering AG, Germany); XMP 300, (XOMA, USA); VGA 1102, (Taisho, Japan); VEGF receptor modulators, (Pharmacopeia, USA); VE-cadherin-2 antagonists , (ImClone Systems, USA); Vasostatin, (National Institutes of Health, USA);vaccine, Flk-1, (ImClone Systems, USA); TZ 93, (Tsumura, Japan); TumStatin, (Beth Israel Hospital, USA); truncated soluble FLT 1 (vascular endothelial growth factor receptor 1), (Merck & Co, USA); Tie-2 ligands, (Regeneron, USA); and, thrombospondin 1 inhibitor, (Allegheny Health, Education and Research Foundation, USA).

Autophagy inhibitors include, but are not limited to chloroquine, 3- methyladenine, hydroxychloroquine (Plaquenil^{™}), bafilomycin A1, 5-amino-4- imidazole carboxamide riboside (AICAR), okadaic acid, autophagy-suppressive algal toxins which inhibit protein phosphatases of type 2A or type 1, analogues of cAMP, and drugs which elevate cAMP levels such as adenosine, LY204002, N6-mercaptopurine riboside, and vinblastine. In addition, antisense or siRNA that inhibits expression of proteins including but not limited to ATG5 (which are implicated in autophagy), may also be used.

Additional pharmaceutically active compounds/agents that can be used in the treatment of cancers and that can be used in combination with one or more compound of the present invention include: epoetin alfa; darbepoetin alfa; panitumumab; pegfilgrastim; palifermin; filgrastim; denosumab; ancestim; AMG 102; AMG 386; AMG 479; AMG 655; AMG 745; AMG 951; and AMG 706, or a pharmaceutically acceptable salt thereof.

In certain embodiments, a composition provided herein is conjointly administered with a chemotherapeutic agent . Suitable chemotherapeutic agents may include, natural products such as vinca alkaloids (e.g., vinblastine, vincristine, and vinorelbine), paclitaxel, epidipodophyllotoxins (e.g., etoposide and teniposide), antibiotics (e.g., dactinomycin (actinomycin D), daunorubicin, doxorubicin, and idarubicin), anthracyclines, mitoxantrone, bleomycins, plicamycin (mithramycin), mitomycin, enzymes (e.g., L-asparaginase which systemically metabolizes L-asparagine and deprives cells which do not have the capacity to synthesize their own asparagine), antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexaamethylmelaamine and thiotepa), CDK inhibitors (e.g., seliciclib, UCN-01, P1446A-05, PD-0332991, dinaciclib, P27-00, AT-7519, RGB286638, and SCH727965), alkyl sulfonates (e.g., busulfan), nitrosoureas (e.g., carmustine (BCNU) and analogs, and streptozocin), trazenes-dacarbazinine (DTIC), antiproliferative/antimitotic antimetabolites such as folic acid analogs (e.g., methotrexate), pyrimidine analogs (e.g., fluorouracil, floxuridine, and cytarabine), purine analogs and related inhibitors (e.g., mercaptopurine, thioguanine, pentostatin and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, LBH 589, romidepsin, ACY-1215, and panobinostat), mTor inhibitors (e.g., temsirolimus, everolimus, ridaforolimus, and sirolimus), KSP(Eg5) inhibitors (e.g., Array 520), DNA binding agents (e.g., Zalypsis), PI3K delta inhibitor (e.g., GS-1101 and TGR-1202), PI3K delta and gamma inhibitor (e.g., CAL-130), multi-kinase inhibitor (e.g., TG02 and sorafenib), hormones (e.g., estrogen) and hormone agonists such as leutinizing hormone releasing hormone (LHRH) agonists (e.g., goserelin, leuprolide and triptorelin), BAFF-neutralizing antibody (e.g., LY2127399), IKK inhibitors, p38MAPK inhibitors, anti-IL-6 (e.g., CNTO328), telomerase inhibitors (e.g., GRN 163L), aurora kinase inhibitors (e.g., MLN8237, AMG 900, AZD-1152), cell surface monoclonal antibodies (e.g., anti-CD38 (HUMAX-CD38), anti-CS1 (e.g., elotuzumab), HSP90 inhibitors (e.g., 17 AAG and KOS 953), P13K / Akt inhibitors (e.g., perifosine), Akt inhibitor (e.g., GSK-2141795), PKC inhibitors (e.g., enzastaurin), FTIs (e.g., Zarnestra^{™}), anti-CD138 (e.g., BT062), Torc1/2 specific kinase inhibitor (e.g., INK128), kinase inhibitor (e.g., GS-1101), ER/UPR targeting agent (e.g., MKC-3946), cFMS inhibitor (e.g., ARRY-382), JAK1/2 inhibitor (e.g., CYT387), PARP inhibitor (e.g., olaparib, Talazoparib, Niraparib veliparib (ABT-888)), BCL-2 antagonist. Other chemotherapeutic agents may include mechlorethamine, camptothecin, ifosfamide, tamoxifen, raloxifene, gemcitabine, navelbine, sorafenib, or any analog or derivative variant of the foregoing.

The compounds of the present invention may also be used in combination with radiation therapy, hormone therapy, surgery and immunotherapy, which therapies are well known to those skilled in the art.

In certain embodiments, a pharmaceutical composition provided herein is conjointly administered with a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts and/or derivatives thereof In a particular embodiment, the compounds of the present invention can also be used in combination with additional pharmaceutically active agents that treat nausea. Examples of agents that can be used to treat nausea include: dronabinol; granisetron; metoclopramide; ondansetron; and prochlorperazine; or a pharmaceutically acceptable salt thereof.

The compounds or pharmaceutical compositions of the disclosure can also be used in combination with an amount of one or more substances selected from EGFR inhibitors, MEK inhibitors, PI3K inhibitors, AKT inhibitors, TOR inhibitors, and immune therapies, including anti-PD-1, anti-PDL-1, anti-CTLA4, anti-LAG1, and anti-OX40 agents, GITR agonists, CAR-T cells, and BiTEs.

EGFR inhibitors include, but are not limited to, small molecule antagonists, antibody inhibitors, or specific antisense nucleotide or siRNA. Useful antibody inhibitors of EGFR include cetuximab (Erbitux), panitumumab (Vectibix), zalutumumab, nimotuzumab, and matuzumab. Small molecule antagonists of EGFR include gefitinib, erlotinib (Tarceva), and most recently, lapatinib (TykerB). See e.g., Yan L, et. al., Pharmacogenetics and Pharmacogenomics In Oncology Therapeutic Antibody Development, BioTechniques 2005; 39(4): 565-8, and Paez J G, et. al., EGFR Mutations In Lung Cancer Correlation With Clinical Response To Gefitinib Therapy, Science 2004; 304(5676): 1497-500.

Non-limiting examples of small molecule EGFR inhibitors include any of the EGFR inhibitors described in the following patent publications, and all pharmaceutically acceptable salts and solvates of said EGFR inhibitors: European Patent Application EP 520722, published Dec. 30, 1992; European Patent Application EP 566226, published Oct. 20, 1993; PCT International Publication WO 96/33980, published Oct. 31, 1996; U.S. Pat. No. 5,747,498, issued May 5, 1998; PCT International Publication WO 96/30347, published Oct. 3, 1996; European Patent Application EP 787772, published Aug. 6, 1997; PCT International Publication WO 97/30034, published Aug. 21, 1997; PCT International Publication WO 97/30044, published Aug. 21, 1997; PCT International Publication WO 97/38994, published Oct. 23, 1997; PCT International Publication WO 97/49688, published Dec. 31, 1997; European Patent Application EP 837063, published Apr. 22, 1998; PCT International Publication WO 98/02434, published Jan. 22, 1998; PCT International Publication WO 97/38983, published Oct. 23, 1997; PCT International Publication WO 95/19774, published Jul. 27, 1995; PCT International Publication WO 95/19970, published Jul. 27, 1995; PCT International Publication WO 97/13771, published Apr. 17, 1997; PCT International Publication WO 98/02437, published Jan. 22, 1998; PCT International Publication WO 98/02438, published Jan. 22, 1998; PCT International Publication WO 97/32881, published Sep. 12, 1997; German Application DE 19629652, published Jan. 29, 1998; PCT International Publication WO 98/33798, published Aug. 6, 1998; PCT International Publication WO 97/32880, published Sep. 12, 1997; PCT International Publication WO 97/32880 published Sep. 12, 1997; European Patent Application EP 682027, published Nov. 15, 1995; PCT International Publication WO 97/02266, published Jan. 23, 197; PCT International Publication WO 97/27199, published Jul. 31, 1997; PCT International Publication WO 98/07726, published Feb. 26, 1998; PCT International Publication WO 97/34895, published Sep. 25, 1997; PCT International Publication WO 96/31510', published Oct. 10, 1996; PCT International Publication WO 98/14449, published Apr. 9, 1998; PCT International Publication WO 98/14450, published Apr. 9, 1998; PCT International Publication WO 98/14451, published Apr. 9, 1998; PCT International Publication WO 95/09847, published Apr. 13, 1995; PCT International Publication WO 97/19065, published May 29, 1997; PCT International Publication WO 98/17662, published Apr. 30, 1998; U.S. Pat. No. 5,789,427, issued Aug. 4, 1998; U.S. Pat. No. 5,650,415, issued Jul. 22, 1997; U.S. Pat. No. 5,656,643, issued Aug. 12, 1997; PCT International Publication WO 99/35146, published Jul. 15, 1999; PCT International Publication WO 99/35132, published Jul. 15, 1999; PCT International Publication WO 99/07701, published Feb. 18, 1999; and PCT International Publication WO 92/20642 published Nov. 26, 1992. Additional non-limiting examples of small molecule EGFR inhibitors include any of the EGFR inhibitors described in Traxler, P., 1998, Exp. Opin. Ther. Patents 8(12):1599-1625.

Antibody-based EGFR inhibitors include any anti-EGFR antibody or antibody fragment that can partially or completely block EGFR activation by its natural ligand. Non-limiting examples of antibody-based EGFR inhibitors include those described in Modjtahedi, H., et al., 1993, Br. J. Cancer 67:247-253; Teramoto, T., et al., 1996, Cancer 77:639-645; Goldstein et al., 1995, Clin. Cancer Res. 1:1311-1318; Huang, S. M., et al., 1999, Cancer Res. 15:59(8):1935-40; and Yang, X., et al., 1999, Cancer Res. 59:1236-1243. Thus, the EGFR inhibitor can be monoclonal antibody Mab E7.6.3 (Yang, 1999 supra), or Mab C225 (ATCC Accession No. HB-8508), or an antibody or antibody fragment having the binding specificity thereof.

MEK inhibitors include, but are not limited to, CI-1040, AZD6244, PD318088, PD98059, PD334581, RDEA119, ARRY-142886, ARRY-438162, and PD-325901.

PI3K inhibitors include, but are not limited to, wortmannin, 17-hydroxywortmannin analogs described in WO 06/044453, 4-[2-(1H-Indazol-4-yl)-6-[[4-(methylsulfonyl)piperazin-1-yl]methyl]thieno[3,2-d]pyrimidin-4-yl]morpholine (also known as GDC 0941 and described in PCT Publication Nos. WO 09/036,082 and WO 09/055,730), 2-Methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in PCT Publication No. WO 06/122806), (S)-1-(4-((2-(2-aminopyrimidin-5-yl)-7-methyl-4-morpholinothieno[3,2-d]pyrimidin-6-yl)methyl)piperazin-1-yl)-2-hydroxypropan-1-one (described in PCT Publication No. WO 2008/070740), LY294002 (2-(4-Morpholinyl)-8-phenyl-4H-1-benzopyran-4-one available from Axon Medchem), PI 103 hydrochloride (3-[4-(4-morpholinylpyrido-[3',2':4,5]furo[3,2-d]pyrimidin-2-yl]phenol hydrochloride available from Axon Medchem), PIK 75 (N'-[(1E)-(6-bromoimidazo[1,2-a]pyridin-3-yl)methylene]-N,2-dimethyl-5-nitrobenzenesulfono-hydrazide hydrochloride available from Axon Medchem), PIK 90 (N-(7,8-dimethoxy-2,3-dihydro-imidazo[1,2-c]quinazolin-5-yl)-nicotinamide available from Axon Medchem), GDC-0941 bismesylate (2-(1H-Indazol-4-yl)-6-(4-methanesulfonyl-piperazin-1-ylmethyl)-4-morpholin-4-yl-thieno[3,2-d]pyrimidine bismesylate available from Axon Medchem), AS-252424 (5-[1-[5-(4-Fluoro-2-hydroxy-phenyl)-furan-2-yl]-meth-(Z)-ylidene]-thiazolidine-2,4-dione available from Axon Medchem), and TGX-221 (7-Methyl-2-(4-morpholinyl)-9-[1-(phenylamino)ethyl]-4H-pyrido-[1,2-a]pyrimidin-4-one available from Axon Medchem), XL-765, and XL-147. Other PI3K inhibitors include demethoxyviridin, perifosine, CAL101, PX-866, BEZ235, SF1126, INK1117, IPI-145, BKM120, XL147, XL765, Palomid 529, GSK1059615, ZSTK474, PWT33597, IC87114, TG100-115, CAL263, PI-103, GNE-477, CUDC-907, and AEZS-136.

AKT inhibitors include, but are not limited to, Akt-1-1 (inhibits Akt1) (Barnett et al. (2005) Biochem. J., 385 (Pt. 2), 399-408); Akt-1-1,2 (inhibits Ak1 and 2) (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (e.g., WO05011700); indole-3-carbinol and derivatives thereof (e.g., U.S. Pat. No. 6,656,963; Sarkar and Li (2004) J Nutr. 134(12 Suppl), 3493S-3498S); perifosine (e.g., interferes with Akt membrane localization; Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); and triciribine (TCN or API-2 or NCI identifier: NSC 154020; Yang et al. (2004) Cancer Res. 64, 4394-9).

TOR inhibitors include, but are not limited to, inhibitors include AP-23573, CCI-779, everolimus, RAD-001, rapamycin, temsirolimus, ATP-competitive TORC1/TORC2 inhibitors, including PI-103, PP242, PP30 and Torin 1. Other TOR inhibitors in FKBP12 enhancer; rapamycins and derivatives thereof, including: CCI-779 (temsirolimus), RAD001 (Everolimus; WO 9409010) and AP23573; rapalogs, e.g. as disclosed in WO 98/02441 and WO 01/14387, e.g. AP23573, AP23464, or AP23841; 40-(2-hydroxyethyl)rapamycin, 40-[3-hydroxy(hydroxymethyl)methylpropanoate]-rapamycin (also called CC1779), 40-epi-(tetrazolyt)-rapamycin (also called ABT578), 32-deoxorapamycin, 16-pentynyloxy-32(S)-dihydrorapanycin, and other derivatives disclosed in WO 05005434; derivatives disclosed in U.S. Pat. No. 5,258,389, WO 94/090101, WO 92/05179, U.S. Pat. No. 5,118,677, U.S. Pat. No. 5,118,678, U.S. Pat. No. 5,100,883, U.S. Pat. No. 5,151,413, U.S. Pat. No. 5,120,842, WO 93/111130, WO 94/02136, WO 94/02485, WO 95/14023, WO 94/02136, WO 95/16691, WO 96/41807, WO 96/41807 and U.S. Pat. No. 5,256,790; phosphorus-containing rapamycin derivatives (e.g., WO 05016252); 4H-1-benzopyran-4-one derivatives (e.g., U.S. Provisional Application No. 60/528,340).

Immune therapies include, but are not limited to, anti-PD-1 agents, anti-PDL-1 agents, anti-CTLA-4 agents, anti-LAG1 agents, and anti-OX40 agents. Exemplary anti-PD-1 antibodies and methods for their use are described by Goldberg et al., Blood 110(1): 186-192 (2007), Thompson et al., Clin. Cancer Res. 13(6):1757-1761 (2007), and Korman et al., International Application No. PCT/JP2006/309606 (publication no. WO 2006/121168 A1),. include: Yervoy^{™} (ipilimumab) or Tremelimumab (to CTLA-4), galiximab (to B7.1), BMS-936558 (to PD-1), MK-3475 (to PD-1), AMP224 (to B7DC), BMS-936559 (to B7-H1), MPDL3280A (to B7-H1), MEDI-570 (to ICOS), AMG557 (to B7H2), MGA271 (to B7H3), IMP321 (to LAG-3), BMS-663513 (to CD137), PF-05082566 (to CD137), CDX-1127 (to CD27), anti-OX40 (Providence Health Services), huMAbOX40L (to OX40L), Atacicept (to TACI), CP-870893 (to CD40), Lucatumumab (to CD40), Dacetuzumab (to CD40), Muromonab-CD3 (to CD3), Ipilumumab (to CTLA-4). Immune therapies also include genetically engineered T-cells (e.g., CAR-T cells) and bispecific antibodies (e.g., BiTEs).

GITR agonists include, but are not limited to, GITR fusion proteins and anti-GITR antibodies (e.g., bivalent anti-GITR antibodies), such as, a GITR fusion protein described in U.S. Pat. No. 6,111,090box.c, European Patent No.: 090505B1, U.S. Pat. No. 8,586,023, PCT Publication Nos.: WO 2010/003118 and 2011/090754, or an anti-GITR antibody described, e.g., in U.S. Pat. No. 7,025,962, European Patent No.: 1947183B1, U.S. Pat. No. 7,812,135, U.S. Pat. No. 8,388,967, U.S. Pat. No. 8,591,886, European Patent No.: EP 1866339, PCT Publication No.: WO 2011/028683, PCT Publication No.: WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Pat. No. 7,618,632, and PCT Publication No.: WO 2011/051726.

The compounds described herein can be used in combination with the agents disclosed herein or other suitable agents, depending on the condition being treated. Hence, in some embodiments the one or more compounds of the disclosure will be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound described herein and any of the agents described above can be formulated together in the same dosage form and administered simultaneously. Alternatively, a compound of the disclosure and any of the agents described above can be simultaneously administered, wherein both the agents are present in separate formulations. In another alternative, a compound of the present disclosure can be administered just followed by and any of the agents described above, or vice versa. In some embodiments of the separate administration protocol, a compound of the disclosure and any of the agents described above are administered a few minutes apart, or a few hours apart, or a few days apart.

As one aspect of the present invention contemplates the treatment of the disease/conditions with a combination of pharmaceutically active compounds that may be administered separately, the invention further relates to combining separate pharmaceutical compositions in kit form. The kit comprises two separate pharmaceutical compositions: a compound of the present invention, and a second pharmaceutical compound. The kit comprises a container for containing the separate compositions such as a divided bottle or a divided foil packet. Additional examples of containers include syringes, boxes, and bags. In some embodiments, the kit comprises directions for the use of the separate components. The kit form is particularly advantageous when the separate components are preferably administered in different dosage forms (e.g., oral and parenteral), are administered at different dosage intervals, or when titration of the individual components of the combination is desired by the prescribing health care professional.

### EXPERIMENTAL

Abbreviations: The following abbreviations may be used herein:

| | |
|---|---|
| AcOH | acetic acid |
| aq or aq. | aqueous |
| BOC or Boc | *tert*-butyloxycarbonyl |
| COMU | (1-cyano-2-ethoxy-2-oxoethylidenaminooxy)dimethylamino-morpholino-carbenium hexafluorophosphate |
| DCE | 1,2-dichloroethane |
| DCM | DCM |
| DMAP | 4-dimethylaminopyridine |
| DMF | *N,N-*dimethylformamide |
| DMSO | dimethyl sulfoxide |
| DMTMM | 4-(4,6-dimethoxy-1,3,5-triazin-2-yl)-4-methylmorpholinium chloride |
| Dppf, DPPF or dppf | 1,1'-bis(diphenylphosphino)ferrocene |
| ESI or ES | electrospray ionization |
| Et | ethyl |
| Et₂O | diethyl ether |
| EtOH | ethyl alcohol |
| EtOAc | EtOAc |
| G | grams |
| H | hour |
| HPLC | high pressure liquid chromatography |
| iPr | isopropyl |
| *i*Pr₂NEt or DIPEA | *N*-ethyl diisopropylamine (Hünig's base) |
| KOAc | potassium acetate |
| LAH | lithium aluminum hydride |
| LDA | lithium diisopropylamide |
| LC MS, LCMS, LC-MS or LC/MS | liquid chromatography mass spectroscopy |
| LG | leaving group (e.g., halogen, mesylate, triflate) |
| LiHMDS | lithium bis(trimethylsilyl)amide |
| m/z | mass divided by charge |
| Me | methyl |
| MeCN/CAN | acetonitrile |
| MeOH | methanol |
| Met | metal species for cross-coupling (e.g., MgX, ZnX, SnR₃, SiR₃, B(OR)₂) |
| Mg | milligrams |
| Min | minutes |
| mL | milliliters |
| MS | mass spectra |
| MsCl | methanesulfonyl chloride |
| MTBE | *tert*-butyl methyl ether |
| NMP | 1-methyl-2-pyrrolidine |
| *n*-BuLi | n-butyllithium |
| NMR | nuclear magnetic resonance |
| Pd₂(dba)₃ | tris(dibenzylideneacetone)dipalladium(0) |
| Pd(dppf)Cl₂· DCM | [1,1'-bis(diphenylphosphino)ferrocene]dichloropalladium(II), complex with DCM |
| Pd(PPh₃)₄ | tetrakis(triphenylphosphine)palladium(0) |
| Ph | phenyl |
| PR or PG or Prot. group | protecting group |
| Rbf | round-bottom flask |
| RP-HPLC | reverse phase high pressure liquid chromatography |
| RT or rt | RT |
| RuPhos | 2-dicyclohexylphosphino-2',6'-diisopropoxybiphenyl |
| sat. or satd. | saturated |
| SFC | supercritical fluid chromatography |
| TBAB | tetra-*n*-butylammonium bromide |
| TBAF | tetra-*n*-butylammonium fluoride |
| TBDMS Cl | *tert*-butyldimethylchlorosilane |
| *t*-BuOH | *tert*-butanol |
| TEA or Et₃N | trimethylamine |
| TFA | trifluoroacetic acid |
| THF | tetrahydrofuran |
| T₃P | 2,4,6-tripropyl-1,3,5,2,4,6-trioxatriphosphinane 2,4,6-trioxide |
| TsCl | *p*-toluenesulfonyl chloride |
| Xantphos | 4,5-bis(diphenylphosphino)-9,9-dimethylxanthene |

Unless otherwise noted, all materials were obtained from commercial suppliers and used without further purification. All parts are by weight and temperatures are in degrees centigrade unless otherwise indicated. All microwave assisted reactions were conducted with a Smith Synthesizer^{™} from Biotage^{™}. All compounds showed NMR spectra consistent with their assigned structures. Melting points were determined on a Buchi apparatus and are uncorrected. Mass spectral data was determined by electrospray ionization technique. All examples were purified to >90% purity as determined by high-performance liquid chromatography. Unless otherwise stated, reactions were run at room temperature.

In synthesizing compounds of the present invention, it may be desirable to use certain leaving groups. The term "leaving groups" ("LG") generally refer to groups that are displaceable by a nucleophile. Such leaving groups are known in the art. Examples of leaving groups include, but are not limited to, halides (e.g., I, Br, F, Cl), sulfonates (e.g., mesylate, tosylate), sulfides (e.g., SCH₃), N-hydroxysuccinimide, N-hydroxybenzotriazole, and the like. Examples of nucleophiles include, but are not limited to, amines, thiols, alcohols, Grignard reagents, anionic species (e.g., alkoxides, amides, carbanions) and the like.

The examples presented below illustrate specific embodiments of the present invention. These examples are meant to be representative.

It is noted that when a percent (%) is used with regard to a liquid, it is a percent by volume with respect to the solution. When used with a solid, it is the percent with regard to the solid composition. Materials obtained from commercial suppliers were typically used without further purification. Reactions involving air or moisture sensitive reagents were typically performed under a nitrogen or argon atmosphere. Purity was measured using high performance liquid chromatography (HPLC) system with UV detection at 254 nm and 215 nm (System A: Agilent Zorbax Eclipse XDB-C8 4.6 x 150 mm, 5 µm, 5 to 100% CH₃CN in H₂O with 0.1% TFA for 15 min at 1.5 mL/min; System B: Zorbax SB-C8, 4.6 x 75 mm, 10 to 90% CH₃CN in H₂O with 0.1% formic acid for 12 min at 1.0 mL/min) (Agilent Technologies, Santa Clara, CA). Silica gel chromatography was generally performed with prepacked silica gel cartidges (Biotage, Uppsala, Sweden or Teledyne-Isco, Lincoln, NE). ¹H NMR spectra were recorded on a Bruker AV-400 (400 MHz) spectrometer (Bruker Corporation, Madison, WI) or a Varian (Agilent Technologies, Santa Clara, CA) 400 MHz spectrometer at ambient temperature. All observed protons are reported as parts per million (ppm) downfield from tetramethylsilane (TMS) or other internal reference in the appropriate solvent indicated. Data are reported as follows: chemical shift, multiplicity (s = singlet, d = doublet, t = triplet, q = quartet, br = broad, m = multiplet), coupling constants, and number of protons. Low-resolution mass spectral (MS) data were determined on an Agilent 1100 Series (Agilent Technologies, Santa Clara, CA) LC/MS with UV detection at 254 nm and 215 nm and a low resonance electrospray mode (ESI).

### GENERAL SYNTHETIC SCHEME

Unless otherwise stated, starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure over a temperature range from about -78 °C to about 150 °C, more preferably from about 0 °C to about 125 °C and most preferably at about room (or ambient) temperature, e.g., about 20 °C.

For the purpose of clarity in this general synthesis section, Compounds of Formula (I) can be schematically drawn to contain Ring Ar¹ and Ring Ar² as follows: 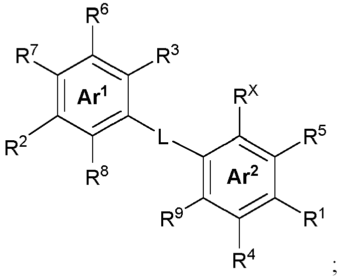 wherein the group L is a linker as defined in the summary of the inventions, i.e., -NR³-(C=O)- or -NR³-(C=O)-; ring Ar¹ is located to the left of the linker, and ring Ar² is located to the right of the linker.

Generally, compounds of Formula (I), can be synthesized via three general steps as follows:
Step 1: Preparation of Ring Ar¹ compound.
Step 2: Preparation of Ring Ar² compound.
Step 3: Coupling of Ring Ar¹ compound to Ring Ar² compound.

The generic schemes below are meant to provide guidance to the ordinarily skilled synthetic chemist, who will readily appreciate that the solvent, concentration, reagent, protecting group, order of synthetic steps, time, temperature, and the like can be modified as necessary, well within the skill and judgment of the ordinarily skilled artisan.

In one embodiment, a compound of Formula (I) having the following formula (Ia): 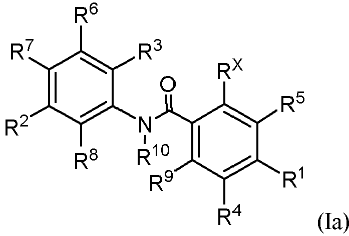 ; can be synthesized according to Schemes A and B.

An example of a compound of formula (Ia) includes, but is not limited to:

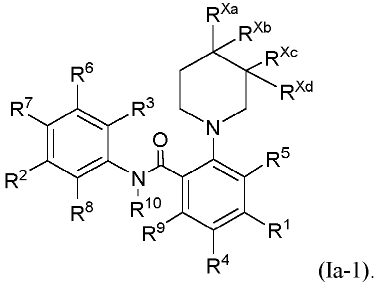

### SCHEME A: PREPARATION OF COMPOUND (Ia):

### Step A-1: Preparation of Ring Ar¹ compound:

According to Scheme A, in one embodiment, a Ring Ar¹ compound can be prepared as follows:

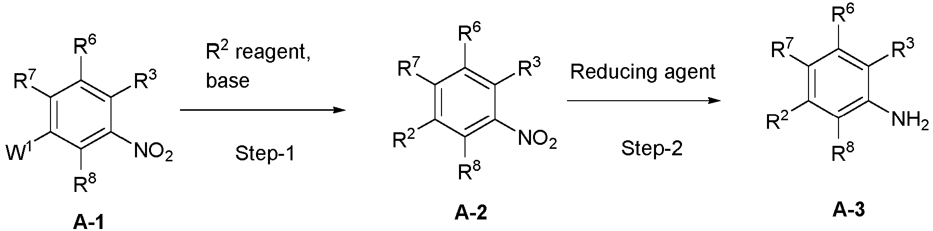

Compound A-1, wherein the group W¹ is a leaving group, for example halo, such as fluoro, chloro, bromo, or iodo; which is commercially available or can be prepared according to known methods and reagents by those skilled in the art, can be reacted with an appropriate R² reagent, such as (1) (*R*)-2-methylmorpholine, (2) 4,4-difluoropiperidine hydrochloride, (3) 3,3-difluoroazetidine hydrochloride, (4) 3,3,3-trifluoropropan-1-ol, (5) 2-aminoethan-1-ol, or (6) 2-amino3-methylpropan-1-ol, in the presence of a base, such as diisopropylethyl amine, potassium carbonate, or sodium hydride, in a suitable organic solvent such as NMP, acetonitrile, tetrahydrofuran, DMF, dioxane, and the like, to form compound A-2. Examples of compound A-1 include, but are not limited to, 1-fluoro-3-nitrobenzene, 1,3-difluoro-5-nitrobenzene, 1-fluoro-3-methylbenzene, or 2-bromo-1-fluoro-4-nitrobenzene. Examples of compound A-2 includes (*R*)-4-(3-fluoro-5-nitrophenyl)-2-methylmorpholine, 4-(3-fluoro-5-nitrophenyl)morpholine, (*R*)-2-methyl-4-(3-methyl-5-nitrophenyl)morpholine, or 4,4-difluoro-1-(3-fluoro-5-nitrophenyl)piperidine. Compound A-2 can then be reacted with a reducing agent, such as hydrogen gas, in the presence of a suitable catalyst, such as Pd/C, in a suitable organic solvent such as MeOH, EtOH, THF and the like, to form compound A-3. Examples of compound A-3 includes (*R*)-3-fluoro-5-(2-methylmorpholino)aniline, (*R*)-3-methyl-5-(2-methylmorpholino)aniline, or 3-(4,4-difluoropiperidin-1-yl)-5-fluoroaniline.

Alternatively, Compound **A-1** may be converted in one pot synthesis as outlined in Step 1a above to Compound A-3, without further purification of Compound **A-2.**

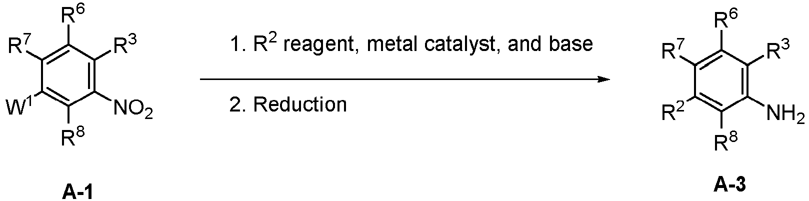

Yet alternatively, Compound **A-1** may be converted to compound **A-2,** as defined in Step 1a above, via metal catalyzed amination, where a suitable palladium or copper catalyst and a base are used. This step can be followed by a reduction with a suitable palladium catalyst and a hydrogen source, such as Pd/C in the presence of hydrogen gas, to form compound **A-2.**

Yet alternatively, Compound **A-2** may be commercially available. Example of commercially available Compound **A-2** is 3-amino-*N*-(*tert*-butyl)benzenesulfonamide.

### Step A-2: Preparation of Ring Ar² compound:

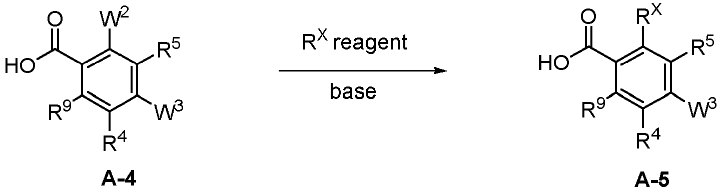

In Step A-2, Compound **A-4,** wherein each of W² and W³ is independently a halogen, for example fluoro, chloro, bromo, or iodo, can be reacted with an R^{X} reagent, such as (1) 6-azaspiro[2.5]octane hydrochloride, (2) 4,4-dimethylpiperidine hydrochloride, (3) 3,4,4-trimethylpiperidine hydrochloride, (4) 4-methyl-6-azaspiro[2.5]octane hydrochloride, or (5) 7-azaspiro[3.5]nonane hydrochloride, in a suitable organic solvent such as NMP, acetonitrile, tetrahydrofuran, DMF, DMSO, and the like, to form Compound A-5. Examples of compound **A-4** that can be reacted in this manner includes 4,6-dichloronicotinic acid, 2-fluoro-4-iodobenzoic acid, or 4,6-difluoronicotinic acid.

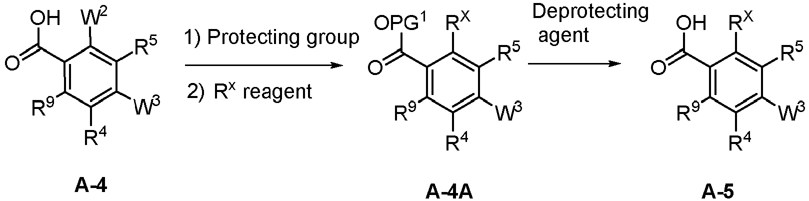

Alternatively, Compound **A-4,** as defined above, may be reacted with an appropriate carboxylic acid protecting group (PG¹ reagent), such as PG¹OH in the presence of SOCl₂, or benzyl bromide in the presence of sodium carbonate, to form a methyl ester or benzyl ester, in a suitable organic solvent such as NMP, acetonitrile, tetrahydrofuran, DMF, methylene chloride, and the like, followed by reaction with an R^{X} reagent, such as (1) 6-azaspiro[2.5]octane in the presence of DIPEA, (2) 4,4-dimethylpiperidine hydrochloride, (3) 3,4,4-trimethylpiperidine hydrochloride, (4) 4-methyl-6-azaspiro[2.5]octane hydrochloride, or (5) 7-azaspiro[3.5]nonane hydrochloride, in a suitable organic solvent such as NMP, acetonitrile, tetrahydrofuran, DMF, and the like, to form compound **A-4A,** wherein W³ is as defined in compound **A-4.** Compound **A-4A** can then be reacted with a deprotecting agent, which can be a base, such as lithium hydroxide, followed by neutralization with HCl, to form compound **A-5,** wherein W³ is as defined in compound **A-4.**

### Step A-3: Coupling of Ring Ar¹ compound to Ring Ar² compound followed by introduction of R¹:

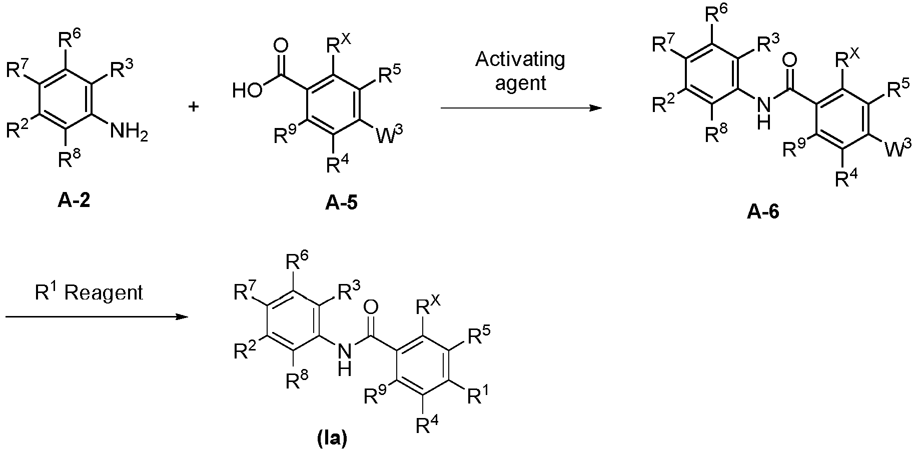

In Step A-3, compound **A-5,** which was obtained from Step A-2, can be reacted with an activating agent such as acid chloride (COCl)₂ or SOCl₂, in a suitable organic solvent such as tetrahydrofuran, methylene chloride, and the like, to form an acid chloride derivative, which can then react with compound **A-2** to form compound **A-6.**

Alternatively, compound **A-2** can be directly reacted with compound **A-5,** which was obtained from Step A-2, in a suitable organic solvent such as acetonitrile, tetrahydrofuran, DMF, methylene chloride, and the like, in the presence of a coupling reagent, such as N, N'-diisopropylcarbodiimide, N-(3-dimethylaminopropyl)-N'-ethylcarbodiimide, benzotriazol-1-yl-oxytripyrrolidinophosphonium hexafluorophosphate, *O*-(benzotriazol-1-yl)-N,N,N',N'-tetramethyluronium hexafluorophosphate, thionyl chloride, carbonyl diimidazole, and polyphosphonic anhydride. Those ordinary skilled synthetic chemists will readily understand that other known coupling agents can be used.

Compound **A-6** can then be converted to Compound (Ia) via transformation reactions such as, metal-catalyzed sulfoamidation, sulfination, or sulfonylation, in a suitable organic solvent such as DMSO, acetonitrile, tetrahydrofuran, DMF, methylene chloride, and the like, in the presence of a metal catalyst and an R¹ reagent, such as (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropanoate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol. Those ordinary skilled in the art will readily understand that coupling reaction such as shown in Step A-3a can be performed under various known conditions.

### SCHEME B: ALTERNATIVE PREPARATION OF COMPOUND (Ia):

According to Scheme B, in another embodiment of the invention, the preparation of Ring Ar¹ compound can be performed as outlined in Step A-1 of SCHEME A above.

### Step B-2: Preparation of Ring Ar² compound:

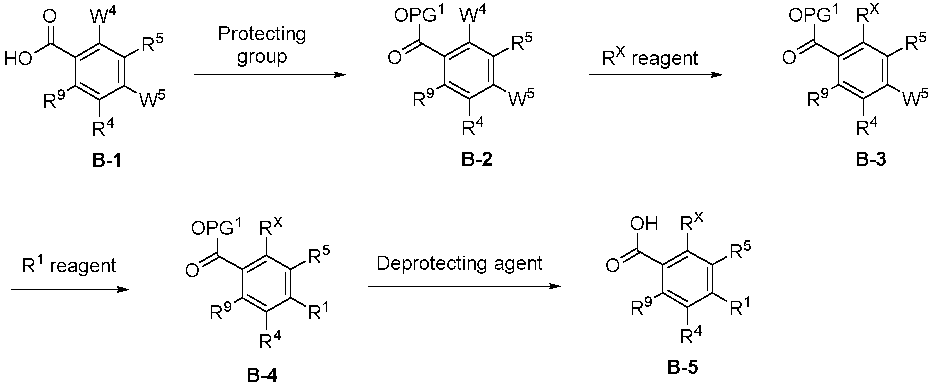

Scheme B provides an alternative method for formation of compounds of Formula (I) as disclosed herein. After Step A-1 as described in Scheme A, the group R¹ can alternatively be introduced to Ring Ar² in Step B-2 rather that in Step A-3 as in Scheme A. According to Step B-2, compound **B-1**, wherein each of W⁴ and W⁵ is independently a halogen, for example fluoro, chloro, bromo, or iodo, can be reacted with an appropriate carboxylic acid protecting group (PG₁ reagent), such as methyl iodide in the presence of a base such as potassium carbonate, to form a methyl ester, or other appropriate protecting group to form other ester such as benzyl ester, in a suitable organic solvent such as NMP, acetonitrile, tetrahydrofuran, DMF, methylene chloride and the like, to form compound **B-2,** wherein each of W⁴ and W⁵ are as defined in compound **B-1**. Compound **B-2** can then be reacted with an R^{X} reagent, such as 6-azaspiro[2.5]octane, in a suitable organic solvent such as NMP, acetonitrile, tetrahydrofuran, DMF, methylene chloride, DMSO, and the like, to form compound **B-3**, wherein W⁵ is as defined in compound **B-1**. Compound **B-3** can then be reacted with an R¹ reagent by a transformation reaction such as, metal-catalyzed sulfoamidation, sulfination, or sulfonylation, in a suitable organic solvent such as DMSO, acetonitrile, tetrahydrofuran, DMF, methylene chloride, and the like, in the presence of a metal catalyst, such as copper iodide, Pd₂(dba)₃ to form compound **B-4**, which can then be reacted further with an appropriate carboxylic acid deprotecting agent to form compound **B-5**. Appropriate carboxylic acid protecting groups and deprotection agents are known to those skilled in the art, e.g., as discussed in Greene's Protective Groups in Organic Synthesis.

### Step B-3: Coupling of Ring Ar¹ compound to Ring Ar² compound:

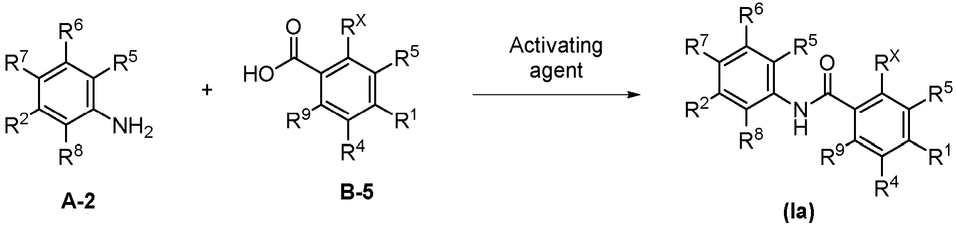

In Step B-3, compound **B-5**, which was obtained from Step B-2, can be reacted with an activating agent such as acid chloride (COCl)₂ or SOCl₂, in a suitable organic solvent such as tetrahydrofuran, methylene chloride, and the like, to form an acid chloride derivative, which can then react with compound **A-2** to form compound **(Ia).**

### SCHEME C

In another embodiment, a compound of formula (I) having a general formula **(Ib):** 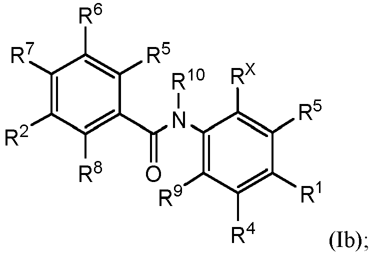 as defined herein can be synthesized according to Scheme C.

An example of a compound of formula (Ib) includes, but is not limited to:

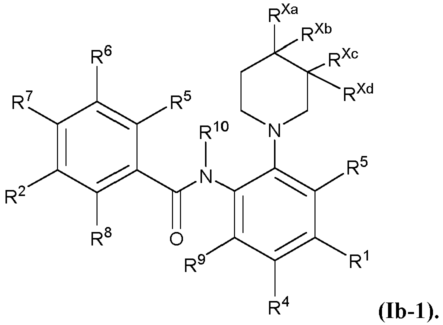

### Step C-1: Preparation of Ring Ar¹ compound:

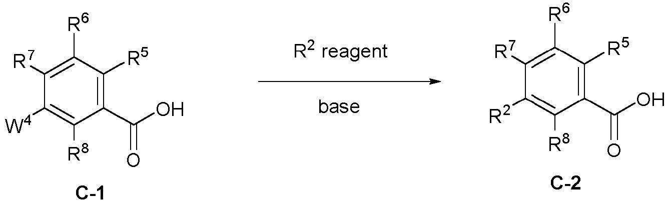

In step C-1, compound **C-1,** wherein W⁴ is a halogen, for example fluoro or chloro, can be reacted with an R² reagent in the presence of a suitable base, in a suitable organic solvent such as NMP, dioxane, acetonitrile, tetrahydrofuran, DMF, methylene chloride, and the like, to form compound **C-2.** Examples of compound **C-1** include, but are not limited to, 3-fluorobenzoic acid or, 3-fluoro-3-methylbenzoic acid. Examples of R² reagents include, but not limited to (1) (R)-2-methylmorpholine, (2) 4,4-difluoropiperidine hydrochloride, or (3) 3,3-difluoroazetidine hydrochloride. Examples of bases include, but are not limited to diisopropylethyl amine, potassium carbonate.

### Step C-2: Preparation of Ring Ar² compound:

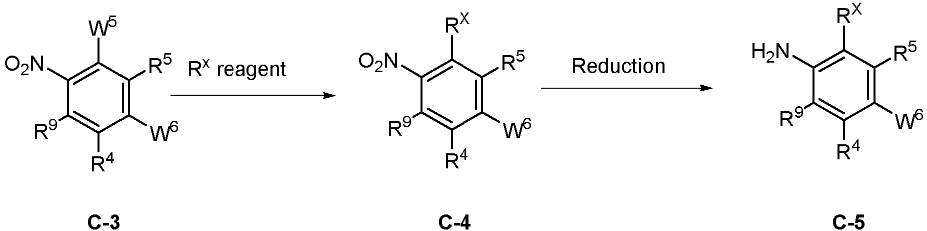

**In** step **C-2,** compound **C-3,** wherein each of W⁵ and W⁶ is independently a halogen, for example fluoro, chloro, bromo, or iodo, can be reacted with an R^{X} reagent, such as (1) 6-azaspiro[2.5]octane hydrochloride, (2) 4,4-dimethylpiperidine hydrochloride, (3) 3,4,4-trimethylpiperidine hydrochloride, (4) 4-methyl-6-azaspiro[2.5]octane hydrochloride, or (5) 7-azaspiro[3.5]nonane hydrochloride, in a suitable organic solvent such as NMP, acetonitrile, tetrahydrofuran, DMF, methylene chloride, DMSO, and the like, to form compound **C-4.** Examples of compound **C-3** include, but are not limited to, (1) 4-bromo-2-fluoro-1-nitrobenzene, (2) 4-iodo-2-fluoro-1-nitrobenzene, or (3) 6-bromo-2-fluoro-3-nitropyridine. The nitro group on compound **C-4** can then be converted into an amino group by reacting with a reducing agent, which includes but is not limited to palladium on carbon, and a hydrogen source such as hydrogen gas to form compound **C-5.**

### Step C-3: Coupling of Ring Ar¹ compound to Ring Ar² compound:

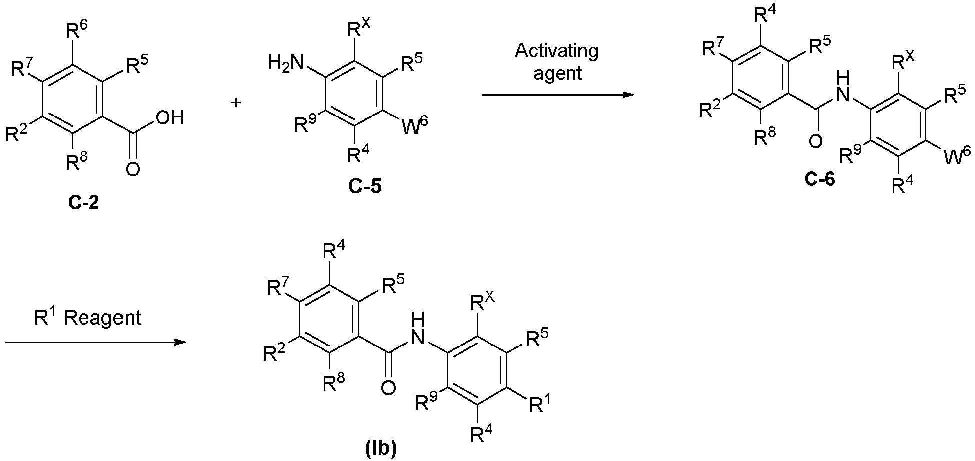

In step C-3, compound **C-2,** which was obtained from Step C-1, can be reacted with compound **C-5,** which was obtained from Step **C-2,** in a suitable organic solvent such as acetonitrile, tetrahydrofuran, DMF, methylene chloride, and the like, in the presence of a coupling agent, such as N, N'-diisopropylcarbodiimide, N-(3-dimethylaminopropyl)-N'-ethylcarbodiimide, benzotriazol-1-yl-oxytripyrrolidinophosphonium hexafluorophosphate, *O*-(benzotriazol-1-yl)-N,N,N',N'-tetramethyluronium hexafluorophosphate, carbonyl diimidazole, or polyphosphonic anhydride to form compound **C-6.** Those skilled in the art will readily understand that other coupling agents can be used. Further manipulation of halogen group W⁶ by transformation reactions such as, SNAr, metal-catalyzed sulfoamidation, sulfination, or sulfonylation, in a suitable organic solvent such as DMSO, acetonitrile, tetrahydrofuran, DMF, and the like, in the presence of a metal catalyst and an R¹ reagent, such as (1) oxetane 3-amine, (2) 2-amino-2-methylpropan-1-ol, (3) (3-aminooxetan-3-yl)methanol, (4) ethyl 2-sulfamoylpropanoate, (5) 2-hydroxypropane-1-sulfonamide, (6) 2-hydroxyethane-1-sulfonamide, (7) 2-mercaptopropan-1-ol, (8) 2-mercapto-2-methylpropan-1-ol, (9) 2-aminoethan-1-ol, or (10) cyclopropanethiol can then be performed to form compound (Ib). Those skilled in the art will readily understand that coupling reaction such as shown in Step C-3 can be performed under various known conditions.

It will be recognized by a person skilled in the art that the above transformations could also be carried out at earlier stages in the synthetic process based on feasibility of the transformations.

### PREPARATION OF SYNTHETIC INTERMEDIATES

### Ring Ar¹ Intermediates:

### Intermediate 1: (R)-3-Fluoro-5-(2-methylmorpholino)aniline.

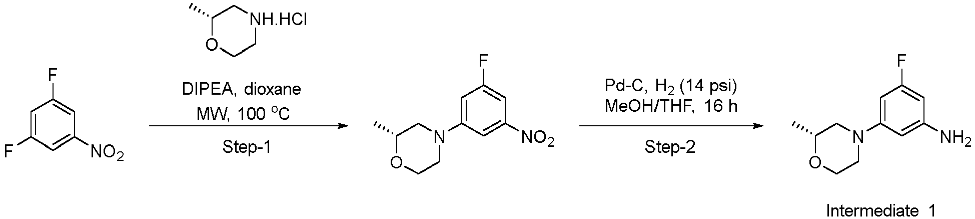

Step 1: A mixture of 1,3-difluoro-5-nitrobenzene (3.0 g, 18.86 mmol, Apollo Scientific), (R)-2-methylmorpholine (2.29 g, 22.63 mmol, Arbor Chemicals) and DIPEA (6.59 mL, 37.7 mmol) in 1,4-dioxane (30 mL) was stirred under microwave at 100 °C for 2 h. The reaction mixture was concentrated and purified by silica gel column chromatography eluting with a gradient of 0 - 40% EtOAc in petroleum ether to provide (*R*)-4-(3-fluoro-5-nitrophenyl)-2-methylmorpholine (1.5 g, 6.24 mmol, 33 % yield) as a yellow solid. ¹H NMR (400 MHz, DMSO-*d*₆): δ ppm 7.54 (d, *J*=2.3 Hz, 1 H), 7.38 (dt, *J*=8.4, 2.1 Hz, 1 H), 7.27 (dt, *J=*12.3, 2.3 Hz, 1 H), 3.92 (ddd, *J=*11.5, 3.7, 1.4 Hz, 1 H), 3.80 (dt, *J=*12.2, 2.2 Hz, 1 H), 3.68 (ddt, *J=*12.2, 3.1, 1.6 Hz, 1 H), 3.53 - 3.67 (m, 2 H), 2.79 (td, *J=*11.9, 3.6 Hz, 1 H), 2.41 - 2.49 (m, 1 H), 1.16 (d, J=6.2 Hz, 3 H). *m*/*z* (ESI): 241.1 (M+H)⁺.

Step 2: To a solution of (R)-4-(3-fluoro-5-nitrophenyl)-2-methylmorpholine (1.8 g, 7.49 mmol) in MeOH (10 mL) and THF (10 mL) was added Pd on Carbon (0.5 g, 4.70 mmol, Hindustan platinum) and was stirred under H₂ pressure (14 psi) for 16 h. The reaction mixture was filtered through a CELITE^{®} bed, washed with MeOH, and the filtrate was concentrated to obtain (*R*)-3-fluoro-5-(2-methylmorpholino)aniline (1.1 g, 5.23 mmol, 70 % yield) as a beige solid. ¹H NMR (400 MHz, DMSO-*d*₆): δ ppm 5.90 (d, *J*=12.5 Hz, 2 H), 5.78 (d, *J=*11.0 Hz, 1 H), 5.19 (d, *J*=7.9 Hz, 2 H), 3.86 (dd, *J=*11.2*,* 3.4 Hz, 1 H), 3.57 (dtd, *J=*14.7*,* 11.5, 10.0, 4.3 Hz, 2 H), 3.43 (d, *J=*11.6 Hz, 1 H), 3.33 (m, 1 H), 2.58 (td, *J=*11.7, 3.3 Hz, 1 H), 2.27 (q, *J*=10.9, 10.3 Hz, 1 H), 1.13 (dd, *J*=9.6, 5.7 Hz, 3 H). *m*/*z* (ESI): 211.2 (M+H)⁺.

### Intermediate 2: (R)-4-Fluoro-3-(2-methylmorpholino)aniline.

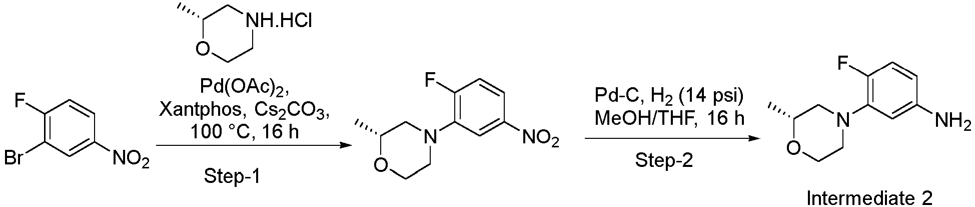

Step 1: A mixture of 2-bromo-1-fluoro-4-nitrobenzene (3.0 g, 13.64 mmol, Apollo Scientific), (*R*)-2 methyl morpholine (1.94 g, 19.23 mmol, Arbor chemicals), Pd(OAc)₂ (0.36 g, 1.63 mmol), Cs₂CO₃ (8.89 g, 27.3 mmol) and xantphos (0.87 g, 1.50 mmol) in dioxane (15 mL) was heated at 100 °C for 16 h. The reaction mixture was filtered through a bed of CELITE^{®} and washed with EtOAc. The filtrate was washed with water, dried over Na₂SO₄, filtered, and concentrated. The residue was purified by silica gel column chromatography eluting with 0-20% EtOAc in hexanes to provide (*R*)-4-(2-fluoro-5-nitrophenyl)-2-methylmorpholine (0.7 g, 2.91 mmol, 21 % yield) as a yellow solid. ¹H NMR (400 MHz, DMSO-*d*₆): δ ppm 7.89 (ddd, *J*=8.9, 3.9, 2.8 Hz, 1 H), 7.79 (dd, *J*=7.6, 2.8 Hz, 1 H), 7.46 (dd, *J=*12.2, 8.9 Hz, 1 H), 3.90 (ddd, *J=*11.5, 3.2, 1.5 Hz, 1 H), 3.68 - 3.77 (m, 2 H), 3.27 - 3.40 (m, 2 H), 2.85 (td, *J=*11.6, 3.2 Hz, 1 H), 2.56 (dd, *J=*11.6, 10.0 Hz, 1 H), 1.15 (d, *J*=6.3 Hz, 3 H). *m*/*z* (ESI): 241.1 (M+H)⁺.

Step 2: To a solution of (*R*)-4-(2-fluoro-5-nitrophenyl)-2-methylmorpholine (0.7 g, 2.91 mmol) in MeOH (10 mL) and THF (10 mL) was added Pd on Carbon (0.35 g, 3.29 mmol, Hindustan Platinum) and the reaction mixture was stirred under H₂ pressure (14 psi) for 16 h. The reaction mixture was filtered through a CELITE^{®} bed, washed with MeOH and concentrated to obtain (*R*)-4-fluoro-3-(2-methylmorpholino)
aniline as beige solid which was directly used for the next step without purification. ¹H NMR (400 MHz, DMSO-*d*₆): δ ppm 6.75 (dd, *J*=12.9, 8.5 Hz, 1 H), 6.22 (dd, *J*=7.7, 2.6 Hz, 1 H), 6.10 (dt, *J*=8.6, 3.1 Hz, 1 H), 4.84 (s, 2 H), 3.79 - 3.87 (m, 1 H), 3.58 - 3.72 (m, 2 H), 3.07 - 3.20 (m, 2 H), 2.61 (td, *J=*11.5, 3.2 Hz, 1 H), 2.32 (t, *J*=10.7 Hz, 1 H), 1.10 (d, *J*=6.3 Hz, 3 H). *m*/*z* (ESI): 211.2 (M+H)⁺.

**Table 1: Intermediates 2-1 and 2-2 were prepared analogous to preparation of Intermediate 2:**

| Int. # | Chemical Structure | Name | LRMS: (ESI + ve ion) *m*/*z* |
|---|---|---|---|
| 2-1 | 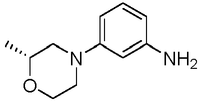 | 3-(4,4-difluoropiperidin-1-yl)aniline | 193.2 |
| 2-2 | 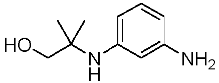 | 2-((3-aminophenyl)amino)-2-methylpropan-1-ol | 181.0 |

### Intermediate 3: 3-(4,4-Difluoropiperidin-1-yl)-5-methylaniline.

Step 1: A mixture of 1-bromo-3-methyl-5-nitrobenzene (5 g, 23.14 mmol), 4,4-difluoropiperidine (4.21 g, 34.7 mmol), sodium *tert*-butoxide (6.67 g, 69.4 mmol), Pd₂(dba)₃ (2.12 g, 2.31 mmol) and xantphos (1.34 g, 2.31 mmol) in toluene (50 mL) was stirred at 100 °C for 1.5 h. The reaction mixture was diluted with water and extracted with EtOAc. The organic extract was washed with brine, dried over Na₂SO₄, filtered, concentrated, and purified by silica gel column chromatography using 10% EtOAc in petroleum ether to provide 4,4-difluoro-1-(3-methyl-5-nitrophenyl)piperidine (3.70 g, 14.44 mmol, 62% yield) as a grey solid. ¹H NMR (400 MHz, DMSO-*d*₆): δ ppm 7.55 (t, *J*=2.3 Hz, 1 H), 7.45 (s, 1 H), 7.32 (d, *J*=2.3 Hz, 1 H), 3.46 (t, *J*=5.8 Hz, 4 H), 2.38 (s, 3 H), 1.96 - 2. 04 (m, 4 H). *m*/*z* (ESI): 257.1 (M+H)⁺.

Step 2: A mixture of 4,4-difluoro-1-(3-methyl-5-nitrophenyl)piperidine (3.7 g, 14.44 mmol), iron powder (8.06 g, 144 mmol) and ammonium chloride (7.72 g, 144 mmol) in EtOH (30 mL) and water (7 mL) was stirred at 75 °C for 16 h. The reaction mixture was filtered through a CELITE^{®®} pad, washed with methanol, and the filtrate was concentrated. The residue was diluted with water and extracted with EtOAc. The organic extract was washed with brine, dried over Na₂SO₄, filtered, concentrated, and purified by silica gel column chromatography eluting with 30-40% EtOAc in petroleum ether to provide 3-(4,4-difluoropiperidin-1-yl)-5-methylaniline (2.6 g, 11.49 mmol, 80% yield) as a brown solid. ¹H NMR (400 MHz, DMSO-*d*₆): δ ppm 6.00 (s, 2 H), 5.89 (s, 1 H), 4.81 (s, 2 H), 3.16 - 3.22 (m, 4 H), 2.09 (s, 3 H), 1.94 - 2.04 (m, 4 H). *m*/*z* (ESI): 227.1 (M+H)⁺.

**Table 2: Intermediate 3-1 was prepared analogous to preparation of Intermediate 3:**

| Int. # | Chemical Structure | Name | LRMS: (ESI + ve ion) *m*/*z* |
|---|---|---|---|
| 3-1 | 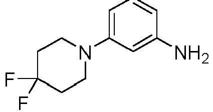 | 3-(4,4-difluoropiperidin-1-yl)aniline | 213.1 |

### Intermediate 4: 3-(4,4-Difluoropiperidin-1-yl)-2-fluoroaniline.

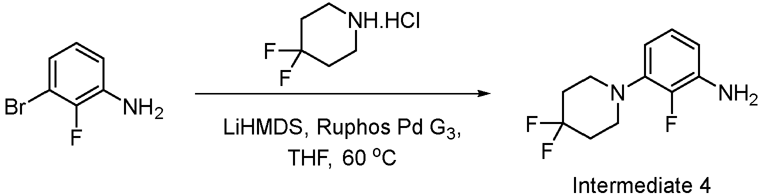

To a solution of 4,4-difluoropiperidine hydrochloride (0.91 g, 5.79 mmol) and lithium bis(trimethylsilyl)amide (1.0 M in THF, 11.84 mL, 11.84 mmol) in THF (50 mL) were added a solution of 3-bromo-2-fluoroaniline (0.5 g, 2.63 mmol) in THF (20 mL) and Ruphos Pd G-3 (0.13 g, 0.16 mmol, Strem chemicals). The reaction mixture was stirred at 60°C for 6 h before it was diluted with water and extracted with EtOAc. The organic extract was washed with brine, dried over Na₂SO₄, filtered, and concentrated. The concentrate was purified by silica gel column chromatography eluting with 5-10% EtOAc in petroleum ether to provide 3-(4,4-difluoropiperidin-1-yl)-2-fluoroaniline (0.45 g, 1.96 mmol, 74% yield) as a black oil. ¹H NMR (400 MHz, DMSO-*d*₆): δ ppm 6.75 (td, *J*=8.0, 1.4 Hz, 1 H), 6.43 (td, *J*=8.1, 1.5 Hz, 1 H), 6.25 (td, *J*=7.9, 1.6 Hz, 1 H), 5.01 (s, 2 H), 3.03 - 3.10 (m, 4 H), 2.02 - 2.17 (m, 4 H). *m*/*z* (ESI): 231.1 (M+H)⁺.

### Intermediate 5: 2-((3-Amino-2-fluorophenyl)amino)-2-methylpropan-1-ol.

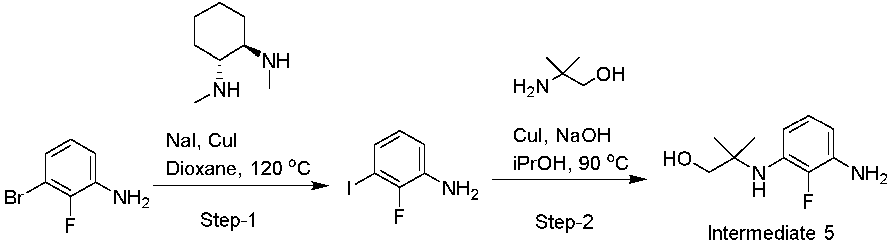

Step 1: A pressure relief vial was charged with copper(I) iodide (0.025 g, 0.132 mmol) and sodium iodide (0.789 g, 5.26 mmol). The vial was evacuated / backfilled with nitrogen 3x. Dioxane (5 mL) was added followed by 3-bromo-2-fluoroaniline (0.50 mL, 2.63 mmol, Oakwood Inc., Estill, SC, USA) and *trans-N,N'-*dimethylcyclohexane-1,2-diamine (0.041 mL, 0.263 mmol). The cap was replaced and the reaction was stirred in a pre-heated 120 °C oil bath for 24 h. The reaction was partitioned between 9:1 satd. NH₄Cl:satd. NH₄OH and EtOAc. The organic phase was separated, washed with brine, dried over magnesium sulfate and concentrated in vacuo to afford 2-fluoro-3-iodoaniline (0.63 g, 2.66 mmol, 100% yield) as a dark brown oil which was used without further purification. ¹H NMR (400 MHz, CHLOROFORM-*d*) δ ppm 7.08 (ddd, *J*=7.52, 5.55, 1.87 Hz, 1 H) 6.63 - 6.76 (m, 2 H) 3.78 (br s, 2 H). *m*/*z* (ESI, +ve ion): 238.1 (M+H)⁺.

Step 2: A pressure relief vial was charged with copper(I) iodide (0.024 g, 0.127 mmol) and sodium hydroxide (0.101 g, 2.53 mmol). The vial was sealed and evacuated / backfilled with nitrogen 3x. Isopropanol (6 mL) was added followed by 2-fluoro-3-iodoaniline (0.30 g, 1.27 mmol) and 2-amino-2-methyl-1-propanol (0.13 mL, 1.52 mmol, Combi-Blocks Inc.). The cap was replaced, and the reaction was stirred in a pre-heated 90 °C oil bath over for 16 h. The reaction was partitioned between satd NH₄Cl:NH₄OH (9:1) and EtOAc. The organic phase was separated, washed with brine, dried over magnesium sulfate, and concentrated in vacuo. The crude material was purified by silica gel chromatography (eluent: 50-100% EtOAc:heptane) to afford 2-((3-amino-2-fluorophenyl)amino)-2-methylpropan-1-ol (0.123 g, 0.620 mmol, 49% yield) as a brown oil. ¹H NMR (400 MHz, DMSO-*d*₆) δ ppm 6.61 (td, *J*=8.03, 1.14 Hz, 1 H) 6.20 (td, *J*=7.88, 1.24 Hz, 1 H) 6.10 (td, *J*=8.09, 1.24 Hz, 1 H) 5.03 (t, *J*=5.49 Hz, 1 H) 4.80 (s, 2 H) 4.31 (br d, *J*=3.94 Hz, 1 H) 3.33 (s, 1 H) 1.19 (s, 6 H). *m*/*z* (ESI, +ve ion): 199.2 (M+H)⁺.

### Intermediate 6: (3-Aminophenyl)(tert-butyl)((tert-butyldimethylsilyl)imino)-sulfanone.

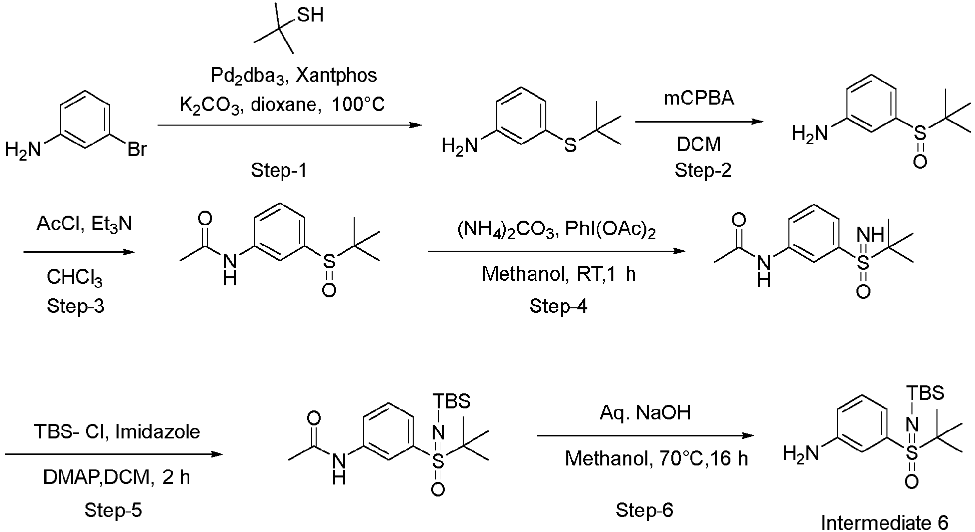

Step 1: To a solution of 3-bromoaniline (5 g, 29.1 mmol) in dioxane (30 mL) were added 2-methylpropane-2-thiol (2.88 g, 32.0 mmol), K₂CO₃ (4.02 g, 29.1 mmol), Pd₂(dba)₃ (26.6 g, 29.1 mmol) followed by Xantphos (16.82 g, 29.1 mmol) and the reaction mixture was heated at 100 °C for 14 h. The reaction mixture was filtered through a CELITE^{®} pad and washed with EtOAc. The filtrate was taken and washed with brine, dried over Na₂SO₄, and concentrated under reduced pressure. The crude material was purified by silica gel column chromatography using 10% EtOAc in petroleum ether to give 3-(*tert-*butylthio)aniline (3.5 g, 19.31 mmol, 66% yield). ¹H NMR (400 MHz, Chloroform-*d*): δ ppm 7.17 (t, *J*=7.8 Hz, 1 H), 6.97 - 7.06 (m, 2 H), 6.81 (ddd, *J*=7.9, 2.4, 1.0 Hz, 1 H), 4.34 (s, 2 H), 1.32 (d, *J=*3.1 Hz, 9 H).

Step 2: To a solution of 3-(*tert*-butylthio)aniline (3.5 g, 19.31 mmol) in DCM (100 mL) was added 3-chlorobenzoperoxoic acid (3.33 g, 19.31 mmol) at 0 °C and the reaction mixture was stirred at rt for 1 h. The reaction mixture was quenched with 100 mL of satd. NaHCO₃ solution and extracted with DCM (2x). The combined organic extracts were dried over Na₂SO₄ and concentrated to get the crude product which was purified by silica gel column chromatography using 60% EtOAc in petroleum ether to give 3-(*tert-*butylsulfinyl) aniline (2.8 g, 14.19 mmol, 73% yield). *m*/*z* (ESI): 198.2 (M+H)⁺.

Step 3: To a solution of 3-(*tert*-butylsulfinyl)aniline (2.8 g, 14.19 mmol) in chloroform (20 mL) were added Et3N (2.87 g, 28.4 mmol) followed by acetyl chloride (1.67 g, 21.29 mmol) at 0 °C and was stirred for 3 h at rt. The reaction mixture was quenched with ice cold water and extracted with chloroform (2x). The combined organic extracts were dried over Na₂SO₄ and concentrated to get the crude material. Purification by silica gel column chromatography using 50% EtOAc in petroleum ether gave *N-*(3-(*tert-*butylsulfinyl)phenyl)acetamide (3.0 g, 12.53 mmol, 88% yield). ¹H NMR (300 MHz, DMSO-*d₆*): δ ppm 10.18 (s, 1 H), 7.84 (t, *J=*1.9 Hz, 1 H), 7.63 - 7.73 (m, 1 H), 7.46 (d, *J*=7.9 Hz, 1 H), 7.19 (dt, *J*=7.8, 1.2 Hz, 1 H), 2.04 (s, 3 H), 1.05 (s, 9 H). *m*/*z* (ESI): 240.1 (M+H)⁺.

Step 4: To a solution of *N*-(3-(*tert*-butylsulfinyl)phenyl)acetamide (3.0 g, 12.53 mmol) in methanol (60 mL) were added phenyl-λ³-iodanediyl diacetate (10.1 g, 31.3 mmol) followed by ammonium carbonate (4.19 g, 62.7 mmol) portion wise and the reaction mixture was stirred for 1 h at rt. The reaction mixture was quenched with water and extracted with EtOAc (2x). The combined organic extracts were dried over Na₂SO₄ and concentrated. The crude product was purified by silica gel column chromatography using 90% EtOAc in petroleum ether to give *N*-(3-(2-methylpropan-2-ylsulfonimidoyl)phenyl)acetamide (2.4 g, 75% yield) as off-white solid. ¹H NMR (300 MHz, DMSO-*d₆*): δ ppm 10.26 (s, 1 H), 8.09 (d, *J=*2.1 Hz, 1 H), 7.89 (dt, *J*=7.1, 2.2 Hz, 1 H), 7.48 - 7.53 (m, 2 H), 4.03 (s, 1 H), 2.06 (s, 3 H), 1.21 (s, 9 H). *m*/*z* (ESI): 255.1 (M+H)⁺.

Step 5: To a solution of *N*-(3-(2-methylpropan-2-ylsulfonimidoyl)phenyl)acetamide (2.4 g, 9.44 mmol) in DCM (48 mL) were added imidazole (1.28 g, 18.87 mmol), DMAP (0.57 g, 4.72 mmol) followed by TBS-Cl (1.7 g, 11.32 mmol) at 0 °C and the reaction mixture was stirred for 2 h at rt. The reaction mass was quenched with water followed by 10% aqueous sodium bicarbonate solution. The aqueous layer was extracted with EtOAc (2x) and combined organic extracts were washed with brine, dried over Na₂SO₄, and concentrated. The crude material was purified by silica gel column chromatography eluting with a gradient of 50-60% EtOAc in petroleum ether to provide *N*-(3-(*N*-(tert-butyldimethylsilyl)-2-methylpropan-2-ylsulfonimidoyl)phenyl)acetamide (3.2 g, 92% yield) as a light yellow liquid. ¹H NMR (300 MHz, DMSO-*d₆*): δ ppm 10.24 (s, 1 H), 8.13 (t, *J*=1.9 Hz, 1 H), 7.82 (d, *J=*2.0 Hz, 1 H), 7.34 - 7.58 (m, 2 H), 2.06 (s, 3 H), 1.18 (s, 9 H), 0.87 (s, 9 H), -0.06 (s, 3 H), -0.07 (s, 3 H). *m*/*z* (ESI): 369.2 (M+H)⁺.

Step 6: To a solution of *N*-(3-(N-(*tert*-butyldimethylsilyl)-2-methylpropan-2-ylsulfonimidoyl)phenyl)acetamide (3.2 g, 8.68 mmol) in methanol (32 mL) was added 2.5 M sodium hydroxide solution (64 mL) and the reaction mixture was stirred for 16 h at 70 °C. The reaction mixture was diluted with water and was extracted with EtOAc. The combined organic extracts were dried over Na₂SO₄ and concentrated. The crude material was purified by silica gel column chromatography using 20% EtOAc in petroleum ether to provide (3-aminophenyl)(*tert*-butyl)((*tert*-butyldimethylsilyl) imino)-λ⁶-sulfanone (2.1 g, 6.43 mmol, 74% yield) as an off-white solid. ¹H NMR (400 MHz, DMSO-*d₆*): δ ppm 7.18 (t, *J*=7.9 Hz, 1 H), 6.99 (t, *J*=2.1 Hz, 1 H), 6.87 (ddd, *J*=7.7, 1.8, 1.0 Hz, 1 H), 6.75 (ddd, *J*=8.0, 2.4, 1.0 Hz, 1 H), 5.51 (s, 2 H), 1.17 (d, *J*=2.3 Hz, 9 H), 0.87 (d, *J=*2.5 Hz, 9 H), -0.06 (s, 3 H), -0.07 (s, 3 H). *m*/*z* (ESI): 327.2 (M+H).

**Table 3: Intermediate 6-1 was prepared analogous to preparation of Intermediate 6:**

| Int. # | Chemical Structure | Name | LRMS: (ESI + ve ion) *m*/*z* |
|---|---|---|---|
| 6-1 | 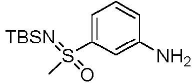 | (3-aminophenyl)((tertbutyldimethylsilyl)imino) (methyl)-λ⁶-sulfanone | 285.2 |

### Intermediate 7: 3-(N-(tert-Butyldimethylsilyl)azetidine-1-sulfonimidoyl)aniline.

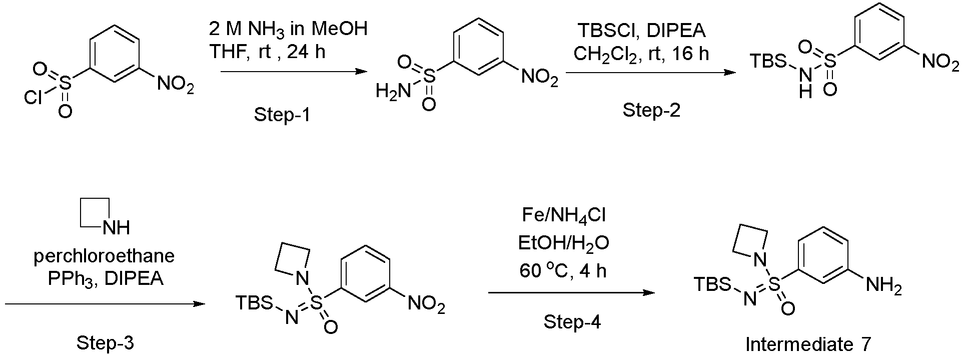

Step 1: A solution of 3-nitrobenzenesulfonyl chloride (2.0 g, 9.02 mmol, Combi-blocks) in THF (10 mL) was treated with ammonia (20 mL, 40.0 mmol, 2M in methanol) and stirred at ambient temperature for 24 h. The solvent was evaporated under reduced pressure and residue was diluted with water to provide solid material which was filtered and dried to afford 3-nitrobenzenesulfonamide (1.3 g, 6.43 mmol, 71% yield) as an off-white solid. ¹H NMR (400 MHz, DMSO-*d*₆): δ ppm 8.59 (t, *J*=2.0 Hz, 1 H), 8.45 (dd, *J*=8.3, 2.3 Hz, 1 H), 8.24 (dd, *J*=7.9, 1.6 Hz, 1 H), 7.89 (t, *J*=8.0 Hz, 1 H), 7.71 (s, 2 H). *m*/*z* (ESI): 201.0 (M-H).

Step 2: To a solution of 3-nitrobenzenesulfonamide (1.3 g, 6.43 mmol) in CH₂Cl₂ (26 mL) were added Et₃N (2.69 mL, 19.29 mmol) followed by TBS-Cl (1.45 g, 9.64 mmol) at 0 °C. The reaction mixture was stirred at rt for 16 h before it was quenched with cold water and extracted with CH₂Cl₂. The CH₂Cl₂ layer was washed with brine, dried over anhydrous Na₂SO₄, concentrated under reduced pressure to givethe crude product which was purified by silica gel column chromatography using a gradient of 0-10% EtOAc in petroleum ether to provide N-(*tert*-butyldimethylsilyl)-3-nitrobenzenesulfonamide (1.0 g, 3.16 mmol, 49% yield) as a pale-yellow solid. ¹H NMR (400 MHz, DMSO-*d*₆): δ ppm 8.57 (t, *J*=2.0 Hz, 1 H), 8.44 (ddd, *J*=8.2, 2.4, 1.1 Hz, 1 H), 8.22 (dt, *J*=7.9, 1.4 Hz, 1 H), 7.96 (s, 1 H), 7.89 (td, *J*=8.0, 1.6 Hz, 1 H), 0.87 (d, *J=*1.7 Hz, 9 H), 0.11 (s, 3 H), 0.12 (s, 3 H). *m*/*z* (ESI): 315.1 (M-H)⁻.

Step 3: To a solution of Ph₃P (0.91 g, 3.48 mmol) in chloroform (10 mL) was added perchloroethane (0.82 g, 3.48 mmol, Aldrich) and the reaction mixture was heated at 70 °C for 6 h. The solution was cooled to 0 °C and was treated with Et₃N (0.66 mL, 4.74 mmol) followed by N-(*tert*-butyldimethylsilyl)-3-nitrobenzenesulfonamide (1.0 g, 3.16 mmol) in chloroform (5 mL) and was stirred for 30 min. Finally, azetidine (0.361 g, 6.32 mmol, Chempure) in chloroform (1.5 mL) was dropwise added at 0 °C and the reaction mixture was stirred for 16 h at rt. The reaction mixture was quenched with cold water, extracted with CH₂Cl₂, washed with brine, dried over anhydrous Na₂SO₄ and concentrated under reduced pressure. The crude product was purified by silica gel column chromatography using a gradient of 0-20 % EtOAc in petroleum ether to afford 1-(*N*-(*tert*-butyldimethylsilyl)-3-nitrophenylsulfonimidoyl)azetidine (0.85 g, 2.39 mmol, 76% yield) as clear syrup. ¹H NMR (400 MHz, DMSO-*d*₆): δ ppm 8.51 (ddd, *J*=8.2, 2.4, 1.0 Hz, 1 H), 8.46 (t, *J*=2.0 Hz, 1 H), 8.19 (dt, *J*=7.8, 1.4 Hz, 1 H), 7.95 (t, *J*=8.0 Hz, 1 H), 3.57 (dq, *J*=20.9, 7.7 Hz, 4 H), 1.89 (pent, *J*=7.6 Hz, 2 H), 0.90 (s, 9 H), 0.11 (s, 3 H), 0.08 (s, 3 H).

Step 4: To a mixture of 1-(*N*-(*tert*-butyldimethylsilyl)-3-nitrophenylsulfonimidoyl)azetidine (0.85 g, 2.39 mmol) in ethanol (7 mL) and water (3 mL) were added iron powder (1.33 g, 23.91 mmol) and ammonium chloride (1.28 g, 23.91 mmol). The reaction mixture was heated at 60 °C for 4 h before it was filtered through a CELITE^{®®} bed and washed with EtOAc. The filtrate was washed with water, 10% NaHCO₃ solution, brine, dried over anhydrous Na₂SO₄, and concentrated under reduced pressure. The crude product was purified by silica gel column chromatography using a gradient of 0-35% EtOAc in petroleum ether to afford 3-(*N*-(*tert*-butyldimethylsilyl)azetidine-1-sulfonimidoyl)aniline (0.54 g, 1.66 mmol, 69% yield) as a clear syrup. *m*/*z* (ESI): 326.2 (M+H)⁺.

### PREPARATION OF RING AR² INTERMEDIATES:

### Intermediate 8: 4-Iodo-2-(6-azaspiro[2.5]octan-6-yl)benzoic acid.

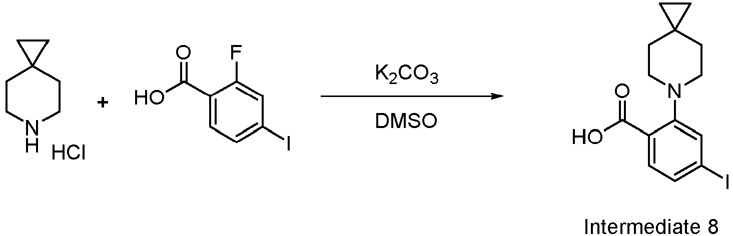

To a solution of 2-fluoro-4-iodobenzoic acid (300 g, 1.13 mol, Combi-Blocks) in DMSO (2.10 L) was added 6-azaspiro[2.5]octane hydrochloride (216 g, 1.47 mol, Wuxi AppTec) at 20°C. Then K₂CO₃ (468 g, 3.38 mol) was added and the reaction mixture was stirred at 140 °C for 48 h under N₂. The reaction solution was slowly poured into ice water (4.20 L), then extracted with hexanes (2 L x 3). The water phase was separated and adjusted to pH = 6 with HCl (2M). Solid was precipitate out and collected. The solid was washed with water (700 mL x 3) and filtered. The moist solid was spread out on a large watch glass and dried in the air at 25°C for 72 h. 4-Iodo-2-(6-azaspiro[2.5]octan-6-yl)benzoic acid (280 g, 777 mmol, 69% yield) was obtained as a light yellow solid. 400 MHz DMSO-d₆ δ ppm 8.07 (s, 1H), 7.76 - 7.66 (m, 2H), 3.10 (t, *J =* 5.2 Hz, 4H), 1.55 (br s, 4H), 0.41 (s, 4H).

**Table 4: Intermediates 8-1 to 8-4 were prepared analogous to preparation of Intermediate 8:**

| Int. # | Chemical Structure | Name | LRMS: (ESI + ve ion) *m*/*z* |
|---|---|---|---|
| 8-1 | 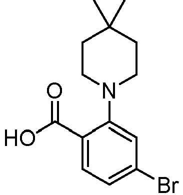 | 4-bromo-2-(6-azaspiro[2.5]octan-6-yl)benzoic acid | 310.2/312.2 |
| 8-2 | 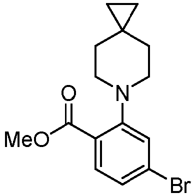 | methyl 4-bromo-2-(6-azaspiro[2.5]octan-6-yl)benzoate | 324.0/326.0 |
| 8-3 | 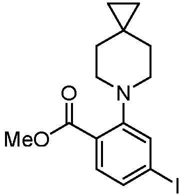 | methyl 4-iodo-2-(6-azaspiro[2.5]octan-6-yl)benzoate | 372.1 |
| 8-4 | 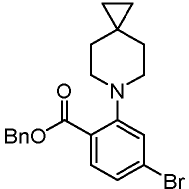 | benzyl 4-bromo-2-(6-azaspiro[2.5]octan-6-yl)benzoate | 400.1/402.1 |

### Intermediate 9: 4-(Methylsulfonyl)-2-(6-azaspiro[2.5]octan-6-yl)benzoic acid.

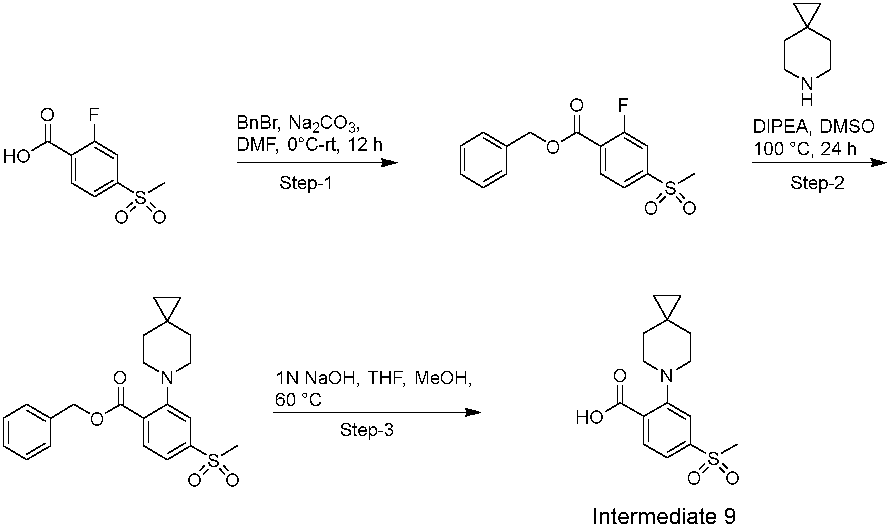

Step 1: To a solution of 2-fluoro-4-(methylsulfonyl)benzoic acid (90.0 g, 412.1 mmol) in *N,N-*dimethylformamide (1.0 L) were added benzyl bromide (78.1 g, 454.0 mmol) and sodium carbonate (52.5 g, 495 mmol) at 0 °C. The reaction mixture was stirred for 12 h at RT. The reaction mixture was quenched with water (1 L) and extracted with MTBE (3 x 1 L). The combined organic extracts were washed with brine (1 L), dried over Na₂SO₄, filtered, and concentrated under reduced pressure. The crude residue was purified by column chromatography over silica gel using 0-30% EtOAc in hexanes to give benzyl 2-fluoro-4-(methylsulfonyl)benzoate (100 g, 79% yield) as a white solid. ¹H NMR (300 MHz, DMSO-*d*₆) *δ* ppm 8.16 (dd, *J=* 8.2, 6.9 Hz, 1H), 7.98 - 7.86 (m, 2H), 7.48 - 7.31 (m, 5H), 5.40 (s, 2H), 3.33 (s, 3H).

Step 2: To a solution of benzyl 2-fluoro-4-(methylsulfonyl)benzoate (55g, 178 mmol) in DMSO (550 mL) was added DIPEA (57.6 g, 446 mmol) followed by 6-azaspiro[2.5]octane (29.8 g, 268 mmol) and the reaction mixture was stirred at 100 °C for 24 h. The reaction mixture was quenched with water (1 L) and extracted with MTBE (3 x 1 L). The combined organic layer was washed with brine solution (1 L), dried over Na₂SO₄, filtered, and concentrated under reduced pressure. The crude product was purified by column chromatography over silica gel (230-400 mesh) using 0-10% EtOAc in hexanes to give benzyl 4-(methylsulfonyl)-2-(6-azaspiro[2.5]octan-6-yl)benzoate (55 g, 77% yield) as a white solid. ¹H NMR (400 MHz, DMSO-*d*₆) *δ* ppm 7.76 (d, *J =* 8.0 Hz, 1H), 7.52 - 7.45 (m, 4H), 7.43 - 7.35 (m, 3H), 5.35 (s, 2H), 3.25 (s, 3H), 3.05 (t, *J =* 5.3 Hz, 4H), 1.36 (t, *J =* 5.3 Hz, 4H), 0.30 (s, 4H). *m*/*z* (ESI): 400.1 (M+H)⁺.

Step 3: To a solution of benzyl 4-(methylsulfonyl)-2-(6-azaspiro[2.5]octan-6-yl)benzoate (65 g, 163 mmol) in THF (108 mL) and methanol (36 mL) was added 1N aqueous sodium hydroxide solution (407 mL, 407 mmol) and the reaction mixture was stirred for 12 h at 60 °C. The reaction mixture was concentrated under reduced pressure to remove THF and methanol. The remaining aqueous solution was acidified to pH ~2 with 1.5 N HCl solution. The precipitated solid was filtered, washed with water (200 mL) followed by hexanes (200 mL), dried under vacuum for 12 h to give 4-(methylsulfonyl)-2-(6-azaspiro[2.5]octan-6-yl)benzoic acid (42 g, 83% yield) as an off-white solid. ¹H NMR (400 MHz, DMSO-*d*₆) *δ* ppm 16.13 (s, 1H), 8.04 (d, *J =* 8.0 Hz, 1H), 7.99 (s, 1H), 7.75 (d, *J =* 8.0 Hz, 1H), 3.29 (s, 3H), 3.17 (b s, 4H), 1.55 (b s, 4H), 0.41 (s, 4H). *m*/*z* (ESI): 308.1 (M- H)⁺.

### Intermediate 10: 4-((1-Methylcyclopropane)-1-sulfonamido)-2-(6-azaspiro[2.5]octan-6-yl)benzoic acid.

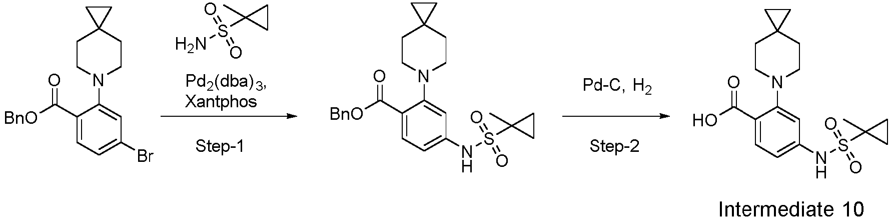

Step 1: To a 250-mL sealed tube were added benzyl 4-bromo-2-(6-azaspiro[2.5]octan-6-yl)benzoate (9 g, 22.48 mmol, Intermediate 8-4), 1-methylcyclopropane-1-sulfonamide (3.95 g, 29.2 mmol, Combi-Blocks) and K₂CO₃ (6.21 g, 45.0 mmol) in dioxane (90 mL) and the reaction mixture was degassed and purged with nitrogen for 5 min. To this reaction mixture was added Xantphos (1.30 g, 2.25 mmol) followed by Pd₂(dba)₃ (1.03 g, 1.12 mmol) and tube was sealed and stirred at 110 °C for 18 h. The reaction mixture was quenched with water (250 mL) and extracted with EtOAc (2 x 150 mL). The combined organic extracts were washed with water (100 mL), dried over Na₂SO₄, filtered and concentrated under reduced pressure. The crude material was purified by column chromatography over silica gel eluting with a gradient of 0-15% EtOAc in hexanes to give benzyl 4-((1-methylcyclopropane)-1-sulfonamido)-2-(6-azaspiro[2.5]octan-6-yl)benzoate (6.1 g, 59% yield) as an orange oil. ¹H NMR (400 MHz, DMSO-*d*₆) *δ* ppm 10.06 (s, 1H), 7.62 (d, *J=* 8.5 Hz, 1H), 7.48 - 7.31 (m, 5H), 6.98 (s, 1H), 6.81 (d, *J=* 8.5 Hz, 1H), 5.27 (s, 2H), 2.92 (t, *J* = 4.96 Hz, 4H), 1.40 - 1.30 (m, 7H), 1.16 (dd, *J* = 6.4, 4.7 Hz, 2H), 0.81 (dd, *J* = 6.4, 4.7 Hz, 2H), 0.28 (s, 4H). *m*/*z* (ESI): 455.2 (M+H)⁺.

Step 2: To a solution of benzyl 4-((1-methylcyclopropane)-1-sulfonamido)-2-(6-azaspiro[2.5]octan-6-yl)benzoate (2.1 g, 4.62 mmol) in MeOH (20 mL) and EtOAc (10 mL) was added 10% Pd-C (1.05 g, 50% wt/wt) under nitrogen atmosphere. The reaction mixture was degassed and stirring under hydrogen pressure (1 atm, balloon pressure) for 4 h. The reaction mixture was filtered through a CELITE^{®} bed and washed with MeOH (20 mL). The filtrate was concentrated under reduced pressure. The residue was triturated with Et₂O (50 mL) to give 4-((1-methylcyclopropane)-1-sulfonamido)-2-(6-azaspiro[2.5]octan-6-yl)benzoic acid (1.2 g, 71% yield) as an off-white solid. ¹H NMR (400 MHz, DMSO-*d*₆) *δ* ppm 13.20 (s, 1H), 10.33 (s, 1H), 7.95 (d, *J =* 8.6 Hz, 1H), 7.41 (s, 1H), 7.20 (d, *J =* 8.6 Hz, 1H), 2.99 (s, 4H), 1.56 (s, 4H), 1.39 (s, 3H), 1.18 (t, *J=* 4.8 Hz, 2H), 0.83 (t, *J=* 4.7 Hz, 2H), 0.42 (s, 4H). *m*/*z* (ESI): 363.2 (M-H).

### Intermediate 11: 4-((Methylsulfonyl)methyl)-2-(6-azaspiro[2.5]octan-6-yl)benzoic acid.

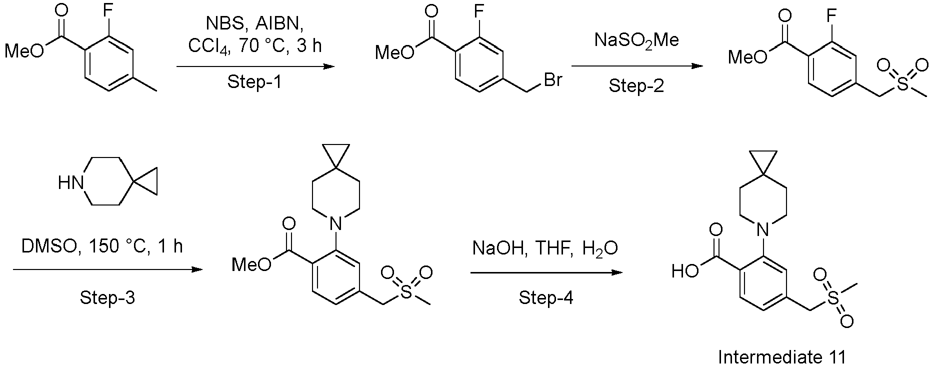

Step 1: To a solution of methyl 2-fluoro-4-methylbenzoate (10.0 g, 59.5 mmol) in carbon tetrachloride (200 mL) were added NBS (11.6 g, 65.4 mmol) and AIBN (0.98 g, 5.95 mmol) at rt. The reaction mixture was stirred at 70 °C for 3 h before quenched with water (250 mL) and extracted with DCM (2 x 200 mL). The combined organic extracts were washed with brine (150 mL), dried over Na₂SO₄, filtered, and concentrated under reduced pressure to give methyl 2-fluoro-4-methylbenzoate (14.0 g, crude) as a pale yellow oil.¹H NMR (400 MHz, Chloroform-d): *δ* 8.04 - 7.88 (m, 1H), 7.27 - 7.13 (m, 2H), 4.46 (s, 2H), 3.96 (s, 3H).

Step 2: A mixture of methyl 2-fluoro-4-methylbenzoate (14.0 g, 56.7 mmol) and sodium methanesulfinate (12.15 g, 119 mmol) in DMF (42 mL) was irradiated in a microwave oven (Biotage initiator+) at 120 °C for 30 min. The reaction mixture was quenched with water (150 mL) and extracted with EtOAc (2 x 200 mL). The combined organic extracts were washed with satd brine solution (150 mL), dried over Na₂SO₄, filtered, and concentrated under reduced pressure to give methyl 2-fluoro-4-((methylsulfonyl)methyl)benzoate (6 g, crude) as an off-white solid.

Step 3: A mixture of methyl 2-fluoro-4-((methylsulfonyl)methyl)benzoate (6.0 g, 24 mmol) and 6-azaspiro [2.5] octane (2.71 g, 24.4 mmol) in DMSO (30 mL) was irradiated in a microwave oven at 150 °C for 1 h. The reaction mixture was quenched with water (50 mL) and extracted with EtOAc (2 x 50 mL). The combined organic extracts were washed with brine (50 mL), dried over Na₂SO₄, filtered, and concentrated under reduced pressure to give methyl 4-((methylsulfonyl)methyl)-2-(6-azaspiro[2.5]octan-6-yl)benzoate (4.0 g, crude) as an off-white solid. The material as such was taken onto the next step without any further purification. ¹H NMR (400 MHz, Chloroform-*d) δ* 7.72 (d, *J =* 7.8 Hz, 1H), 7.11 (s, 1H), 6.96 (dd, *J* = 7.8, 1.6 Hz, 1H), 4.24 (s, 2H), 3.93 (s, 3H), 3.12 (t, *J =* 5.4 Hz, 4H), 2.78 (s, 3H), 1.55 (t, *J =* 5.5 Hz, 4H), 0.37 (s, 4H). *m*/*z* (ESI): 338.1 (M-H)⁺.

Step 4: To a solution of methyl 4-((methylsulfonyl)methyl)-2-(6-azaspiro[2.5]octan-6-yl)benzoate (2.0 g, 3.0 mmol) in THF (15 mL) was added sodium hydroxide (0.474 g, 11.8 mmol) in water (7 mL) and stirred at RT for 12 h. The reaction mixture was acidified with 1.5 N HCl solution to pH ~3 and extracted with EtOAc (5 x 20 mL). The combined organic extracts were washed with brine solution (20 mL), dried over Na₂SO₄, filtered, and concentrated under reduced pressure to give 4-((methylsulfonyl)methyl)-2-(6-azaspiro[2.5]octan-6-yl)benzoic acid (1.0 g, 10% yield over 3 steps) as a white solid. *m*/*z* (ESI): 324.1 (M-H)⁺.

### Intermediate 12: 4-(N-(3-Methyloxetan-3-yl)sulfamoyl)-2-(6-azaspiro[2.5]octan-6-yl)benzoic acid.

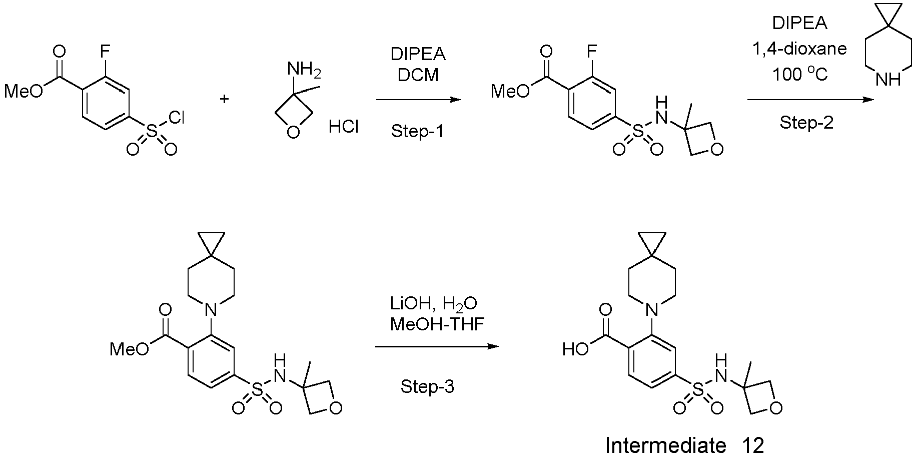

Step 1: To a solution of 3-methyl-3-oxetanamine hydrochloride (5.50 g, 44.5 mmol) and DIPEA (23.26 mL, 134 mmol) in DCM (200 mL) at 0 °C, methyl 4-(chlorosulfonyl)-2-fluorobenzoate (12.37 g, 49.0 mmol) was added and the mixture stirred from 0 °C to RT for 1 h. The mixture was diluted with 1.0 N HCl (200 mL) and extracted with DCM (150 mL x 2). The combined organic extracts were washed with brine, dried with anhydrous Na₂SO₄, filtered and concentrated under reduced pressure to afford crude product. The crude product was purified with Biotage SNAP 100 g column eluting with 0-30% EtOAc-EtOH (3:1) in heptane to afford methyl 2-fluoro-4-(*N*-(3-methyloxetan-3-yl)sulfamoyl)benzoate (13.59 g, 44.8 mmol, 100% yield) as a white solid. ¹H NMR (500 MHz, DMSO-d₆) δ ppm 8.67 (s, 1H), 8.11 (t, *J =* 7.37 Hz, 1H), 7.76-7.82 (m, 1H), 7.69-7.76 (m, 1H), 4.56 (d, *J* = 6.23 Hz, 2H), 4.18 (d, *J =* 6.75 Hz, 2H), 3.90 (s, 3H), 1.42 (s, 3H).

Step 2: A mixture of *N*, *N*-diisopropylethylamine (16.23 mL, 93 mmol), 6-azaspiro[2.5]octane (6.22 g, 55.9 mmol), and methyl 2-fluoro-4-(*N*-(3-methyloxetan-3-yl)sulfamoyl)benzoate (14.13 g, 46.6 mmol) in anhydrous dioxane was stirred at 100 °C for 20 h. The mixture was cooled down to RT, quenched with water, and extracted with EtOAc (2x). The combined organic extracts were washed with brine, dried and evaporated to dryness under reduced pressure. The crude product was purified using the Biotage SNAP 340 g column eluting with 0-40% 3:1 EtOAc-EtOH in heptane to afford methyl 4-(*N*-(3-methyloxetan-3-yl)sulfamoyl)-2-(6-azaspiro[2.5]octan-6-yl)benzoate (14.15 g, 35.9 mmol, 77% yield) as an off-white solid. ¹H NMR (500 MHz, DMSO-*d*₆) δ ppm 8.42 (s, 1H), 7.72 (d, *J =* 8.04 Hz, 1H), 7.47 (d, *J =* 1.56 Hz, 1H), 7.36 (dd, *J=* 1.82, 8.04 Hz, 1H), 4.55 (d, *J =* 5.97 Hz, 2H), 4.14 (d, *J =* 6.49 Hz, 2H), 3.85 (s, 3H), 3.02-3.09 (m, 4H), 1.44-1.50 (m, 4H), 1.42 (s, 3H), 0.35 (s, 4H).

Step 3: A mixture of methyl 4-(*N*-(3-methyloxetan-3-yl)sulfamoyl)-2-(6-azaspiro[2.5]octan-6-yl)benzoate (14.15 g, 35.9 mmol) and lithium hydroxide monohydrate (22.58 g, 538 mmol) in THF-water-MeOH (1:1:1, 300 mL) was stirred at RT overnight. The mixture was concentrated under reduced pressure to partially remove the organic solvent. The solution was acidified with 2N HCl to reach pH < 3. The precipitated solid was filtered and dried in air to give 4-(*N*-(3-methyloxetan-3-yl)sulfamoyl)-2-(6-azaspiro[2.5]octan-6-yl)benzoic acid (9.94 g, 26.1 mmol, 73% yield) as a white solid. ¹H NMR (500 MHz, DMSO-*d*₆) δ ppm 8.51 (s, 1H), 8.04 (d, *J*=8.04 Hz, 1H), 7.89 (d, *J=*1.30 Hz, 1H), 7.66 (dd, *J*=1.69, 8.17 Hz, 1H), 4.55 (d, *J*=6.23 Hz, 2H), 4.09-4.17 (m, 2H), 3.06-3.19 (m, 4H), 1.56 (t, *J*=5.19 Hz, 4H), 1.40 (s, 3H), 0.40 (s, 4H).

### Intermediate 13: 4-(N-(tert-Butyl)sulfamoyl)-2-(6-azaspiro[2.5]octan-6-yl)benzoic acid.

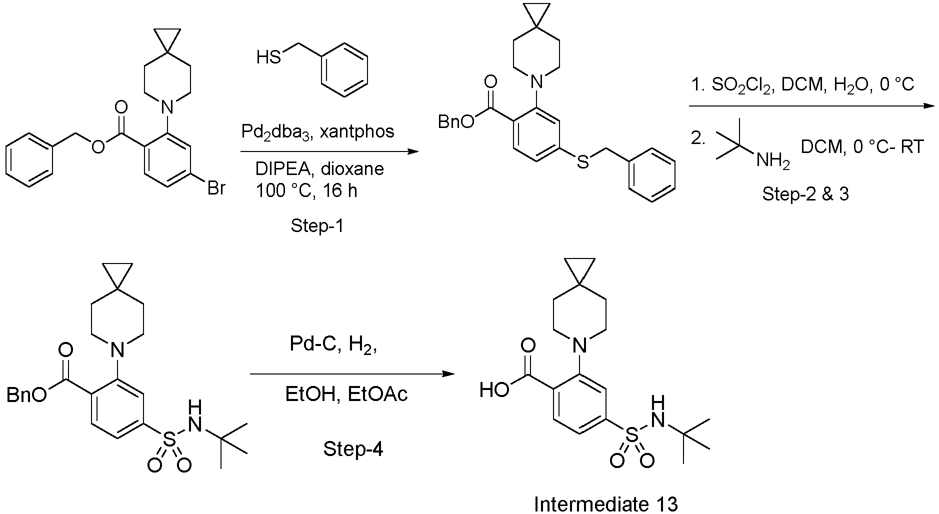

Step 1: A solution of benzyl 4-bromo-2-(6-azaspiro[2.5]octan-6-yl)benzoate (25 g, 62 mmol, Intermediate 8-4), DIPEA (21.8 mL, 125 mmol), xantphos (1.81 g, 3.12 mmol), Pd₂(dba)₃ (1.14 g, 1.25 mmol) and benzyl mercaptan (10 g, 81 mmol) in dioxane (250 mL) was degassed and purged with nitrogen for 15 min. The reaction mixture was heated at 100 °C for 16 h in a sealed pressure vessel, and then quenched with water (500 mL), and extracted with EtOAc (2 x 500 mL). The combined organic extracts were dried over anhydrous Na₂SO₄, filtered, and concentrated under reduced pressure. The crude residue was purified by column chromatography over silica gel (60-120 mesh) using 5-10% of EtOAc in hexanes to give benzyl 4-(benzylthio)-2-(6-azaspiro[2.5]octan-6-yl)benzoate (20 g, 72% yield) as a pale yellow liquid. ¹H NMR (400 MHz, DMSO-*d*₆): δ 7.57 - 7.53 (m, 1H), 7.48 - 7.26 (m, 10H), 6.93 - 6.87 (m, 2H), 5.27 (s, 2H), 4.32 (s, 2H), 2.95 - 2.87 (m, 4H), 1.35 (t, *J =* 5.3 Hz, 4H), 0.28 (s, 4H). *m*/*z* (ESI): 442.2 (M-H)⁺.

Step 2 & 3: To a solution of benzyl 4-(benzylthio)-2-(6-azaspiro[2.5]octan-6-yl)benzoate (20 g, 45 mmol) in DCM (160 mL) and water (40 mL), was added sulfuryl chloride (18.3 mL, 225 mmol) at 0 °C. The reaction mixture was stirred for 1 h before diluted with water (200 mL) and extracted with DCM (200 mL). The organic extract was dried over anhydrous Na₂SO₄, filtered, and cooled to 0 °C. *tert*-Butylamine (47.8 mL, 451 mmol) was added to the above solution. The reaction mixture was stirred at RT for 1 h before quenched with water (200 mL) and extracted with DCM (2 x 100 mL). The combined organic extracts were dried over anhydrous Na₂SO₄, filtered, and concentrated under reduced pressure. The crude residue was purified by column chromatography over silica gel (60-120 mesh) using 15% EtOAc in hexanes to give benzyl 4-(*N*-(tert-butyl)sulfamoyl)-2-(6-azaspiro[2.5]octan-6-yl)benzoate (12 g, 61% yield) as a pale yellow solid. ¹H NMR (400 MHz, DMSO-*d*₆): δ 7.71 (d, *J =* 8.0 Hz, 1H), 7.63 (s, 1H), 7.55 - 7.46 (m, 3H), 7.45 - 7.33 (m, 4H), 5.33 (s, 2H), 3.01 (b s, 4H), 1.38 (b s, 4H), 1.10 (s, 9H), 0.31 (2, 4H). *m*/*z* (ESI): 457.2 (M-H)⁺.

Step 4: To a solution of benzyl 4-(*N*-(*tert*-butyl)sulfamoyl)-2-(6-azaspiro[2.5]octan-6-yl)benzoate (10 g, 21.9 mmol) in ethanol (50 mL) and EtOAc (50 mL) was added 10% palladium on carbon (4.66 g, 4.38 mmol) at RT under nitrogen atmosphere. The reaction mixture was degassed and stirred under hydrogen atmosphere (1 atm) at RT for 16 h. The reaction mixture was filtered through a CELITE^{®} bed and the filter bed was washed with EtOAc (200 mL). The filtrate was concentrated under reduced pressure. The crude residue was triturated with diethyl ether (200 mL) to give the title compound (6.0 g, 75% yield) as an off-white solid. ¹H NMR (400 MHz, DMSO-*d*₆): δ 8.06 (d, *J =* 8.2 Hz, 1H), 7.98 (s, 1H), 7.73 - 7.68 (m, 2H), 3.12 (t, *J =* 5.3 Hz, 4H), 1.57 (t, *J =* 5.3 Hz, 4H), 1.10 (s, 9H), 0.43 (s, 4H). *m*/*z* (ESI): 365.2 (M-H)⁺.

### Intermediate 14: 4-(((2-Hydroxyethyl)sulfonyl)methyl)-2-(6-azaspiro[2.5]octan-6-yl)benzoic acid.

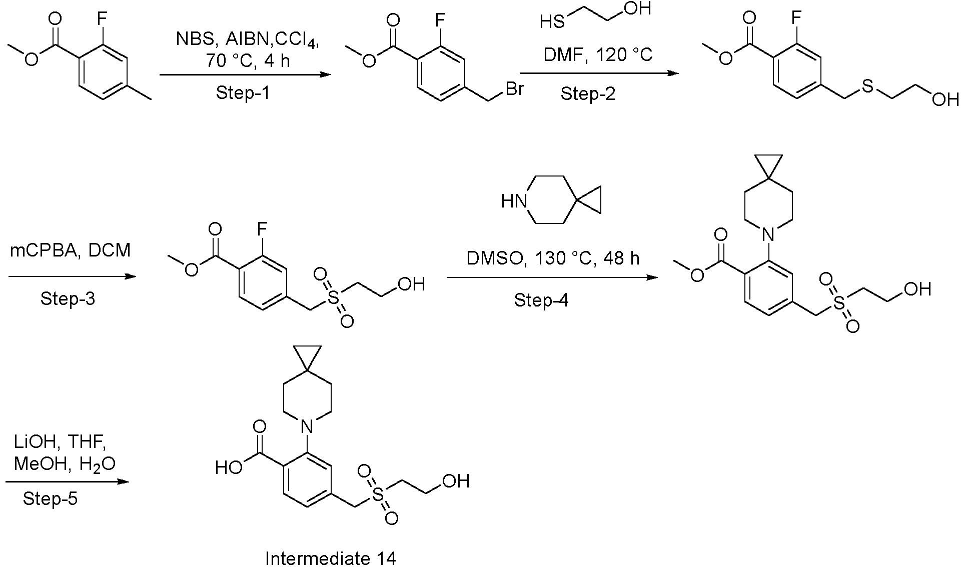

Step 1: To a stirred solution of methyl 2-fluoro-4-methylbenzoate (7.5 g, 44.6 mmol) in carbon tetrachloride (75 mL) were added NBS (7.94 g, 44.6 mmol) and AIBN (0.366 g, 2.23 mmol) and stirred at 70 °C for 4 h. The reaction mixture was quenched with water (200 mL) and extracted with DCM (2 x 200 mL). The combined organic extracts were washed with brine solution (100 mL), dried over Na₂SO₄, filtered, and concentrated under reduced pressure to give methyl 4-(bromomethyl)-2-fluorobenzoate (8.5 g, crude) as a gummy solid. The crude material showed a mixture of mono and dibromo compounds, and hence taken to the next step without any purification. *m*/*z* (ESI): 247.1 [M+1]

Step 2: A mixture of methyl 4-(bromomethyl)-2-fluorobenzoate (7 g, 28.3 mmol) and 2-mercaptoethan-1-ol (2.214 g, 28.3 mmol) in *N,N-*dimethylformamide (25.0 mL) was taken in a microwave vial. The vial was sealed and irradiated in a microwave reactor at 120 °C for 1 h. The reaction mixture was quenched with water (100 mL) and extracted with EtOAc (2 x 100 mL). The combined organic extracts were washed with brine solution (100 mL), dried over Na₂SO₄, filtered, and concentrated under reduced pressure. The crude residue was purified by column chromatography over silica gel using 50% EtOAc in hexane to give methyl 2-fluoro-4-(((2-hydroxyethyl)thio)methyl)benzoate (2 g, 29% yield) as a viscous oil.¹H NMR (300 MHz, Chloroform-*d*): *δ* 8.06 - 7.86 (m, 1H), 7.17 (td, *J=* 10.5, 3.8 Hz, 2H), 3.90 (s, 3H), 3.85 - 3.67 (m, 4H), 2.65 (td, *J =* 6.0, 1.4 Hz, 2H), 1.42 - 1.18 (m, 1H). *m*/*z* (ESI): 245.1 [M+1].

Step 3: To a stirred solution of methyl 2-fluoro-4-(((2-hydroxyethyl)thio)methyl)benzoate (2 g, 8.19 mmol) in DCM (20 mL) was added mCPBA (2.826 g, 16.38 mmol) at 0 °C and stirred at RT for 1 h. The reaction mixture was quenched with aqueous satd. NaHCO₃ solution (25 mL) and extracted with DCM (2 x 25 mL). The organic layer was washed with brine solution (25 mL), dried over Na₂SO₄, filtered and concentrated under reduced pressure to afford a gummy solid. The crude residue was triturated with diethyl ether (40 mL) to give methyl 2-fluoro-4-(((2-hydroxyethyl)sulfonyl)methyl)benzoate (1.0 g, 44% yield) as an off-white solid. ¹H NMR (400 MHz, DMSO-*d*₆): *δ* 8.07 - 7.78 (m, 1H), 7.50 - 7.23 (m, 2H), 5.29 (br s, 1H), 4.62 (s, 2H), 3.87 (s, 3H), 3.85 - 3.79 (m, 2H), 3.21 (t, *J=* 5.8 Hz, 2H). *m*/*z* (ESI): 277.1 [M+1].

Step 4: A glass tube was charged with methyl 2-fluoro-4-(((2-hydroxyethyl)sulfonyl)methyl)benzoate (1.0 g, 3.62 mmol), 6-azaspiro[2.5]octane (0.48 g, 4.34 mmol), DIPEA (0.76 mL, 4.34 mmol), and DMSO (10 mL). The tube was sealed and heated at 100 °C for 72 h. The reaction mixture was quenched with water (50 mL) and extracted with EtOAc (2 x 50 mL). The combined organic extracts were washed with brine solution (50 mL), dried over Na₂SO₄, filtered, and concentrated under reduced pressure. The crude material triturated with diethyl ether (100 mL) to give methyl 4-(((2-hydroxyethyl)sulfonyl)methyl)-2-(6-azaspiro[2.5]octan-6-yl)benzoate (900 mg, 67% yield) as a gummy oil. ¹H NMR (300 MHz, DMSO-*d*₆): *δ* 7.55 (d, *J =* 7.6 Hz, 1H), 7.13 - 7.01 (m, 2H), 5.24 (s, 1H), 4.48 (s, 2H), 3.88 - 3.72 (m, 5H), 3.31 (bs, 2H), 3.16 (bs, 4H), 1.43 (t, *J=* 6.5 Hz, 4H), 0.31 (s, 4H). *m*/*z* (ESI): 368.1 [M+1].

Step 5: To a stirred solution of methyl 4-(((2-hydroxyethyl)sulfonyl)methyl)-2-(6-azaspiro[2.5]octan-6-yl)benzoate (0.9 g, 2.45 mmol) in THF (6 mL) and methanol (4 mL) was added sodium hydroxide (98 mg, 2.45 mmol) in water (4 mL) and stirred at RT for 2 h. The reaction mixture was concentrated under reduced pressure to remove the solvents. The resulting aqueous layer was acidified with 6 N aqueous HCl solution to pH~2 and extracted with 10% methanol in DCM (3 x 50 mL). The organic layer was washed with brine solution (50 mL), dried over Na₂SO₄, filtered, and concentrated under reduced pressure. The crude residue was triturated with diethyl ether (50 mL) to give 4-(((2-hydroxyethyl)sulfonyl)methyl)-2-(6-azaspiro[2.5]octan-6-yl)benzoic acid (0.6 g, 69% yield) as a white solid. ¹H NMR (400 MHz, DMSO-*d*₆): *δ* 8.03 (d, *J =* 8.0 Hz, 1H), 7.74 (d, *J =* 1.6 Hz, 1H), 7.45 (dd, *J =* 8.0, 1.6 Hz, 1H), 5.28 (t, *J =* 4.9 Hz, 1H), 4.60 (s, 2H), 3.88 - 3.80 (m, 2H), 3.22 (t, *J =* 5.8 Hz, 2H), 3.09 (t, *J=* 5.4 Hz, 4H), 1.72 - 1.47 (m, 4H), 0.44 (s, 4H). *m*/*z* (ESI): 352.1 [M-1]. [Note: the COOH proton was not observed].

### Intermediate 15: 4-((3-Methyloxetan-3-yl)sulfonyl)-2-(6-azaspiro[2.5]octan-6-yl)benzoic acid.

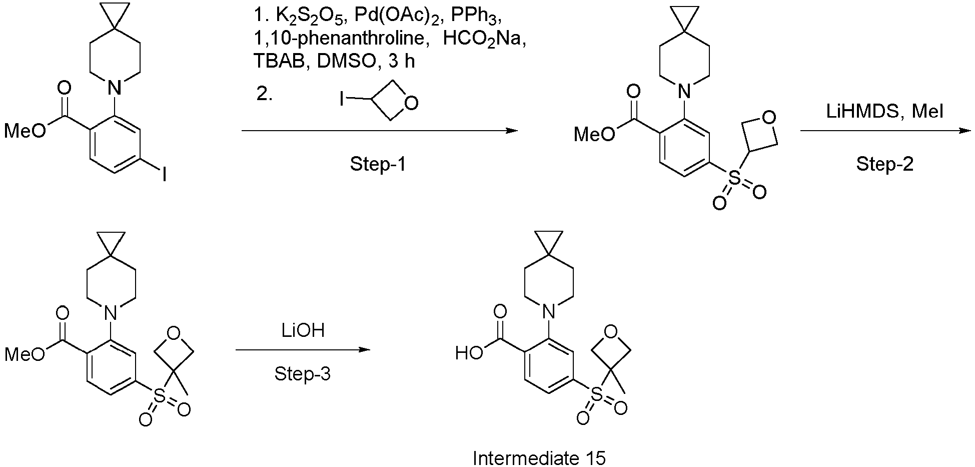

Step 1: In a glass microwave reaction vessel (20 mL) a solution of methyl 4-iodo-2-(6-azaspiro[2.5]octan-6-yl)benzoate (2.0 g, 5.39 mmol, Intermediate 8-3) in DMSO (15.0 mL) were added potassium metabisulfite (2.40 g, 10.78 mmol), TBAB (1.91 g, 5.93 mmol), sodium formate (0.81 g, 11.85 mmol), triphenyl phosphine (0.212 g, 0.81 mmol), 1,10-phenanthroline (0.146 g, 0.81 mmol) and palladium acetate (0.060 g, 0.27 mmol) under nitrogen atmosphere. The reaction mixture was degassed and purged with nitrogen for 10 min. The reaction vessel was sealed and heated at 70 °C for 3 h. The reaction mixture was cooled to RT and 3-iodooxetane (2.39 g, 12.97 mmol) was added and stirred at 120 °C for 4 h. The reaction mixture was quenched with water (100 mL) and extracted with EtOAc (2 x 100 mL). The combined organic extracts were washed with brine (100 mL), dried over Na₂SO₄, filtered, and concentrated under reduced pressure. The crude residue was adsorbed onto a plug of silica gel (60-120 mesh) and purified by silica gel chromatography through a Redi-Sep pre-packed silica gel column (40 g), eluting with a gradient of 1-40% EtOAc in hexanes to give methyl 4-(oxetan-3-ylsulfonyl)-2-(6-azaspiro[2.5]octan-6-yl)benzoate (360 mg, 15% yield) as a yellow solid. ¹H NMR (400 MHz, Chloroform-*d*): *δ* 7.79 (dd, *J* = 8.1, 1.6 Hz, 1H), 7.51 (d, *J=* 1.8 Hz, 1H), 7.38 (dd, *J =* 8.0, 1.8 Hz, 1H), 4.98 (dd, *J=* 7.4, 6.2 Hz, 2H), 4.80 (dd, *J =* 8.4, 7.1 Hz, 2H), 4.45 (tt, *J =* 8.4, 6.2 Hz, 1H), 3.94 (s, 3H), 3.22 - 3.10 (m, 4H), 1.52 (t, *J =* 5.2 Hz, 4H), 0.38 (s, 4H). *m*/*z* (ESI): 366.1 [M+1].

Step 2: To a solution of methyl 4-(oxetan-3-ylsulfonyl)-2-(6-azaspiro[2.5]octan-6-yl)benzoate (350 mg, 0.96 mmol) in THF (5 mL) was added LiHMDS (1.0 M solution in hexanes, 1.92 mL, 1.91 mmol) at - 78 °C under nitrogen atmosphere and stirred for 1 h. Iodomethane (71.9 µL, 1.15 mmol) was added slowly to the reaction mixture and slowly warmed to RT. The reaction mixture was quenched with satd. aqueous NH₄Cl solution (25 mL) and extracted with EtOAc (2 x 50 mL). The combined organic extracts were washed with brine (50 mL), dried over Na₂SO₄ filtered, and concentrated under reduced pressure. The crude residue was adsorbed onto a plug of silica gel (60-120 mesh) and purified by silica gel chromatography through a Redi-Sep pre-packed silica gel column (12 g), eluting with a gradient of 1-50% EtOAc in hexanes, to give methyl 4-((3-methyloxetan-3-yl)sulfonyl)-2-(6-azaspiro[2.5]octan-6-yl)benzoate (260 mg, 72% yield) as a pale yellow solid. ¹H NMR (400 MHz, Chloroform-d): *δ* 7.82 (d, *J =* 8.0 Hz, 1H), 7.53 (d, *J* = 1.6 Hz, 1H), 7.41 (dd, *J =* 8.0, 1.6 Hz, 1H), 5.20 (d, *J =* 6.9 Hz, 2H), 4.43 (d, *J =* 6.9 Hz, 2H), 3.97 (s, 3H), 3.24 - 3.12 (m, 4H), 1.70 (s, 3H), 1.58 (t, *J =* 5.4 Hz, 4H), 0.40 (s, 4H). *m*/*z* (ESI): 380.2 [M+1].

Step 3: To a solution of methyl 4-((3-methyloxetan-3-yl)sulfonyl)-2-(6-azaspiro[2.5]octan-6-yl)benzoate (250 mg, 0.66 mmol) in THF (5 mL), water (5 mL) and methanol (1 mL) was added lithium hydroxide (63 mg, 2.64 mmol) and stirred at RT for 5 h. The reaction mixture was acidified with 1.5N HCl pH ~4. The aqueous layer was extracted with EtOAc (3 x 50 mL), washed with brine (25 mL), dried over Na₂SO₄, filtered, and concentrated under reduced pressure to give 4-((3-methyloxetan-3-yl)sulfonyl)-2-(6-azaspiro[2.5]octan-6-yl)benzoic acid (200 mg, 83% yield) as an off-white solid. ¹H NMR (400 MHz, DMSO-*d*₆): *δ* 16.01 (s, 1H), 8.05 (d, *J =* 8.1 Hz, 1H), 7.91 (d, *J =* 1.7 Hz, 1H), 7.72 (dd, *J =* 8.0, 1.8 Hz, 1H), 5.01 (d, *J =* 7.4 Hz, 2H), 4.48 (d, *J =* 7.4 Hz, 2H), 3.19 (t, *J =* 5.2 Hz, 4H), 1.60 - 1.52 (m, 7H), 0.41 (s, 4H). *m*/*z* (ESI): 366.2 [M+1].

### Intermediate 16: 4-(3-Methyloxetan-3-yl)-2-(6-azaspiro[2.5]octan-6-yl)benzoic acid.

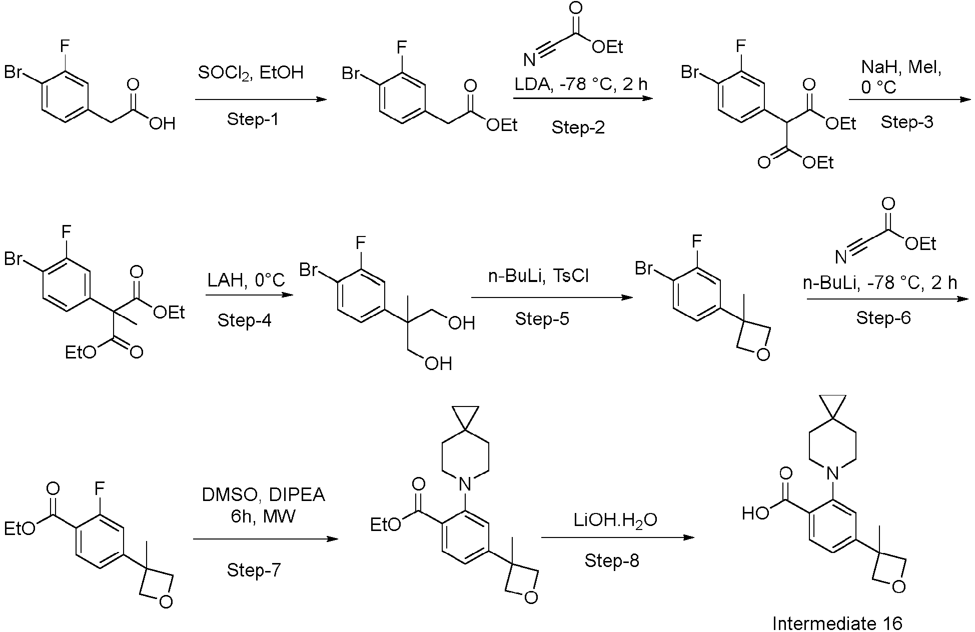

Step 1: To a solution of 2-(4-bromo-3-fluorophenyl)acetic acid (180 g, 772 mmol) in EtOH (1500 mL) was added thionyl chloride (92.1 g, 772 mmol) at 0 °C and heated at 70 °C for 2 h. The reaction mixture was concentrated under reduced pressure. The crude residue was extracted with EtOAc (3 x 1000 mL) and washed with satd. sodium bicarbonate solution (2 x 2000 mL). The organic layer was dried over Na₂SO₄, filtered and concentrated under reduced pressure to give ethyl 2-(4-bromo-3-fluorophenyl)acetate (155 g, 77% yield) as a colourless gum. ¹H NMR (400 MHz, Chloroform-*d*): *δ* 7.54 - 7.48 (m, 1H), 7.11 (dd, *J =* 9.3, 2.1 Hz, 1H), 6.98 (ddt, *J =* 8.2, 2.0, 0.8 Hz, 1H), 4.19 (q, *J =* 7.1 Hz, 2H), 3.60 (s, 2H), 1.28 (t, *J =* 7.2 Hz, 3H).

Step 2: To a solution of ethyl 2-(4-bromo-3-fluorophenyl)acetate (155 g, 594 mmol) in dry THF (1500 mL) was added LDA (2.0 M solution in THF, 297 mL, 594 mmol) at -78 °C and stirred for at -78 °C for 1 h. Ethyl cyanoformate (64.7 g, 653 mmol) was added to the reaction mixture at -78 °C and the reaction mixture was stirred for 1 h at -78 °C. The mixture was quenched with 1.5 N HCl solution (1 L) and extracted with EtOAc (3 x 2 L). The organic layer was washed with water (1 L), dried over Na₂SO₄, filtered and concentrated under reduced pressure. The crude product was purified by column chromatography over silica gel (60-120 mesh) using 10% EtOAc in hexanes to give diethyl 2-(4-bromo-3-fluorophenyl)malonate (120.0 g, 60% yield) as a viscous oil. ¹H NMR (400 MHz, Chloroform-*d*): δ 7.59 - 7.49 (m, 1H), 7.31 - 7.25 (m, 1H), 7.10 (dd, *J=* 8.0, 2.0 Hz, 1H), 4.59 (s, 1H), 4.37 - 4.14 (m, 4H), 1.36 - 1.24 (m, 6H).

Step 3: To a solution of diethyl 2-(4-bromo-3-fluorophenyl)malonate (60.0 g, 180 mmol) in DMF (600 mL) was added sodium hydride (8.64 g, 360 mmol) at 0 °C followed by the addition of iodo methane (30.7 g, 216 mmol) and the reaction mixture was stirred at RT for 2 h. The reaction mixture was quenched with ice and extracted with EtOAc (3 x 1 L). The combined organic extracts were washed with water (1 L), dried over Na₂SO₄, filtered, and concentrated under reduced pressure. The crude residue was purified by silica gel chromatography using 10% EtOAc in hexanes to give diethyl 2-(4-bromo-3-fluorophenyl)methylmalonate (45.0 g, 72% yield) as a pale brown gum. ¹H NMR (400 MHz, Chloroform-*d*): *δ* 7.53 (t, *J=* 7.6 Hz, 1H), 7.22 (dd, *J=* 10.0, 2.4 Hz, 1H), 7.11 - 7.05 (m, 1H), 4.25 (q, *J =* 7.1 Hz, 4H), 1.85 (s, 3H), 1.27 (t, *J=* 7.1 Hz, 6H).

Step 4: To a solution of diethyl 2-(4-bromo-3-fluorophenyl)methylmalonate (45.0 g, 130 mmol) in dry THF (500 mL) was added lithium aluminium hydride (2.0 M solution in THF, 130 mL, 260 mmol) at 0 °C and the reaction mixture was stirred at RT for 2 h. The reaction mixture was quenched with MeOH (40 mL) and then 1.5N HCl (500 mL) was added. The mixture was extracted with EtOAc (3 x 500 mL). The combined organic extracts were dried over Na₂SO₄, filtered, and concentrated under reduced pressure. The crude product was purified by silica gel chromatography using 20% EtOAc in hexanes to give 2-(4-bromo-3-fluorophenyl)-2-methylpropane-1,3-diol (20 g, 59% yield) as a gum.¹H NMR (400 MHz, Chloroform-*d*): *δ* 7.53 (dd, *J=* 8.4, 7.5 Hz, 1H), 7.29 - 7.21 (m, 1H), 7.12 (dd, *J=* 8.4, 2.2 Hz, 1H), 3.92 (d, *J =* 11.0 Hz, 2H), 3.77 (d, *J=* 11.0 Hz, 2H), 2.44 (br s, 2H), 1.25 (s, 3H).

Step 5: To a solution of 2-(4-bromo-3-fluorophenyl)-2-methylpropane-1,3-diol (2.5 g, 9.50 mmol) in dry THF (30 mL) was added n-butyl lithium (2.5 M solution in hexanes, 3.8 mL, 9.5 mmol) at 0 °C and stirred for 30 min. To this reaction mixture was added tosyl chloride (1.08 g, 5.70 mmol) at 0 °C and stirred at RT for 1 h. After cooling the reaction mixture to 0 °C was added n-butyl lithium (3.8 mL, 9.5 mmol) at 0 °C and slowly heated to 70 °C for 2 h. The reaction mixture was quenched with satd. ammonium chloride solution (100 mL) and extracted by EtOAc (3 x 50 mL). The combined organic extracts were dried over Na₂SO₄, filtered, and concentrated under reduced pressure. The crude residue was purified by silica gel chromatography using 5% EtOAc in hexanes to give 3-(4-bromo-3-fluorophenyl)-3-methyloxetane (0.25 g, 11% yield) as a white solid. ¹H NMR (400 MHz, Chloroform-d): *δ* 7.54 (td, *J =* 7.6, 7.1 Hz, 1H), 7.04 - 6.98 (m, 1H), 6.92 (dd, *J=* 8.2, 2.2 Hz, 1H), 4.89 (d, *J=* 5.7 Hz, 2H), 4.65 (d, *J=* 5.6 Hz, 2H), 1.72 (s, 3H).

Step 6: To a solution of 3-(4-bromo-3-fluorophenyl)-3-methyloxetane (2.5 g, 10.20 mmol) in THF (50 mL) was added n-butyl lithium (4.9 mL, 12.24 mmol) at -78 °C and the reaction mixture was stirred for 15 min at -78 °C. To this, ethyl cyanoformate (1.21 g, 12.24 mmol) was added at -78 °C and the reaction mixture was slowly warmed to 0 °C. The reaction mixture was quenched with satd. ammonium chloride solution (15 mL) and extracted by EtOAc (3 x 100 mL). The combined organic extracts were dried over Na₂SO₄ and concentrated over vacuum. The crude product was purified by silica gel chromatography using 10% EtOAc in hexanes to give ethyl 2-fluoro-4-(3-methyloxetan-3-yl)benzoate (1.25 g, 51% yield) as a white solid. ^{l}H NMR (400 MHz, Chloroform-*d*): *δ* 7.95 (t, *J=* 7.9 Hz, 1H), 7.08 (dd, *J=* 8.1, 1.8 Hz, 1H), 7.03 - 6.97 (m, 1H), 4.93 (d, *J =* 5.7 Hz, 2H), 4.67 (d, *J =* 5.7 Hz, 2H), 4.41 (q, *J =* 7.1 Hz, 2H), 1.74 (s, 3H), 1.41 (t, *J=* 7.1 Hz, 3H). *m*/*z* (ESI): 239.2 [M+1].

Step 7: To a solution of ethyl 2-fluoro-4-(3-methyloxetan-3-yl)benzoate (0.5 g, 2.10 mmol) in DMSO (5 mL) was added azaspiro[2,5]octane (0.28 g, 2.52 mmol) and heated to 130 °C for 6 h in microwave. The reaction mixture was quenched with water (50 mL) and extracted with EtOAc (3 x 50 mL). The organic layer was dried over Na₂SO₄, filtered, and concentrated under reduced pressure. The crude residue was purified by silica gel chromatography using 10% EtOAc in hexanes to give ethyl 4-(3-methyloxetan-3-yl)-2-(6-azaspiro[2.5]octan-6-yl)benzoate (0.4 g, 58% yield) as a white solid. ¹H NMR (400 MHz, Chloroform-*d*): *δ* 7.70 (d, *J =* 8.0 Hz, 1H), 6.94 - 6.79 (m, 2H), 4.97 (d, *J =* 5.6 Hz, 2H), 4.64 (d, *J=* 5.6 Hz, 2H), 4.39 (q, *J =* 7.1 Hz, 2H), 3.12 (s, 4H), 1.74 (s, 3H), 1.55 (b s, 4H), 1.41 (t, *J=* 7.1 Hz, 3H), 0.37 (s, 4H). *m*/*z* (ESI): 330.8 [M+1].

Step 8: To a solution of ethyl 4-(3-methyloxetan-3-yl)-2-(6-azaspiro[2.5]octan-6-yl)benzoate (0.8 g, 2.428 mmol) in THF (5 mL), water (5 mL) and MeOH (3 mL) was added lithium hydroxide monohydrate (117 mg, 4.86 mmol) and stirred at RT for 12 h. The reaction mixture was diluted with water (30 mL) and neutralized with 1.5 N HCl solution to make pH ~7. The reaction mixture was extracted with EtOAc (3 x 70 mL), dried over Na₂SO₄, filtered, and concentrated under reduced pressure. The crude product was triturated with diethyl ether (20 mL), filtered and dried under vacuum to give 4-(3-methyloxetan-3-yl)-2-(6-azaspiro[2.5]octan-6-yl)benzoic acid (0.45 g, 62% yield) as a white solid. ¹H NMR (400 MHz, DMSO-*d*₆): *δ* 8.01 (d, *J=* 8.0 Hz, 1H), 7.64 (d, *J=* 1.2 Hz, 1H), 7.30 (dd, *J =* 8.4, 1.6 Hz, 1H), 4.85 (d, *J =* 5.6 Hz, 2H), 4.58 (d, *J =* 5.6 Hz, 2H), 3.13 (t, *J =* 5.4 Hz, 4H), 1.65 (s, 3H), 1.60 (dd, *J =* 8.7, 4.6 Hz, 4H), 0.44 (s, 4H). *Note: The acid proton was not visible. m*/*z* (ESI): 302.2 [M+1].

### Intermediate 17: 4-(3-Hydroxyoxetan-3-yl)-2-(6-azaspiro[2.5]octan-6-yl)benzoic acid.

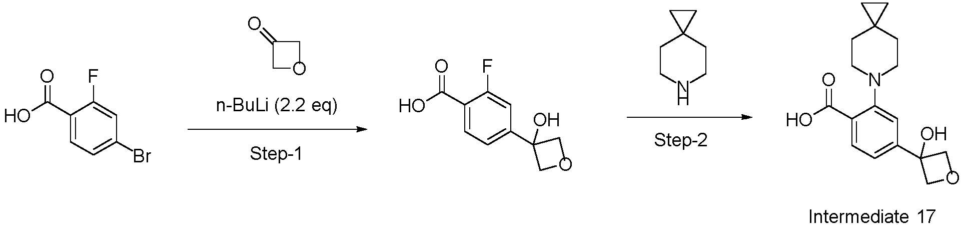

Step 1: To a solution of 4-bromo-2-fluorobenzoic acid (10.0 g, 45.7 mmol) in THF (150 mL) was added *n*-butyl lithium (2.5 M solution in hexanes, 36.5 mL, 91 mmol) at -78 °C and stirred for 30 min at - 78 °C. To this reaction mixture was added oxetan-3-one (6.5 g, 91.4 mmol) at -78 °C and stirred further for 1 h. The reaction mixture was quenched with 2.0 M aqueous sodium hydroxide solution (100 mL) and extracted with EtOAc (100 mL). The organic layer was discarded, and the aqueous layer was cooled to 0 °C and acidified with 2.0 N HCl to *p*H~5 and extracted with EtOAc (3 x 300 mL). The combined organic extracts were washed with water (200 mL), dried over Na₂SO₄, filtered, and concentrated under reduced pressure. The crude residue was purified by silica gel chromatography eluting with 50% EtOAc in hexanes to give 2-fluoro-4-(3-hydroxyoxetan-3-yl)benzoic acid (1.7 g, 17% yield) as a white solid. ¹H NMR (300 MHz, DMSO-*d*₆): *δ* 7.92 (t, *J =* 8.0 Hz, 1H), 7.57 (t, *J =* 8.2 Hz, 1H), 7.45 (d, *J =* 12.3 Hz, 1H), 6.65 (s, 1H), 4.78 (d, *J =* 6.6 Hz, 2H), 4.67 (d, *J =* 6.6 Hz, 2H). *Note: The acid proton was not visible. m*/*z* (ESI): 211.1 [M-1].

Step 2: To a solution of 2-fluoro-4-(3-hydroxyoxetan-3-yl)benzoic acid (3.8 g, 17.91 mmol) in DMSO (40 mL) was added 6-azaspiro[2.5]octane (4.0 g, 35.8 mmol) and heated to 160 °C in microwave (Biotage microwave initiator+) for 4 h. The reaction mixture was quenched with water (100 mL) and extracted with EtOAc (3 x 100 mL). The organic layer was dried over Na₂SO₄, filtered and concentrated under reduced pressure. The crude residue was purified by reverse phase silica gel chromatography using 40% CH₃CN in water to give 4-(3-hydroxyoxetan-3-yl)-2-(6-azaspiro[2.5]octan-6-yl)benzoic acid (1.1 g, 20% yield) as a white solid. ¹H NMR (400 MHz, DMSO-*d*₆): *δ* 8.08 (dd, *J* = 8.2, 1.8 Hz, 1H), 7.86 (d, *J =* 1.9 Hz, 1H), 7.69 (dt, *J =* 8.3, 1.8 Hz, 1H), 6.62 (d, *J=* 1.7 Hz, 1H), 4.79 (d, *J =* 6.4 Hz, 2H), 4.72 (d, *J =* 6.4 Hz, 2H), 3.14 - 3.08 (m, 4H), 1.59 (bs, 4H), 0.44 (s, 4H). *Note: The acid proton was not visible. m*/*z* (ESI): 302.2 [M-1].

### Intermediate 18: 4-Bromo-2-(6-azaspiro[2.5]octan-6-yl)aniline.

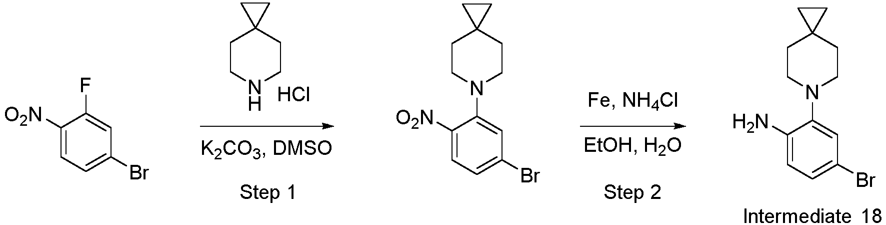

Step 1: A mixture of 4-bromo-2-fluoro-1-nitrobenzene (3.17 g, 14.4 mmol, Combi-Blocks), 6-azaspiro[2.5]octane hydrochloride (2.45 g, 16.57 mmol, AstaTech) and potassium carbonate (5.97 g, 43.2 mmol, Sigma-Aldrich Corporation) in DMSO (12 mL) was heated in an oil bath at 60 °C for 10 min then heated to 90 °C for 1 h. The mixture was cooled to RT, treated with 20 mL of water, and extracted with EtOAc (2 x 50 mL). The combined organic extracts were washed with water (2 x 5 mL), dried (Na₂SO₄), and the solvent was removed under vacuum. The residue was purified on a silica gel column (15-45% EtOAc in heptane) to give 6-(5-bromo-2-nitrophenyl)-6-azaspiro[2.5]octane (4.26 g, 13.7 mmol, 95% yield) as an orange solid. *m*/*z* (ESI): 311.0/313.0 (M+H)⁺.

Step 2: To a mixture of 6-(5-bromo-2-nitrophenyl)-6-azaspiro[2.5]octane (2.91 g, 9.35 mmol) and NH₄Cl (1.50 g, 28.1 mmol, Sigma-Aldrich Corporation) in EtOH (16 mL) and water (4 mL) was added iron powder (3.13 g, 56.1 mmol, Sigma-Aldrich Corporation). The heterogeneous mixture was heated in an oil bath at 85 °C for 2 h. The dark mixture was diluted with 50 mL of MeOH and filtered through a pad of CELITE^{®}. The filter cake was rinsed with 2 x 5 mL of MeOH and the filtrate was concentrated under vacuum. The residue was partitioned between 10 mL of water and 75 mL of EtOAc. The organic layer was taken, dried over Na₂SO₄, and concentrated to afford 4-bromo-2-(6-azaspiro[2.5]octan-6-yl)aniline ( 2.23 g, 7.95 mmol, 85% yield) as a brown oil. ¹H NMR (400 MHz, METHANOL-d₄) δ 6.87 (d, *J =* 2.28 Hz, 1H), 6.77 (dd, *J =* 2.18, 8.40 Hz, 1H), 6.48 (d, *J =* 8.50 Hz, 1H), 4.65 (s, 4H), 1.23-1.50 (br s, 4H), 0.18 (s, 4H). *m*/*z* (ESI): 281.0 /283.0 (M+H)⁺.

### COUPLING OF AR¹ AND AR² INTERMEDIATES

### Intermediate 19: N-(3-(N-(tert-Butyl)sulfamoyl)phenyl)-4-iodo-2-(6-azaspiro[2.5]octan-6-yl)benzamide.

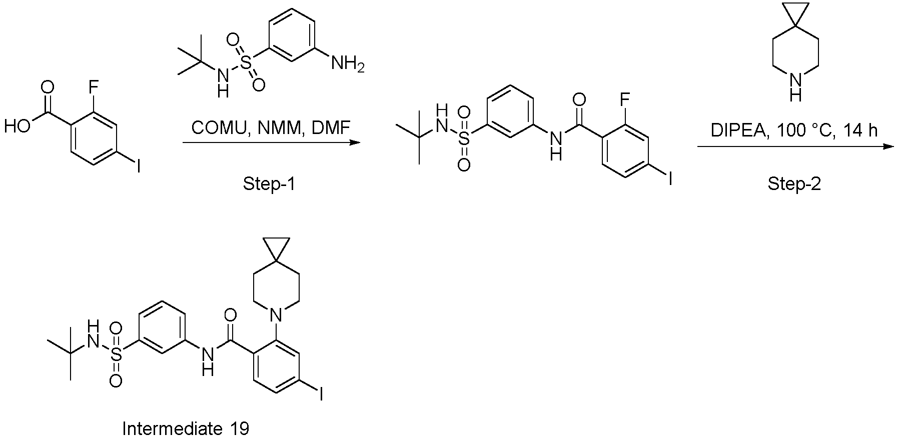

Step-1: To a solution of 2-fluoro-4-iodobenzoic acid (2.0 g, 7.52 mmol) in DMF (20 mL) were added 3-amino-*N*-(*tert*-butyl)benzenesulfonamide (1.72 g, 7.52 mmol), (1-cyano-2-ethoxy-2-oxoethylidenaminooxy)dimethylaminomorpholinocarbenium hexafluorophosphate (3.86 g, 9.02 mmol) and *N*-methylmorpholine (2.48 mL, 22.56 mmol) slowly at RT and stirred for 2 h. The reaction mixture was quenched with water (100 mL) and extracted with EtOAc (2x 100 mL). The combined organic extracts were washed with brine (50 mL), dried over Na₂SO₄, filtered, and concentrated under reduced pressure. The crude residue was absorbed onto a plug of silica gel and purified by flash chromatography through a Redi-Sep pre-packed silica gel column, eluting with a gradient of 0-50% EtOAc in hexanes, to give *N-*(3-(*N-*(*tert-*butyl)sulfamoyl)phenyl)-2-fluoro-4-iodobenzamide (2.8 g, 78% yield) as a yellow solid. ¹H NMR (400 MHz, DMSO-*d*₆): δ 10.72 (s, 1H), 8.29 (s, 1H), 7.86 - 7.71 (m, 3H), 7.61 - 7.51 (m, 3H), 7.48 (t, *J*=7.83 Hz, 1H), 1.12 (s, 9H). *m*/*z* (ESI): 475.0 [M-1].

Step-2: To a solution of *N*-(3-(*N*-(*tert*-butyl)sulfamoyl)phenyl)-2-fluoro-4-iodobenzamide (8.0 g, 16.80 mmol) in dimethyl sulfoxide (50 mL) was added 6-azaspiro[2.5]octane (1.87 g, 16.80 mmol) and DIPEA (2.93 mL, 16.80 mmol) under nitrogen atmosphere and stirred at 100 °C for 16 h. The reaction mixture was quenched with water (150 mL). The precipitated solid was filtered, washed with water (200 mL), and dried under vacuum. The solid cake was absorbed onto a plug of silica gel and purified by flash chromatography through a Redi-Sep pre-packed silica gel column, eluting with a gradient of 0-30% EtOAc in hexanes, to give *N*-(3-(*N*-(*tert*-butyl)sulfamoyl)phenyl)-4-iodo-2-(6-azaspiro[2.5]octan-6-yl)benzamide (6.0 g, 63% yield) as an off-white foamy solid. ¹H NMR (400 MHz, DMSO-*d₆*): *δ* 11.37 (s, 1H), 8.32 (s, 1H), 7.90 (d, *J =* 4.4 Hz, 1H), 7.59 - 7.54 (m, 5H), 7.47 - 7.44 (m, 1H), 3.35 (s, 4H), 1.41 (s, 4H), 1.18 (s, 9H), 0.28 (s, 4H). *m*/*z* (ESI): 568.1 [M+1].

**Table 5: Intermediates 19-1 and 19-2 were prepared analogous to preparation of Intermediate 19.**

| Int. # | Chemical Structure | Name | LRMS: (ESI + ve ion) *m*/*z* |
|---|---|---|---|
| 19-1 | 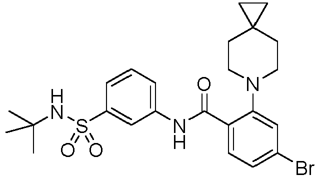 | 4-bromo-*N*-(3-(*N*-(*tert-*butyl)sulfamoyl)phenyl )-2-(6-azaspiro[2.5]octan-6-yl)benzamide | 520.2/522.2 |
| 19-2 | 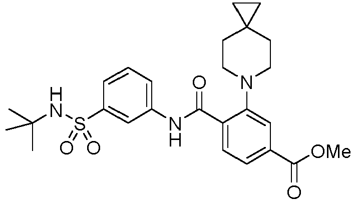 | methyl 4-((3-(*N-*(*tert-*butyl)sulfamoyl)phenyl )carbamoyl)-3-(6-azaspiro[2.5]octan-6-yl)benzoate | 500.3 |

### Intermediate 20: 4-Bromo-N-(3-(4,4-difluoropiperidin-1-yl)-5-methylphenyl)-2-(6-azaspiro[2.5]octan-6-yl)benzamide.

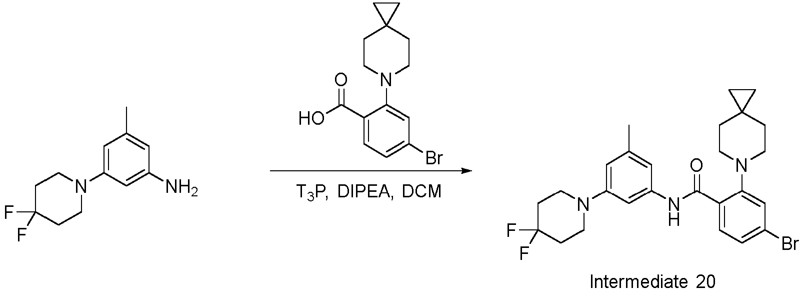

A mixture of 4-bromo-2-(6-azaspiro[2.5]octan-6-yl)benzoic acid (2.47 g, 7.96 mmol, Intermediate 8-1), *N*-ethyl-*N*-isopropylpropan-2-amine (2.57 g, 19.89 mmol), 2,4,6-tripropyl-1,3,5,2,4,6-trioxatriphosphorinane-2,4,6-trioxide (50% in EtOAc, 12.66 g, 19.89 mmol) and 3-(4,4-difluoropiperidin-1-yl)-5-methylaniline (1.5 g, 6.63 mmol, Intermediate 3) in DCM (15 mL) was stirred at RT for 6 h. The reaction mixture was quenched with water and extracted with DCM. The organic layer was washed with brine, dried over Na₂SO₄, filtered, concentrated and purified by silica gel column chromatography using 60-70% EtOAc in petroleum ether to provide 4-bromo-*N*-(3-(4,4-difluoropiperidin-1-yl)-5-methylphenyl)-2-(6-azaspiro[2.5]octan-6-yl)benzamide (1 g, 1.93 mmol, 29% yield) as a pale brown oil. ¹H NMR (400 MHz, DMSO-*d*₆): δ ppm 11.19 (s, 1 H), 7.68 (d, *J*=8.3 Hz, 1 H), 7.45 (d, *J*=1.9 Hz, 1 H), 7.39 (dd, *J*=8.3, 1.9 Hz, 1 H), 7.17 (s, 1 H), 7.14 (d, *J*=2.4 Hz, 1 H), 6.61 (s, 1 H), 3.30 - 3.34 (m, 4 H), 3.02 (t, *J=*5.2 Hz, 4 H), 2.27 (s, 3 H), 1.97 - 2.13 (m, 4 H), 1.49 (t, *J=*5.2 Hz, 4 H), 0.32 (s, 4 H). *m*/*z* (ESI): 518.1(M+H)⁺.

### Intermediate 21: 4-Bromo-N-(3-(4,4-difluoropiperidin-1-yl)-2-fluorophenyl)-2-(6-azaspiro[2.5]octan-6-yl)benzamide.

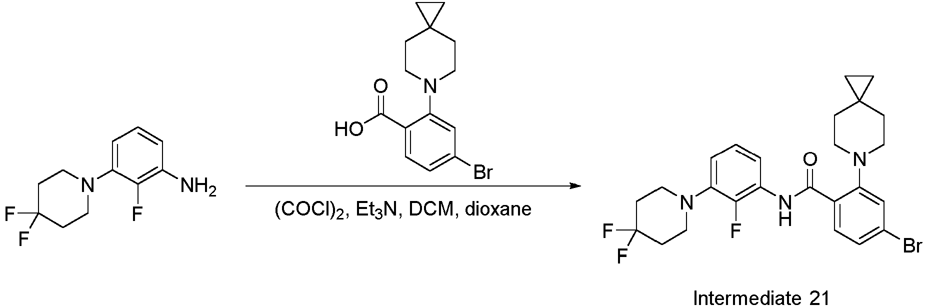

A mixture of 4-bromo-2-(6-azaspiro[2.5]octan-6-yl)benzoic acid (0.2 g, 0.64 mmol, Intermediate 8-1), oxalyl dichloride (0.123 g, 0.97 mmol) and catalytic DMF in DCM (3 mL) was stirred at 0°C for 30 min. The reaction mixture was concentrated, and the residue was dissolved in dioxane (2 mL). This solution was treated with a solution of 3-(4,4-difluoropiperidin-1-yl)-2-fluoroaniline (0.15 g, 0.64 mmol, Intermediate 4) and Et3N (0.27 mL, 1.93 mmol) in dioxane (4 mL). The reaction mixture was stirred at 100 °C for 16 h before it was diluted with water and extracted with EtOAc. The organic extract was washed with brine, dried over Na₂SO₄, filtered, and concentrated. The concentrate was purified by silica gel column chromatography eluting with 10-20% EtOAc in petroleum ether to provide 4-bromo-*N*-(3-(4,4-difluoropiperidin-1-yl)-2-fluorophenyl)-2-(6-azaspiro[2.5]octan-6-yl)benzamide (0.160 g, 0.31 mmol, 48% yield) as an off-white solid. ¹H NMR (300 MHz, DMSO-*d*₆): δ ppm 11.86 (s, 1 H), 7.99 (t, *J*=7.5 Hz, 1 H), 7.89 (d, *J*=8.4 Hz, 1 H), 7.60 (d, *J=*1.9 Hz, 1 H), 7.49 (dd, *J*=8.3, 1.8 Hz, 1 H), 7.13 (t, *J*=8.2 Hz, 1 H), 6.92 (t, *J*=8.2 Hz, 1 H), 3.16 (t, *J*=6.0 Hz, 4 H), 3.03 (t, *J=*5.3 Hz, 4 H), 2.07 - 2.22 (m, 4 H), 1.53 (s, 4 H), 0.36 (s, 4 H). *m*/*z* (ESI): 524.1(M+H)⁺.

**Table 6: Intermediates 22 to 26 were prepared analogous to preparations of Intermediate 20 or 21:**

| Int. # | Chemical Structure | Name | LRMS: (ESI + ve ion) *m*/*z* |
|---|---|---|---|
| 22 | 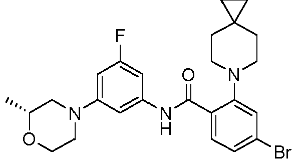 | (*R*)-4-bromo-*N*-(3-fluoro-5-(2-methylmorpholino)phenyl)-2-(6-azaspiro[2.5]octan-6-yl)benzamide | 502.1/504.1 |
| 23 | 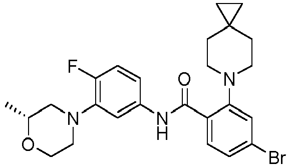 | (*R*)-4-bromo-*N*-(4-fluoro-3-(2-methylmorpholino)phenyl)-2-(6-azaspiro[2.5]octan-6-yl)benzamide | 502.1/504.1 |
| 24 | 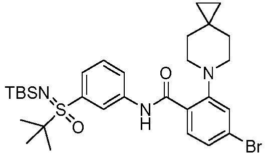 | 4-bromo-*N-*(3-(*N-*(*tert-*butyldimethylsilyl)-2-methylpropan-2-ylsulfonimidoyl)phenyl)-2-(6-azaspiro[2.5]octan-6-yl)benzamide | 618.2/620.2 |
| 25 | 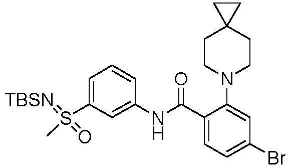 | 4-bromo-*N-*(3-(*N-*(*tert-*butyldimethylsilyl)-methylsulfonimidoyl)phenyl )-2-(6-azaspiro[2.5]octan-6-yl)benzamide | 576.2/578.2 |
| 26 | 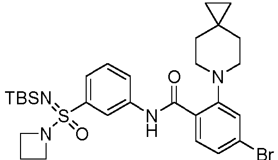 | 4-bromo-*N-*(3-(*N*-(*tert-*butyldimethylsilyl)azetidine-1-sulfonimidoyl)phenyl)-2-(6-azaspiro[2.5]octan-6-yl)benzamide | 617.2/619.2 |

### Intermediate 27: 4-Bromo-N-(3-(2-hydroxy-2-methylpropoxy)phenyl)-2-(6-azaspiro[2.5]octan-6-yl)benzamide.

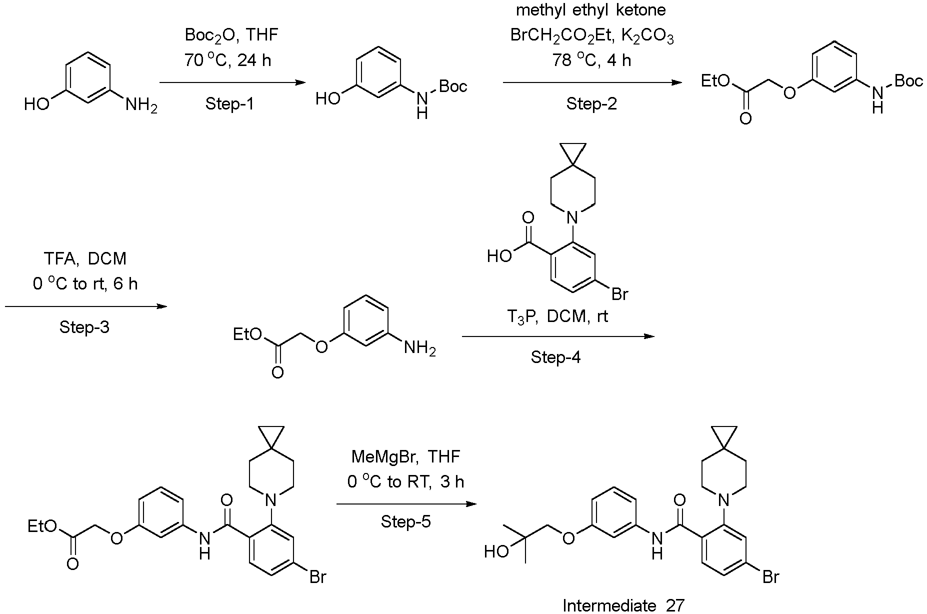

Step 1: A mixture of 3-aminophenol (5 g, 45.8 mmol) and Boc-anhydride (9.0 g, 41.2 mmol) in THF (50 mL) was stirred at 70 °C for 24 h. The reaction mixture diluted with EtOAc, washed with 1.5 N HCl. The organic layer was washed with brine, dried over Na₂SO₄, filtered, and concentrated. The concentrate was purified by silica gel column chromatography eluting with 15-20% EtOAc in petroleum ether to provide *tert-*butyl (3-hydroxyphenyl)carbamate (8.0 g, 38.2 mmol, 83% yield) as off-white solid. ¹H NMR (400 MHz, DMSO-*d*₆): δ ppm 9.25 (d, *J*=1.7 Hz, 1 H), 9.20 (s, 1 H), 6.90 - 7.13 (m, 2 H), 6.74 - 6.90 (m, 1 H), 6.35 (ddt, *J=*1.3, 2.7, 8.1 Hz, 1 H), 1.47 (s, 9 H).

Step 2: A mixture of *tert-*butyl (3-hydroxyphenyl)carbamate (3.0 g, 14.34 mmol), K₂CO₃ (5.94 g, 43.0 mmol) and ethyl 2-bromoacetate (2.39 mL, 21.51 mmol) ) in methyl ethyl ketone (40 mL) was stirred at 78°C for 4 h. The reaction mixture was diluted with water and extracted with EtOAc. The organic extract was washed with brine, dried over Na₂SO₄, filtered, and concentrated. The concentrate was purified by silica gel column chromatography eluting with 10-15% EtOAc in petroleum ether to provide ethyl 2-(3-((*tert-*butoxycarbonyl)amino)phenoxy)acetate (3.4 g, 11.51 mmol, 80% yield) as a colorless oil. ¹H NMR (300 MHz, DMSO-*d*₆): δ ppm 9.32 (s, 1 H), 6.98 - 7.14 (m, 3 H), 6.49 (dd, *J*=2.6, 8.0 Hz, 1 H), 4.67 (s, 2 H), 3.75 - 4.37 (m, 2 H), 1.45 (s, 9 H), 1.20 (t, *J=*7.2 Hz, 3 H). *m*/*z* (ESI): 196.1 (M-Boc)⁺.

Step 3: A solution of ethyl 2-(3-((*tert*-butoxycarbonyl)amino)phenoxy)acetate (1.0 g, 3.39 mmol) and TFA (1.30 mL, 16.93 mmol) in DCM (10 mL) was stirred at RT for 6 h. The reaction was concentrated, neutralized with a satd. aqueous solution of sodium bicarbonate and extracted with DCM. The organic extract was washed with brine, dried over Na₂SO₄, filtered, and concentrated to provide ethyl 2-(3-aminophenoxy)acetate (0.6 g, 3.07 mmol, 91% yield) as a yellow oil. ¹H NMR (300 MHz, DMSO-*d*₆): δ ppm 7.27 (t, *J*=8.1 Hz, 2 H), 6.66 - 6.80 (m, 3 H), 4.77 (s, 2 H), 4.17 (q, *J*=7.1 Hz, 2 H), 1.22 (t, *J*=7.1 Hz, 3 H). *m*/*z* (ESI): 196.1 (M+H)⁺.

Step 4: A mixture of 4-bromo-2-(6-azaspiro[2.5]octan-6-yl)benzoic acid (0.5 g, 1.61 mmol, Intermediate 8-1), ethyl 2-(3-aminophenoxy)acetate (0.47 g, 2.42 mmol), DIPEA (0.84 mL, 4.84 mmol) and T₃P (50% in EtOAc, 2.05 g, 3.22 mmol) in DCM (10 mL) was stirred at rt for 48 h. The reaction mixture was diluted with water and extracted with DCM. The organic extract was washed with brine, dried over Na₂SO₄, filtered, and concentrated. The concentrate was purified by silica gel column chromatography eluting with 10% EtOAc in petroleum ether to provide ethyl 2-(3-(4-bromo-2-(6-azaspiro[2.5]octan-6-yl)benzamido)phenoxy)acetate (0.41 g, 0.84 mmol, 52% yield) as a light yellow oil. ¹H NMR (300 MHz, DMSO-*d*₆): δ ppm 11.16 (s, 1 H), 7.65 (d, *J=*8.2 Hz, 1 H), 7.48 (s, 1 H), 7.40 (dd, *J*=1.8, 14.8 Hz, 1 H), 7.32 (d, *J*=17.3 Hz, 1 H), 7.28 - 7.32 (m, 2 H), 6.64 - 6.70 (m, 1 H), 4.76 (s, 2 H), 4.18 (q, *J*=7.1 Hz, 2 H), 3.03 (t, *J*=6.4 Hz, 4 H), 1.46 (br s, 4 H), 1.21 (t, *J*=7.4 Hz, 3 H), 0.32 (s, 4 H). *m*/*z* (ESI): 487.1 (M+H)⁺.

Step 5: To a solution of ethyl 2-(3-(4-bromo-2-(6-azaspiro[2.5]octan-6-yl)benzamido)phenoxy) acetate (0.5 g, 1.03 mmol) in THF (15 mL) was added methyl magnesium bromide (2.05 mL, 6.16 mmol) at 0 °C and the reaction mixture was stirred at RT for 3 h. The reaction mixture was quenched with a satd. aqueous solution of NH₄Cl and extracted with EtOAc. The organic extract was washed with brine, dried over Na₂SO₄, filtered, and concentrated. The concentrate was purified by silica gel column chromatography eluting with 15% EtOAc in petroleum to provide 4-bromo-*N*-(3-(2-hydroxy-2 methylpropoxy)phenyl)-2-(6-azaspiro[2.5]octan-6-yl)benzamide (0.26 g, 0.55 mmol, 53% yield) as an off-white solid. ¹H NMR (300 MHz, DMSO-*d*₆): δ ppm 11.18 (s, 1 H), 7.66 (d, *J*=8.3 Hz, 1 H), 7.56 (t, *J*=2.2 Hz, 1 H), 7.29 - 7.48 (m, 2 H), 7.18 - 7.30 (m, 2 H), 6.66 - 6.70 (m, 1 H), 4.66 (s, 1 H), 3.70 (s, 2 H), 3.02 (t, *J*=5.4 Hz, 4 H), 1.46 (br s, 4 H), 1.21 (s, 6 H), 0.32 (s, 4 H). *m*/*z* (ESI): 473.1 (M+H)⁺.

### Intermediate 28: Ethyl 2-sulfamoylpropanoate

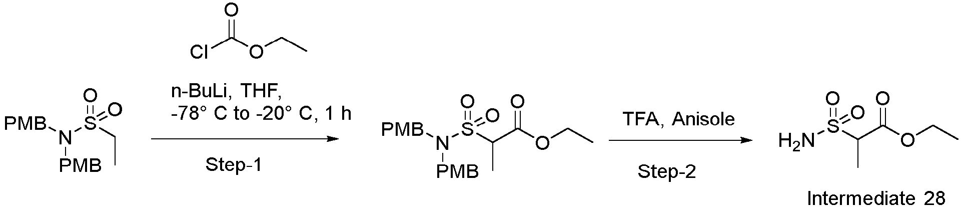

Step 1: To a solution of *N,N-*bis(4-methoxybenzyl)ethanesulfonamide (200.0 g, 572.0 mmol) in THF (4000 mL) was added nBuLi (1.6 M in hexane, 608.0 mL, 973.0 mmol) at -78 °C slowly and stirred for 30 min. Ethyl carbonochloridate (92.0 mL, 973.0 mmol) in THF (50 mL) was added to the reaction mixture and stirred at -78 °C for 1 h. The reaction mixture was quenched with HCl (1.5 N, 3000 mL) and extracted with EtOAc (2 x 3000 mL). The organic extract was dried over Na₂SO₄, filtered, and concentrated in reduced pressure to give the crude material of ethyl 2-(*N*,*N*-bis(4-methoxybenzyl)sulfamoyl)propanoate (250.0 g, 60% pure) as a yellow oil, which was proceeded to next step without any purification.

Step 2: To solution of ethyl 2-(*N,N*-bis(4-methoxybenzyl)sulfamoyl)propanoate (600.0 g, 1.4 mol) in trifluoroacetic acid (2.50 L, 32.45 mol) was added anisole (500.0 mL, 4.57 mol) and stirred at RT for 3 h. The reaction mixture was concentrated under reduced pressure, quenched with 10% cold aqueous NaHCO₃ solution (3 L) and extracted with EtOAc (2 x 3 L). The combined organic extracts were dried over Na₂SO₄, filtered, and concentrated under reduced pressure. The crude residue was purified by column chromatography over silica gel using 25% EtOAc in hexanes to give a pale, yellow solid (168 g) which was dissolved in DCM (1 L) and precipitated by the addition of hexanes (3000 mL). The solid was filtered, dried under vacuum to give the title compound (109.0 g, 42% yield) as a white solid. ¹H NMR (400 MHz, DMSO-*d*₆) *δ* ppm 7.14 (s, 2H), 4.15 (q, *J =* 7.1 Hz, 2H), 3.98 (q, *J =* 7.0 Hz, 1H), 1.45 (d, *J =* 7.0 Hz, 3H), 1.21 (t, *J=* 7.1 Hz, 3H). *m*/*z* (ESI): 180.1 (M-H)⁺.

### EXAMPLES

### Example 100: N-(3-(N-(tert-Butyl)sulfamoyl)phenyl)-4-((3-methyloxetan-3-yl)sulfonyl)-2-(6-azaspiro[2.5]octan-6-yl)benzamide

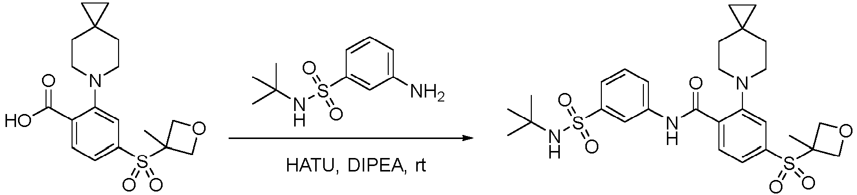

To a solution of 4-((3-methyloxetan-3-yl)sulfonyl)-2-(6-azaspiro[2.5]octan-6-yl)benzoic acid (120 mg, 0.33 mmol, Intermediate 15) in DMF (2 mL) were added HATU (187 mg, 0.49 mmol) and DIPEA (143 µL, 0.821 mmol) at RT and stirred for 10 min. To this reaction mixture, 3-amino-*N*-(tert-butyl)benzenesulfonamide (82 mg, 0.36 mmol) was added and stirred for 12 h at RT. The reaction mixture was quenched with water (20 mL) and extracted by EtOAc (3 x 25 mL). The combined organic extracts were washed with brine solution (20 mL), dried over Na₂SO₄, filtered, and concentrated under reduced pressure. The crude residue was purified by silica gel column chromatography using 30% EtOAc in hexanes to give the title compound (110 mg, 58% yield) as an off-white solid. ¹H NMR (400 MHz, Chloroform-*d*): *δ* 12.33 (s, 1H), 8.47 (d, *J=* 8.2 Hz, 1H), 8.31 (d, *J=* 2.1 Hz, 1H), 8.06 - 7.95 (m, 1H), 7.87 (d, *J=* 1.8 Hz, 1H), 7.79 (dd, *J* = 8.2, 1.7 Hz, 1H), 7.69 (d, *J =* 8.3 Hz, 1H), 7.54 (t, *J* = 8.0 Hz, 1H), 5.19 (d, *J =* 7.0 Hz, 2H), 4.52 (s, 1H), 4.47 (d, *J=* 7.0 Hz, 2H), 3.16 (t, *J =* 5.5 Hz, 4H), 1.73 (s, 3H), 1.70 - 1.60 (b s, 3H), 1.30 (s, 9H), 0.48 (s, 4H). *m*/*z* (ESI): 576.2 [M+1].

**Table 7: Examples 100-1 to 100-15 were prepared analogous to preparation of Example 100:**

| Ex. # | Chemical Structure | Name | LRMS: (ESI + ve ion) *m*/*z* |
|---|---|---|---|
| 100-1 | 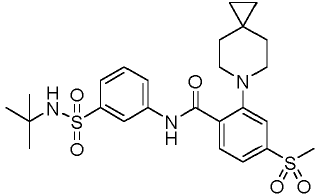 | N-(3-(*N-*(*tert-*butyl)sulfamoyl)phenyl)-4-(methylsulfonyl)-2-(6-azaspiro[2.5]octan-6-yl)benzamide | 520.1 |
| 100-2 | 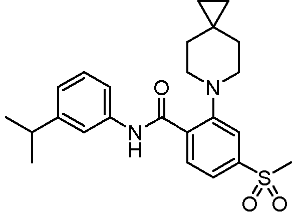 | *N*-(3-isopropylphenyl)-4-(methylsulfonyl)-2-(6-azaspiro[2.5]octan-6-yl)benzamide | 427.2 |
| 100-3 | 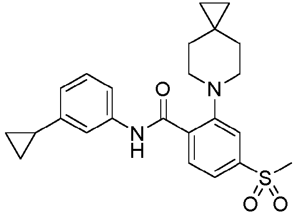 | *N*-(3-cyclopropylphenyl)-4-(methylsulfonyl)-2-(6-azaspiro[2.5]octan-6-yl)benzamide | 425.2 |
| 100-4 | 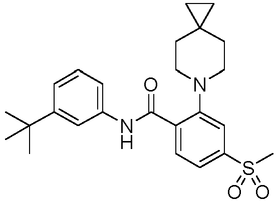 | *N*-(3-(*tert*-butyl)phenyl)-4-(methylsulfonyl)-2-(6-azaspiro[2.5]octan-6-yl)benzamide | 441.2 |
| 100-5 | 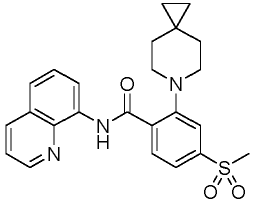 | 4-(methylsulfonyl)-*N-*(quinolin-8-yl)-2-(6-azaspiro[2.5]octan-6-yl)benzamide | 436.2 |
| 100-6 | 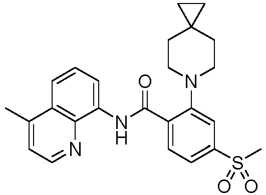 | *N*-(4-methylquinolin-8-yl)-4-(methylsulfonyl)-2-(6-azaspiro[2.5]octan-6-yl)benzamide | 495.2 |
| 100-7 | 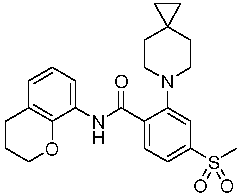 | *N*-(chroman-8-yl)-4-(methylsulfonyl)-2-(6-azaspiro[2.5]octan-6-yl)benzamide | 440.8 |
| 100-8 | 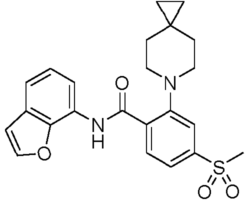 | *N*-(benzofuran-7-yl)-4-(methylsulfonyl)-2-(6-azaspiro[2.5]octan-6-yl)benzamide | 425.1 |
| 100-9 | 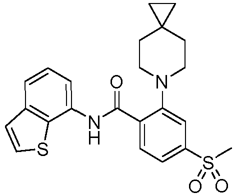 | *N*-(benzo[b]thiophen-7-yl)-4-(methylsulfonyl)-2-(6-azaspiro[2.5]octan-6-yl)benzamide | 441.2 |
| 100-10 | 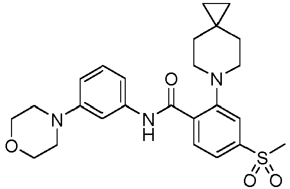 | 4-(methylsulfonyl)-*N*-(3-morpholinophenyl)-2-(6-azaspiro[2.5]octan-6-yl)benzamide | 470.1 |
| 100-11 | 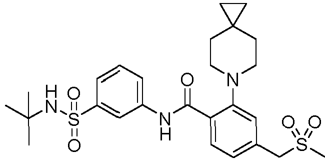 | N-(*3-*(*N*-(*tert-*butyl)sulfamoyl)phenyl)-4-((methylsulfonyl)methyl)-2-(6-azaspiro[2.5]octan-6-yl)benzamide | 534.2 |
| 100-12 | 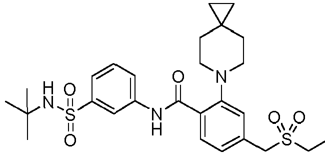 | *N*-(3-(*N-*(*tert-*butyl)sulfamoyl)phenyl)-4-(((2-hydroxyethyl)sulfonyl)meth yl)-2-(6-azaspiro[2.5]octan-6-yl)benzamide | 564.2 |
| 100-13 | 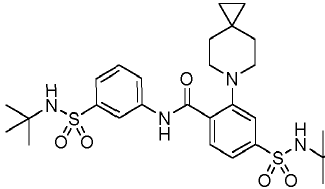 | 4-(*N*-(*tert*-butyl)sulfamoyl)-N-(3-(*N*-(*tert-*butyl)sulfamoyl)phenyl)-2-(6-azaspiro[2.5]octan-6-yl)benzamide | 577.2 |
| 100-14 | 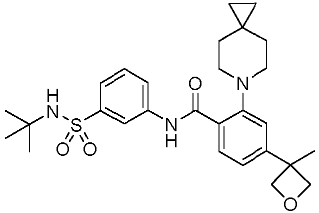 | *N-(*3-(*N-*(*tert-*butyl)sulfamoyl)phenyl)-4-(3-methyloxetan-3-yl)-2-(6-azaspiro[2.5]octan-6-yl)benzamide | 512.3 |
| 100-15 | 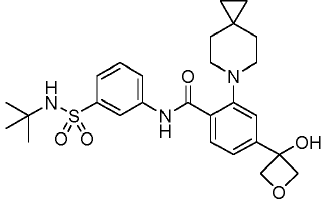 | *N-*(3-(*N-*(*tert-*butyl)sulfamoyl)phenyl)-4-(3-hydroxyoxetan-3-yl)-2-(6-azaspiro[2.5]octan-6-yl)benzamide | 514.1 |

### Example 101: N-(3-((1-hydroxy-2-methylpropan-2-yl)amino)phenyl)-4-(N-(3-methyloxetan-3-yl)sulfamoyl)-2-(6-azaspiro[2.5]octan-6-yl)benzamide

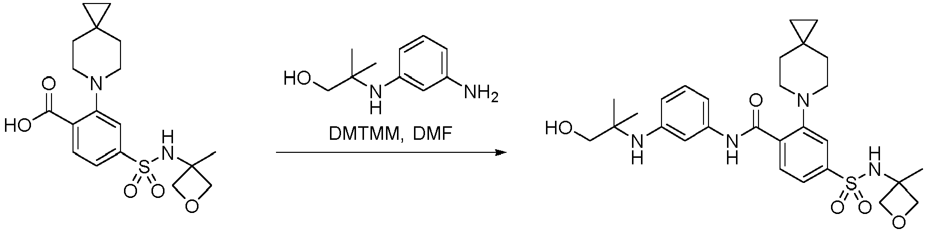

To a solution of 4-(*N*-(3-methyloxetan-3-yl)sulfamoyl)-2-(6-azaspiro[2.5]octan-6-yl)benzoic acid (0.25 g, 0.66 mmol, Intermediate 12) and 2-((3-aminophenyl)amino)-2-methylpropan-1-ol (0.130 g, 0.72 mmol, Intermediate 2-2) in DMF (3 mL) was added 4-(4,6-dimethoxy-1,3,5-triazin-2-yl)-4-methylmorpholinium chloride (0.291 g, 0.986 mmol) and stirred at rt for 18 h. The reaction was partitioned between water and EtOAc. The organic phase was separated, washed with brine, dried over magnesium sulfate, and concentrated under reduced pressure. Purification by prep SFC gave *N*-(3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide (0.26 g, 0.48 mmol, 73% yield). ¹H NMR (500 MHz, DMSO-d6) δ ppm 11.61 (s, 1 H) 8.16 (s, 1 H) 7.87 (d, J=8.48 Hz, 1 H) 7.26 (d, J=2.06 Hz, 1 H) 7.17 (t, J=1.95 Hz, 1 H) 7.05 - 7.10 (m, 2 H) 6.99 - 7.04 (m, 1 H) 6.42 - 6.46 (m, 1 H) 3.39 (s, 2 H) 3.18 (s, 3 H) 2.96 (br t, J=5.16 Hz, 4 H) 1.58 (br s, 4 H) 1.42 (s, 3 H) 1.26 (s, 6 H) 1.18 - 1.22 (m, 2 H) 0.84 (d, J=2.06 Hz, 2 H) 0.37 (s, 4 H). *m*/*z* (ESI, +ve ion): 527.3 (M+H)⁺.

**Table 8: Examples 101-1 to 101-3 were prepared analogous to preparation of Example 101:**

| Ex. # | Chemical Structure | Name | LRMS: (ESI + ve ion) *m*/*z* |
|---|---|---|---|
| 101-1 | 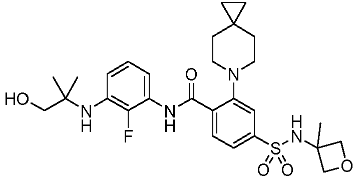 | *N*-(2-fluoro-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide | 561.2 |
| 101-2 | 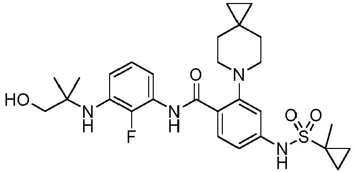 | *N*-(2-fluoro-3-((1-hydroxy-2-methylpropan-2-yl)amino)phenyl)-4-((1-methylcyclopropane)-1-sulfonamido)-2-(6-azaspiro[2.5]octan-6-yl)benzamide | 545.4 |
| 101-3 | 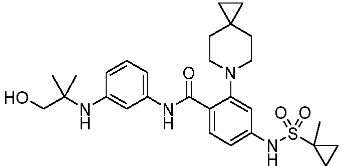 | *N-*(3-((1-hydroxy-2-methylpropan-2-yl)amino)phenyl)-4-((1-methylcyclopropane)-1-sulfonamido)-2-(6-azaspiro[2.5]octan-6-yl)benzamide | 527.2 |

### Example 102: N-(3-(N-(tert-Butyl)sulfamovl)phenyl)-4-((1-methylcyclopropane)-1-sulfonamido)-2-(6-azaspiro[2.5]octan-6-yl)benzamide.

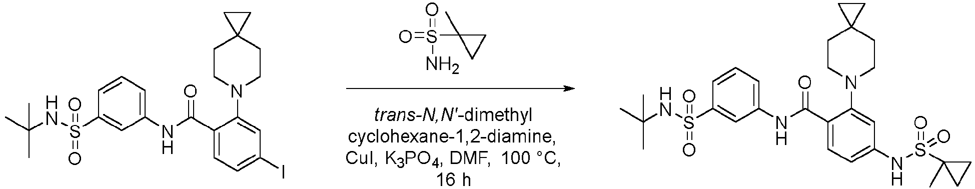

A 250-mL glass tube was charged with a solution of*N*-(3-(*N*-(tert-butyl)sulfamoyl)phenyl)-4-iodo-2-(6-azaspiro[2.5]octan-6-yl)benzamide (15.0 g, 26.4 mmol, Intermediate 19) and DMF (100 mL). To this solution potassium phosphate tribasic (16.83 g, 79.0 mmol), copper(I) iodide (1.26 g, 6.61 mmol), trans-*N,N*'-dimethylcyclohexane-1,2-diamine (1.0 mL, 6.61 mmol), and 1-methylcyclopropane-1-sulfonamide (4.3 g, 31.7 mmol) were added. The reaction mixture was degassed and purged with nitrogen for 10 min. The tube was sealed and stirred at 100 °C for 16 h. The reaction mixture was cooled to RT and quenched with satd. aqueous NH₄Cl solution (100 mL). The reaction mixture was extracted with DCM (3 x 50 mL) and washed with water (50 mL). The organic layer was dried over Na₂SO₄, filtered, and concentrated under reduced pressure. The crude residue was purified by silica gel silica gel chromatography eluting with a gradient of 40% EtOAc in hexanes to give a brown solid. This solid was further purified by triturating with heptane (75 mL) and EtOAc (25 mL) to give the title compound (10.0 g, 66% yield) as a white solid. ¹H NMR (400 MHz, DMSO-*d*₆): *δ* 11.63 (s, 1H), 10.16 (s, 1H), 8.34 (d, *J* = 2.1 Hz, 1H), 7.91 (dt, *J =* 8.0, 1.7 Hz, 1H), 7.78 (d, *J=* 8.4 Hz, 1H), 7.62 - 7.52 (m, 3H), 7.22 (d, *J =* 2.1 Hz, 1H), 7.05 (dd, *J=* 8.4, 2.0 Hz, 1H), 2.96 (t, *J =* 5.4 Hz, 4H), 1.48 (d, *J =* 5.6 Hz, 4H), 1.42 (s, 3H), 1.21 (q, *J =* 4.5 Hz, 2H), 1.12 (s, 9H), 0.89 - 0.80 (m, 2H), 0.33 (s, 4H). *m*/*z* (ESI): 575.2 [M+1].

**Table 9: Examples 102-1 to 102-6 were prepared analogous to preparation of Example 102:**

| x. # | Chemical Structure | Name | LRMS: (ESI + ve ion) *m*/*z* |
|---|---|---|---|
| 102-1 | 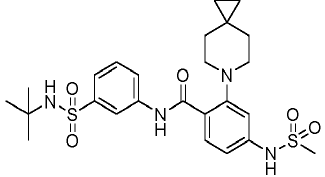 | *N-*(3-(*N-*(*tert-*butyl)sulfamoyl)phenyl)-4-(methylsulfonamido)-2-(6-azaspiro[2.5]octan-6-yl)benzamide | 535.2 |
| 102-2 | 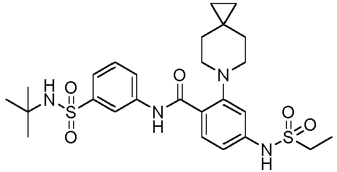 | *N-*(3-(*N-*(*tert-*butyl)sulfamoyl)phenyl)-4-(ethylsulfonamido)-2-(6-azaspiro[2.5]octan-6-yl)benzamide | 549.2 |
| 102-3 | 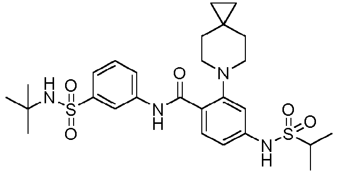 | *N-*(3-(*N-*(*tert-*butyl)sulfamoyl)phenyl)-4-((1-methylethyl)sulfonamido)-2-(6-azaspiro[2.5]octan-6-yl)benzamide | 563.2 |
| 102-4 | 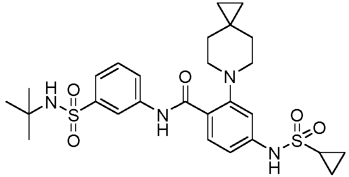 | *N-*(3-(*N-*(*tert-*butyl)sulfamoyl)phenyl)-4-(cyclopropanesulfonamido) -2-(6-azaspiro[2.5]octan-6-yl)benzamide | 561.2 |
| 102-5 | 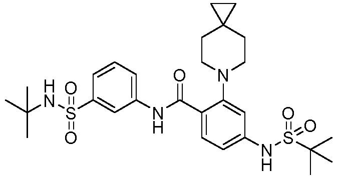 | *N-*(3-(*N-*(*tert-*butyl)sulfamoyl)phenyl)-4-((1,1-dimethylethyl)sulfonamido) -2-(6-azaspiro[2.5]octan-6-yl)benzamide | 577.2 |
| 102-6 | 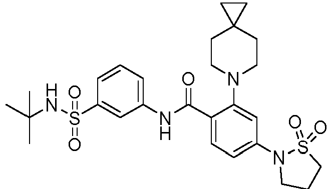 | *N-*(3-(*N-*(*tert-*butyl)sulfamoyl)phenyl)-4-(1,1-dioxidoisothiazolidin-2-yl)-2-(6-azaspiro [2. 5]octan-6-yl)benzamide | 561.2 |

### Example 103: N-(3-(N-(tert-Butyl)sulfamoyl)phenyl)-4-((2-hydroxyethyl)sulfonamido)-2-(6-azaspiro[2.5]octan-6-yl)benzamide.

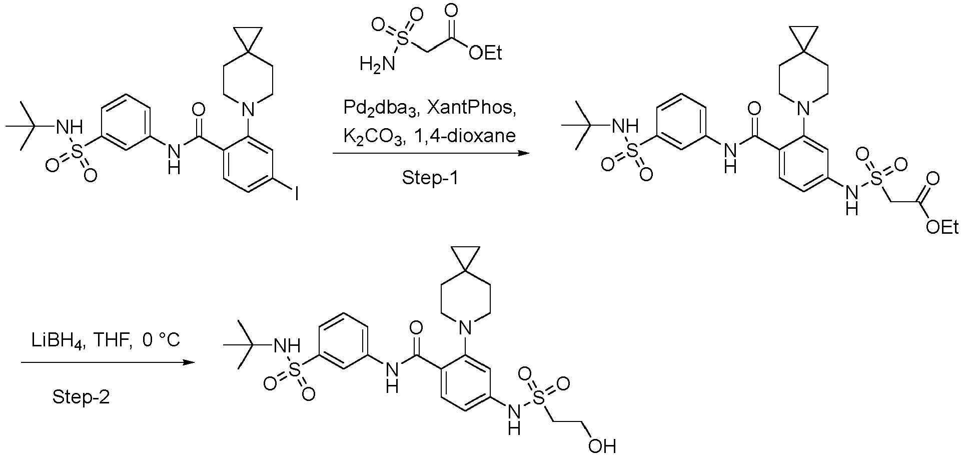

Step 1: In a sealed tube (10 mL) *N*-(3-(*N*-(*tert*-butyl)sulfamoyl)phenyl)-4-iodo-2-(6-azaspiro[2.5]octan-6-yl)benzamide (0.25 g, 0.44 mmol, Intermediate 19) in dioxane (2.5 mL) was taken. To this solution at RT, was added ethyl 2-sulfamoylacetate (0.15 g, 0.88 mmol) and potassium carbonate (0.15 g, 1.10 mmol). The reaction mixture was degassed and purged with nitrogen for 10 min. To this was added Xantphos (0.013 g, 0.022 mmol) and Pd₂dba₃ (0.020 g, 0.022 mmol). The reaction tube was sealed and stirred at 110 °C for 16 h. The reaction mixture was quenched with water (20 mL) and extracted with EtOAc (2 x 50 mL). The organic layer was washed with brine (10 mL), dried over Na₂SO₄, filtered, and concentrated under reduced pressure. The crude residue was purified by column chromatography over silica gel (60-120 mesh) using 50% EtOAc in hexanes to give ethyl 2-(*N-*(4-((3-(*N-*(*tert-*butyl)sulfamoyl)phenyl)carbamoyl)-3-(6-azaspiro[2.5]octan-6-yl)phenyl)sulfamoyl)acetate (100 mg, 37% yield) as a brown spongy solid. ¹H NMR (400 MHz, DMSO-*d*₆): *δ* 11.50 (s, 1H), 10.45 (s, 1H), 8.32 (d, *J* = 8.0 Hz, 1H), 7.90 - 7.70 (m, 1H), 7.54 - 7.44 (m, 3H), 7.12 - 6.97 (m, 2H), 4.33 (s, 2H), 4.09 (q, *J =* 7.08 Hz, 2H), 3.07 (s, 1H), 3.00 (b s, 4H), 1.47 (b s, 4H), 1.20 - 1.02 (m, 12H), 0.33 (s, 4H). *m*/*z* (ESI): 607.2 [M+1].

Step 2: To a solution of ethyl 2-(*N*-(4-((3-(*N*-(*tert*-butyl)sulfamoyl)phenyl)carbamoyl)-3-(6-azaspiro[2.5]octan-6-yl)phenyl)sulfamoyl)acetate (0.18 g, 0.297 mmol) in THF (4 mL) was added lithium borohydride (2.0 M in THF, 0.22 mL, 0.445 mmol) at 0 °C and stirred for 1 h. The reaction mixture was quenched with satd. aqueous NH₄Cl solution (10 mL) and extracted with EtOAc (2x 15 mL). The combined organic extracts were washed with satd. brine solution (10 mL), dried over Na₂SO₄, filtered, and concentrated under reduced pressure. The crude residue was purified by column chromatography over silica gel using 50% EtOAc in hexanes to give *N*-(3-(*N*-(*tert*-butyl)sulfamoyl)phenyl)-4-((2-hydroxyethyl)sulfonamido)-2-(6-azaspiro[2.5]octan-6-yl)benzamide (80 mg, 48% yield) as an off-white solid. ¹H NMR (400 MHz, DMSO-*d*₆): *δ* 11.56 (s, 1H), 10.10 (s, 1H), 8.34 (s, 1H), 7.90 (d, *J =* 7.52 Hz, 1H), 7.77 (d, *J=* 8.48 Hz, 1H), 7.60 - 7.51 (m, 3H), 7.14 (s, 1H), 7.01 (d, *J =* 8.48 Hz, 1H), 4.98 (t*, J =* 5.44 Hz, 1H), 3.76 (dt, *J=* 6.24, 5.44 Hz, 2H), 3.34 (t, *J =* 6.24 Hz, 2H), 2.98 (br s, 4H), 1.48 (br s, 4H), 1.12 (s, 9H), 0.32 (s, 4H). *m*/*z* (ESI): 565.2.1 [M+1].

**Table 10: Examples 103-1 to 103-4 were prepared analogous to preparation of Example 103:**

| Ex. # | Chemical Structure | Name | LRMS: (ESI + ve ion) *m*/*z* |
|---|---|---|---|
| 103-1 | 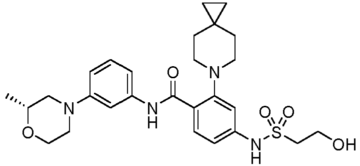 | (*R*)-4-((2-hydroxyethyl)sulfona mido)-*N*-(3-(2-methylmorpholino)phe nyl)-2-(6-azaspiro[2.5]octan-6-yl)benzamide | 529.2 |
| 103-2 | 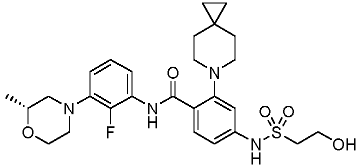 | (*R*)-*N*-(2-fluoro-3-(2-methylmorpholino)phe nyl)-4-((2-hydroxyethyl)sulfona mido)-2-(6-azaspiro[2.5]octan-6-yl)benzamide | 547.2 |
| 103-3 | 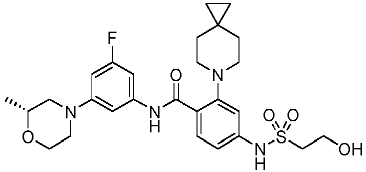 | (*R*)-*N*-(3-fluoro-5-(2-methylmorpholino)phe nyl)-4-((2-hydroxyethyl)sulfona mido)-2-(6-azaspiro[2.5]octan-6-yl)benzamide | 547.2 |
| 103-4 | 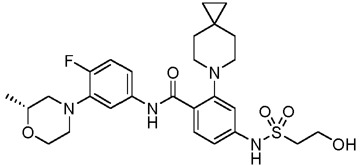 | (*R*)-*N*-(4-fluoro-3-(2-methylmorpholino)phe nyl)-4-((2-hydroxyethyl)sulfona mido)-2-(6-azaspiro[2.5]octan-6-yl)benzamide | 547.2 |

### Example 104: N-(3-(4,4-difluoropiperidin-1-yl)-5-methylphenyl)-4-((2-hydroxyethyl)sulfonamido)-2-(6-azaspiro[2.5]octan-6-yl)benzamide.(REFERENCE EXAMPLE)

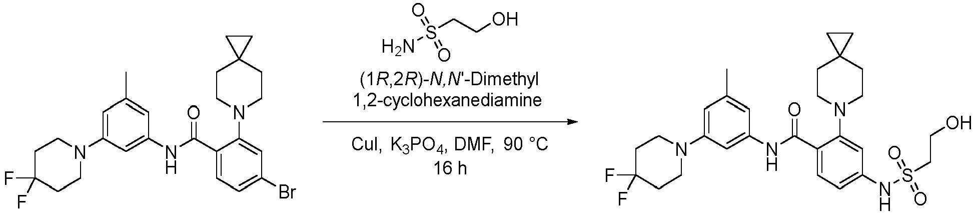

A mixture of 4-bromo-*N*-(3-(4,4-difluoropiperidin-1-yl)-5-methylphenyl)-2-(6-azaspiro[2.5]octan-6-yl)benzamide (0.5 g, 0.96 mmol, Intermediate 20), potassium phosphate (0.614 g, 2.89 mmol), 2-hydroxyethane-1-sulfonamide (0.181 g, 1.45 mmol), (1*R*,2*R*)-*N*1,*N*2-dimethylcyclohexane-1,2-diamine (0.069 g, 0.48 mmol) and copper(I) iodide (0.092 g, 0.48 mmol) in DMF (5 mL) was stirred at 90 °C for 16 h. The reaction mixture was quenched with ice water, filtered through a CELITE^{®} bed, and extracted with EtOAc. The organic extract was washed with brine, dried over Na₂SO₄, filtered, concentrated, and purified by silica gel column chromatography using 40% EtOAc in petroleum ether to provide *N*-(3-(4,4-difluoropiperidin-1-yl)-5-methylphenyl)-4-((2-hydroxyethyl)sulfonamido)-2-(6-azaspiro[2.5]octan-6-yl)benzamide (0.31 g, 0.54 mmol, 56% yield) as an off-white solid. ¹H NMR (400 MHz, DMSO-*d*₆): δ ppm 11.55 (s, 1 H), 10.09 (s, 1 H), 7.83 (d, *J=*8.5 Hz, 1 H), 7.12 - 7.16 (m, 3 H), 7.03 (dd, *J=*8.5, 2.1 Hz, 1 H), 6.60 (s, 1 H), 4.97 (br s, 1 H), 3.76 (t, *J*=6.6 Hz, 2 H), 3.30 - 3.34 (m, 6 H), 2.97 (t, *J=*5.3 Hz, 4 H), 2.27 (s, 3 H), 2.00 - 2.10 (m, 4 H), 1.55 (br s, 4 H), 0.36 (s, 4 H). *m*/*z* (ESI): 563.2 (M+H)⁺.

**Table 11: Examples 104-1 to 104-2 were prepared analogous to preparation of Example 104:**

| Ex. # | Chemical Structure | Name | LRMS: (ESI + ve ion) *m*/*z* |
|---|---|---|---|
| 104-1 (REF. EX) | 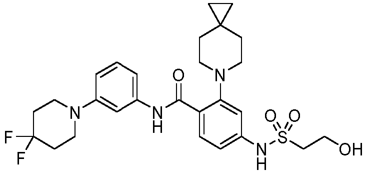 | *N*-(3-(4,4-difluoropiperidin-1-yl)phenyl)-4-((2-hydroxyethyl)sulfonamid o)-2-(6-azaspiro[2.5]octan-6-yl)benzamide | 549.2 |
| 104-2 (REF. EX) | 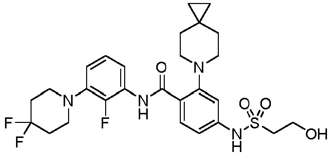 | *N*-(3-(4,4-difluoropiperidin-1-yl)-2-fluorophenyl)-4-((2-hydroxyethyl)sulfonamid o)-2-(6-azaspiro[2.5]octan-6-yl)benzamide | 567.2 |

### Examples 105-1 and 105-2: (S)-N-(3-(N-(tert-butyl)sulfamoyl)phenyl)-4-((2-hydroxy-1-methylethyl)sulfonamido)-2-(6-azaspiro[2.5]octan-6-yl)benzamide and (R)-N-(3-(N-(tert-butyl)sulfamoyl)phenyl)-4-((2-hydroxy-1-methylethyl)sulfonamido)-2-(6-azaspiro[2.5]octan-6-yl)benzamide.

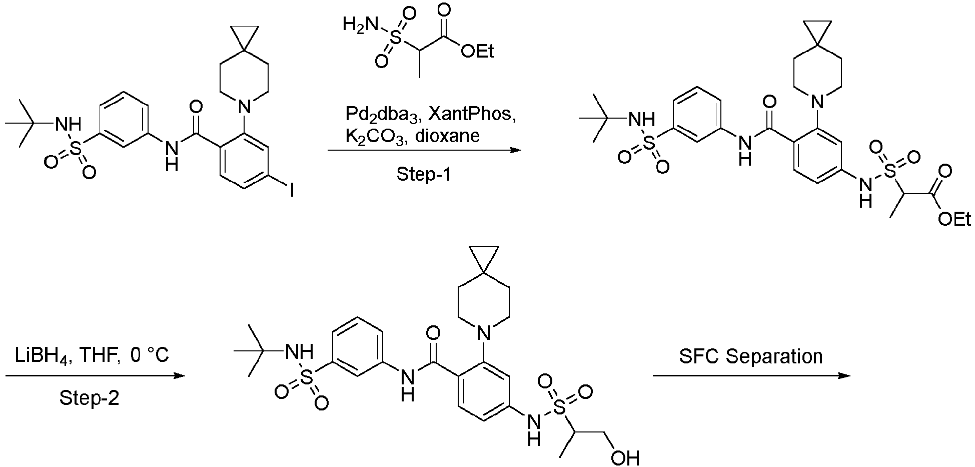

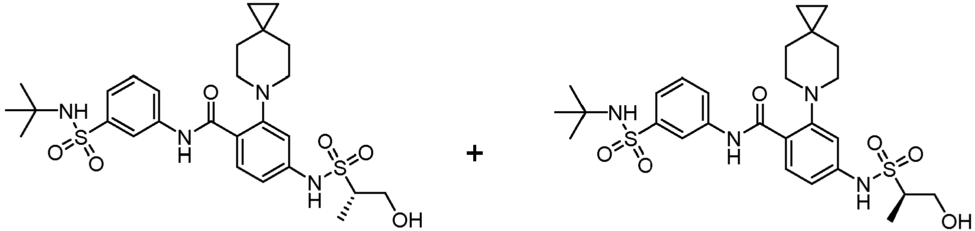

Step 1: A glass tube was charged with *N*-(3-(*N*-(*tert*-butyl)sulfamoyl)phenyl)-4-iodo-2-(6-azaspiro[2.5]octan-6-yl)benzamide (1.0 g, 1.76 mmol, Intermediate 19) and dioxane (10 mL). To this solution, was added ethyl 2-sulfamoylpropanoate (0.64 g, 3.52 mmol, Intermediate 28) and potassium carbonate (0.61 g, 4.41 mmol). The reaction mixture was degassed and purged nitrogen for 10 min. Xantphos (0.051 g, 0.088 mmol) and Pd₂dba₃ (0.081 g, 0.088 mmol) were added to the reaction mixture. The reaction vessel was sealed and stirred at 110 °C for 16 h. The reaction mixture was quenched with water (50 mL) and extracted with EtOAc (2 x 50 mL). The combined organic extracts were washed with brine (50 mL), dried over Na₂SO₄, filtered, and concentrated under reduced pressure. The crude residue was purified by column chromatography over silica gel using 50% EtOAc in hexanes to give ethyl 2-(N-(4-((3-(*N*-(*tert*-butyl)sulfamoyl)phenyl)carbamoyl)-3-(6-azaspiro[2.5]octan-6-yl)phenyl)sulfamoyl)propanoate

(400 mg, 38% yield) as a brown solid. ¹H NMR (400 MHz, Chloroform-d): *δ* 8.28 (d, *J =* 8.1 Hz, 1H), 7.98 (d, *J =* 8.1 Hz, 1H), 7.66 (t, *J=* 9.0 Hz, 2H), 7.54 - 7.50 (m, 2H), 7.39 (s, 1H), 7.21 (s, 1H), 7.14 (d, *J=* 8.3 Hz, 1H), 6.79 (d, *J =* 93.0 Hz, 1H), 4.59 (d, *J =* 9.7 Hz, 1H), 4.28 (dd, *J =* 8.0, 5.6 Hz, 2H), 3.83 - 3.66 (m, 8H), 3.10 (d, *J =* 5.3 Hz, 3H), 1.29 (b s, 12H), 0.45 (s, 4H). *m*/*z* (ESI): 621.2 [M+1].

Step 2: To a solution of 2-(*N*-(4-((3-(*N*-(*tert*-butyl)sulfamoyl)phenyl)carbamoyl)-3-(6-azaspiro[2.5]octan-6-yl)phenyl)sulfamoyl)propanoate (0.4 g, 0.64 mmol) in THF (4 mL) was added lithium borohydride 2M in THF (0.387 mL, 0.773 mmol) at 0 °C and stirred for 2h. The reaction mixture was quenched with sat.NH₄Cl solution (25 mL) and extracted with EtOAc (2x 50 mL).The combined organic extracts were washed with satd. brine solution (20 mL), dried over Na₂SO₄, filtered, and concentrated under reduced pressure. The crude was purified by column chromatography over silica gel using 50-100% EtOAc in hexanes to give *N*-(3-(*N*-(*tert*-butyl)sulfamoyl)phenyl)-4-((2-hydroxy-1-methylethyl)sulfonamido)-2-(6-azaspiro[2.5]octan-6-yl)benzamide (300 mg, 80% yield) as an off-white solid. ¹H NMR (400 MHz, DMSO-*d*₆): *δ* 11.53 (s, 1H), 10.11 (s, 1H), 8.34 (d, *J =* 1.9 Hz, 1H), 7.90 (dt, *J =* 7.8, 1.8 Hz, 1H), 7.76 (d, *J =* 8.5 Hz, 1H), 7.66 - 7.47 (m, 3H), 7.16 (d, *J=* 2.1 Hz, 1H), 7.03 (dd, *J=* 8.4, 2.1 Hz, 1H), 5.06 (d, *J=* 6.6 Hz, 1H), 3.85 (dd, *J* = 9.8, 5.2 Hz, 1H), 3.49 (s, 1H), 3.25 (dt, *J =* 7.6, 3.9 Hz, 1H), 3.04 - 2.92 (m, 4H), 1.48 (b s, 4H), 1.30 (d, *J =* 6.8 Hz, 3H), 1.12 (s, 9H), 0.32 (s, 4H). *m*/*z* (ESI): 579.1 [M+1]. The racemic material was via preparative SFC using a Chiralpak AD-H (250 × 30 mm, 5 µm) with a mobile phase of 75% Liquid CO₂ and 25% MeOH and a flow rate of 100 mL/min to generate the following compounds of Examples 105-1 and 105-2:

### Example 105-1: (S)-N-(3-(N-(tert-butyl)sulfamoyl)phenyl)-4-((2-hydroxy-1-methylethyl)sulfonamido)-2-(6-azaspiro[2.5]octan-6-yl)benzamide.

First eluting peak, ee 100%; ¹H NMR (400 MHz, DMSO-*d*₆): *δ* 11.53 (s, 1H), 10.11 (s, 1H), 8.34 (d, *J =* 1.9 Hz, 1H), 7.90 (dt, *J* = 7.8, 1.8 Hz, 1H), 7.76 (d, *J =* 8.5 Hz, 1H), 7.66 - 7.47 (m, 3H), 7.16 (d, *J* = 2.1 Hz, 1H), 7.03 (dd, *J =* 8.4, 2.1 Hz, 1H), 5.06 (d, *J =* 6.6 Hz, 1H), 3.85 (dd, *J =* 9.8, 5.2 Hz, 1H), 3.49 (s, 1H), 3.25 (dt, *J =* 7.6, 3.9 Hz, 1H), 3.04 - 2.92 (m, 4H), 1.48 (b s, 4H), 1.30 (d, *J =* 6.8 Hz, 3H), 1.12 (s, 9H), 0.32 (s, 4H). *m*/*z* (ESI): 579.1 [M+1].

### Example 105-2: (R)-N-(3-(N-(tert-butyl)sulfamoyl)phenyl)-4-((2-hydroxy-1-methylethyl)sulfonamido)-2-(6-azaspiro[2.5]octan-6-yl)benzamide.

Second eluting peak, ee 97.5%; ¹H NMR (400 MHz, DMSO-*d*₆): *δ* 11.53 (s, 1H), 10.11 (s, 1H), 8.34 (d, *J=* 1.9 Hz, 1H), 7.90 (dt, *J =* 7.8, 1.8 Hz, 1H), 7.76 (d, *J =* 8.5 Hz, 1H), 7.66 - 7.47 (m, 3H), 7.16 (d, *J=* 2.1 Hz, 1H), 7.03 (dd, *J =* 8.4, 2.1 Hz, 1H), 5.06 (d, *J =* 6.6 Hz, 1H), 3.85 (dd, *J =* 9.8, 5.2 Hz, 1H), 3.49 (s, 1H), 3.25 (dt, *J=* 7.6, 3.9 Hz, 1H), 3.04 - 2.92 (m, 4H), 1.48 (b s, 4H), 1.30 (d, *J =* 6.8 Hz, 3H), 1.12 (s, 9H), 0.32 (s, 4H). *m*/*z* (ESI): 579.1 [M+1].

The stereochemistry assignments were arbitrary.

### Example 106: N-(3-(2-Hydroxy-2-methylpropoxy)phenyl)-4-((2-hydroxyethyl)sulfonamido)-2-(6-azaspiro[2.5]octan-6-yl)benzamide.

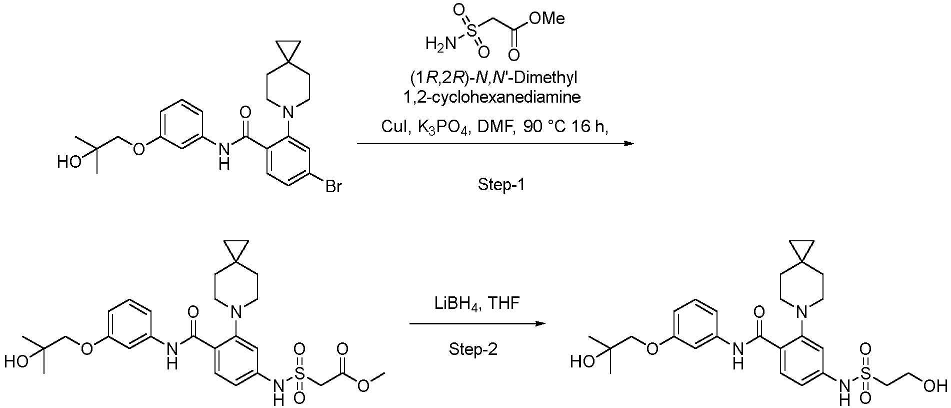

Step 1: A mixture of 4-bromo-*N*-(3-(2-hydroxy-2-methylpropoxy)phenyl)-2-(6-azaspiro[2.5]octan-6-yl)benzamide (0.260 g, 0.549 mmol, Intermediate 27), methyl 2-sulfamoylacetate (0.126 g, 0.82 mmol), potassium phosphate tribasic (0.233 g, 1.10 mmol), copper(I) iodide (0.105 g, 0.55 mmol) and (1*R*,2*R*)-*N*, *N'*-dimethyl-1,2-cyclohexanediamine (0.039 g, 0.27 mmol) in DMF (5 mL) was stirred at 90 °C for 16 h. The reaction mixture was diluted with water and extracted with EtOAc. The organic extract was washed with brine, dried over Na₂SO₄, filtered, and concentrated. The concentrate was purified by silica gel column chromatography eluting with 40-50 % EtOAc in petroleum ether to provide methyl 2-(*N*- (4-((3-(2-hydroxy-2-methylpropoxy)phenyl)carbamoyl)-3-(6-azaspiro[2.5]octan-6-yl)phenyl)sulfamoyl)acetate (0.260 g, 0.476 mmol, 87% yield) as a light yellow oil. ¹H NMR (300 MHz, DMSO-*d*₆): δ ppm 11.53 (s, 1 H), 10.53 (s, 1 H), 7.95 (s, 1 H), 7.82 (d, *J*=8.5 Hz, 1 H), 7.59 (s, 1 H), 7.27 (t, *J*=8.0 Hz, 1 H), 7.14 - 7.20 (m, 1 H), 6.96 - 7.07 (m, 1 H), 6.67 (d, *J*=8.3 Hz, 1 H), 4.65 (s, 1 H), 4.36 (s, 2 H), 3.70 (s, 2 H), 3.65 (s, 3 H), 2.95 - 3.02 (m, 4 H), 1.53 (br s, 4 H), 1.21 (s, 6 H), 0.35 (s, 4 H). *m*/*z* (ESI): 546.2 (M+H)⁺.

Step 2: To a solution of methyl 2-(*N*-(4-((3-(2-hydroxy-2- methylpropoxy)phenyl)carbamoyl)-3-(6-azaspiro[2.5]octan-6-yl)phenyl)sulfamoyl)acetate (0.260 g, 0.476 mmol) in THF (10 mL), was added lithium borohydride (0.95 mL, 1.91 mmol) at 0 °C and the reaction mixture was stirred at RT for 1 h. The reaction mixture was quenched with a satd. aqueous solution of NH₄Cl and extracted with EtOAc. The organic extract was washed with brine, dried over Na₂SO₄, filtered, and was concentrated. The concentrate was purified by silica gel column chromatography eluting with 45-50% EtOAc in petroleum ether to provide *N*-(3-(2-hydroxy-2-methylpropoxy)phenyl)-4-((2-hydroxyethyl)sulfonamido)-2-(6-azaspiro[2.5]octan -6-yl)benzamide (0.032 g, 0.062 mmol, 13% yield) as a white solid. ¹H NMR (400 MHz, DMSO-*d*₆): δ 11.54 (s, 1 H), 10.08 (s, 1 H), 7.81 (d, *J =* 8.5 Hz, 1 H), 7.47 (t, *J =* 2.1 Hz, 1 H), 7.27 (t, *J =* 8.2 Hz, 1 H), 7.19 - 7.10 (m, 2 H), 7.01 (dd, *J=* 8.5, 2.1 Hz, 1 H), 6.75 - 6.64 (m, 1 H), 4.95 (s, 1 H), 4.65 (s, 1 H), 3.82 - 3.62 (m, 4 H), 2.94 - 3.01 (m, 4 H), 1.52 (br s, 4 H), 1.40 - 1.46 (m, 2 H), 1.21 (s, 6 H), 0.34 (s, 4 H). *m*/*z* (ESI): 518.2 (M+H)⁺.

### Example 107: 4-(Azetidin-1-ylsulfonyl)-N-(3-(N-(tert-butyl)sulfamoyl)phenyl)-2-(6-azaspiro[2.5]octan-6-yl)benzamide.

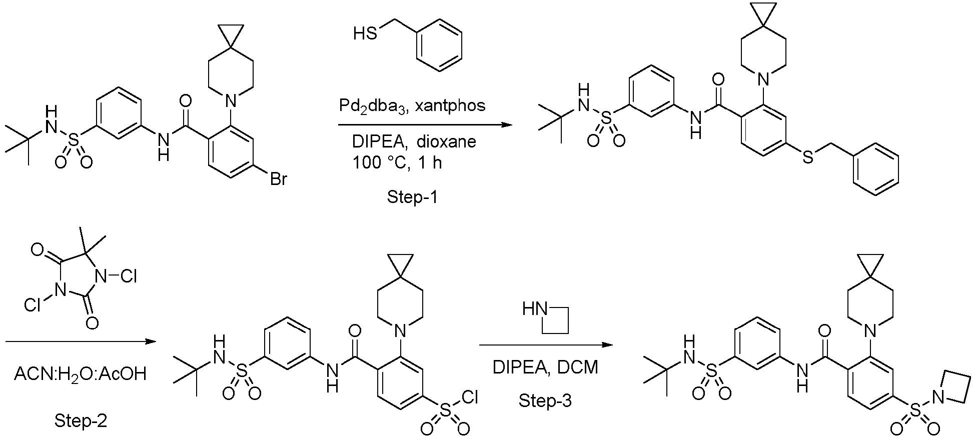

Step 1: A 250-mL round-bottomed flask was charged with 4-bromo-*N*-(3-(*N*-(*tert-*butyl)sulfamoyl)phenyl)-2-(6-azaspiro[2.5]octan-6-yl)benzamide (3.98 g, 7.65 mmol, Intermediate 19-1), xantphos (0.227 g, 0.39 mmol), Pd₂dba₃ (0.18 g, 0.2 mmol) and dioxane (25 mL) under Ar atmosphere. While the mixture was degassed by bubbling Ar through, DIPEA (2.96 mL, 22.94 mmol) was added followed by benzyl mercaptan (0.95 mL, 8.03 mmol). The reaction mixture was heated at 100 °C for 1 h. The mixture was cooled to rt and partitioned between water (40 mL) and EtOAc (30 mL). The aqueous phase was extracted with EtOAc (20 mL). The combined organic extracts were washed with water (30 mL). The organic phase was dried by passing through a Chem Elut extraction cartridge eluting with EtOAc (2 x 10 mL) and the solvent was removed under vacuum. The crude product was carried forward to next step. ¹H NMR (400 MHz, CHLOROFORM-d) δ 12.78 (s, 1H), 8.28 (s, 1H), 8.18 (d, *J*=8.22 Hz, 1H), 7.98 (dd, *J*=1.08, 8.12 Hz, 1H), 7.63 (d, *J*=7.82 Hz, 1H), 7.45-7.52 (m, 1H), 7.28-7.40 (m, 5H), 7.22 (dd, *J*=1.66, 8.31 Hz, 1H), 7.17 (d, *J*=1.56 Hz, 1H), 4.56 (s, 1H), 4.22 (s, 2H), 2.99 (t, *J*=5.28 Hz, 4H), 1.60-1.67 (m, 4H), 1.28 (s, 9H), 0.43 (s, 4H). in DMSO-d6: ¹H NMR (400 MHz, DMSO-d₆) δ 11.62 (s, 1H), 8.32 (s, 1H), 7.88 (br d, *J*=7.24 Hz, 1H), 7.70 (d, *J*=8.61 Hz, 1H), 7.50-7.59 (m, 3H), 7.44 (d, *J=*7.24 Hz, 2H), 7.33 (t, *J*=7.34 Hz, 2H), 7.22-7.29 (m, 1H), 7.14 (dd, *J*=2.54, 4.11 Hz, 2H), 4.36 (s, 2H), 2.96 (br t, *J*=4.99 Hz, 4H), 1.45 (br s, 4H), 1.11 (s, 9H), 0.31 (s, 4H).

Step 2: A 100-mL round-bottomed flask was charged with 4-(benzylthio)-*N-*(3-(*N*-(*tert-*butyl)sulfamoyl)phenyl)-2-(6-azaspiro[2.5]octan-6-yl)benzamide (1.01 g, 1.781 mmol), ACN (8 mL), water (0.2 mL) and acetic acid (0.3 mL). The mixture was cooled in an ice-water bath and 1,3-dichloro-5,5-dimethylhydantoin (0.57 g, 2.91 mmol) was added in portions. The mixture was stirred at 0 °C for 20 min before adding satd. NaHCO₃ (20 mL) and extracting with EtOAc (2 x 20 mL). The combined organic extracts were washed with brine and dried by passing through a Chem Elut extraction cartridge eluting with EtOAc (2 x 10 mL). The organic extracts were concentrated and purified by silica gel chromatography 2-50% EtOAc in hexanes to afford 4-((3-(*N*-(*tert*-butyl)sulfamoyl)phenyl)carbamoyl)-3-(6-azaspiro[2.5]octan-6-yl)benzenesulfonyl chloride. ¹H NMR (400 MHz, CHLOROFORM-d) δ 12.14 (s, 1H), 8.48 (d, *J=*8.22 Hz, 1H), 8.30 (s, 1H), 7.91-8.00 (m, 3H), 7.69 (d, *J*=7.82 Hz, 1H), 7.51-7.57 (m, 1H), 4.62 (s, 1H), 3.18 (t, *J*=5.18 Hz, 4H), 1.67 (br s, 4H), 1.29 (s, 9H), 0.47 (s, 4H).

Step 3: A glass vial was charged with 4-((3-(*N*-(*tert*-butyl)sulfamoyl)phenyl)carbamoyl)-3-(6-azaspiro[2.5]octan-6-yl)benzene-1-sulfonyl chloride (0.085 g, 0.157 mmol), DIPEA (0.082 mL, 0.47 mmol), azetidine (0.013 mL, 0.19 mmol) and DCM (1 mL). The mixture was stirred at rt for 20 min before concentrating. The crude material was purified by Biotage (SNAP25, Ultra, eluent 20-80% EtOAc in heptane) then lyophilized to obtain 0.071 g of the title compound as a white solid. ¹H NMR (400MHz, DMSO-d₆) δ = 11.05 (s, 1H), 8.33 (s, 1H), 7.98 - 7.91 (m, 1H), 7.87 (d, *J*=7.9 Hz, 1H), 7.58 (br d, *J=*6.2 Hz, 3H), 7.50 (d, *J*=8.1 Hz, 1H), 7.43 (s, 1H), 3.73 (t, *J*=7.6 Hz, 4H), 3.10 (br t, *J*=4.7 Hz, 4H), 2.03 (quin, *J*=7.7 Hz, 2H), 1.41 (br s, 4H), 1.12 (s, 9H), 0.28 (s, 4H).

**Table 12: Examples 107-1 to 107-8 were prepared analogous to preparation of Example 107:**

| Ex. # | Chemical Structure | Name | LRMS: (ESI + ve ion) *m*/*z* |
|---|---|---|---|
| 107-1 | 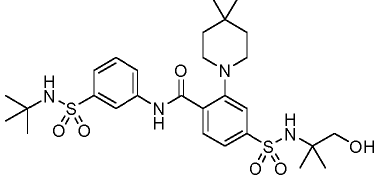 | *N-*(3-(*N-*(*tert-*butyl)sulfamoyl)phenyl)-4-(*N*-(1-hydroxy-2-methylpropan-2-yl)sulfamoyl)-2-(6-azaspiro[2.5]octan-6-yl)benzamide | 593.2 |
| 107-2 | 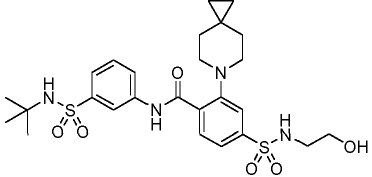 | *N-*(3-(*N-*(*tert-*butyl)sulfamoyl)phenyl)-4-(*N-*(2-hydroxyethyl)sulfamoyl)-2-(6-azaspiro[2.5]octan-6-yl)benzamide | 565.2 |
| 107-3 | 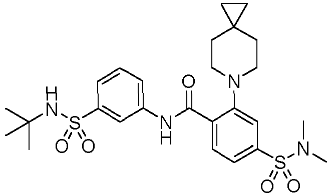 | *N-*(3-(*N-*(*tert-*butyl)sulfamoyl)phenyl)-4-(*N,N*-dimethylsulfamoyl)-2-(6-azaspiro[2.5]octan-6-yl)benzamide | 549.2 |
| 107-4 | 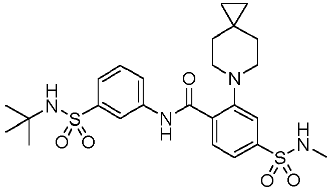 | *N-*(3-(*N-*(*tert-*butyl)sulfamoyl)phenyl)-4-(*N*-methylsulfamoyl)-2-(6-azaspiro[2.5]octan-6-yl)benzamide | 535.2 |
| 107-5 | 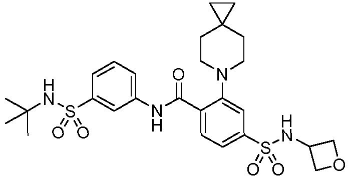 | *N-(*3-(*N-*(*tert-*butyl)sulfamoyl)phenyl)-4-(*N*-(oxetan-3-yl)sulfamoyl)-2-(6-azaspiro[2.5]octan-6-yl)benzamide | 577.2 |
| 107-6 | 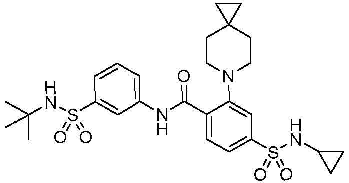 | *N-*(3-(*N-*(*tert-*butyl)sulfamoyl)phenyl)-4-(*N*-cyclopropylsulfamoyl)-2-(6-azaspiro[2.5]octan-6-yl)benzamide | 561.2 |
| 107-7 | 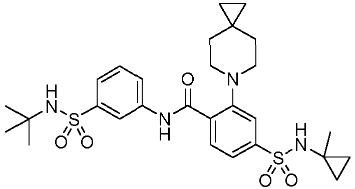 | *N-*(3-(*N-*(*tert-*butyl)sulfamoyl)phenyl)-4-(*N-*(1-methylcyclopropyl)sulfamoy 1)-2-(6-azaspiro[2.5]octan-6-yl)benzamide | 575.2 |
| 107-8 | 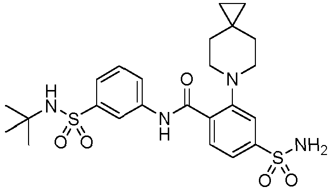 | *N-*(3-(*N-*(*tert-*butyl)sulfamoyl)phenyl)-2-(6-azaspiro[2.5]octan-6-yl)-4-sulfamoylbenzamide | 521.2 |

### Examples 108-1 and 108-2: (R)-N-(3-(N-(tert-butyl)sulfamoyl)phenyl)-4-(1,2-dihydroxypropan-2-yl)-2-(6-azaspiro[2.5]octan-6-yl)benzamide and (S)-N-(3-(N-(tert-butyl)sulfamoyl)phenyl)-4-(1,2-dihydroxypropan-2-yl)-2-(6-azaspiro[2.5]octan-6-yl)benzamide.

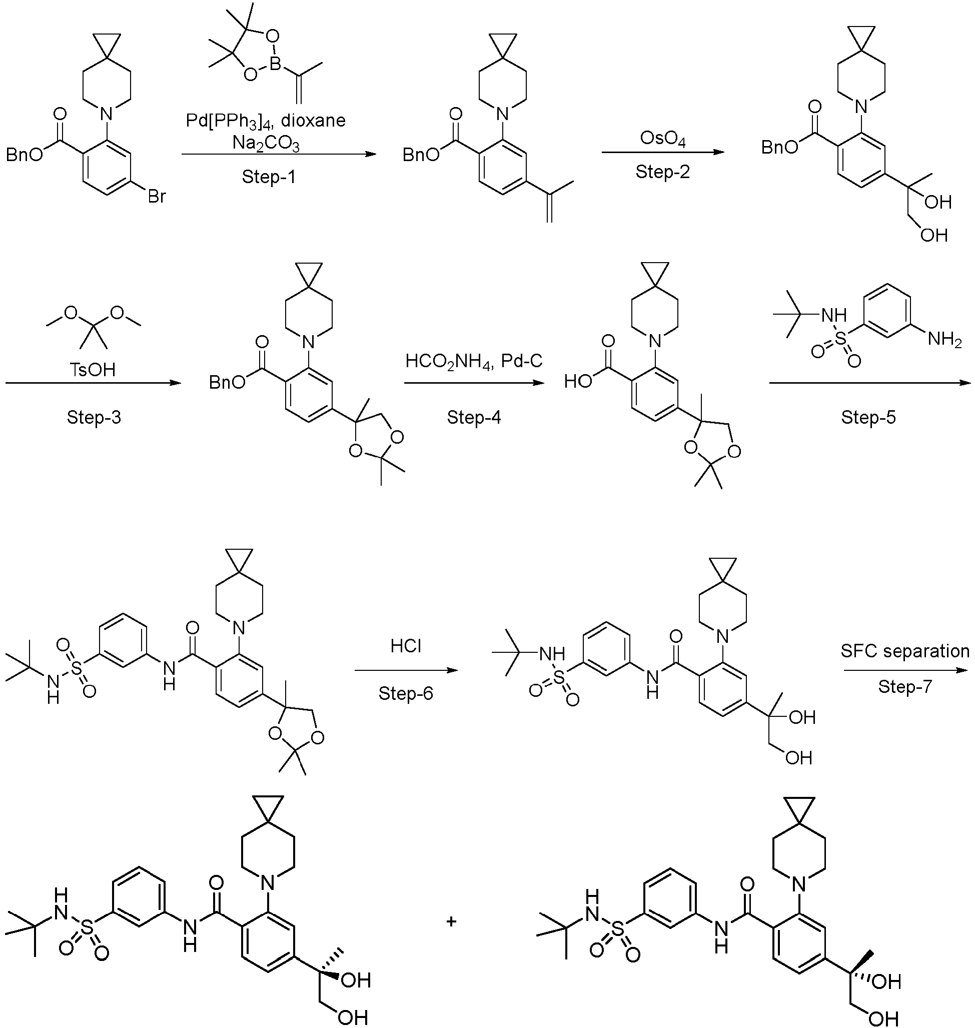

Step 1: A solution of benzyl 4-bromo-2-(6-azaspiro[2.5]octan-6-yl)benzoate (10.0 g, 25 mmol, Intermediate 8-4) in dioxane (100 mL) was taken in a glass tube (250 mL). To this reaction mixture were added 4,4,5,5-tetramethyl-2-(prop-1-en-2-yl)-1,3,2-dioxaborolane (5 g, 27.5 mmol) and aqueous Na₂CO₃ solution (2M solution, 31.2 mL, 62.5 mmol) at RT. The reaction mixture was degassed and purged nitrogen gas for 15 min. Tetrakis(triphenylphosphine)palladium (1.44 g, 1.25 mmol) was added to the reaction mixture and the reaction vessel was sealed and heated at 110 °C for 16 h. The reaction mixture was concentrated under reduced pressure. The crude residue was purified by column chromatography over silica gel using EtOAc in hexanes (0-10%) to give benzyl 4-(prop-1-en-2-yl)-2-(6-azaspiro[2.5]octan-6-yl)benzoate (8.0 g, 89% yield) as a yellow oil. ¹H NMR (400 MHz, Chloroform-*d*): δ 7.72 (d, *J =* 8.12 Hz, 1H), 7.47 (d, *J=* 7.24 Hz, 2H), 7.31 - 7.42 (m, 3H), 7.13 (s, 1H), 7.04 (d, *J=* 8.12 Hz, 1H), 5.40 (s, 1H), 5.36 (s, 2H), 5.14 (s, 1H), 3.03 - 3.13 (m, 4H), 2.14 (s, 3H), 1.44 - 1.51 (m, 4H), 0.31 (s, 4H). *m*/*z* (ESI): 362.2 [M+1].

Step 2: To a solution of benzyl 4-(prop-1-en-2-yl)-2-(6-azaspiro[2.5]octan-6-yl)benzoate (8.0 g, 22.13 mmol) in acetone (80 mL) and water (40 mL) were added 4-methylmorpholine-*N*-oxide (5.19 mL, 44.3 mmol) and osmium tetroxide (4 wt% in H₂O, 3.47 mL, 11.07 mmol) at RT and stirred for 3 h. The reaction mixture was diluted with water (50mL) and extracted with EtOAc (2 x 100 mL). The combined organic extracts were washed with brine solution (50 mL), dried over Na₂SO₄, filtered, and concentrated under reduced pressure to give benzyl 4-(1,2-dihydroxypropan-2-yl)-2-(6-azaspiro[2,5]octan-6-yl)benzoate (9.0 g, crude) as a viscous oil. The crude product was proceeded further without any purification. *m*/*z* (ESI): 396.2 [M+1].

Step 3: To a stirred solution of benzyl 4-(1,2-dihydroxypropan-2-yl)-2-(6-azaspiro[2,5]octan-6-yl)benzoate (9.0 g, 22.70 mmol) in THF (90 mL) were added 2,2-dimethoxypropane (8.34 mL, 68.1 mmol) and *p*-toluenesulfonic acid monohydrate (0.864 g, 4.54 mmol) under nitrogen atmosphere and the reaction mixture was heated at 60 °C for 5 h. The reaction mixture was quenched with water (100 mL) and extracted with EtOAc (2 x 100 mL). The combined organic extracts washed with brine solution (50 mL), dried over Na₂SO₄, filtered, and concentrated under reduced pressure to give benzyl 2-(6-azaspiro[2,5]octan-6-yl)-4-(2,2,4-trimethyl-1,3-dioxolan-4-yl)benzoate (7.0 g, crude) as a yellow viscous oil. The crude product was proceeded further without any purification. *m*/*z* (ESI): 436.2 [M+1].

Step 4: To a stirred solution of benzyl 2-(6-azaspiro[2,5]octan-6-yl)-4-(2,2,4-trimethyl-1,3-dioxolan-4-yl)benzoate (7.0 g, 16.03 mmol) in EtOH were added ammonium formate (2.02 g, 32.1 mmol) and Pd-C (10%, 5.12 g, 4.81 mmol) under N₂ atmosphere and heated at 60 °C for 30 min. The reaction mixture was cooled to RT, filtered through a CELITE^{®} bed and washed with EtOAc (100 mL). The filtrate was concentrated under reduced pressure. The crude residue was extracted with EtOAc (200 mL) and washed with water (100 mL) and brine solution (100 mL). The organic layer was dried over Na₂SO₄, filtered, and concentrated under reduced pressure to give 2-(6-azaspiro[2.5]octan-6-yl)-6-(2,2,4-trimethyl-1,3-dioxolan-4-yl)nicotinic acid (5.0 g, crude) as an off-white solid. The crude product was proceeded further without any purification. ¹H NMR (400 MHz, DMSO-*d₆*): *δ* 8.00 (d, *J =* 8.22 Hz, 1H), 7.73 (s, 1H), 7.48 (dd, *J=* 1.27, 8.12 Hz, 1H), 4.11 - 4.17 (m, 1H), 4.04 - 4.09 (m, 1H), 3.10 (t, *J =* 5.38 Hz, 4H), 1.59 (bs, 4H), 1.52 (s, 3H), 1.45 (s, 3H), 1.31 (s, 3H), 0.44 (s, 4H).

Step 5: To a solution of 2-(6-azaspiro[2.5]octan-6-yl)-6-(2,2,4-trimethyl-1,3-dioxolan-4-yl)nicotinic acid (2.0 g, 5.77 mmol) in DMF (20 mL) were added 3-amino-*N*-(*tert-*butyl)benzenesulfonamide (1.32 g, 5.77 mmol), HATU (2.195 g, 5.77 mmol) and DIPEA (1.01 mL, 5.77 mmol) at rt and stirred for 3 h. The reaction mixture was quenched with water (50 mL) and extracted with EtOAc (2 x 50 mL). The combined organic extracts were washed with brine solution (50 mL), dried over Na₂SO₄, filtered, and concentrated under reduced pressure. The residue was triturated with diethyl ether (50 mL), filtered, and dried under vacuum to give *N*-(3-(*N*-(*tert*-butyl)sulfamoyl)phenyl)-2-(6-azaspiro[2.5]octan-6-yl)-6-(2,2,4-trimethyl-1,3-dioxolan-4-yl)nicotinamide (2.0 g, 62% yield) as an off-white solid. ¹H NMR (400 MHz, Chloroform-*d*): *δ* 12.94 (s, 1H), 8.31 (s, 1H), 8.27 (bs, 1H), 8.01 (d, *J =* 8.22 Hz, 1H), 7.64 (d, *J=* 7.82 Hz, 1H), 7.46 - 7.55 (m, 2H), 7.23 (d, *J=* 8.22 Hz, 1H), 4.54 (d, *J=* 8.61 Hz, 1H), 4.08 - 4.17 (m, 1H), 3.04 - 3.18 (m, 4H), 1.66 (s, 3H), 1.67 - 1.59 (m, 4H), 1.57 (s, 3H), 1.43 (s, 3H), 1.29 (s, 9H), 0.45 (s, 4H). *m*/*z* (ESI): 556.2 [M+1].

Step 6: To a stirred solution of *N*-(3-(*N*-(*tert*-butyl)sulfamoyl)phenyl)-2-(6-azaspiro[2.5]octan-6-yl)-6-(2,2,4-trimethyl-1,3-dioxolan-4-yl)nicotinamide (2.1 g, 3.78 mmol) in dioxane (21 mL) was added hydrochloric acid (2N, 8.89 mL, 37.8 mmol) and stirred at RT for 5 h. The reaction mixture was quenched with satd. aqueous NaHCO₃ solution (50 mL) and extracted with EtOAc (2 x 100 mL). The organic layer was washed with brine solution (50 mL), dried over Na₂SO₄, filtered, and concentrated under reduced pressure. The crude residue was triturated with diethyl ether to afford a solid. The solid was filtered and dried to give *N*-(3-(*N*-(*tert*-butyl)sulfamoyl)phenyl)-4-(1,2-dihydroxypropan-2-yl)-2-(6-azaspiro[2.5]octan-6-yl)benzamide (1.4 g, 72% yield) as an off-white solid. ¹H NMR (400 MHz, DMSO-*d₆*): *δ* 11.95 (s, 1H), 8.35 (s, 1H), 7.90 (d, *J =* 7.43 Hz, 1H), 7.77 (d, *J=* 8.22 Hz, 1H), 7.51 - 7.61 (m, 3H), 7.47 (s, 1H), 7.28 (d, *J =* 8.41 Hz, 1H), 5.04 (s, 1H), 4.73 (t, *J =* 5.77 Hz, 1H), 3.44 (d, *J =* 4.89 Hz, 2H), 3.02 (t, *J* = 4.89 Hz, 4H), 1.49 (s, 4H), 1.41 (s, 3H), 1.12 (s, 9H), 0.33 (s, 4H). *m*/*z* (ESI): 516.2 [M+1]. The racemic mixture was separated via preparative SFC using a Chiralpak IC (250 × 30mm, 5µm) with a mobile phase of 60% Liquid CO₂ and 40% MeOH and a flow rate of 100 mL/min to generate:

Example 108-1: (*R*)-*N*-(3-(*N*-(*tert*-butyl)sulfamoyl)phenyl)-4-(1,2-dihydroxypropan-2-yl)-2-(6-azaspiro[2.5]octan-6-yl)benzamide. First eluting peak, ee 100%; ¹H NMR (400 MHz, DMSO-*d₆*): *δ* 11.95 (s, 1H), 8.35 (s, 1H), 7.90 (d, *J =* 7.43 Hz, 1H), 7.77 (d, *J* = 8.22 Hz, 1H), 7.51 - 7.61 (m, 3H), 7.47 (s, 1H), 7.28 (d, *J=* 8.41 Hz, 1H), 5.04 (s, 1H), 4.73 (t, *J* = 5.77 Hz, 1H), 3.44 (d, *J* = 4.89 Hz, 2H), 3.02 (t, *J =* 4.89 Hz, 4H), 1.49 (s, 4H), 1.41 (s, 3H), 1.12 (s, 9H), 0.33 (s, 4H). *m*/*z* (ESI): 516.2 [M+1].

Example 108-2: (*S*)-*N*-(3-(*N*-(*tert*-butyl)sulfamoyl)phenyl)-4-(1,2-dihydroxypropan-2-yl)-2-(6-azaspiro[2.5]octan-6-yl)benzamide. Second eluting peak, ee 100%; ¹H NMR (400 MHz, DMSO-*d₆*): *δ* 11.95 (s, 1H), 8.35 (s, 1H), 7.90 (d, *J=* 7.43 Hz, 1H), 7.77 (d, *J=* 8.22 Hz, 1H), 7.51 - 7.61 (m, 3H), 7.47 (s, 1H), 7.28 (d, *J =* 8.41 Hz, 1H), 5.04 (s, 1H), 4.73 (t, *J =* 5.77 Hz, 1H), 3.44 (d, *J* = 4.89 Hz, 2H), 3.02 (t, *J* = 4.89 Hz, 4H), 1.49 (s, 4H), 1.41 (s, 3H), 1.12 (s, 9H), 0.33 (s, 4H). *m*/*z* (ESI): *m*/*z:* 516.2 [M+1].

The stereochemistry assignments were arbitrary.

### Example 109: N-(3-(N-(tert-butyl)sulfamoyl)phenyl)-4-(1-methyl-1H-imidazol-2-yl)-2-(6-azaspiro[2.5]octan-6-yl)benzamide.

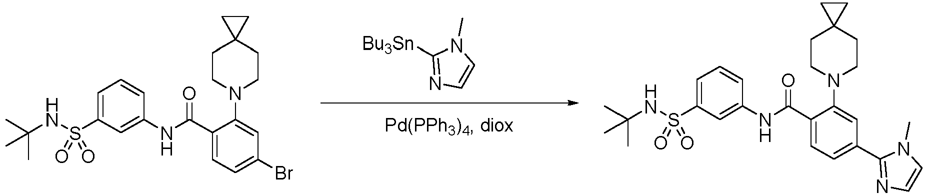

To a round bottomed flask was added 4-bromo-*N*-(3-(*N*-(*tert*-butyl)sulfamoyl)phenyl)-2-(6-azaspiro[2.5]octan-6-yl)benzamide (94 mg, 0.181 mmol, Intermediate 19-1), tetrakis(triphenylphosphine)palladium, polymer bound (0.06 mmol/g) (300 mg, 0.260 mmol, Sigma-Aldrich), 1-methyl-2-(tributylstannyl)imidazole (0.087 mL, 0.271 mmol, Alfa Aesar), and dioxane (2 mL). The solution was stirred at 90 °C for 48 h. The reaction was allowed to cool to RT and filtered through a pad of CELITE^{®}. The residue was washed with EtOAc (2 x 10 mL). The filtrate was adsorbed onto a plug of silica gel and chromatographed through a Redi-Sep^{®} pre-packed silica gel column eluting with 0-100% EtOAc in heptane, to provide the title compound (6 mg, 0.012 mmol, 7% yield), as a light, yellow solid. ¹H NMR (400 MHz, CHLOROFORM-*d*) δ ppm 12.82 (s, 1 H), 8.31 - 8.38 (m, 2 H), 8.01 (d, *J=*7.7 Hz, 1 H), 7.66 (d, *J*=8.0 Hz, 1 H), 7.59 (s, 1 H), 7.53 (t, *J*=8.0 Hz, 1 H), 7.39 (s, 1 H), 7.34 (dd, *J*=8.1, 1.3 Hz, 1 H), 7.20 - 7.25 (m, 1 H), 4.57 (s, 1 H), 3.75 (s, 3 H), 3.14 (t, *J=5.3* Hz, 4 H), 1.58 - 1.64 (m, 4 H), 1.30 (s, 9 H), 0.46 (s, 4 H). *m*/*z* (ESI): 522.2 (M+H)⁺.

**Table 13: Examples 109-1 to 109-3 were prepared analogous to preparation of Example 109:**

| Ex. # | Chemical Structure | Name | LRMS: (ESI + ve ion) *m*/*z* |
|---|---|---|---|
| 109-1 | 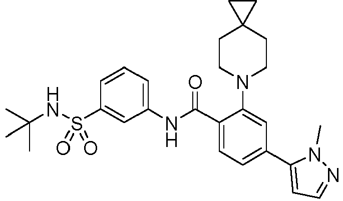 | *N-*(3-(*N-*(*tert-*butyl)sulfamoyl)phenyl)-4-(1-methyl-1H-pyrazol-5-yl)-2-(6-azaspiro [2. 5]octan-6-yl)benzamide | 522.2 |
| 109-2 | 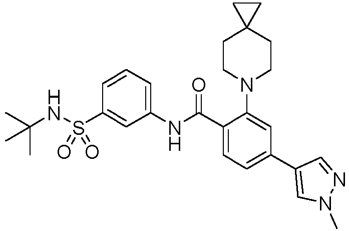 | *N-*(3-(*N-*(*tert-*butyl)sulfamoyl)phenyl)-4-(1-methyl-1H-pyrazol-4-yl)-2-(6-azaspiro [2. 5]octan-6-yl)benzamide | 522.2 |
| 109-3 | 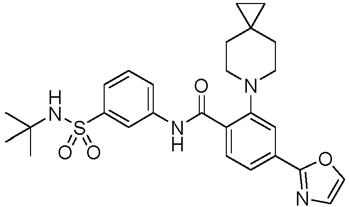 | *N-*(3-(*N-*(*tert-*butyl)sulfamoyl)phenyl)-4-(oxazol-2-yl)-2-(6-azaspiro[2.5]octan-6-yl)benzamide | 509.1 |

### Example 110: N-(3-(N-(tert-Butyl)sulfamoyl)phenyl)-4-((1-hydroxy-2-methylpropan-2-yl)amino)-2-(6-azaspiro[2.5]octan-6-yl)benzamide.

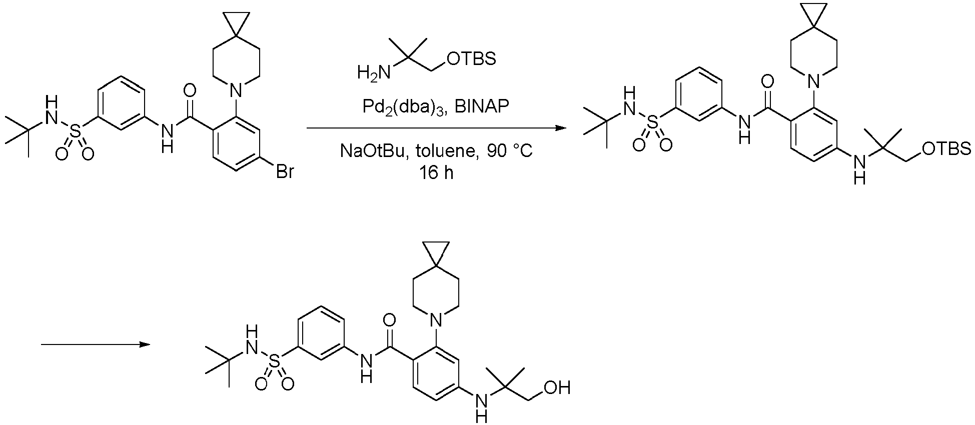

Step 1: To a RBF was added 2,2'-bis(diphenylphosphino)-1,1-binaphthyl (10 mg, 0.015 mmol, Sigma-Aldrich), tris(dibenzylideneacetone)dipalladium (8.7 mg, 9.50 µmol, Strem Chemicals, Inc.) and toluene (1 mL). The solution was stirred for 5 min. The reaction was then treated with 4-bromo-*N*-(3-(*N-*(*tert*-butyl)sulfamoyl)phenyl)-2-(6-azaspiro[2.5]octan-6-yl)benzamide (103 mg, 0.198 mmol, Intermediate 19-1), 1-((*tert*-butyldimethylsilyl)oxy)-2-methylpropan-2-amine (59 mg, 0.290 mmol), and sodium *tert-*butoxide (63 mg, 0.656 mmol, Sigma-Aldrich). The solution was stirred at 90°C for 16 h.

The reaction was allowed to cool to RT and diluted with water (20 mL) and EtOAc (10 mL). The layers were separated, and the aqueous layer extracted with EtOAc (10 mL). The combined EtOAc layers were concentrated in vacuo and adsorbed onto a plug of silica gel and chromatographed through a Redi-Sep^{®} pre-packed silica gel column, eluting with 0-75% EtOAc in heptane, to provide (38 mg, 0.059 mmol, 30% yield), as a golden film. *m*/*z* (ESI): 643.2 (M+H)⁺.

Step 2: To a solution of *N*-(3-(*N*-(*tert*-butyl)sulfamoyl)phenyl)-4-((1-((*tert*butyldimethylsilyl)oxy)-2-methylpropan-2-yl)amino)-2-(6-azaspiro[2.5]octan-6-yl)benzamide (38 mg, 0.041 mmol) in THF (1 mL) at 0 °C, was added tetrabutylammonium fluoride, (1M in THF, 0.124 mL, 0.124 mmol, Sigma-Aldrich). The solution was stirred in the cooling bath as is expired. After 20 h, the reaction was concentrated in vacuo and adsorbed onto a plug of silica gel and chromatographed through a REDI-SEP^{®} pre-packed silica gel column eluting with 0-70% EtOAc in heptane, to provide the title compound (18 mg, 0.034 mmol, 82% yield), as an off-white solid. ¹H NMR (400 MHz, CHLOROFORM-*d*) δ ppm 12.92 (s, 1 H), 8.31 (s, 1 H), 7.98 (d, *J*=8.6 Hz, 1 H), 7.68 (br d, *J*=8.0 Hz, 1 H), 7.59 (d, *J*=7.8 Hz, 1 H), 7.43 (t, *J*=7.9 Hz, 1 H), 6.67 (dd, *J*=8.6, 2.2 Hz, 1 H), 6.57 (d, *J=*2.2 Hz, 1 H), 5.29 (s, 1 H), 3.77 (s, 2 H), 2.89 (br t, *J*=4.9 Hz, 4 H), 1.39 - 1.45 (m, 6 H), 1.16 - 1.35 (m, 13 H), 0.38 (s, 4 H). [Note: protons for OH and 1 NH not seen]. *m*/*z* (ESI): 529.1 (M+H)⁺.

**Table 14: Example 110-1 was prepared analogous to preparation of Example 110:**

| Ex. # | Chemical Structure | Name | LRMS: (ESI + ve ion) *m*/*z* |
|---|---|---|---|
| 110-1 | 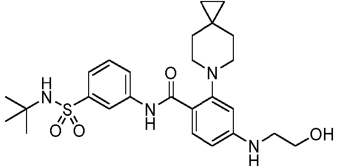 | *N-*(3-(*N-*(*tert-*butyl)sulfamoyl)phenyl)-4-((2-hydroxyethyl)amino)-2-(6-azaspiro[2.5]octan-6-yl)benzamide | 501.1 |

### Example 111: N¹-(3-(N-(tert-Butyl)sulfamoyl)phenyl)-N⁴-(2-hydroxyethyl)-2-(6-azaspiro[2.5]octan-6-yl)terephthalamide.

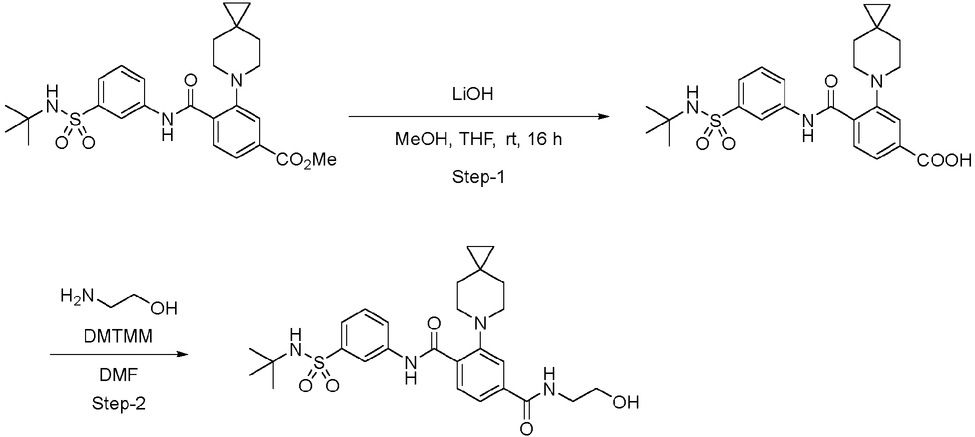

Step 1: To a solution of methyl 4-((3-(*N*-(*tert*-butyl)sulfamoyl)phenyl)carbamoyl)-3-(6-azaspiro[2.5]octan-6-yl)benzoate (197 mg, 0.394 mmol, Intermediate 19-2) and THF: MeOH (3:2, 8 mL) was added LiOH (2 mL, 2 mmol, 1M). The solution was stirred at RT for 16 h. The reaction was concentrated in vacuo to remove organic solvents. The aqueous solution was acidified with 2N HCl and extracted with EtOAc (3 x 10 mL). The combined EtOAc layers were dried over MgSO₄ and concentrated in vacuo to provide crude title compound (200 mg, 0.412 mmol), as a white solid that was carried forward. *m*/*z* (ESI): 486.0 (M+H)⁺.

Step 2: To a solution of 4-((3-(*N*-(*tert*-butyl)sulfamoyl)phenyl)carbamoyl)-3-(6-azaspiro[2.5]octan-6-yl)benzoic acid (60 mg, 0.124 mmol) and DMF (2 mL) was added 4-(4,6-dimethoxy-1,3,5-triazin-2-yl)-4-methylmorpholin-4-ium chloride (51.3 mg, 0.185 mmol, Sigma-Aldrich). The solution was stirred at RT for 30 min, then ethanolamine (17 µL, 0.283 mmol, Sigma-Aldrich) was added and stirred at rt for 2h. The reaction was diluted with water (25 mL) and stirred for 30 min. The aqueous solution was extracted with EtOAc (3 x 10 mL). The combined EtOAc extracts were concentrated in vacuo and adsorbed onto a plug of silica gel and chromatographed through a Redi-Sep^{®} pre-packed silica gel column eluting with 0-75% EtOAc:EtOH (3: 1) in heptane, to provide the title compound (52 mg, 0.1 mmol, 80% yield), as a white solid. ¹H NMR (400 MHz, DMSO-*d*₆) δ ppm 11.58 (s, 1 H), 8.62 (t, *J*=5.6 Hz, 1 H), 8.35 (s, 1 H), 7.92 (br d, *J=*7.2 Hz, 1 H), 7.81 (d, *J*=8.0 Hz, 1 H), 7.75 (s, 1 H), 7.65 (d, *J*=8.0 Hz, 1 H), 7.54 - 7.62 (m, 3 H), 4.75 (br s, 1 H), 3.53 (q, *J*=5.6 Hz, 2 H), 3.34 - 3.39 (m, 2 H), 3.05 (br t, *J*=4.9 Hz, 4 H), 1.47 (br s, 4 H), 1.12 (s, 9 H), 0.32 (s, 4 H). *m*/*z* (ESI): 529.1 (M+H)⁺.

**Table 15: Examples 111-1 to 111-4 were prepared analogous to preparation of Example 111:**

| Ex. # | Chemical Structure | Name | LRMS: (ESI + ve ion) *m*/*z* |
|---|---|---|---|
| 111-1 | 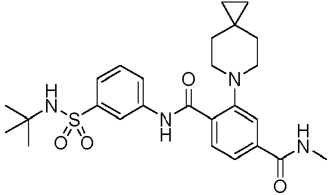 | *N¹*-(3-(*N*-(*tert-*butyl)sulfamoyl)phenyl)-*N⁴*-methyl-2-(6-azaspiro[2.5]octan-6-yl)terephthalamide | 499.1 |
| 111-2 | 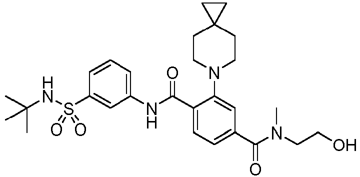 | *N*¹-(3-(*N*-(*tert-*butyl)sulfamoyl)phenyl)-*N⁴*-(2-hydroxyethyl)-*N⁴*-methyl-2-(6-azaspiro[2.5]octan-6-yl)terephthalamide | 543.2 |
| 111-3 | 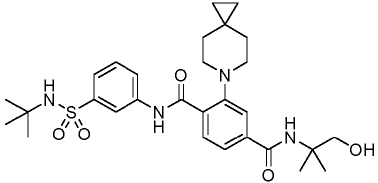 | *N¹*-(3-(*N*-(*tert-*butyl)sulfamoyl)phenyl)-*N⁴*-(1-hydroxy-2-methylpropan-2-yl)-2-(6-azaspiro[2.5]octan-6-yl)terephthalamide | 557.2 |
| 111-4 | 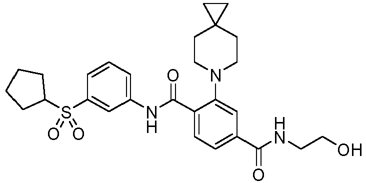 | *N¹*-(3-(cyclopentylsulfonyl)phen yl)-*N⁴*-(2-hydroxyethyl)-2-(6-azaspiro[2.5]octan-6-yl)terephthalamide | 526.1 |

### Examples 112-1 and 112-2: (R)-N-(3-(Azetidine-1-sulfonimidoyl)phenyl)-4-((2-hydroxyethyl)sulfonamido)-2-(6-azaspiro[2.5]octan-6-yl)benzamide and (S)-N-(3-(azetidine-1-sulfonimidoyl)phenyl)-4-((2-hydroxyethyl)sulfonamido)-2-(6-azaspiro[2.5]octan-6-yl)benzamide.

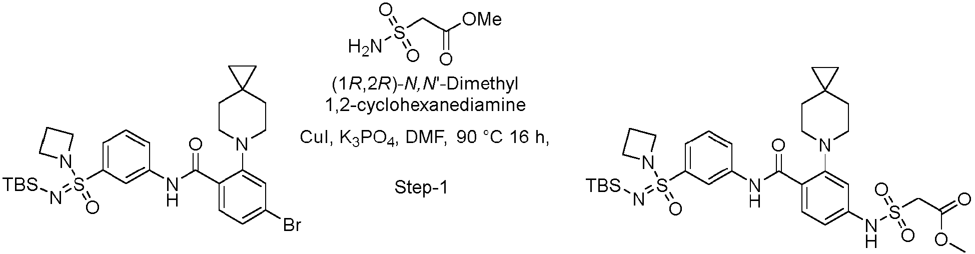

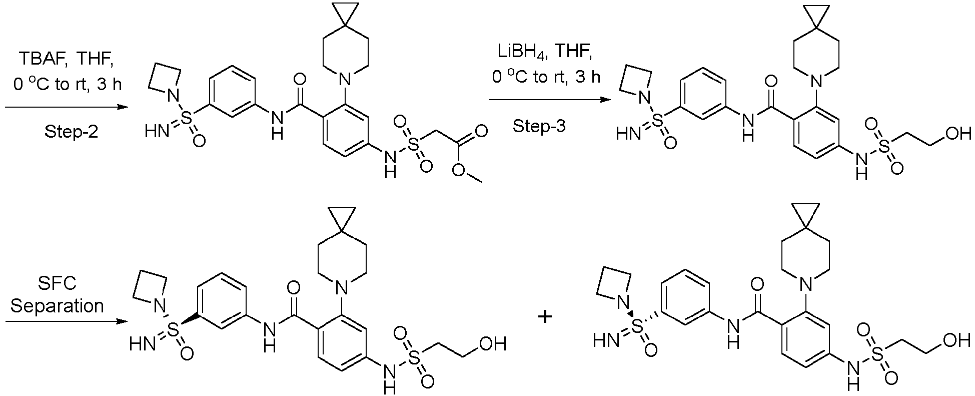

Step 1: A solution of 4-bromo-*N*-(3-(*N*-(*tert*-butyldimethylsilyl)azetidine-1-sulfonimidoyl)phenyl)-2-(6-azaspiro[2.5]octan-6-yl)benzamide (0.2 g, 0.32 mmol, Intermediate 26) in DMF (3 mL) was treated with methyl 2-sulfamoylacetate (0.074 g, 0.486 mmol), potassium phosphate tribasic (0.137 g, 0.65 mmol), copper(I) iodide (0.062 g, 0.324 mmol) followed by (1*R*,2*R*)-*N,N'*-dimethyl-1,2-cyclohexanediamine (0.023 g, 0.162 mmol) and the resulting mixture was heated at 90 °C for 16 h. The reaction was quenched with cold water and filtered through CELITE^{®} pad, washed with EtOAc. The filtrate was washed with brine, dried over anhydrous Na₂SO₄, and concentrated under reduced pressure to get the crude product. The crude product was purified by silica gel column chromatography using a gradient of 0-35% EtOAc in petroleum ether to afford methyl 2-(*N*-(4-((3-(*N*-(*tert*-butyldimethylsilyl)azetidine-1-sulfonimidoyl)phenyl)carbamoyl)-3-(6-azaspiro[2.5]octan-6-yl)phenyl)sulfamoyl)acetate (0.18 g, 0.26 mmol, 81% yield) as a pale yellow gum. ¹H NMR (400 MHz, DMSO-*d*₆): δ ppm 11.66 (s, 1 H), 10.53 (s, 1 H), 8.10 - 8.17 (m, 2 H), 7.78 (d, *J*=8.4 Hz, 1 H), 7.63 (t, *J*=7.9 Hz, 1 H), 7.46 (d, *J=*7.7 Hz, 1 H), 7.13 (d, *J*=2.2 Hz, 1 H), 7.01 (dd, *J*=8.5, 2.0 Hz, 1 H), 4.32 (s, 2 H), 3.64 (s, 3 H), 3.53 (dt, *J*=22.3, 7.6 Hz, 4 H), 2.98 (t, *J=5.3* Hz, 4 H), 1.85 - 1.87 (m, 2 H), 1.48 (s, 4 H), 0.88 (s, 9 H), 0.30 (s, 4 H), 0.08 (s, 3 H), 0.06 (s, 3 H). *m*/*z* (ESI): 690.2 (M+H)⁺.

Step 2: To a solution of methyl 2-(*N*-(4-((3-(*N*-(*tert*-butyldimethylsilyl)azetidine-1-sulfonimidoyl)phenyl)carbamoyl)-3-(6-azaspiro[2.5]octan-6-yl)phenyl)sulfamoyl)acetate (0.18 g, 0.261 mmol) in THF (4 mL) was added tetrabutylammonium fluoride (1.0 M in THF, 0.391 mL, 0.391 mmol) dropwise at 0 °C and the reaction mixture was stirred at ambient temperature for 3 h. The reaction mixture was quenched with ammonium chloride solution and was extracted with EtOAc (2x). The combined EtOAc layers were washed with water, brine, dried over anhydrous Na₂SO₄, and concentrated to afford methyl 2-(*N*-(4-((3-(azetidine-1-sulfonimidoyl)phenyl)carbamoyl)-3-(6-azaspiro[2.5]octan-6-yl)phenyl)sulfamoyl)acetate (0.13 g, 0.23 mmol, 87% yield) as an off-white solid. ¹H NMR (400 MHz, DMSO-*d*₆): δ ppm 11.68 (s, 1 H), 10.52 (s, 1 H), 8.29 (s, 1 H), 8.08 (d, *J*=8.1 Hz, 1 H), 7.79 (dd, *J*=8.5, 2.7 Hz, 1 H), 7.64 (td, *J*=7.9, 2.6 Hz, 1 H), 7.54 (d, *J*=7.9 Hz, 1 H), 7.13 (d, *J*=2.5 Hz, 1 H), 6.99 - 7.05 (m, 1 H), 4.34 (d, *J*=2.6 Hz, 2 H), 4.26 (d, *J*=2.6 Hz, 1 H), 3.64 (d, *J*=2.6 Hz, 3 H), 3.57 (t, *J*=7.5 Hz, 4 H), 2.98 (d, *J=*6.2 Hz, 4 H), 1.81 - 1.92 (m, 2 H), 1.49 (s, 4 H), 0.31 (d, *J*=2.6 Hz, 4 H). *m*/*z* (ESI): 576.2 (M+H)⁺.

Step 3: To a solution of methyl 2-(*N*-(4-((3-(azetidine-1-sulfonimidoyl)phenyl)carbamoyl)-3-(6-azaspiro[2.5]octan-6-yl)phenyl)sulfamoyl)acetate (0.13 g, 0.226 mmol) in THF (2 mL) was added lithium borohydride (2.0 M in THF, 0.226 mL, 0.452 mmol) dropwise at 0 °C and was stirred at rt for 3 h. The reaction mixture was quenched with satd. NH₄Cl solution and was extracted with EtOAc. The EtOAc layer was washed with water, brine, dried over anhydrous Na₂SO₄, and concentrated under reduced pressure. The crude product was purified by preparative HPLC to afford *N*-(3-(azetidine-1-sulfonimidoyl)phenyl)-4-((2-hydroxyethyl)sulfonamido)-2-(6-azaspiro[2.5]octan-6-yl)benzamide (0.1 g, 0.18 mmol, 81% yield) as a white solid. ¹H NMR (400 MHz, DMSO-*d*₆) δ ppm 11.69 (s, 1 H), 10.11 (s, 1 H), 8.30 (t, *J*=1.9 Hz, 1 H), 8.13 - 8.05 (m, 1 H), 7.79 (d, *J*=8.4 Hz, 1 H), 7.65 (t, *J*=7.9 Hz, 1 H), 7.55 (d, *J*=7.9 Hz, 1 H), 7.15 (d, *J=*2.1 Hz, 1 H), 7.02 (dd, *J*=8.4, 2.1 Hz, 1 H), 4.98 (s, 1 H), 4.27 (s, 1 H), 3.77 (t, *J*=6.5 Hz, 2 H), 3.62 - 3.54 (m, 4 H), 3.35 (s, 1 H), 3.32 (s, 1 H), 2.99 (t, *J=*5.0 Hz, 4 H), 1.86 - 1.88 (m, 2 H), 1.49 (d, *J*=5.7 Hz, 4 H), 0.31 (s, 4 H). *m*/*z* (ESI): 548.1 (M+H)⁺.

The racemic compound was subjected to chiral separation by SFC [Column: Chiralpak IC (250 x 30 mm, 5 µm); mobile phase: 70:30 (A : B), A = liquid CO₂, B = methanol, flow rate: 120 mL/min. to provide the following compounds of Example 112-1 and 112-2:

Example 112-1: (*R*)-*N*-(3-(Azetidine-1-sulfonimidoyl)phenyl)-4-((2-hydroxyethyl)sulfonamido)-2-(6-azaspiro[2.5]octan-6-yl)benzamide. First eluting peak; ¹H NMR (400 MHz, DMSO-*d*₆) δ ppm 11.83 (s, 1 H), 8.29 (s, 1 H), 8.08 (d, *J*=7.8 Hz, 1 H), 7.82 - 7.75 (m, 1 H), 7.64 (t, *J*=7.8 Hz, 1 H), 7.55 (d, *J*=7.8 Hz, 1 H), 7.11 (s, 1 H), 6.98 (d, *J*=8.5 Hz, 1 H), 4.31 - 4.23 (m, 1 H), 3.80 - 3.70 (m, 2 H), 3.58 (t, *J*=7.7 Hz, 4H), 3.31 - 3.27 (m, 2 H), 2.99 (d, *J=5.3* Hz, 4H), 1.86 - 1.88 (m, 2 H), 1.51 (d, *J=*5.4 Hz, 4 H), 0.32 (s, 4 H). *m*/*z* (ESI): 548.1 (M+H)⁺.

Example 112-2: (*S*)-*N*-(3-(Azetidine-1-sulfonimidoyl)phenyl)-4-((2-hydroxyethyl)sulfonamido)-2-(6-azaspiro[2.5]octan-6-yl)benzamide.

Second eluting peak; ¹H NMR (400 MHz, DMSO-*d*₆) δ ppm 11.86 (s, 1 H), 8.30 (s, 1 H), 8.08 (d, *J*=8.1 Hz, 1 H), 7.78 (d, *J*=8.5 Hz, 1 H), 7.64 (t, *J*=7.9 Hz, 1 H), 7.55 (d, *J=*7.7 Hz, 1 H), 7.10 (s, 1 H), 6.98 (d, *J*=8.7 Hz, 1 H), 4.26 (s, 1 H), 3.76 (t, *J*=6.6 Hz, 2 H), 3.58 (t, *J*=7.9 Hz, 4 H), 3.28 (t, *J*=6.5 Hz, 2 H), 2.98 (t, *J=*5.1 Hz, 4 H), 1.86 - 1.88 (m, 2 H), 1.51 (s, 4 H), 0.32 (s, 4 H). *m*/*z* (ESI): 548.1 (M+H)⁺.

**Table 16: Examples 113-1 to 113-2 and 114-1 to 114-2 were prepared analogous to preparation of Examples 112-1 and 112-2:**

| Ex. # | Chemical Structure | Name | LRMS: (ESI + ve ion) *m*/*z* |
|---|---|---|---|
| 113-1 | 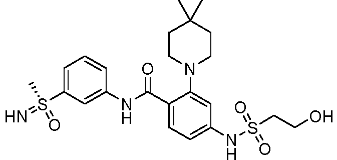 | (*R*)-4-((2-hydroxyethyl)sulfonamido)-*N-*(3-(*S-*methylsulfonimidoyl)phenyl )-2-(6-azaspiro[2.5]octan-6-yl)benzamide | 507.1 |
| 113-2 | 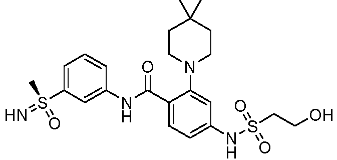 | (*S*)-4-((2-hydroxyethyl)sulfonamido)-*N-*(3-(*S-*methylsulfonimidoyl)phenyl )-2-(6-azaspiro[2.5]octan-6-yl)benzamide | 507.1 |
| 114-1 | 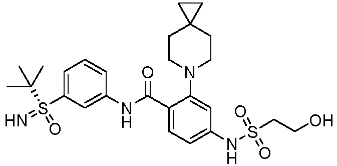 | (*R*)-4-((2-hydroxyethyl)sulfonamido)-*N*-(3-(2-methylpropan-2-ylsulfonimidoyl)phenyl)-2-(6-azaspiro[2.5]octan-6-yl)benzamide | 549.2 |
| 114-2 | 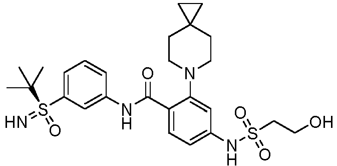 | (*S*)-4-((2-hydroxyethyl)sulfonamido)-*N*-(3-(2-methylpropan-2-ylsulfonimidoyl)phenyl)-2-(6-azaspiro[2.5]octan-6-yl)benzamide | 549.2 |

### Example 115: N-(4-((2-Hydroxyethyl)sulfonamido)-2-(6-azaspiro[2.5]octan-6-yl)phenyl)-3-(piperidin-1-yl)benzamide.(REFERENCE EXAMPLE)

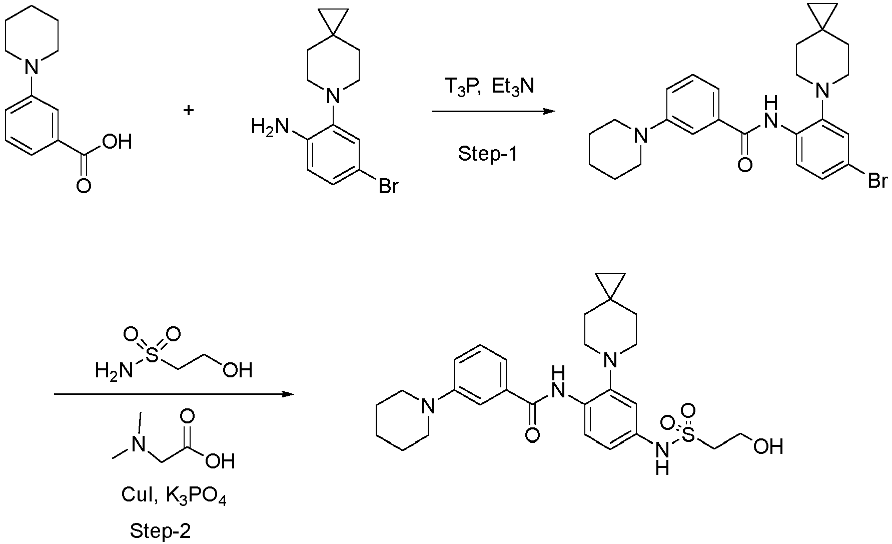

Step 1: To a solution of 3-piperidinobenzoic acid (0.40 g, 1.95 mmol, Matrix Innovation Inc.), 4-bromo-2-(6-azaspiro[2.5]octan-6-yl)aniline (0.50 g, 1.78 mmol, Intermediate 18), and Et₃N (0.49 mL, 3.56 mmol) in DCM (10 mL) at 0 °C was added T₃P (50% wt. in EtOAc) (1.58 mL, 2.67 mmol). The mixture was stirred at RT for 3 h, then diluted with 50 mL of DCM, and washed with 5 mL of water followed by 5 mL of 1 N NaOH. The organic layer was concentrated in vacuo and adsorbed onto a plug of silica gel and chromatographed through a Redi-Sep^{®} pre-packed silica gel column, eluting with 10-35% EtOAc in heptane, to provide *N*-(4-bromo-2-(6-azaspiro[2.5]octan-6-yl)phenyl)-3-(piperidin-1-yl)benzamide (0.77 g, 1.64 mmol, 92% yield) as a brown amorphous solid. *m*/*z* (ESI): 468.1 / 470.1(M+H)⁺. ¹H NMR (400 MHz, DMSO-d₆) δ 9.53 (s, 1H), 8.15 (d, *J*=8.71 Hz, 1H), 7.27-7.46 (m, 5H), 7.17 (dd, *J*=1.97, 8.19 Hz, 1H), 3.22-3.28 (m, 4H), 2.88 (t, *J*=5.29 Hz, 4H), 1.47-1.67 (m, 10H), 0.35 (s, 4H).

Step 2: A mixture of 2-hydroxyethane-1-sulfonamide (90 mg, 0.72 mmol, Enamine), potassium phosphate (381 mg, 1.79 mmol), dimethylglycine (37 mg, 0.36 mmol, Oakwood), copper(I) iodide (34 mg, 0.17 mmol, Strem) in 3 mL DMF in a glass tube was degassed for 3 min. The mixture was heated at 50 °C for 5 min under argon then treated with *N*-(4-bromo-2-(6-azaspiro[2.5]octan-6-yl)phenyl)-3-(piperidin-1-yl)benzamide (168 mg, 0.36 mmol). The glass tubed was sealed then heated at 100 °C for 24 h. The mixture was cooled to RT then partitioned between 5 mL of water and 50 mL of EtOAc. The layers were separated. The organic layer was washed with 3 mL of brine and concentrated. The residue was purified on a silica gel column (15-90% EtOAc in heptane) to give N-(4-((2-hydroxyethyl)sulfonamido)-2-(6-azaspiro[2.5]octan-6-yl)phenyl)-3-(piperidin-1-yl)benzamide (151 mg, 0.29 mmol, 82% yield) as an off-white solid. *m*/*z* (ESI): 513.2 (M+H)⁺. ¹H NMR (400 MHz, DMSO-d₆) δ 9.43 (s, 1H), 8.06 (d, *J*=8.71 Hz, 1H), 7.35-7.46 (m, 2H), 7.27-7.31 (m, 1H), 7.15 (d, *J*=7.65 Hz, 1H), 7.10-7.13 (m, 1H), 6.96 (d, *J*=8.91 Hz, 1H), 6.73 (s, 1H), 4.88 (t, *J*=5.70 Hz, 1H), 3.12-3.28 (m, 8H), 2.85 (m, 4H), 1.43-1.71 (m, 10H), 0.35 (s, 4H).

### BIOLOGICAL EXAMPLES

The following assays were used in testing the exemplary compounds of the invention. Data for those examples tested in accordance with the procedures described below are presented in Table A below.

KIF18A Enzyme Assay: Microtubule-stimulated ATPase activity assay is used to measure KIF18A enzyme activity after treatment with compound. Compounds were 2-fold serially diluted in DMSO (Sigma Inc) over 22-point concentration range. Recombinant human KIF18A (1-467 His-tagged) protein was expressed using a baculovirus system and purified by affinity chromatography by Amgen Inc. Concentrations of KIF18A protein, microtubules (MT), and ATP in the reaction were optimized for standardized homogenous enzyme assay using ADP-Glo^{™} Kinase/ATPase Assay Kit (Promega Inc). The assay measures ADP formed from the ATPase reaction. Prepare reaction buffer [(15 mM Tris, pH 7.5 (Teknova Inc), 10 mM MgCl2 (JT Baker Inc), 0.01% Pluronic F-68 (Life Technologies Inc), 1 µM Taxol (Cytoskeleton Inc), and 30 µg/mL pig microtubules (Cytoskeleton Inc)]. Add compound and KIF18A protein (30 nM) to prepared reaction buffer and incubated for 15 minutes at RT, next add ATP (at Kₘ, 75 µM) to the reaction mixture and incubated for an additional 15 minutes at RT. Mix 5 µl of ADP-Glo^{™} Reagent and 2.5 µl of the reaction mixture and incubate for 40 minutes at RT. Add 10 µl ADP-Glo^{™} Detection Reagent and incubate for 40 minutes at RT. Read luminescence using EnVision microplate reader with ultra-luminescence module (Perkin Elmer Inc). Concentration-response curve-fitting and IC₅₀ determination was performed using Genedata Screener Software (Standard 15.0.1, Genedata Inc) with a four-parameter logistic regression fit model.

Table A provides data for compounds exemplified in the present application and priority document thereof, as representative compounds of the present invention, as follows: chemical name (as named by either ACD software or ChemDraw (Professional 15.0)) and biological data (IC₅₀ in µM). Ex. # refers to Example No.

**TABLE A: BIOLOGICAL DATA**

| Ex. # | Compound Name | KIF 18A ATPase IC₅₀ (µM) |
|---|---|---|
| 100 | *N*-(3-(*N*-(*tert*-butyl)sulfamoyl)phenyl)-4-((3-methyloxetan-3-yl)sulfonyl)-2-(6-azaspiro[2.5]octan-6-yl)benzamide | 0.173 |
| 100-1 | *N*-(3-(*N*-(*tert*-butyl)sulfamoyl)phenyl)-4-(methylsulfonyl)-2-(6-azaspiro[2.5]octan-6-yl)benzamide | 0.061 |
| 100-2 | *N*-(3-isopropylphenyl)-4-(methylsulfonyl)-2-(6-azaspiro[2.5]octan-6-yl)benzamide | 0.094 |
| 100-3 | *N*-(3-cyclopropylphenyl)-4-(methylsulfonyl)-2-(6-azaspiro[2.5]octan-6-yl)benzamide | 0.187 |
| 100-4 | *N*-(3-(*tert*-butyl)phenyl)-4-(methylsulfonyl)-2-(6-azaspiro[2.5]octan-6-yl)benzamide | 0.362 |
| 100-5 | 4-(methylsulfonyl)-*N*-(quinolin-8-yl)-2-(6-azaspiro[2.5]octan-6-yl)benzamide | 0.246 |
| 100-6 | *N*-(4-methylquinolin-8-yl)-4-(methylsulfonyl)-2-(6-azaspiro[2.5]octan-6-yl)benzamide | 0.061 |
| 100-7 | *N*-(chroman-8-yl)-4-(methylsulfonyl)-2-(6-azaspiro[2.5]octan-6-yl)benzamide | 0.045 |
| 100-8 | *N*-(benzofuran-7-yl)-4-(methylsulfonyl)-2-(6-azaspiro[2.5]octan-6-yl)benzamide | 0.200 |
| 100-9 | *N*-(benzo[b]thiophen-7-yl)-4-(methylsulfonyl)-2-(6-azaspiro[2.5]octan-6-yl)benzamide | 0.630 |
| 100-10 | 4-(methylsulfonyl)-*N-*(3-morpholinophenyl)-2-(6-azaspiro[2.5]octan-6-yl)benzamide | 0.862 |
| 100-11 | *N*-(3-(*N*-(*tert*-butyl)sulfamoyl)phenyl)-4-((methylsulfonyl)methyl)-2-(6-azaspiro[2.5]octan-6-yl)benzamide | 0.051 |
| 100-12 | *N*-(3-(*N*-(*tert*-butyl)sulfamoyl)phenyl)-4-(((2-hydroxyethyl)sulfonyl)methyl)-2-(6-azaspiro[2.5]octan-6-yl)benzamide | 0.075 |
| 100-13 | 4-(*N*-(*tert*-butyl)sulfamoyl)-*N-*(3-(*N-*(*tert-*butyl)sulfamoyl)phenyl)-2-(6-azaspiro[2.5]octan-6-yl)benzamide | 0.076 |
| 100-14 | *N*-(3-(*N*-(*tert*-butyl)sulfamoyl)phenyl)-4-(3-methyloxetan-3-yl)-2-(6-azaspiro[2.5]octan-6-yl)benzamide | 0.130 |
| 100-15 | *N*-(3-(*N*-(*tert*-butyl)sulfamoyl)phenyl)-4-(3-hydroxyoxetan-3-yl)-2-(6-azaspiro[2.5]octan-6-yl)benzamide | 0.088 |
| 101 | *N*-(3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide | 0.198 |
| 101-1 | *N*-(2-fluoro-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide | 0.066 |
| 101-2 | *N*-(2-fluoro-3-((1-hydroxy-2-methylpropan-2-yl)amino)phenyl)-4-((1-methylcyclopropane)-1-sulfonamido)-2-(6-azaspiro[2.5]octan-6-yl)benzamide | 0.298 |
| 101-3 | *N*-(3-((1-hydroxy-2-methylpropan-2-yl)amino)phenyl)-4-((1-methylcyclopropane)-1-sulfonamido)-2-(6-azaspiro[2.5]octan-6-yl)benzamide | 0.115 |
| 102 | *N*-(3-(*N*-(*tert*-Butyl)sulfamoyl)phenyl)-4-((1-methylcyclopropane)-1-sulfonamido)-2-(6-azaspiro[2.5]octan-6-yl)benzamide | 0.061 |
| 102-1 | *N*-(3-(*N*-(*tert*-butyl)sulfamoyl)phenyl)-4-(methylsulfonamido)-2-(6-azaspiro[2.5]octan-6-yl)benzamide | 0.027 |
| 102-2 | *N*-(3-(*N*-(*tert*-butyl)sulfamoyl)phenyl)-4-(ethylsulfonamido)-2-(6-azaspiro[2.5]octan-6-yl)benzamide | 0.034 |
| 102-3 | *N*-(3-(*N*-(*tert*-butyl)sulfamoyl)phenyl)-4-((1-methylethyl)sulfonamido)-2-(6-azaspiro[2.5]octan-6-yl)benzamide | 0.026 |
| 102-4 | *N*-(3-(*N*-(*tert*-butyl)sulfamoyl)phenyl)-4-(cyclopropanesulfonamido)-2-(6-azaspiro[2.5]octan-6-yl)benzamide | 0.030 |
| 102-5 | *N*-(3-(*N*-(*tert*-butyl)sulfamoyl)phenyl)-4-((1,1-dimethylethyl)sulfonamido)-2-(6-azaspiro[2.5]octan-6-yl)benzamide | 0.063 |
| 102-6 | *N*-(3-(*N*-(*tert*-butyl)sulfamoyl)phenyl)-4-(1,1-dioxidoisothiazolidin-2-yl)-2-(6-azaspiro[2.5]octan-6-yl)benzamide | 0.045 |
| 103 | *N*-(3-(*N*-(*tert*-butyl)sulfamoyl)phenyl)-4-((2-hydroxyethyl)sulfonamido)-2-(6-azaspiro[2.5]octan-6-yl)benzamide | 0.051 |
| 103-1 | (*R*)-4-((2-hydroxyethyl)sulfonamido)-*N*-(3-(2-methylmorpholino)phenyl)-2-(6-azaspiro[2.5]octan-6-yl)benzamide | 0.071 |
| 103-2 | (*R*)-*N*-(2-fluoro-3-(2-methylmorpholino)phenyl)-4-((2-hydroxyethyl)sulfonamido)-2-(6-azaspiro[2.5]octan-6-yl)benzamide | 0.101 |
| 103-2 | (*R*)-*N*-(3-fluoro-5-(2-methylmorpholino)phenyl)-4-((2-hydroxyethyl)sulfonamido)-2-(6-azaspiro[2.5]octan-6-yl)benzamide | 0.131 |
| 103-4 | (*R*)-*N*-(4-fluoro-3-(2-methylmorpholino)phenyl)-4-((2-hydroxyethyl)sulfonamido)-2-(6-azaspiro[2.5]octan-6-yl)benzamide | 0.177 |
| 104 (REF. EX) | *N*-(3-(4,4-difluoropiperidin-1-yl)-5-methylphenyl)-4-((2-hydroxyethyl)sulfonamido)-2-(6-azaspiro[2.5]octan-6-yl)benzamide | 0.034 |
| 104-1 (REF. EX) | *N*-(3-(4,4-difluoropiperidin-1-yl)phenyl)-4-((2-hydroxyethyl)sulfonamido)-2-(6-azaspiro[2.5]octan-6-yl)benzamide | 0.012 |
| 104-2 (REF. EX) | *N*-(3-(4,4-difluoropiperidin-1-yl)-2-fluorophenyl)-4-((2-hydroxyethyl)sulfonamido)-2-(6-azaspiro[2.5]octan-6-yl)benzamide | 0.029 |
| 105-1 | (*S*)-*N*-(3-(*N*-(*tert*-butyl)sulfamoyl)phenyl)-4-((2-hydroxy-1-methylethyl)sulfonamido)-2-(6-azaspiro[2.5]octan-6-yl)benzamide | 0.042 |
| 105-2 | (*R*)*-N-*(3-(*N-*(*tert*-butyl)sulfamoyl)phenyl)-4-((2-hydroxy-1-methylethyl)sulfonamido)-2-(6-azaspiro[2.5]octan-6-yl)benzamide | 0.057 |
| 106 | *N*-(3-(2-Hydroxy-2-methylpropoxy)phenyl)-4-((2-hydroxyethyl)sulfonamido)-2-(6-azaspiro[2.5]octan-6-yl)benzamide | 0.078 |
| 107 | 4-(Azetidin-1-ylsulfonyl)-*N*-(3-(*N*-(*tert-*butyl)sulfamoyl)phenyl)-2-(6-azaspiro[2.5]octan-6-yl)benzamide | 0.044 |
| 107-1 | *N*-(3-(*N*-(*tert*-butyl)sulfamoyl)phenyl)-4-(*N*-(1-hydroxy-2-methylpropan-2-yl)sulfamoyl)-2-(6-azaspiro[2.5]octan-6-yl)benzamide | 0.060 |
| 107-2 | *N*-(3-(*N*-(tert-butyl)sulfamoyl)phenyl)-4-(*N*-(2-hydroxyethyl)sulfamoyl)-2-(6-azaspiro[2.5]octan-6-yl)benzamide | 0.043 |
| 107-3 | *N*-(3-(*N*-(*tert*-butyl)sulfamoyl)phenyl)-4-(*N*,*N-*dimethylsulfamoyl)-2-(6-azaspiro[2.5]octan-6-yl)benzamide | 0.070 |
| 107-4 | *N*-(3-(*N*-(*tert*-butyl)sulfamoyl)phenyl)-4-(*N*-methylsulfamoyl)-2-(6-azaspiro[2.5]octan-6-yl)benzamide | 0.029 |
| 107-5 | *N*-(3-(*N*-(*tert*-butyl)sulfamoyl)phenyl)-4-(*N*-(oxetan-3-yl)sulfamoyl)-2-(6-azaspiro[2.5]octan-6-yl)benzamide | 0.060 |
| 107-6 | *N*-(3-(*N*-(*tert*-butyl)sulfamoyl)phenyl)-4-(*N-*cyclopropylsulfamoyl)-2-(6-azaspiro[2.5]octan-6-yl)benzamide | 0.036 |
| 107-7 | *N*-(3-(*N*-(*tert*-butyl)sulfamoyl)phenyl)-4-(*N-*(1-methylcyclopropyl)sulfamoyl)-2-(6-azaspiro[2.5]octan-6-yl)benzamide | 0.052 |
| 107-8 | *N*-(3-(*N*-(*tert*-butyl)sulfamoyl)phenyl)-2-(6-azaspiro[2.5]octan-6-yl)-4-sulfamoylbenzamide | 0.040 |
| 108-1 | (*R*)-*N*-(3-(*N-*(*tert*-butyl)sulfamoyl)phenyl)-4-(1,2-dihydroxypropan-2-yl)-2-(6-azaspiro[2.5]octan-6-yl)benzamide | 0.092 |
| 108-2 | (*S*)-*N*-(3-(*N*-(*tert*-butyl)sulfamoyl)phenyl)-4-(1,2-dihydroxypropan-2-yl)-2-(6-azaspiro[2.5]octan-6-yl)benzamide | 0.141 |
| 109 | *N*-(3-(*N*-(*tert*-butyl)sulfamoyl)phenyl)-4-(1-methyl-1H-imidazol-2-yl)-2-(6-azaspiro[2.5]octan-6-yl)benzamide | 0.124 |
| 109-1 | *N*-(3-(*N*-(*tert*-butyl)sulfamoyl)phenyl)-4-(1-methyl-1H-pyrazol-5-yl)-2-(6-azaspiro[2.5]octan-6-yl)benzamide | 0.601 |
| 109-2 | *N*-(3-(*N*-(*tert*-butyl)sulfamoyl)phenyl)-4-(1-methyl-1H-pyrazol-4-yl)-2-(6-azaspiro[2.5]octan-6-yl)benzamide | 1.09 |
| 109-3 | *N*-(3-(*N*-(*tert*-butyl)sulfamoyl)phenyl)-4-(oxazol-2-yl)-2-(6-azaspiro[2.5]octan-6-yl)benzamide | 0.534 |
| 110 | *N*-(3-(*N*-(*tert*-Butyl)sulfamoyl)phenyl)-4-((1-hydroxy-2-methylpropan-2-yl)amino)-2-(6-azaspiro[2.5]octan-6-yl)benzamide | 0.058 |
| 110-1 | *N*-(3-(*N*-(*tert*-butyl)sulfamoyl)phenyl)-4-((2-hydroxyethyl)amino)-2-(6-azaspiro[2.5]octan-6-yl)benzamide | 0.134 |
| 111 | *N*¹-(3-(*N*-(*tert*-Butyl)sulfamoyl)phenyl)-*N*⁴-(2-hydroxyethyl)-2-(6-azaspiro[2.5]octan-6-yl)terephthalamide | 0.124 |
| 111-1 | *N*¹-(3-(*N*-(*tert-*butyl)sulfamoyl)phenyl)-*N*⁴-methyl-2-(6-azaspiro[2.5]octan-6-yl)terephthalamide | 0.132 |
| 111-2 | *N*¹-(3-(*N*-(*tert*-butyl)sulfamoyl)phenyl)-*N*⁴-(2-hydroxyethyl)-*N*⁴-methyl-2-(6-azaspiro[2.5]octan-6-yl)terephthalamide | 0.201 |
| 111-3 | *N*¹-(3-(*N-*(*tert*-butyl)sulfamoyl)phenyl)-*N*⁴-(1-hydroxy-2-methylpropan-2-yl)-2-(6-azaspiro[2.5]octan-6-yl)terephthalamide | 0.272 |
| 111-4 | *N*¹-(3-(cyclopentylsulfonyl)phenyl)-*N*⁴-(2-hydroxyethyl)-2-(6-azaspiro[2.5]octan-6-yl)terephthalamide | 0.139 |
| 112-1 | (*R*)-*N*-(3-(Azetidine-1-sulfonimidoyl)phenyl)-4-((2-hydroxyethyl)sulfonamido)-2-(6-azaspiro[2.5]octan-6-yl)benzamide | 0.142 |
| 112-2 | (*S*)-*N*-(3-(Azetidine-1-sulfonimidoyl)phenyl)-4-((2-hydroxyethyl)sulfonamido)-2-(6-azaspiro[2.5]octan-6-yl)benzamide | 0.160 |
| 113-1 | (*R*)-4-((2-hydroxyethyl)sulfonamido)-*N*-(3-(*S-*methylsulfonimidoyl)phenyl)-2-(6-azaspiro[2.5]octan-6-yl)benzamide | 0.429 |
| 113-2 | (*S*)-4-((2-hydroxyethyl)sulfonamido)-*N*-(3-(S-methylsulfonimidoyl)phenyl)-2-(6-azaspiro[2.5]octan-6-yl)benzamide | 0.488 |
| 114-1 | (*R*)-4-((2-hydroxyethyl)sulfonamido)-*N*-(3-(2-methylpropan-2-ylsulfonimidoyl)phenyl)-2-(6-azaspiro[2.5]octan-6-yl)benzamide | 0.194 |
| 114-2 | (*S*)-4-((2-hydroxyethyl)sulfonamido)-*N*-(3-(2-methylpropan-2-ylsulfonimidoyl)phenyl)-2-(6-azaspiro[2.5]octan-6-yl)benzamide | 0.323 |
| 115 (REF. EX.) | *N*-(4-((2-hydroxyethyl)sulfonamido)-2-(6-azaspiro[2.5]octan-6-yl)phenyl)-3-(piperidin-1-yl)benzamide | 0.026 |

## Claims

1. A compound of formula I: 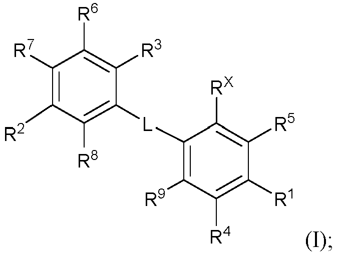 or any pharmaceutically-acceptable salt thereof, wherein:
R^{X} is 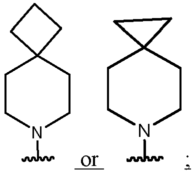
R¹ is a group -Z-R¹²; wherein Z is absent, -C₀₋₄alk-S-C₀₋₄alk-, C₀₋₄alk-S(=O)-C₀₋₄alk-, -C₀₋₄alk-SO₂-C₀₋₄alk-, -C₀₋₄alk-NR¹¹-C₀₋₄alk-, -C₀₋₄alk-NR¹¹SO₂-C₀₋₄alk-, -C₀₋₄alk-SO₂NR¹¹-C₀₋₄alk-, -C₀₋₄alk-NR¹¹SO₂NR¹¹-C₀₋₄alk-, -C₀₋₄alk-O-C₀₋₄alk-, -C₀₋₄alk-C(=O)-C₀₋₄alk-, -C₀₋₄alk-C(=O)-O-C₀₋₄alk-, -C₀₋₄alk-(C=O)NR¹¹-C₀₋₄alk-, -C₀₋₄alk-NR¹¹(C=O)-C₀₋₄alk-, -C₀₋₄alk-S(=O)(=NH)-, -N=S(=O)<, -(C=O)-,
or -C(=N-OH)-;
R² is a group -Y-R¹³, wherein Y is -C₀₋₄alk-S-C₀₋₄alk-, C₀₋₄alk-S(=O)-C₀₋₄alk-, -C₀₋₄alk-SO₂-C₀₋₄alk-, -C₀₋₄alk-NR^{13c}-C₀₋₄alk-, -C₀₋₄alk-SO₂NR^{13c}-C₀₋₄alk-, -C₀₋₄alk-NR^{13c}SO₂-C₀₋₄alk-, -C₀₋₄alk-S(=O)(=NH)-, -C₀₋₄alk-O-C₀₋₄alk-, -C₀₋₄alk-C(=O)-C₀₋₄alk-, -C₀₋₄alk-C(=O)-O-C₀₋₄alk-, -C₀₋₄alk-(C=O)NR^{13c}-C₀₋₄alk-, -C₀₋₄alk-NR^{13c}(C=O)-C₀₋₄alk-, -or -N=S(=O)<;
R³ is H, halo, C₁₋₄alk, or C₁₋₄haloalk;
R⁴ is H, halo, C₁₋₄alk, or C₁₋₄haloalk;
R⁵ is H, halo, C₁₋₈alk, or C₁₋₄haloalk;
R⁶ is H, halo, C₁₋₈alk, or C₁₋₄haloalk;
R⁷ is H, halo, C₁₋₄alk, or C₁₋₄haloalk;
R⁸ is H, halo, C₁₋₈alk, or C₁₋₄haloalk;
or alternatively, R² and R⁸ can combine with the carbon atoms attached to each of them to form a saturated or partially-saturated 5- or 6-membered monocyclic ring fused to the phenyl ring; wherein said 5-or 6-membered monocyclic ring contains 0, 1, 2 or 3 N atoms and 0 or 1 atoms selected from O and S, and further wherein said 5- or 6-membered monocyclic ring is substituted by 0, 1, 2 or 3 group(s) selected from F, Cl, Br, C₁₋₆alk, C₁₋₄haloalk, -OR^{a}, -OC₁₋₄haloalk, CN, -NR^{a}R^{a}, or oxo;
R⁹ is H, halo, C₁₋₄alk, or C₁₋₄haloalk;
L is -(C=O)-NR¹⁰- or -NR¹⁰-(C=O)-;
R¹⁰ is H or C₁₋₄alk;
R¹¹ is H or C₁₋₄alk;
R¹² is R^{12a}, or R^{12b};
R¹³ is halo, R^{13a} or R^{13b};
R^{13c} is H or C₁₋₄alk;
R^{12a} and R^{13a} is independently, at each instance, selected from the group consisting of a saturated, partially-saturated or unsaturated 3-, 4-, 5-, 6-, or 7-membered monocyclic or 8-, 9-, 10-, 11-, or 12-membered bicyclic ring containing 0, 1, 2 or 3 N atoms and 0 or 1 atoms selected from O and S, which is substituted by 0, 1, 2 or 3 group(s) selected from the group consisting of F, Cl, Br, C₁₋₆alk, C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b},-N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S( =O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, a saturated, partially-saturated or unsaturated 3-, 4-, 5-, or 6-membered monocyclic ring, and oxo;
R^{12b} and R^{13b} is independently, at each instance, selected from the group consisting of C₁₋₆alk substituted by 0, 1, 2, 3, 4, or 5 group(s) selected from the group consisting of F, Cl, Br, -CH₂F, -CHF₂, -CF₃, -C(=O)OR^{a}, -OR^{a}, -OC₁₋₄haloalk, CN, NH₂, NH(CH₃), N(CH₃)₂, and a saturated, partially-saturated or unsaturated 3-, 4-, 5-, or 6-membered monocyclic ring;
R^{a} is independently, at each instance, H or R^{b}; and
R^{b} is independently, at each instance, C₁₋₆alk, phenyl, or benzyl, wherein the C₁₋₆alk is being substituted by 0, 1, 2 or 3 substituents selected from halo, -OH, -OC₁₋₄alk, -NH₂, -NHC₁₋₄alk, -OC(=O)C₁₋₄alk, or -N(C₁₋₄alk)C₁₋₄alk; and the phenyl or benzyl is being substituted by 0, 1, 2 or 3 substituents selected from halo, C₁₋₄alk, C₁₋₃haloalk, -OH, -OC₁₋₄alk, -NH₂, -NHC₁₋₄alk, -OC(=O)C₁₋₄alk, or -N(C₁₋₄alk)C₁₋₄alk.

2. The compound of claim 1 wherein L is -NR¹⁰-(C=O)-.

3. The compound of claim 1 wherein L is -(C=O)-NR¹⁰-.

4. The compound of claim 1 wherein R¹⁰ is H or methyl.

5. The compound of claim 1 wherein Z is absent, -SO₂-, -CH₂-SO₂, -NH-, -NHSO₂-, -SO₂NH-, -SO₂N(CH₃)-, -O-, -(C=O)O-, -(C=O)NH-, -(C=O)N(CH₃)-, -S(=O)(=NH)-, -CH₂-N(CH₃)-, or -C(=N-OH)-.

6. The compound of claim 1 wherein R¹² is selected from:
a) H, F, Br, OH, or CN;
b) R^{12a} selected from a saturated, partially-saturated or unsaturated 3-, 4-, 5-, 6-, or 7-membered monocyclic ring containing 0, 1, 2 or 3 N atoms and 0 or 1 atoms selected from O and S, which is substituted by 0, 1, 2 or 3 group(s) selected from F, Cl, Br, methyl, ethyl, -CF₃, -CH₂OH, -OH, -OCH₃, -NH₂, or oxo; or
c) R^{12b} selected from C₁₋₆alk substituted by 0, 1, 2 or 3 group(s) selected from F, Cl, Br, -CF₃, or -OH.

7. The compound of claim 1 wherein R¹² is R^{12a} selected from cyclopropyl, oxetanyl, imidazolyl, isothiazolidinyl, azetidinyl, oxazolyl, pyrazolyl, or diazirinyl; each of which is independently substituted by 0, 1, 2 or 3 group(s) selected from methyl, ethyl, -CF₃, or oxo; or R^{12b} selected from methyl, ethyl, isopropyl, *tert*-butyl,-ethenyl, substituted by 0, 1, 2 or 3 group(s) selected from F, Cl, Br, -CF₃, or -OH.

8. The compound of claim 1 wherein R¹ is a group -Z-R¹², wherein Z is absent, -SO₂-, -CH₂SO₂-, -(C=O)NH-, -NH-, -NHSO₂- or -SO₂NH-; and R¹² is cyclopropyl, oxetanyl, azetidinyl, or imidazolyl ring, each of which is independently substituted by 0, 1, or 2 group(s) selected from methyl, - CF₃, or oxo; or R¹² is methyl, ethyl, isopropyl, or *tert*-butyl*,* each of which is independently substituted by 0, 1, 2 or 3 F, -CF₃ or OH group(s).

9. The compound of claim 1 wherein R¹ is a group -Z-R¹², wherein Z is -NHSO₂- and R¹² is -CH₂-CH₂-OH or -CH(CH₃)CH₂OH.

10. The compound of claim 1 wherein Y is absent, -SO₂NH-, NH-, - SO₂-, -S(=O)(=NH)-, or -O-.

11. The compound of claim 1 wherein R¹³ is H or F; R^{13a} selected from morpholinyl, piperidinyl, cyclopentyl, cyclopropyl, azetidinyl, or oxetanyl; wherein each said ring is substituted by 0, 1, 2 or 3 OH group(s) selected from methyl or -OH; or R^{13b} selected from methyl, ethyl, propyl, isopropyl, *tert*-butyl*,* or isopentyl; each of which is independently substituted by 0, 1, 2 or 3 OH group(s).

12. The compound of claim 1 wherein R² and R⁸ can combine with the carbon atoms attached to each of them to form a saturated or partially-saturated 6-membered monocyclic ring fused to the phenyl ring; wherein said 6-membered monocyclic ring contains 0, 1, 2 or 3 N atoms and 0 or 1 atoms selected from O and S, and further wherein said 6-membered monocyclic ring is substituted by 0, 1, 2 or 3 group(s) selected from F, Cl, Br, C₁₋₆alk, C₁₋₄haloalk, or oxo.

13. The compound of claim 1 wherein R⁴ is H.

14. The compound of claim 1 wherein R⁵ is H.

15. The compound of claim 1 wherein R⁶ is H or F.

16. The compound of claim 1 wherein R⁷ is H or F.

17. The compound of claim 1 wherein R³ is H, F, or methyl; or alternatively, R² and R⁸ can combine with the carbon atoms attached to each of them to form a saturated 6-membered monocyclic ring fused to the phenyl ring; selected from the group:

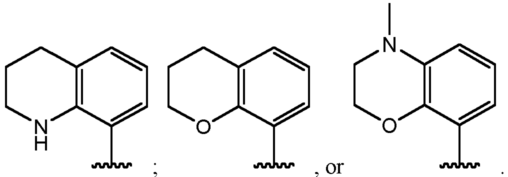

18. The compound claim 1 wherein R² is:
a) a group -Y-R^{13a}, wherein Y is absent or -S(=O)(=NH)-; and R¹³ is piperidinyl or azetidinyl; wherein each said ring is independently substituted by 0, 1, 2 or 3 F group(s);
b) a group -Y-R^{13b}, wherein Y is -SO₂NH-, -O-. NH-; and wherein R^{13b} is *tert*-butyl substituted by 0, 1, 2, or 3 OH group(s); or
c) alternatively, the carbon atoms attached to R² and R³ combine to form a saturated 6-membered monocyclic ring fused to the phenyl ring; which is 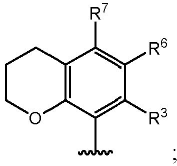 wherein said 6-membered monocyclic ring is unsubstituted.

19. The compound of claim 1 wherein R² is -SO₂NH-*tert*-butyl group or -NH- *tert*-butyl-OH group.

20. The compound of claim 1 wherein R⁸ is H.

21. The compound of claim 1 wherein R⁹ is H.

22. The compound of claim 1 wherein R¹⁰ is H.

23. The compound of claim 1, or the pharmaceutically acceptable salt thereof, selected from the group consisting of:
N-(3-(N-(tert-butyl)sulfamoyl)phenyl)-4-((3-methyloxetan-3-yl)sulfonyl)-2-(6-azaspiro[2.5]octan-6-yl)benzamide;
*N*-(3-(*N*-(*tert*-butyl)sulfamoyl)phenyl)-4-(methylsulfonyl)-2-(6-azaspiro[2.5]octan-6-yl)benzamide;
*N*-(3-isopropylphenyl)-4-(methylsulfonyl)-2-(6-azaspiro[2.5]octan-6-yl)benzamide;
*N*-(3-cyclopropylphenyl)-4-(methylsulfonyl)-2-(6-azaspiro[2.5]octan-6-yl)benzamide;
*N*-(3-(*tert*-butyl)phenyl)-4-(methylsulfonyl)-2-(6-azaspiro[2.5]octan-6-yl)benzamide;
4-(methylsulfonyl)-*N*-(quinolin-8-yl)-2-(6-azaspiro[2.5]octan-6-yl)benzamide;
*N*-(4-methylquinolin-8-yl)-4-(methylsulfonyl)-2-(6-azaspiro[2.5]octan-6-yl)benzamide;
*N*-(chroman-8-yl)-4-(methylsulfonyl)-2-(6-azaspiro[2.5]octan-6-yl)benzamide;
*N*-(benzofuran-7-yl)-4-(methylsulfonyl)-2-(6-azaspiro[2.5]octan-6-yl)benzamide;
*N*-(benzo[b]thiophen-7-yl)-4-(methylsulfonyl)-2-(6-azaspiro[2.5]octan-6-yl)benzamide;
4-(methylsulfonyl)-*N*-(3-morpholinophenyl)-2-(6-azaspiro[2.5]octan-6-yl)benzamide;
*N*-(3-(*N*-(*tert*-butyl)sulfamoyl)phenyl)-4-((methylsulfonyl)methyl)-2-(6-azaspiro[2.5]octan-6-yl)benzamide;
*N*-(3-(*N*-(*tert*-butyl)sulfamoyl)phenyl)-4-(((2-hydroxyethyl)sulfonyl)methyl)-2-(6-azaspiro[2.5]octan-6-yl)benzamide;
4-(*N*-(*tert*-butyl)sulfamoyl)-N-(3-(*N*-(*tert*-butyl)sulfamoyl)phenyl)-2-(6-azaspiro[2.5]octan-6-yl)benzamide;
*N*-(3-(*N*-(*tert*-butyl)sulfamoyl)phenyl)-4-(3-methyloxetan-3-yl)-2-(6-azaspiro[2.5]octan-6-yl)benzamide;
*N*-(3-(*N*-(*tert*-butyl)sulfamoyl)phenyl)-4-(3-hydroxyoxetan-3-yl)-2-(6-azaspiro[2.5]octan-6-yl)benzamide;
N-(3-((1-hydroxy-2-methylpropan-2-yl)amino)phenyl)-4-(N-(3-methyloxetan-3-yl)sulfamoyl)-2-(6-azaspiro[2.5]octan-6-yl)benzamide;
*N*-(2-fluoro-3-((1-hydroxy-2-methylpropan-2-yl)amino)phenyl)-4-(N-(3-methyloxetan-3-yl)sulfamoyl)-2-(6-azaspiro[2.5]octan-6-yl)benzamide;
*N*-(2-fluoro-3-((1-hydroxy-2-methylpropan-2-yl)amino)phenyl)-4-((1-methylcyclopropane)-1-sulfonamido)-2-(6-azaspiro[2.5]octan-6-yl)benzamide;
*N*-(3-((1-hydroxy-2-methylpropan-2-yl)amino)phenyl)-4-((1-methylcyclopropane)-1-sulfonamido)-2-(6-azaspiro[2.5]octan-6-yl)benzamide;
N-(3-(N-(tert-Butyl)sulfamoyl)phenyl)-4-((1-methylcyclopropane)-1-sulfonamido)-2-(6-azaspiro[2.5]octan-6-yl)benzamide;
*N*-(3-(*N*-(*tert*-butyl)sulfamoyl)phenyl)-4-(methylsulfonamido)-2-(6-azaspiro[2.5]octan-6-yl)benzamide;
*N*-(3-(*N*-(*tert*-butyl)sulfamoyl)phenyl)-4-(ethylsulfonamido)-2-(6-azaspiro[2.5]octan-6-yl)benzamide;
*N*-(3-(*N*-(*tert*-butyl)sulfamoyl)phenyl)-4-((1-methylethyl)sulfonamido)-2-(6-azaspiro[2.5]octan-6-yl)benzamide;
*N*-(3-(*N*-(*tert*-butyl)sulfamoyl)phenyl)-4-(cyclopropanesulfonamido)-2-(6-azaspiro[2.5]octan-6-yl)benzamide;
*N*-(3-(*N*-(*tert*-butyl)sulfamoyl)phenyl)-4-((1,1-dimethylethyl)sulfonamido)-2-(6-azaspiro[2.5]octan-6-yl)benzamide;
*N*-(3-(*N*-(*tert*-butyl)sulfamoyl)phenyl)-4-(1,1-dioxidoisothiazolidin-2-yl)-2-(6-azaspiro[2.5]octan-6-yl)benzamide;
N-(3-(N-(tert-butyl)sulfamoyl)phenyl)-4-((2-hydroxyethyl)sulfonamido)-2-(6-azaspiro[2.5]octan-6-yl)benzamide;
(*R*)-4-((2-hydroxyethyl)sulfonamido)-N-(3-(2-methylmorpholino)phenyl)-2-(6-azaspiro[2.5]octan-6-yl)benzamide;
(*R*)-*N*-(2-fluoro-3-(2-methylmorpholino)phenyl)-4-((2-hydroxyethyl)sulfonamido)-2-(6-azaspiro[2.5]octan-6-yl)benzamide;
(*R*)-*N*-(3-fluoro-5-(2-methylmorpholino)phenyl)-4-((2-hydroxyethyl)sulfonamido)-2-(6-azaspiro[2.5]octan-6-yl)benzamide;
(*R*)-*N*-(4-fluoro-3-(2-methylmorpholino)phenyl)-4-((2-hydroxyethyl)sulfonamido)-2-(6-azaspiro[2.5]octan-6-yl)benzamide;
*N*-(3-(4,4-difluoropiperidin-1-yl)-5-methylphenyl)-4-((2-hydroxyethyl)sulfonamido)-2-(6-azaspiro[2.5]octan-6-yl)benzamide;
*N*-(3-(4,4-difluoropiperidin-1-yl)phenyl)-4-((2-hydroxyethyl)sulfonamido)-2-(6-azaspiro[2.5]octan-6-yl)benzamide;
*N*-(3-(4,4-difluoropiperidin-1-yl)-2-fluorophenyl)-4-((2-hydroxyethyl)sulfonamido)-2-(6-azaspiro[2.5]octan-6-yl)benzamide;
(*S*)-*N*-(3-(*N*-(*tert*-butyl)sulfamoyl)phenyl)-4-((2-hydroxy-1-methylethyl)sulfonamido)-2-(6-azaspiro[2.5]octan-6-yl)benzamide;
(*R*)-*N*-(3-(*N*-(*tert*-butyl)sulfamoyl)phenyl)-4-((2-hydroxy-1-methylethyl)sulfonamido)-2-(6-azaspiro[2.5]octan-6-yl)benzamide;
*N*-(3-(2-Hydroxy-2-methylpropoxy)phenyl)-4-((2-hydroxyethyl)sulfonamido)-2-(6-azaspiro[2.5]octan-6-yl)benzamide;
4-(Azetidin-1-ylsulfonyl)-*N*-(3-(*N*-(*tert*-butyl)sulfamoyl)phenyl)-2-(6-azaspiro[2.5]octan-6-yl)benzamide;
*N*-(3-(*N*-(*tert*-butyl)sulfamoyl)phenyl)-4-(*N*-(1-hydroxy-2-methylpropan-2-yl)sulfamoyl)-2-(6-azaspiro[2.5]octan-6-yl)benzamide;
*N*-(3-(*N*-(*tert*-butyl)sulfamoyl)phenyl)-4-(*N*-(2-hydroxyethyl)sulfamoyl)-2-(6-azaspiro[2.5]octan-6-yl)benzamide;
*N*-(3-(*N*-(*tert*-butyl)sulfamoyl)phenyl)-4-(*N,N-*dimethylsulfamoyl)-2-(6-azaspiro[2.5]octan-6-yl)benzamide;
*N*-(3-(*N*-(*tert*-butyl)sulfamoyl)phenyl)-4-(*N*-methylsulfamoyl)-2-(6-azaspiro[2.5]octan-6-yl)benzamide;
*N*-(3-(*N*-(*tert*-butyl)sulfamoyl)phenyl)-4-(N-(oxetan-3-yl)sulfamoyl)-2-(6-azaspiro[2.5]octan-6-yl)benzamide;
*N*-(3-(*N*-(*tert*-butyl)sulfamoyl)phenyl)-4-(N-cyclopropylsulfamoyl)-2-(6-azaspiro[2.5]octan-6-yl)benzamide;
*N*-(3-(*N*-(*tert*-butyl)sulfamoyl)phenyl)-4-(*N*-(1-methylcyclopropyl)sulfamoyl)-2-(6-azaspiro[2.5]octan-6-yl)benzamide;
*N*-(3-(*N*-(*tert*-butyl)sulfamoyl)phenyl)-2-(6-azaspiro[2.5]octan-6-yl)-4-sulfamoylbenzamide;
*(R)-N-(3-(N-(tert-butyl)sulfamoyl)phenyl)-4-(1,2-dihydroxypropan-2-yl)-2-(6-azaspiro[2.5]octan-*6-yl)benzamide;
(*S*)-*N*-(3-(*N*-(*tert*-butyl)sulfamoyl)phenyl)-4-(1,2-dihydroxypropan-2-yl)-2-(6-azaspiro[2.5]octan-6-yl)benzamide;
*N*-(3-(*N*-(*tert*-butyl)sulfamoyl)phenyl)-4-(1-methyl-1H-imidazol-2-yl)-2-(6-azaspiro[2.5]octan-6-yl)benzamide;
*N*-(3-(*N*-(*tert*-butyl)sulfamoyl)phenyl)-4-(1-methyl-1H-pyrazol-5-yl)-2-(6-azaspiro[2.5]octan-6-yl)benzamide;
*N*-(3-(*N*-(*tert*-butyl)sulfamoyl)phenyl)-4-(1-methyl-1H-pyrazol-4-yl)-2-(6-azaspiro[2.5]octan-6-yl)benzamide;
*N*-(3-(*N*-(*tert*-butyl)sulfamoyl)phenyl)-4-(oxazol-2-yl)-2-(6-azaspiro[2.5]octan-6-yl)benzamide;
*N*-(3-(*N*-(*tert*-Butyl)sulfamoyl)phenyl)-4-((1-hydroxy-2-methylpropan-2-yl)amino)-2-(6-azaspiro[2.5]octan-6-yl)benzamide;
*N*-(3-(*N*-(*tert*-butyl)sulfamoyl)phenyl)-4-((2-hydroxyethyl)amino)-2-(6-azaspiro[2.5]octan-6-yl)benzamide;
*N*¹-(3-(*N*-(*tert*-Butyl)sulfamoyl)phenyl)-*N*⁴-(2-hydroxyethyl)-2-(6-azaspiro[2.5]octan-6-yl)terephthalamide;
*N¹*-(3-(*N*-(*tert*-butyl)sulfamoyl)phenyl)-*N⁴*-methyl-2-(6-azaspiro[2.5]octan-6-yl)terephthalamide;
*N¹*-(3-(*N*-(*tert*-butyl)sulfamoyl)phenyl)-*N⁴-*(2-hydroxyethyl)-*N⁴-*methyl-2-(6-azaspiro[2.5]octan-6-yl)terephthalamide;
*N¹*-(3-(*N*-(*tert*-butyl)sulfamoyl)phenyl)-*N⁴*-(1-hydroxy-2-methylpropan-2-yl)-2-(6-azaspiro[2.5]octan-6-yl)terephthalamide;
*N¹*-(3-(cyclopentylsulfonyl)phenyl)-*N⁴*-(2-hydroxyethyl)-2-(6-azaspiro[2.5]octan-6-yl)terephthalamide;
(*R*)-*N*-(3-(Azetidine-1-sulfonimidoyl)phenyl)-4-((2-hydroxyethyl)sulfonamido)-2-(6-azaspiro[2.5]octan-6-yl)benzamide;
(*S*)-*N*-(3-(Azetidine-1-sulfonimidoyl)phenyl)-4-((2-hydroxyethyl)sulfonamido)-2-(6-azaspiro[2.5]octan-6-yl)benzamide;
(*R*)-4-((2-hydroxyethyl)sulfonamido)-*N*-(3-(*S*-methylsulfonimidoyl)phenyl)-2-(6-azaspiro[2.5]octan-6-yl)benzamide;
(*S*)-4-((2-hydroxyethyl)sulfonamido)-*N*-(3-(*S*-methylsulfonimidoyl)phenyl)-2-(6-azaspiro[2.5]octan-6-yl)benzamide;
(*R*)-4-((2-hydroxyethyl)sulfonamido)-*N*-(3-(2-methylpropan-2-ylsulfonimidoyl)phenyl)-2-(6-azaspiro[2.5]octan-6-yl)benzamide;
(*S*)-4-((2-hydroxyethyl)sulfonamido)-*N*-(3-(2-methylpropan-2-ylsulfonimidoyl)phenyl)-2-(6-azaspiro[2.5]octan-6-yl)benzamide; or
*N*-(4-((2-hydroxyethyl)sulfonamido)-2-(6-azaspiro[2.5]octan-6-yl)phenyl)-3-(piperidin-1-yl)benzamide.

24. The compound of any one of the previous Claims, selected from the group consisting of:
| Example # | Chemical Structure | Name |
|---|---|---|
| 100 | 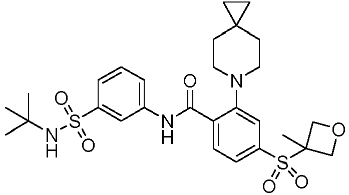 | N-(3-(N-(tert-Butyl)sulfamoyl)phenyl)-4-((3-methyloxetan-3-yl)sulfonyl)-2-(6-azaspiro[2.5]octan-6-yl)benzamide |
| 100-7 | 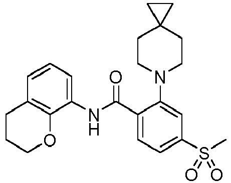 | *N*-(Chroman-8-yl)-4-(methylsulfonyl)-2-(6-azaspiro[2.5]octan-6-yl)benzamide |
| 100-11 | 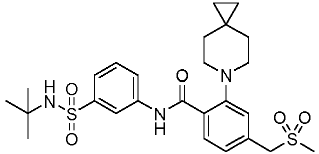 | *N*-(3-(*N-*(*tert-*Butyl)sulfamoyl)phenyl)-4-((methylsulfonyl)methyl)-2-(6-azaspiro[2.5]octan-6-yl)benzamide |
| 100-13 | 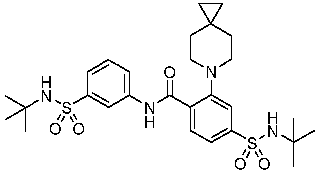 | 4-(*N*-(*tert*-Butyl)sulfamoyl)-N-(3-(*N*-(*tert*-butyl)sulfamoyl)phenyl)-2-(6-azaspiro[2.5]octan-6-yl)benzamide |
| 101 | 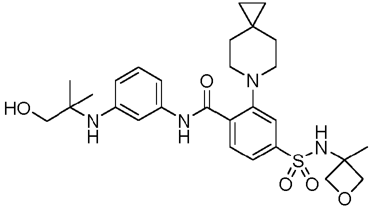 | N-(3-((1-Hydroxy-2-methylpropan-2-yl)amino)phenyl)-4-(N-(3-methyloxetan-3-yl)sulfamoyl)-2-(6-azaspiro[2.5]octan-6-yl)benzamide |
| 101-1 | 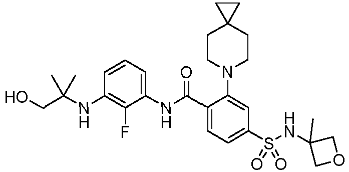 | *N*-(2-Fluoro-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamide |
| 102 | 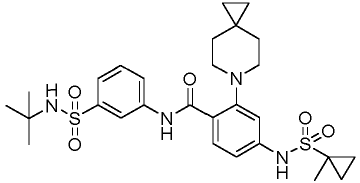 | N-(3-(N-(tert-Butyl)sulfamoyl)phenyl)-4-((1-methylcyclopropane)-1-sulfonamido)-2-(6-azaspiro[2.5]octan-6-yl)benzamide |
| 102-3 | 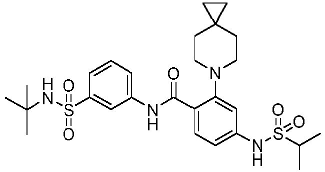 | *N*-(3-(*N*-(*tert-*Butyl)sulfamoyl)phenyl)-4-((1-methylethyl)sulfonamido)-2-(6-azaspiro[2.5]octan-6-yl)benzamide |
| 102-4 | 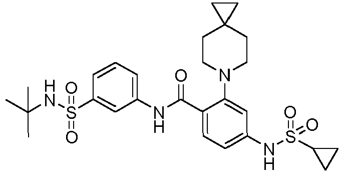 | *N-*(3-(*N-*(*tert-*Butyl)sulfamoyl)phenyl)-4-(cyclopropanesulfonamido)-2-(6-azaspiro[2.5]octan-6-yl)benzamide |
| 103 | 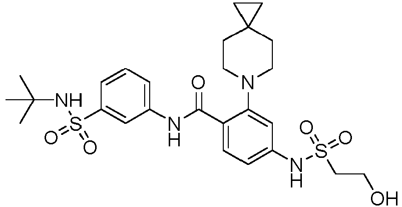 | N-(3-(N-(tert-Butyl)sulfamoyl)phenyl)-4-((2-hydroxyethyl)sulfonamido)-2-(6-azaspiro[2.5]octan-6-yl)benzamide |
| 104 | 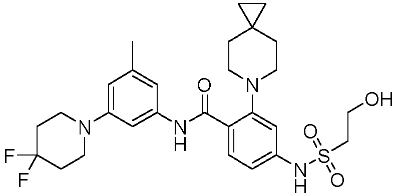 | *N*-(3-(4,4-Difluoropiperidin- 1-yl)-5-methylphenyl)-4-((2-hydroxyethyl)sulfonamido)-2-(6-azaspiro[2.5]octan-6-yl)benzamide |
| 104-1 | 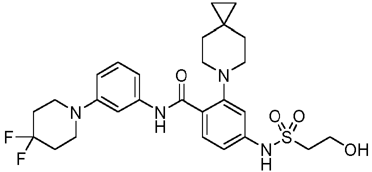 | *N*-(3-(4,4-Difluoropiperidin- 1-yl)phenyl)-4-((2-hydroxyethyl)sulfonamido)-2-(6-azaspiro[2.5]octan-6-yl)benzamide |
| 104-2 | 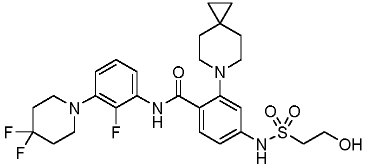 | *N*-(3-(4,4-Difluoropiperidin- 1-yl)-2-fluorophenyl)-4-((2-hydroxyethyl)sulfonamido)-2-(6-azaspiro[2.5]octan-6-yl)benzamide |
| 105-1 | 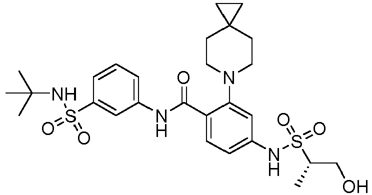 | (*S*)-*N*-(3-(*N-*(*tert-*Butyl)sulfamoyl)phenyl)-4-((2-hydroxy-1-methylethyl)sulfonamido)-2-(6-azaspiro[2.5]octan-6-yl)benzamide |
| 105-2 | 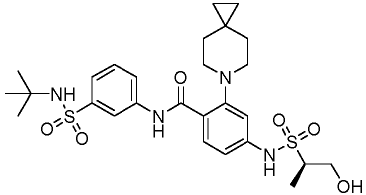 | (*R*)*-N*-(3-(*N*-(*tert-*Butyl)sulfamoyl)phenyl)-4-((2-hydroxy-1-methylethyl)sulfonamido)-2-(6-azaspiro[2.5]octan-6-yl)benzamide |
| 106 | 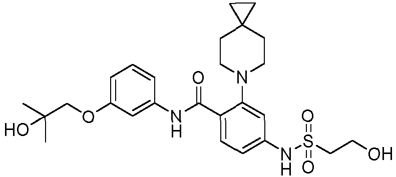 | *N*-(3-(2-Hydroxy-2-methylpropoxy)phenyl)-4-((2-hydroxyethyl)sulfonamido)-2-(6-azaspiro[2.5]octan-6-yl)benzamide |
| 107 | 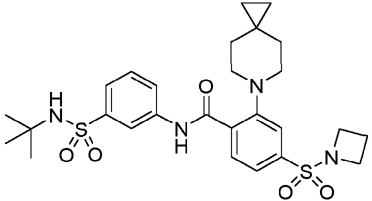 | 4-(Azetidin-1-ylsulfonyl)-*N*-(3-(*N*-(*tert*-butyl)sulfamoyl)phenyl)-2-(6-azaspiro[2.5]octan-6-yl)benzamide |
| 108-1 | 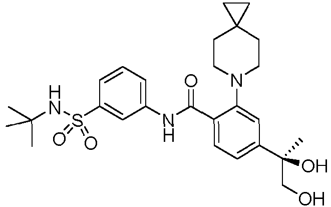 | (*R*)-*N*-(3-(*N*-(*tert-*Butyl)sulfamoyl)phenyl)-4-(1,2-dihydroxypropan-2-yl)-2-(6-azaspiro[2.5]octan-6-yl)benzamide |
| 108-2 | 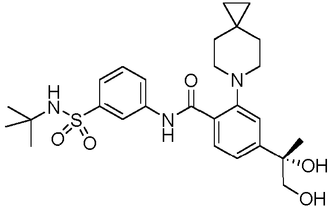 | (*S*)*-N*-(3-(*N*-(*tert-*Butyl)sulfamoyl)phenyl)-4-(1,2-dihydroxypropan-2-yl)-2-(6-azaspiro[2.5]octan-6-yl)benzamide |
| 109 | 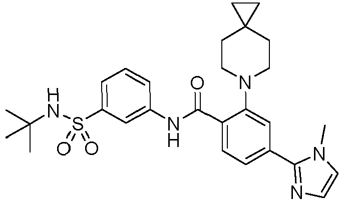 | *N-*(*3-*(*N-*(*tert-*Butyl)sulfamoyl)phenyl)-4-(1-methyl-1H-imidazol-2-yl)-2-(6-azaspiro[2.5]octan-6-yl)benzamide |
| 110 | 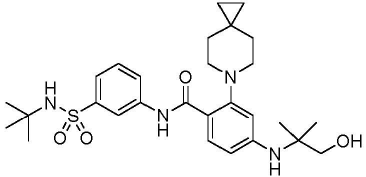 | *N-*(3*-*(*N-*(*tert-*Butyl)sulfamoyl)phenyl)-4-((1-hydroxy-2-methylpropan-2-yl)amino)-2-(6-azaspiro[2.5]octan-6-yl)benzamide |
| 111 | 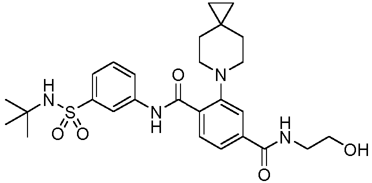 | *N*¹-(3-(*N*-(*tert-*Butyl)sulfamoyl)phenyl)-*N⁴*-(2-hydroxyethyl)-2-(6-azaspiro[2.5]octan-6-yl)terephthalamide |
| 112-1 | 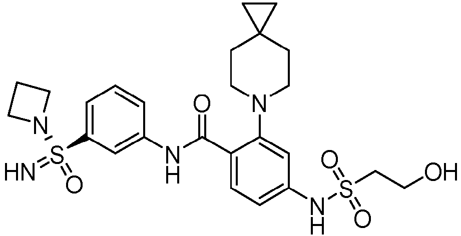 | (*R*)-*N*-(3-(Azetidine-1-sulfonimidoyl)phenyl)-4-((2-hydroxyethyl)sulfonamido)-2-(6-azaspiro[2.5]octan-6-yl)benzamide |
| 112-2 | 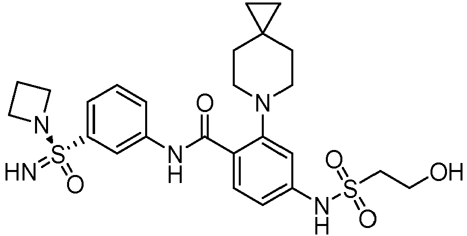 | (*S*)-*N*-(3-(Azetidine-1-sulfonimidoyl)phenyl)-4-((2-hydroxyethyl)sulfonamido)-2-(6-azaspiro[2.5]octan-6-yl)benzamide |
| 115 | 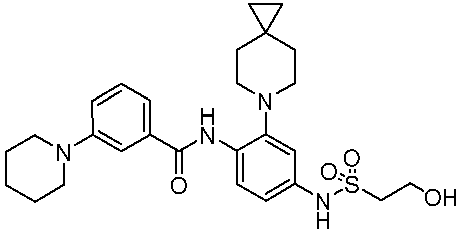 | *N-*(4-((2-Hydroxyethyl)sulfonamido)-2-(6-azaspiro[2.5]octan-6-yl)phenyl)-3-(piperidin-1-yl)benzamide |
; or any pharmaceutically-acceptable salt thereof.

25. A pharmaceutical composition comprising the compound according to any one of the previous Claims or the pharmaceutically acceptable salt thereof, and a pharmaceutically-acceptable diluent or carrier.

26. A compound according to claims 1-24 or the pharmaceutically acceptable salt thereof, or a pharmaceutical composition according to claim 25 for use as a medicine.

27. A compound according to claims 1-24 or the pharmaceutically acceptable salt thereof, or a pharmaceutical composition according to claim 25 for use in a method of treating a condition that may be treated with KIF18a inhibitors, the method comprising administering to a patient in need thereof a therapeutically effective amount of the compound in accordance with any one of claims 1 to 24, or the composition according to claim 25, wherein said condition is a proliferative disorder selected from cancer, psoriasis, atopic dermatitis, an autoimmune disorder, or inflammatory bowel disease; wherein said cancer can be melanoma, prostate cancer, cervical cancer, breast cancer, colon cancer, sarcoma, or leukemia; wherein said autoimmune disorder can be rheumatoid arthritis, systemic lupus erythematosus, Sjögren's syndrome, Scleroderma, mixed connective tissue disease, dermatomyositis, polymyositis, Reiter's syndrome, autoimmune lymphoproliferative syndrome (ALPS), also known as Canale-Smith syndrome, or autoimmune disease of the central nervous system, such as multiple Sclerosis, myasthenia gravis, and encephalomyelitis; and wherein the. inflammatory bowel disease can be ulcerative colitis or Crohn's Disease.

28. A compound according to claims 1-24 or the pharmaceutically acceptable salt thereof, or a pharmaceutical composition according to claim 25 for use in a method of reducing the size of a solid tumor in a subject, the method comprising administering to the subject in need thereof a therapeutically effective amount of the compound in accordance with any one of claims 1 to 24, or the composition according to claim 25.

29. A compound according to claims 1-24 or the pharmaceutically acceptable salt thereof, or a pharmaceutical composition according to claim 25 for use in a method of treating a cell proliferation disorder in a subject, the method comprising administering to the subject in need thereof a therapeutically effective amount of the compound in accordance with any one of claims 1 to 24, or the composition according to claim 25.

30. A compound according to claims 1-24 or the pharmaceutically acceptable salt thereof, or a pharmaceutical composition according to claim 25 for use in a *"ex vivo"* method of inhibiting KIF18A in a cell, comprising contacting the cell with a compound, or pharmaceutically acceptable salts thereof, in accordance with any one of claims 1 to 24, or the composition according to claim 25.

## Patentansprüche

1. Eine Verbindung der Formel I: 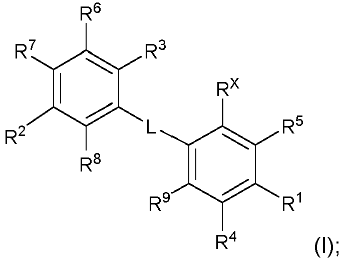 oder ein beliebiges pharmazeutisch annehmbares Salz davon, wobei:
R^{X} 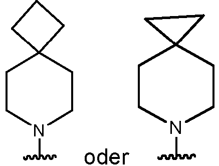 ist;
R¹ eine Gruppe -Z-R¹² ist; wobei Z nicht vorhanden, -C₀₋₄-Alkyl-S-C₀₋₄-Alkyl-, -C₀₋₄-Alkyl-S(=O)-C₀₋₄-Alkyl-, -C₀₋₄-Alkyl-SO₂-C₀₋₄-Alkyl-, -C₀₋₄-Alkyl-NR¹¹-C₀₋₄-Alkyl-, -C₀₋₄-Alkyl-NR¹¹SO₂-C₀₋₄-Alkyl-, -C₀₋₄-Alkyl-SO₂NR¹¹-C₀₋₄-Alkyl-, -C₀₋₄-Alkyl-NR¹¹SO₂NR¹¹-C₀₋₄-Alkyl-, -C₀₋₄-Alkyl-O-C₀₋₄-Alkyl-, -C₀₋₄-Alkyl-C(=O)-C₀₋₄-Alkyl-, -C₀₋₄-Alkyl-C(=O)-O-C₀₋₄-Alkyl-, -C₀₋₄-Alkyl-(C=O)NR¹¹-C₀₋₄-Alkyl-, -C₀₋₄-Alkyl-NR¹¹(C=O)-C₀₋₄-Alkyl-, -C₀₋₄-Alkyl-S(=O)(=NH)-, -N=S(=O)<, -(C=O)-
oder -C(=N-OH)- ist;
R² eine Gruppe -Y-R¹³ ist, wobei Y -C₀₋₄-Alkyl-S-C₀₋₄-Alkyl-, C₀₋₄-Alkyl-S(=O)-C₀₋₄-Alkyl-, -C₀₋₄-Alkyl-SO₂-C₀₋₄-Alkyl-, -C₀₋₄-Alkyl-NR^{13c}-C₀₋₄-Alkyl-, -C₀₋₄-Alkyl-SO₂NR^{13c}-C₀₋₄-Alkyl-, -C₀₋₄-Alkyl-NR^{13c}SO₂-C₀₋₄-Alkyl-, -C₀₋₄-Alkyl-S(=O)(=NH)-, -C₀₋₄-Alkyl-O-C₀₋₄-Alkyl-, -C₀₋₄-Alkyl-C(=O)-C₀₋₄-Alkyl-, -C₀₋₄-Alkyl-C(=O)-O-C₀₋₄-Alkyl-, -C₀₋₄-Alkyl-(C=O)NR^{13c}-C₀₋₄-Alkyl-, -C₀₋₄-Alkyl-NR^{13c}(C=O)-C₀₋₄-Alkyl- oder -N=S(=O)< ist;
R³ H, Halogen, C₁₋₄-Alkyl oder C₁₋₄-Halogenalkyl ist;
R⁴ H, Halogen, C₁₋₄-Alkyl oder C₁₋₄-Halogenalkyl ist;
R⁵ H, Halogen, C₁₋₈-Alkyl oder C₁₋₄-Halogenalkyl ist;
R⁶ H, Halogen, C₁₋₈-Alkyl oder C₁₋₄-Halogenalkyl ist;
R⁷ H, Halogen, C₁₋₄-Alkyl oder C₁₋₄-Halogenalkyl ist;
R⁸ H, Halogen, C₁₋₈-Alkyl oder C₁₋₄-Halogenalkyl ist;
oder alternativ R² und R⁸ sich mit den jeweils daran gebundenen Kohlenstoffatomen verbinden können, sodass sie einen gesättigten oder teilweise gesättigten 5- oder 6-gliedrigen monocyclischen Ring bilden, der an den Phenylring anelliert ist; wobei der 5-oder 6-gliedrige monocyclische Ring 0, 1, 2 oder 3 N-Atome und 0 oder 1 aus O und S ausgewählte Atome enthält und wobei ferner der 5- oder 6-gliedrige monocyclische Ring mit 0, 1, 2 oder 3 Gruppe(n), ausgewählt aus F, Cl, Br, C₁₋₆-Alkyl, C₁₋₄-Halogenalkyl, -OR^{a}, -OC₁₋₄-Halogenalkyl, CN, -NR^{a}R^{a} oder Oxo, substituiert ist; R⁹ H, Halogen, C₁₋₄-Alkyl oder C₁₋₄-Halogenalkyl ist;
L -(C=O)-NR¹⁰- oder -NR¹⁰-(C=O)- ist;
R¹⁰ H oder C₁₋₄-Alkyl ist;
R¹¹ H oder C₁₋₄-Alkyl ist;
R¹² R^{12a} oder R^{12b} ist;
R¹³ Halogen, R^{13a} oder R^{13b} ist;
R^{13c} H oder C₁₋₄-Alkyl ist;
R^{12a} und R^{13a} unabhängig in jedem Fall aus der Gruppe ausgewählt sind, die aus einem gesättigten, teilweise gesättigten oder ungesättigten 3-, 4-, 5-, 6- oder 7-gliedrigen monocyclischen oder 8-, 9-, 10-, 11- oder 12-gliedrigen bicyclischen Ring, der 0, 1, 2 oder 3 N-Atome und 0 oder 1 aus O und S ausgewählte Atome enthält und mit 0, 1, 2 oder 3 Gruppe(n), ausgewählt aus der Gruppe, bestehend aus F, Cl, Br, C₁₋₆-Alkyl, C₁₋₄-Halogenalkyl, -OR^{a}, -OC₁₋₄-Halogenalkyl, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆-Alkyl-NR^{a}R^{a}, -OC₂₋₆-Alkyl-OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-Alkyl-NR^{a}R^{a}, -NR^{a}C₂₋₆-Alkyl-OR^{a}, -C₁₋₆-Alkyl-NR^{a}R^{a}, -C₁₋₆-Alkyl-OR^{a}, -C₁₋₆-Alkyl-N(R^{a})C(=O)R^{b}, -C₁₋₆-Alkyl-OC(=O)R^{b}, -C₁₋₆-Alkyl-C(=O)NR^{a}R^{a}, -C₁₋₆-Alkyl-C(=O)OR^{a}, einem gesättigten, teilweise gesättigten oder ungesättigten 3-, 4-, 5- oder 6-gliedrigen monocyclischen Ring und Oxo, substituiert ist, besteht;
R^{12b} und R^{13b} unabhängig in jedem Fall aus der Gruppe ausgewählt sind, die aus C₁₋₆-Alkyl, das mit 0, 1, 2, 3, 4 oder 5 Gruppe(n), ausgewählt aus der Gruppe, bestehend aus F, Cl, Br, -CH₂F, -CHF₂, -CF₃, -C(=O)OR^{a}, -OR^{a}, -OC₁₋₄-Halogenalkyl, CN, NH₂, NH(CH₃), N(CH₃)₂ und einem gesättigten, teilweise gesättigten oder ungesättigten 3-, 4-, 5- oder 6-gliedrigen monocyclischen Ring, substituiert ist, besteht;
R^{a} unabhängig in jedem Fall H oder R^{b} ist; und
R^{b} unabhängig in jedem Fall C₁₋₆-Alkyl, Phenyl oder Benzyl ist, wobei das C₁₋₆-Alkyl mit 0, 1, 2 oder 3 Substituenten, ausgewählt aus Halogen, -OH, -OC₁₋₄-Alkyl, -NH₂, -NHC₁₋₄-Alkyl, -OC(=O)C₁₋₄-Alkyl oder -N(C₁₋₄-Alkyl)C₁₋₄-Alkyl, substituiert ist; und das Phenyl oder Benzyl mit 0, 1, 2 oder 3 Substituenten, ausgewählt aus Halogen, C₁₋₄-Alkyl, C₁₋₃-Halogenalkyl, -OH, -OC₁₋₄-Alkyl, -NH₂, -NHC₁₋₄-Alkyl, -OC(=O)C₁₋₄-Alkyl oder -N(C₁₋₄-Alkyl)C₁₋₄-Alkyl, substituiert ist.

2. Verbindung gemäß Anspruch 1, wobei L -NR¹⁰-(C=O)- ist.

3. Verbindung gemäß Anspruch 1, wobei L -(C=O)-NR¹⁰- ist.

4. Verbindung gemäß Anspruch 1, wobei R¹⁰ H oder Methyl ist.

5. Verbindung gemäß Anspruch 1, wobei Z nicht vorhanden, -SO₂-, -CH₂-SO₂, -NH-, -NHSO₂-, -SO₂NH-, -SO₂N(CH₃)-, -O-, -(C=O)O-, -(C=O)NH-, -(C=O)N(CH₃)-, -S(=O)(=NH)-, -CH₂-N(CH₃)- oder -C(=N-OH)- ist.

6. Verbindung gemäß Anspruch 1, wobei R¹² aus Folgendem ausgewählt ist:
a) H, F, Br, OH oder CN;
b) R^{12a}, ausgewählt aus einem gesättigten, teilweise gesättigten oder ungesättigten 3-, 4-, 5-, 6- oder 7-gliedrigen monocyclischen Ring, der 0, 1, 2 oder 3 N-Atome und 0 oder 1 aus O und S ausgewählte Atome enthält und mit 0, 1, 2 oder 3 Gruppe(n), ausgewählt aus F, Cl, Br, Methyl, Ethyl, -CF₃, -CH₂OH, -OH, -OCH₃, -NH₂ oder Oxo, substituiert ist; oder
c) R^{12b}, ausgewählt aus C₁₋₆-Alkyl, das mit 0, 1, 2 oder 3 Gruppe(n), ausgewählt aus F, Cl, Br, -CF₃ oder -OH, substituiert ist.

7. Verbindung gemäß Anspruch 1, wobei R¹² eines der Folgenden ist: R^{12a}, ausgewählt aus Cyclopropyl, Oxetanyl, Imidazolyl, Isothiazolidinyl, Azetidinyl, Oxazolyl, Pyrazolyl oder Diazirinyl, die jeweils unabhängig mit 0, 1, 2 oder 3 Gruppe(n), ausgewählt aus Methyl, Ethyl, -CF₃ oder Oxo, substituiert sind; oder R^{12b}, ausgewählt aus Methyl, Ethyl, Isopropyl, tert-Butyl,-ethenyl, substituiert mit 0, 1, 2 oder 3 Gruppe(n), ausgewählt aus F, Cl, Br, -CF₃ oder -OH.

8. Verbindung gemäß Anspruch 1, wobei R¹ eine Gruppe -Z-R¹² ist, wobei Z nicht vorhanden, -SO₂-, -CH₂SO₂-, -(C=O)NH-, -NH-, -NHSO₂- oder -SO₂NH- ist; und R¹² ein Cyclopropyl-, Oxetanyl-, Azetidinyl- oder Imidazolylring ist, die jeweils unabhängig mit 0, 1 oder 2 Gruppe(n), ausgewählt aus Methyl, -CF₃ oder Oxo, substituiert sind; oder R¹² Methyl, Ethyl, Isopropyl oder *tert*-Butyl ist, die jeweils unabhängig mit 0, 1, 2 oder 3 F-, -CF₃- oder OH-Gruppe(n) substituiert sind.

9. Verbindung gemäß Anspruch 1, wobei R¹ eine Gruppe -Z-R¹² ist, wobei Z -NHSO₂- ist und R¹² -CH₂-CH₂-OH oder -CH(CH₃)CH₂OH ist.

10. Verbindung gemäß Anspruch 1, wobei Y nicht vorhanden, -SO₂NH-, NH-, - SO₂-, -S(=O)(=NH)- oder -O- ist.

11. Verbindung gemäß Anspruch 1, wobei R¹³ eines der Folgenden ist: H oder F; R^{13a}, ausgewählt aus Morpholinyl, Piperidinyl, Cyclopentyl, Cyclopropyl, Azetidinyl oder Oxetanyl; wobei jeder dieser Ringe mit 0, 1, 2 oder 3 OH-Gruppe(n), ausgewählt aus Methyl oder -OH, substituiert ist; oder R^{13b}, ausgewählt aus Methyl, Ethyl, Propyl, Isopropyl, *tert*-Butyl oder Isopentyl, die jeweils unabhängig mit 0, 1, 2 oder 3 OH-Gruppe(n) substituiert sind.

12. Verbindung gemäß Anspruch 1, wobei R² und R⁸ sich mit den jeweils daran gebundenen Kohlenstoffatomen verbinden können, sodass sie einen gesättigten oder teilweise gesättigten 6-gliedrigen monocyclischen Ring bilden, der an den Phenylring anelliert ist; wobei der 6-gliedrige monocyclische Ring 0, 1, 2 oder 3 N-Atome und 0 oder 1 aus O und S ausgewählte Atome enthält und wobei ferner der 6-gliedrige monocyclische Ring mit 0, 1, 2 oder 3 Gruppe(n), ausgewählt aus F, Cl, Br, C₁₋₆-Alkyl, C₁₋₄-Halogenalkyl oder Oxo, substituiert ist.

13. Verbindung gemäß Anspruch 1, wobei R⁴ H ist.

14. Verbindung gemäß Anspruch 1, wobei R⁵ H ist.

15. Verbindung gemäß Anspruch 1, wobei R⁶ H oder F ist.

16. Verbindung gemäß Anspruch 1, wobei R⁷ H oder F ist.

17. Verbindung gemäß Anspruch 1, wobei R⁸ H, F oder Methyl ist; oder alternativ R² und R⁸ sich mit den jeweils daran gebundenen Kohlenstoffatomen verbinden können, sodass sie einen gesättigten 6-gliedrigen monocyclischen Ring bilden, der an den Phenylring anelliert ist; ausgewählt aus der folgenden Gruppe:

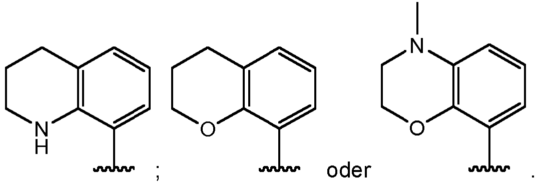

18. Verbindung gemäß Anspruch 1, wobei R² Folgendes ist:
a) eine Gruppe -Y-R^{13a}, wobei Y nicht vorhanden oder -S(=O)(=NH)- ist; und R¹³ Piperidinyl oder Azetidinyl ist; wobei jeder dieser Ringe unabhängig mit 0, 1, 2 oder 3 F-Gruppe(n) substituiert ist;
b) eine Gruppe -Y-R^{13b}, wobei Y -SO₂NH-, -O-, NH- ist; und wobei R^{13b} mit 0, 1, 2 oder 3 OH-Gruppe(n) substituiertes tert-Butyl ist; oder
c) alternativ die an R² und R⁸ gebundenen Kohlenstoffatome sich verbinden, sodass sie einen gesättigten 6-gliedrigen monocyclischen Ring bilden, der an den Phenylring anelliert ist; was 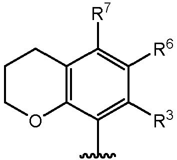 ist; wobei der 6-gliedrige monocyclische Ring nicht substituiert ist.

19. Verbindung gemäß Anspruch 1, wobei R² eine -SO₂NH-*tert*-Butyl-Gruppe oder -NH-*tert-*Butyl-OH-Gruppe ist.

20. Verbindung gemäß Anspruch 1, wobei R⁸ H ist.

21. Verbindung gemäß Anspruch 1, wobei R⁹ H ist.

22. Verbindung gemäß Anspruch 1, wobei R¹⁰ H ist.

23. Verbindung gemäß Anspruch 1 oder das pharmazeutisch annehmbare Salz davon, ausgewählt aus der Gruppe, die aus Folgendem besteht:
N-(3-(N-(tert-Butyl)sulfamoyl)phenyl)-4-((3-methyloxetan-3-yl)sulfonyl)-2-(6-azaspiro[2.5]octan-6-yl)benzamid;
*N*-(3-(*N*-(*tert*-Butyl)sulfamoyl)phenyl)-4-(methylsulfonyl)-2-(6-azaspiro[2.5]octan-6-yl)benzamid;
*N*-(3-Isopropylphenyl)-4-(methylsulfonyl)-2-(6-azaspiro[2.5]octan-6-yl)benzamid;
*N*-(3-Cyclopropylphenyl)-4-(methylsulfonyl)-2-(6-azaspiro[2.5]octan-6-yl)benzamid;
*N*-(3-(*tert*-Butyl)phenyl)-4-(methylsulfonyl)-2-(6-azaspiro[2.5]octan-6-yl)benzamid;
4-(Methylsulfonyl)-*N*-(chinolin-8-yl)-2-(6-azaspiro[2.5]octan-6-yl)benzamid;
*N*-(4-Methylchinolin-8-yl)-4-(methylsulfonyl)-2-(6-azaspiro[2.5]octan-6-yl)benzamid;
*N*-(Chroman-8-yl)-4-(methylsulfonyl)-2-(6-azaspiro[2.5]octan-6-yl)benzamid;
*N*-(Benzofuran-7-yl)-4-(methylsulfonyl)-2-(6-azaspiro[2.5]octan-6-yl)benzamid;
*N*-(Benzo[b]thiophen-7-yl)-4-(methylsulfonyl)-2-(6-azaspiro[2.5]octan-6-yl)benzamid;
4-(Methylsulfonyl)-*N*-(3-morpholinophenyl)-2-(6-azaspiro[2.5]octan-6-yl)benzamid;
*N*-(3-(*N*-(*tert*-Butyl)sulfamoyl)phenyl)-4-((methylsulfonyl)methyl)-2-(6-azaspiro[2.5]octan-6-yl)benzamid;
*N*-(3-(*N*-(*tert*-Butyl)sulfamoyl)phenyl)-4-(((2-hydroxyethyl)sulfonyl)methyl)-2-(6-azaspiro[2.5]octan-6-yl)benzamid;
4-(*N*-(*tert*-Butyl)sulfamoyl)-N-(3-(*N*-(*tert*-butyl)sulfamoyl)phenyl)-2-(6-azaspiro[2.5]octan-6-yl)benzamid;
*N*-(3-(*N*-(*tert*-Butyl)sulfamoyl)phenyl)-4-(3-methyloxetan-3-yl)-2-(6-azaspiro[2.5]octan-6-yl)benzamid;
*N*-(3-(*N*-(*tert*-Butyl)sulfamoyl)phenyl)-4-(3-hydroxyoxetan-3-yl)-2-(6-azaspiro[2.5]octan-6-yl)benzamid;
N-(3-((1-Hydroxy-2-methylpropan-2-yl)amino)phenyl)-4-(N-(3-methyloxetan-3-yl)sulfamoyl)-2-(6-azaspiro[2.5]octan-6-yl)benzamid;
*N*-(2-Fluor-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamid;
*N*-(2-Fluor-3-((1-hydroxy-2-methylpropan-2-yl)amino)phenyl)-4-((1-methylcyclopropan)-1-sulfonamido)-2-(6-azaspiro[2.5]octan-6-yl)benzamid;
*N*-(3-((1-Hydroxy-2-methylpropan-2-yl)amino)phenyl)-4-((1-methylcyclopropan)-1-sulfonamido)-2-(6-azaspiro[2.5]octan-6-yl)benzamid;
N-(3-(N-(tert-Butyl)sulfamoyl)phenyl)-4-((1-methylcyclopropan)-1-sulfonamido)-2-(6-azaspiro[2.5]octan-6-yl)benzamid;
*N*-(3-(*N*-(*tert*-Butyl)sulfamoyl)phenyl)-4-(methylsulfonamido)-2-(6-azaspiro[2.5]octan-6-yl)benzamid;
*N*-(3-(*N*-(*tert*-Butyl)sulfamoyl)phenyl)-4-(ethylsulfonamido)-2-(6-azaspiro[2.5]octan-6-yl)benzamid;
*N*-(3-(*N*-(*tert*-Butyl)sulfamoyl)phenyl)-4-((1-methylethyl)sulfonamido)-2-(6-azaspiro[2.5]octan-6-yl)benzamid;
*N*-(3-(*N*-(*tert*-Butyl)sulfamoyl)phenyl)-4-(cyclopropansulfonamido)-2-(6-azaspiro[2.5]octan-6-yl)benzamid;
*N*-(3-(*N*-(*tert*-Butyl)sulfamoyl)phenyl)-4-((1,1-dimethylethyl)sulfonamido)-2-(6-azaspiro[2.5]octan-6-yl)benzamid;
*N*-(3-(*N*-(*tert*-Butyl)sulfamoyl)phenyl)-4-(1,1-dioxidoisothiazolidin-2-yl)-2-(6-azaspiro[2.5]octan-6-yl)benzamid;
N-(3-(N-(tert-Butyl)sulfamoyl)phenyl)-4-((2-hydroxyethyl)sulfonamido)-2-(6-azaspiro[2.5]octan-6-yl)benzamid;
(*R*)-4-((2-Hydroxyethyl)sulfonamido)-*N*-(3-(2-methylmorpholino)phenyl)-2-(6-azaspiro[2.5]octan-6-yl)benzamid;
(*R*)-*N*-(2-Fluor-3-(2-methylmorpholino)phenyl)-4-((2-hydroxyethyl)sulfonamido)-2-(6-azaspiro[2.5]octan-6-yl)benzamid;
(*R*)-*N*-(3-Fluor-5-(2-methylmorpholino)phenyl)-4-((2-hydroxyethyl)sulfonamido)-2-(6-azaspiro[2.5]octan-6-yl)benzamid;
(*R*)-*N*-(4-Fluor-3-(2-methylmorpholino)phenyl)-4-((2-hydroxyethyl)sulfonamido)-2-(6-azaspiro[2.5]octan-6-yl)benzamid;
*N*-(3-(4,4-Difluorpiperidin-1-yl)-5-methylphenyl)-4-((2-hydroxyethyl)sulfonamido)-2-(6-azaspiro[2.5]octan-6-yl)benzamid;
*N*-(3-(4,4-Difluorpiperidin-1-yl)phenyl)-4-((2-hydroxyethyl)sulfonamido)-2-(6-azaspiro[2.5]octan-6-yl)benzamid;
*N*-(3-(4,4-Difluorpiperidin-1-yl)-2-fluorphenyl)-4-((2-hydroxyethyl)sulfonamido)-2-(6-azaspiro[2.5]octan-6-yl)benzamid;
(*S*)-*N*-(3-(*N*-(*tert*-Butyl)sulfamoyl)phenyl)-4-((2-hydroxy-1-methylethyl)sulfonamido)-2-(6-azaspiro[2.5]octan-6-yl)benzamid;
(*R*)-*N*-(3-(*N*-(*tert*-Butyl)sulfamoyl)phenyl)-4-((2-hydroxy-1-methylethyl)sulfonamido)-2-(6-azaspiro[2.5]octan-6-yl)benzamid;
*N*-(3-(2-Hydroxy-2-methylpropoxy)phenyl)-4-((2-hydroxyethyl)sulfonamido)-2-(6-azaspiro[2.5]octan-6-yl)benzamid;
4-(Azetidin-1-ylsulfonyl)-*N*-(3-(*N*-(*tert*-butyl)sulfamoyl)phenyl)-2-(6-azaspiro[2.5]octan-6-yl)benzamid;
*N*-(3-(*N*-(*tert*-Butyl)sulfamoyl)phenyl)-4-(*N*-(1-hydroxy-2-methylpropan-2-yl)sulfamoyl)-2-(6-azaspiro[2.5]octan-6-yl)benzamid;
*N*-(3-(*N*-(*tert*-Butyl)sulfamoyl)phenyl)-4-(*N*-(2-hydroxyethyl)sulfamoyl)-2-(6-azaspiro[2.5]octan-6-yl)benzamid;
*N*-(3-(*N*-(*tert*-Butyl)sulfamoyl)phenyl)-4-(*N*,*N*-dimethylsulfamoyl)-2-(6-azaspiro[2.5]octan-6-yl)benzamid;
*N*-(3-(*N*-(*tert*-Butyl)sulfamoyl)phenyl)-4-(*N*-methylsulfamoyl)-2-(6-azaspiro[2.5]octan-6-yl)benzamid;
*N*-(3-(*N*-(*tert*-Butyl)sulfamoyl)phenyl)-4-(N-(oxetan-3-yl)sulfamoyl)-2-(6-azaspiro[2.5]octan-6-yl)benzamid;
*N*-(3-(*N*-(*tert*-Butyl)sulfamoyl)phenyl)-4-(*N*-cyclopropylsulfamoyl)-2-(6-azaspiro[2.5]octan-6-yl)benzamid;
*N*-(3-(*N*-(*tert*-Butyl)sulfamoyl)phenyl)-4-(*N*-(1-methylcyclopropyl)sulfamoyl)-2-(6-azaspiro[2.5]octan-6-yl)benzamid;
*N*-(3-(*N*-(*tert*-Butyl)sulfamoyl)phenyl)-2-(6-azaspiro[2.5]octan-6-yl)-4-sulfamoylbenzamid;
(*R*)-*N*-(3-(*N*-(*tert*-Butyl)sulfamoyl)phenyl)-4-(1,2-dihydroxypropan-2-yl)-2-(6-azaspiro[2.5]octan-6-yl)benzamid;
(*S*)-*N*-(3-(*N*-(*tert*-Butyl)sulfamoyl)phenyl)-4-(1,2-dihydroxypropan-2-yl)-2-(6-azaspiro[2.5]octan-6-yl)benzamid;
*N*-(3-(*N*-(*tert*-Butyl)sulfamoyl)phenyl)-4-(1-methyl-1H-imidazol-2-yl)-2-(6-azaspiro[2.5]octan-6-yl)benzamid;
*N*-(3-(*N*-(*tert*-Butyl)sulfamoyl)phenyl)-4-(1-methyl-1H-pyrazol-5-yl)-2-(6-azaspiro[2.5]octan-6-yl)benzamid;
*N-*(3-(*N*-(*tert*-Butyl)sulfamoyl)phenyl)-4-(1-methyl-1H-pyrazol-4-yl)-2-(6-azaspiro[2.5]octan-6-yl)benzamid;
*N*-(3-(*N*-(*tert*-Butyl)sulfamoyl)phenyl)-4-(oxazol-2-yl)-2-(6-azaspiro[2.5]octan-6-yl)benzamid;
*N*-(3-(*N*-(*tert*-Butyl)sulfamoyl)phenyl)-4-((1-hydroxy-2-methylpropan-2-yl)amino)-2-(6-azaspiro[2.5]octan-6-yl)benzamid;
*N*-(3-(*N*-(*tert*-Butyl)sulfamoyl)phenyl)-4-((2-hydroxyethyl)amino)-2-(6-azaspiro[2.5]octan-6-yl)benzamid;
*N*¹-(3-(*N*-(*tert*-Butyl)sulfamoyl)phenyl)-*N⁴*-(2-hydroxyethyl)-2-(6-azaspiro[2.5]octan-6-yl)terephthalamid;
*N¹*-(3-(*N*-(*tert*-Butyl)sulfamoyl)phenyl)-*N⁴*-methyl-2-(6-azaspiro[2.5]octan-6-yl)terephthalamid;
*N*¹-(3-(*N*-(*tert*-Butyl)sulfamoyl)phenyl)-*N⁴*-(2-hydroxyethyl)-*N⁴*-methyl-2-(6-azaspiro[2.5]octan-6-yl)terephthalamid;
*N¹*-(3-(*N*-(*tert*-Butyl)sulfamoyl)phenyl)-*N⁴*-(1-hydroxy-2-methylpropan-2-yl)-2-(6-azaspiro[2.5]octan-6-yl)terephthalamid;
*N¹*-(3-(Cyclopentylsulfonyl)phenyl)-*N⁴*-(2-hydroxyethyl)-2-(6-azaspiro[2.5]octan-6-yl)terephthalamid;
(*R*)-*N*-(3-(Azetidin-1-sulfonimidoyl)phenyl)-4-((2-hydroxyethyl)sulfonamido)-2-(6-azaspiro[2.5]octan-6-yl)benzamid;
(*S*)-*N*-(3-(Azetidin-1-sulfonimidoyl)phenyl)-4-((2-hydroxyethyl)sulfonamido)-2-(6-azaspiro[2.5]octan-6-yl)benzamid;
(*R*)-4-((2-Hydroxyethyl)sulfonamido)-*N*-(3-(*S*-methylsulfonimidoyl)phenyl)-2-(6-azaspiro[2.5]octan-6-yl)benzamid;
(*S*)-4-((2-Hydroxyethyl)sulfonamido)-*N*-(3-(*S*-methylsulfonimidoyl)phenyl)-2-(6-azaspiro[2.5]octan-6-yl)benzamid;
(*R*)-4-((2-Hydroxyethyl)sulfonamido)-*N*-(3-(2-methylpropan-2-ylsulfonimidoyl)phenyl)-2-(6-azaspiro[2.5]octan-6-yl)benzamid;
(*S*)-4-((2-Hydroxyethyl)sulfonamido)-*N*-(3-(2-methylpropan-2-ylsulfonimidoyl)phenyl)-2-(6-azaspiro[2.5]octan-6-yl)benzamid; oder
*N*-(4-((2-Hydroxyethyl)sulfonamido)-2-(6-azaspiro[2.5]octan-6-yl)phenyl)-3-(piperidin-1-yl)benzamid.

24. Verbindung gemäß einem der vorhergehenden Ansprüche, ausgewählt aus der Gruppe, die aus Folgendem besteht:
| Beispiel Nr. | chemische Struktur | Bezeichnung |
|---|---|---|
| 100 | 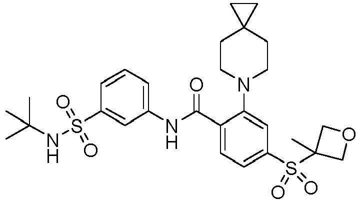 | N-(3-(N-(tert-Butyl)sulfamoyl)phenyl)-4-((3-methyloxetan-3-yl)sulfonyl)-2-(6-azaspiro[2.5]octan-6-yl)benzamid |
| 100-7 | 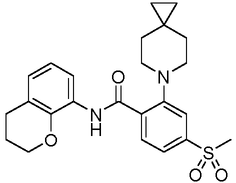 | *N*-(Chroman-8-yl)-4-(methylsulfonyl)-2-(6-azaspiro[2.5]octan-6-yl)benzamid |
| 100-11 | 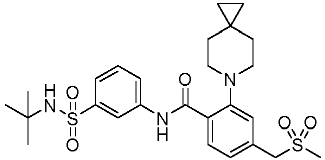 | *N*-(3-(*N*-(*tert-*Butyl)sulfamoyl)phenyl)-4-((methylsulfonyl)methyl)-2-(6-azaspiro[2.5]octan-6-yl)benzamid |
| 100-13 | 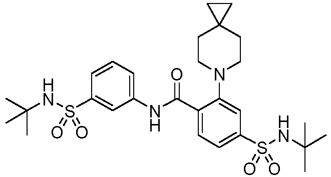 | 4-(*N*-(*tert*-Butyl)sulfamoyl)-N-(3-(*N*-(*tert-*butyl)sulfamoyl)phenyl)-2-(6-azaspiro[2.5]octan-6-yl)benzamid |
| 101 | 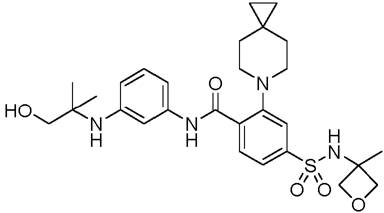 | N-(3-((1-Hydroxy-2-methylpropan-2-yl)amino)phenyl)-4-(N-(3-methyloxetan-3-yl)sulfamoyl)-2-(6-azaspiro[2.5]octan-6-yl)benzamid |
| 101-1 | 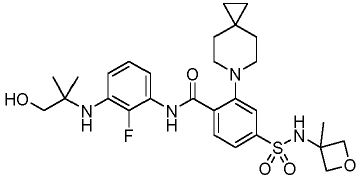 | *N*-(2-Fluor-3-((1-hydroxy-2-methylpropan-2-yl)amino)phenyl)-4-(*N*-(3-methyloxetan-3-yl)sulfamoyl)-2-(6-azaspiro[2.5]octan-6-yl)benzamid |
| 102 | 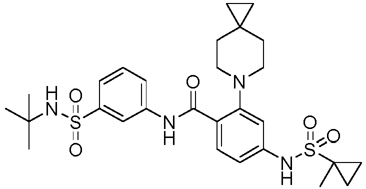 | N-(3-(N-(tert-Butyl)sulfamoyl)phenyl)-4-((1-methylcyclopropan)-1-sulfonamido)-2-(6-azaspiro[2.5]octan-6-yl)benzamid |
| 102-3 | 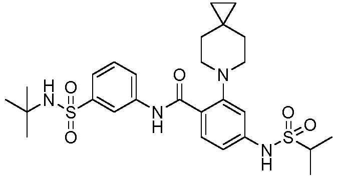 | *N*-(3-(*N*-(*tert-*Butyl)sulfamoyl)phenyl)-4-((1-methylethyl)sulfonamido)-2-(6-azaspiro[2.5]octan-6-yl)benzamid |
| 102-4 | 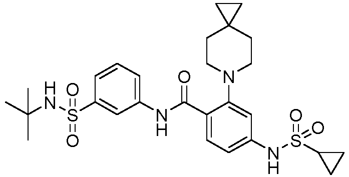 | *N*-(3-(*N*-(*tert-*Butyl)sulfamoyl)phenyl)-4-(cyclopropansulfonamido)-2-(6-azaspiro[2.5]octan-6-yl)benzamid |
| 103 | 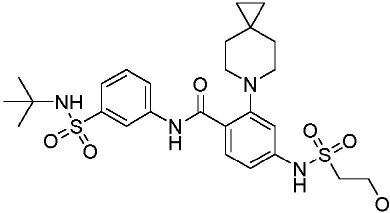 | N-(3-(N-(tert-Butyl)sulfamoyl)phenyl)-4-((2-hydroxyethyl)sulfonamido)-2-(6-azaspiro[2.5]octan-6-yl)benzamid |
| 104 | 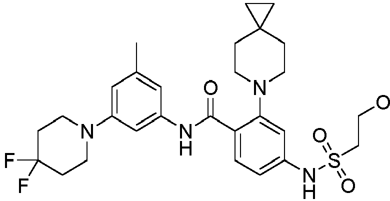 | *N*-(3-(4,4-Difluorpiperidin-1-yl)-5-methylphenyl)-4-((2-hydroxyethyl)sulfonamido)-2-(6-azaspiro[2.5]octan-6-yl)benzamid |
| 104-1 | 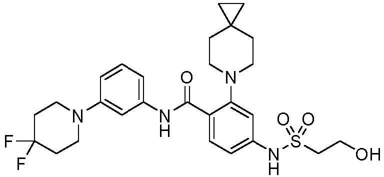 | *N*-(3-(4,4-Difluorpiperidin-1-yl)phenyl)-4-((2-hydroxyethyl)sulfonamido)-2-(6-azaspiro[2.5]octan-6-yl)benzamid |
| 104-2 | 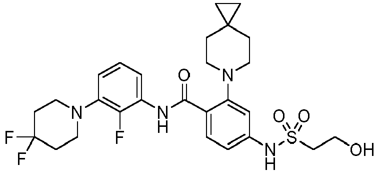 | *N*-(3-(4,4-Difluorpiperidin-1-yl)-2-fluorphenyl)-4-((2-hydroxyethyl)sulfonamido)-2-(6-azaspiro[2.5]octan-6-yl)benzamid |
| 105-1 | 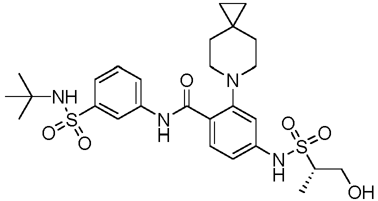 | (*S*)-*N*-(3-(*N*-(*tert-*Butyl)sulfamoyl)phenyl)-4-((2-hydroxy-1-methylethyl)sulfonamido)-2-(6-azaspiro[2.5]octan-6-yl)benzamid |
| 105-2 | 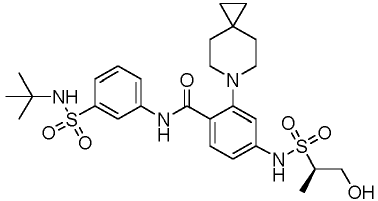 | (*R*)-*N*-(3-(*N*-(*tert-*Butyl)sulfamoyl)phenyl)-4-((2-hydroxy-1-methylethyl)sulfonamido)-2-(6-azaspiro[2.5]octan-6-yl)benzamid |
| 106 | 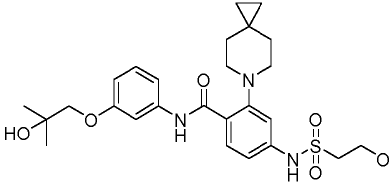 | *N*-(3-(2-Hydroxy-2-methylpropoxy)phenyl)-4-((2-hydroxyethyl)sulfonamido)-2-(6-azaspiro[2.5]octan-6-yl)benzamid |
| 107 | 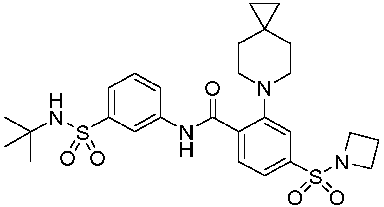 | 4-(Azetidin-1-ylsulfonyl)-*N-*(3-(*N*-(*tert-*butyl)sulfamoyl)phenyl)-2-(6-azaspiro[2.5]octan-6-yl)benzamid |
| 108-1 | 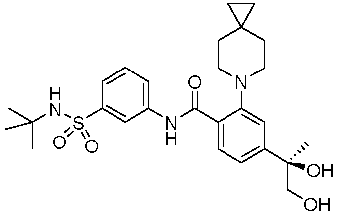 | (*R*)-*N*-(3-(*N*-(*tert-*Butyl)sulfamoyl)phenyl)-4-(1,2-dihydroxypropan-2-yl)-2-(6-azaspiro[2.5]octan-6-yl)benzamid |
| 108-2 | 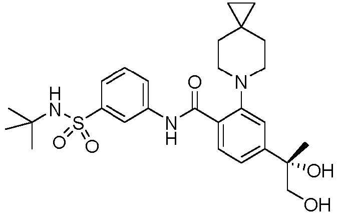 | (*S*)-*N*-(3-(*N*-(*tert-*Butyl)sulfamoyl)phenyl)-4-(1,2-dihydroxypropan-2-yl)-2-(6-azaspiro[2.5]octan-6-yl)benzamid |
| 109 | 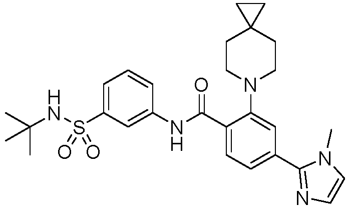 | *N*-(3-(*N*-(*tert-*Butyl)sulfamoyl)phenyl)-4-(1-methyl-1H-imidazol-2-yl)-2-(6-azaspiro[2.5]octan-6-yl)benzamid |
| 110 | 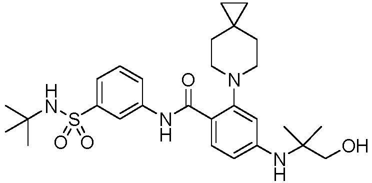 | *N*-(3-(*N*-(*tert-*Butyl)sulfamoyl)phenyl)-4-((1-hydroxy-2-methylpropan-2-yl)amino)-2-(6-azaspiro[2.5]octan-6-yl)benzamid |
| 111 | 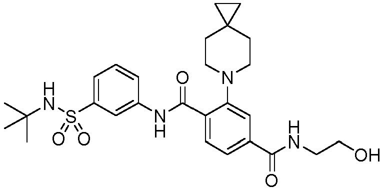 | *N*¹-(3-(*N*-(*tert-*Butyl)sulfamoyl)phenyl)-*N⁴-*(2-hydroxyethyl)-2-(6-azaspiro[2.5]octan-6-yl)terephthalamid |
| 112-1 | 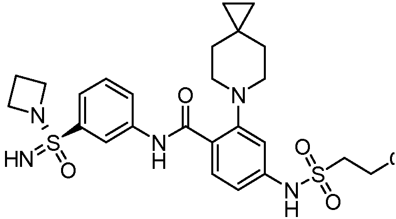 | (*R*)-*N*-(3-(Azetidin-1-sulfonimidoyl)phenyl)-4-((2-hydroxyethyl)sulfonamido)-2-(6-azaspiro[2.5]octan-6-yl)benzamid |
| 112-2 | 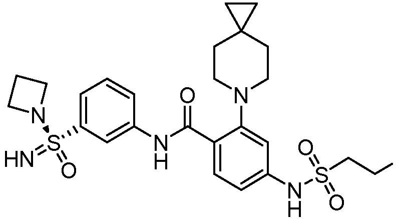 | (*S*)-*N*-(3-(Azetidin-1-sulfonimidoyl)phenyl)-4-((2-hydroxyethyl)sulfonamido)-2-(6-azaspiro[2.5]octan-6-yl)benzamid |
| 115 | 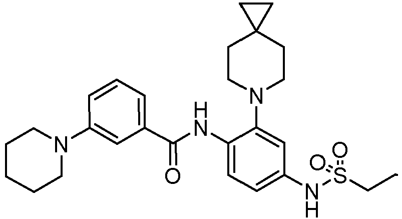 | *N*-(4-((2-Hydroxyethyl)sulfonamido)-2-(6-azaspiro[2.5]octan-6-yl)phenyl)-3-(piperidin-1-yl)benzamid |
; oder ein beliebiges pharmazeutisch annehmbares Salz davon.

25. Eine pharmazeutische Zusammensetzung, beinhaltend die Verbindung gemäß einem der vorhergehenden Ansprüche oder das pharmazeutisch annehmbare Salz davon und ein pharmazeutisch annehmbares Verdünnungsmittel oder Trägermittel.

26. Verbindung gemäß den Ansprüchen 1-24 oder das pharmazeutisch annehmbare Salz davon oder pharmazeutische Zusammensetzung gemäß Anspruch 25 zur Verwendung als ein Medikament.

27. Verbindung gemäß den Ansprüchen 1-24 oder das pharmazeutisch annehmbare Salz davon oder pharmazeutische Zusammensetzung gemäß Anspruch 25 zur Verwendung in einem Verfahren zum Behandeln einer Erkrankung, die mit KIF18A-Inhibitoren behandelt werden kann, wobei das Verfahren das Verabreichen einer therapeutisch wirksamen Menge der Verbindung gemäß einem der Ansprüche 1-24 oder der Zusammensetzung gemäß Anspruch 25 an einen Patienten, der dessen bedarf, beinhaltet, wobei die Erkrankung eine proliferative Störung, ausgewählt aus Krebs, Psoriasis, atopischer Dermatitis, einer Autoimmunstörung oder entzündlicher Darmkrankheit, ist; wobei der Krebs Melanom, Prostatakrebs, Gebärmutterhalskrebs, Brustkrebs, Darmkrebs, Sarkom oder Leukämie sein kann; wobei die Autoimmunstörung rheumatoide Arthritis, systemischer Lupus erythematodes, Sjögren-Syndrom, Sklerodermie, Mischkollagenose, Dermatomyositis, Polymyositis, Reiter-Syndrom, autoimmun-lymphoproliferatives Syndrom (ALPS), auch bekannt als Canale-Smith-Syndrom, oder eine Autoimmunkrankheit des zentralen Nervensystems, wie Multiple Sklerose, Myasthenia gravis und Enzephalomyelitis, sein kann; und wobei die entzündliche Darmkrankheit ulzerative Kolitis oder Morbus Crohn sein kann.

28. Verbindung gemäß den Ansprüchen 1-24 oder das pharmazeutisch annehmbare Salz davon oder pharmazeutische Zusammensetzung gemäß Anspruch 25 zur Verwendung in einem Verfahren zum Verringern der Größe eines soliden Tumors bei einem Individuum, wobei das Verfahren das Verabreichen einer therapeutisch wirksamen Menge der Verbindung gemäß einem der Ansprüche 1 bis 24 oder der Zusammensetzung gemäß Anspruch 25 an das Individuum, das dessen bedarf, beinhaltet.

29. Verbindung gemäß den Ansprüchen 1-24 oder das pharmazeutisch annehmbare Salz davon oder pharmazeutische Zusammensetzung gemäß Anspruch 25 zur Verwendung in einem Verfahren zum Behandeln einer Zellproliferationsstörung bei einem Individuum, wobei das Verfahren das Verabreichen einer therapeutisch wirksamen Menge der Verbindung gemäß einem der Ansprüche 1 bis 24 oder der Zusammensetzung gemäß Anspruch 25 an das Individuum, das dessen bedarf, beinhaltet.

30. Verbindung gemäß den Ansprüchen 1-24 oder das pharmazeutisch annehmbare Salz davon oder pharmazeutische Zusammensetzung gemäß Anspruch 25 zur Verwendung in einem "*Ex-vivo*"-Verfahren zum Hemmen von *KIF18A* in einer Zelle, beinhaltend das In-Kontakt-Bringen der Zelle mit einer Verbindung oder pharmazeutisch annehmbaren Salzen davon gemäß einem der Ansprüche 1 bis 24 oder der Zusammensetzung gemäß Anspruch 25.

## Revendications

1. Un composé de formule I : 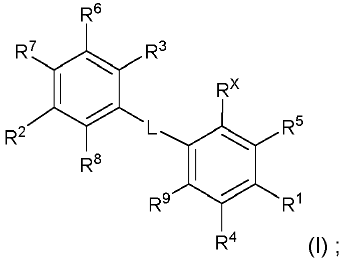 ou n'importe quel sel pharmaceutiquement acceptable de celui-ci, où :
R^{X} est 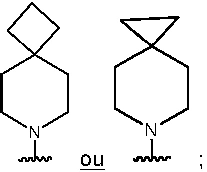
R¹ est un groupe -Z-R¹² ; où Z est absent, -alk en C₀₋₄-S-alk en C₀₋₄-, alk en C₀₋₄-S(=O)-alk en C₀₋₄-, -alk en C₀₋₄-SO₂-alk en C₀₋₄-, -alk en C₀₋₄-NR¹¹-alk en C₀₋₄-, -alk en C₀₋₄-NR¹¹SO₂-alk en C₀₋₄-, -alk en C₀₋₄-SO₂NR¹¹-alk en C₀₋₄-, -alk en C₀₋₄-NR¹¹SO₂NR¹¹-alk en C₀₋₄-, -alk en C₀₋₄-O-alk en C₀₋₄-, -alk en C₀₋₄-C(=O)-alk en C₀₋₄-, -alk en C₀₋₄-C(=O)-O-alk en C₀₋₄-, -alk en C₀₋₄-(C=O)NR¹¹-alk en C₀₋₄-, -alk en C₀₋₄-NR¹¹(C=O)-alk en C₀₋₄-, -alk en C₀₋₄-S(=O)(=NH)-, -N=S(=O)<, -(C=O)-, ou -C(=N-OH)- ;
R² est un groupe -Y-R¹³, où Y est-alk en C₀₋₄-S-alk en C₀₋₄-, alk en C₀₋₄-S(=O)-alk en C₀₋₄-, -alk en C₀₋₄-SO₂-alk en C₀₋₄-, -alk en C₀₋₄-NR^{13c}-alk en C₀₋₄-, -alk en C₀₋₄-SO₂NR^{13c}-alk en C₀₋₄-, -alk en C₀₋₄-NR^{13c}SO₂-alk en C₀₋₄-, -alk en C₀₋₄-S(=O)(=NH)-, -alk en C₀₋₄-O-alk en C₀₋₄-, -alk en C₀₋₄-C(=O)-alk en C₀₋₄-, -alk en C₀₋₄-C(=O)-O-alk en C₀₋₄-, -alk en C₀₋₄-(C=O)NR^{13c}-alk en C₀₋₄-, -alk en C₀₋₄-NR^{13c}(C=O)-alk en C₀₋₄-, ou - N=S(=O)< ;
R³ est H, un halo, un alk en C₁₋₄, ou un haloalk en C₁₋₄ ;
R⁴ est H, un halo, un alk en C₁₋₄, ou un haloalk en C₁₋₄ ;
R⁵ est H, un halo, un alk en C₁₋₈, ou un haloalk en C₁₋₄ ;
R⁶ est H, un halo, un alk en C₁₋₈, ou un haloalk en C₁₋₄ ;
R⁷ est H, un halo, un alk en C₁₋₄, ou un haloalk en C₁₋₄ ;
R⁸ est H, un halo, un alk en C₁₋₈, ou un haloalk en C₁₋₄ ;
ou autrement, R² et R⁸ peuvent se combiner avec les atomes de carbone attachés à chacun d'eux pour former un anneau monocyclique saturé ou partiellement saturé à 5 ou 6 chaînons fusionné à l'anneau phényle ; où ledit anneau monocyclique à 5 ou 6 chaînons contient 0, 1, 2 ou 3 atomes de N et 0, ou 1 atome sélectionné parmi O et S, et où en outre ledit anneau monocyclique à 5 ou 6 chaînons est substitué par 0, 1, 2 ou 3 groupe(s) sélectionné(s) parmi F, CI, Br, un alk en C₁₋₆, un haloalk en C₁₋₄, - OR^{a}, -Ohaloalk en C₁₋₄, CN, -NR^{a}R^{a}, ou un oxo ;
R⁹ est H, un halo, un alk en C₁₋₄, ou un haloalk en C₁₋₄ ;
L est -C=O)-NR¹⁰- ou -NR¹⁰-(C=O)- ;
R¹⁰ est H, ou un alk en C₁₋₄ ;
R¹¹ est H, ou un alk en C₁₋₄ ;
R¹² est R^{12a}, ou R^{12b} ;
R¹³ est un halo, R^{13a} ou R^{13b} ;
R ^{13c} est H ou un alk en C₁₋₄ ;
R^{12a} et R^{13a} sont indépendamment, dans chaque cas, sélectionnés dans le groupe constitué par un anneau monocyclique saturé, partiellement saturé ou insaturé à 3, 4, 5, 6, ou 7 chaînons ou un anneau bicyclique à 8, 9, 10, 11, ou 12 chaînons saturé, partiellement saturé ou insaturé contenant 0, 1, 2 ou 3 atomes de N et 0 ou 1 atome sélectionné parmi O et S, lequel est substitué par 0, 1, 2 ou 3 groupe(s) sélectionné(s) dans le groupe constitué par F, CI, Br, un alk en C₁₋₆, un haloalk en C₁₋₄, -OR^{a}, -Ohaloalk en C₁₋₄, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -Oalk en C₂₋₆NR^{a}R^{a}, -Oalk en C₂₋₆OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}alk en C₂₋₆NR^{a}R^{a}, -NR^{a}alk en C₂₋₆OR^{a}, -alk en C₁₋₆NR^{a}R^{a}, -alk en C₁₋₆OR^{a}, -alk en C₁₋₆N(R^{a})C(=O)R^{b}, -alk en C₁₋₆OC(=O)R^{b}, -alk en C₁₋₆C(=O)NR^{a}R^{a}, -alk en C₁₋₆C(=O)OR^{a}, un anneau monocyclique saturé, partiellement saturé ou insaturé à 3, 4, 5, ou 6 chaînons, et un oxo ;
R^{12b} et R^{13b} sont indépendamment, dans chaque cas, sélectionnés dans le groupe constitué par un alk en C₁₋₆ substitué par 0, 1, 2, 3, 4, ou 5 groupe(s) sélectionné(s) dans le groupe constitué par F, CI, Br, -CH₂F, -CHF₂, -
CF₃, -C(=O)OR^{a}, -OR^{a}, -Ohaloalk en C₁₋₄, CN, NH₂, NH(CH₃), N(CH₃)₂, et un anneau monocyclique saturé, partiellement saturé ou insaturé à 3, 4, 5, ou 6 chaînons ; R^{a} est indépendamment, dans chaque cas, H ou R^{b} ; et
R^{b} est indépendamment, dans chaque cas, un alk en C₁₋₆, un phényle, ou un benzyle, où l'alk en C₁₋₆ est substitué par 0, 1, 2 ou 3 substituants sélectionnés parmi un halo, -OH, -Oalk en C₁₋₄, -NH₂, -NHalk en C₁₋₄, -OC(=O)alk en C₁₋₄, ou -N(alk en C₁₋₄)alk en C₁₋₄ ; et le phényle ou benzyle est substitué par 0, 1, 2 ou 3 substituants sélectionnés parmi un halo, un alk en C₁₋₄, un haloalk en C₁₋₃, -OH, -Oalk en C₁₋₄, -NH₂, -NHalk en C₁₋₄, -OC(=O)alk en C₁₋₄, ou -N(alk en C₁₋₄)alk en C₁₋₄.

2. Le composé de la revendication 1 où L est -NR¹⁰-(C=O)-.

3. Le composé de la revendication 1 où L est -(C=O)-NR¹⁰-.

4. Le composé de la revendication 1 où R¹⁰ est H ou un méthyle.

5. Le composé de la revendication 1 où Z est absent, -SO₂-, -CH₂-SO₂, -NH-, -NHSO₂-, -SO₂NH-, -SO₂N(CH₃)-, -O-, -(C=O)O-, -(C=O)NH-, -(C=O)N(CH₃)-, -S(=O)(=NH)-, -CH₂-N(CH₃)-, ou -C(=N-OH)-.

6. Le composé de la revendication 1, où R¹² est sélectionné parmi :
a) H, F, Br, OH, ou CN ;
b) R^{12a} est sélectionné parmi un anneau monocyclique saturé, partiellement saturé ou insaturé à 3, 4, 5, 6, ou 7 chaînons contenant 0, 1, 2 ou 3 atomes de N et 0 ou 1 atome sélectionné parmi O et S, lequel est substitué par 0, 1, 2, ou 3 groupe(s) sélectionné(s) parmi F, CI, Br, un méthyle, un éthyle, -CF₃, -CH₂OH, -OH, -OCH₃, -NH₂, ou un oxo ; ou
c) R^{12b} sélectionné parmi un alk en C₁₋₆ substitué par 0, 1, 2 ou 3 groupe(s) sélectionné(s) parmi F, CI, Br, -CF₃, ou -OH.

7. Le composé de la revendication 1 où R¹² est R^{12a} sélectionné parmi un cyclopropyle, un oxétanyle, un imidazolyle, un isothiazolidinyle, un azétidinyle, un oxazolyle, un pyrazolyle, ou un diazirinyle ; dont chacun est indépendamment substitué par 0, 1, 2, ou 3 groupe(s) sélectionné(s) parmi un méthyle, un éthyle, -CF₃, ou un oxo ; ou R^{12b} sélectionné parmi un méthyle, un éthyle, un isopropyle, un tert-butyle, -éthényl, substitué par 0, 1, 2 ou 3 groupe(s) sélectionné(s) parmi F, CI, Br, -CF₃, ou -OH.

8. Le composé de la revendication 1 où R¹ est un groupe -Z-R¹², où Z est absent, -SO₂-, -CH₂SO₂-, -(C=O)NH-, -NH-, -NHSO₂- ou -SO₂NH- ; et R¹² est un anneau cyclopropyle, oxétanyle, azétidinyle, ou imidazolyle, dont chacun est indépendamment substitué par 0, 1, ou 2 groupe(s) sélectionné(s) parmi un méthyle, -CF₃, ou un oxo ; ou R¹² est un méthyle, un éthyle, un isopropyle, ou un tert-butyle, dont chacun est indépendamment substitué par 0, 1, 2, ou 3 groupe(s) F, -CF₃ ou OH.

9. Le composé de la revendication 1 où R¹ est un groupe -Z-R¹², où Z est -NHSO₂- et R¹² est -CH₂-CH₂-OH ou -CH(CH₃)CH₂OH.

10. Le composé de la revendication 1 où Y est absent, -SO₂NH-, NH-, -SO₂-, -S(=O)(=NH)-, ou -O-.

11. Le composé de la revendication 1 où R¹³ est H ou F ; R^{13a} sélectionné parmi un morpholinyle, un pipéridinyle, un cyclopentyle, un cyclopropyle, un azétidinyle, ou un oxétanyle ; où chaque dit anneau est substitué par 0, 1, 2 ou 3 groupe(s) OH sélectionnés parmi un méthyle ou -OH ; ou R^{13b} sélectionné parmi un méthyle, un éthyle, un propyle, un isopropyle, un tert-butyle, ou un isopentyle ; dont chacun est indépendamment substitué par 0, 1, 2, ou 3 groupe(s) OH.

12. Le composé de la revendication 1 où R² et R⁸ peuvent se combiner avec les atomes de carbone attachés à chacun d'eux pour former un anneau monocyclique saturé ou partiellement saturé à 6 chaînons fusionné à l'anneau phényle ; où ledit anneau monocyclique à 6 chaînons contient 0, 1, 2 ou 3 atomes de N et 0, ou 1 atome sélectionné parmi O et S, et où en outre ledit anneau monocyclique à 6 chaînons est substitué par 0, 1, 2 ou 3 groupe(s) sélectionné(s) parmi F, CI, Br, un alk en C₁₋₆, un haloalk en C₁₋₄, ou un oxo.

13. Le composé de la revendication 1 où R⁴ est H.

14. Le composé de la revendication 1 où R⁵ est H.

15. Le composé de la revendication 1 où R⁶ est H ou F.

16. Le composé de la revendication 1 où R⁷ est H ou F.

17. Le composé de la revendication 1 où R⁸ est H, F, ou un méthyle ; ou autrement, R² et R⁸ peuvent se combiner avec les atomes de carbone attachés à chacun d'eux pour former un anneau monocyclique saturé à 6 chaînons fusionné à l'anneau phényle ; sélectionné dans le groupe :

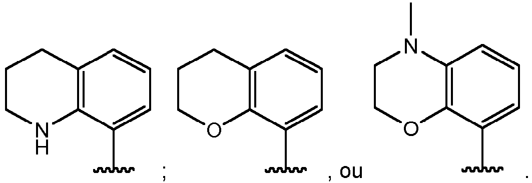

18. Le composé de la revendication 1 où R² est :
a) un groupe -Y-R^{13a}, où Y est absent ou -S(=O)(=NH)- ; et R¹³ est un pipéridinyle ou un azétidinyle ; où chaque dit anneau est indépendamment substitué par 0, 1, 2 ou 3 groupe(s) F ;
b) un groupe -Y-R^{13b}, où Y est -SO₂NH-, -O-, NH- ; et où R^{13b} est un tert-butyle substitué par 0, 1, 2, ou 3 groupe(s) OH ; ou
c) autrement, les atomes de carbone attachés à R² et R⁸ se combinent pour former un anneau monocyclique saturé à 6 chaînons fusionné à l'anneau phényle ;lequel est 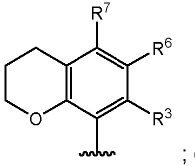 où chaque dit anneau monocyclique saturé à 6 chaînons est non substitué.

19. Le composé de la revendication 1 où R² est un groupe -SO₂NH-*tert*-butyle ou un groupe -NH-tert-butyl-OH.

20. Le composé de la revendication 1 où R⁸ est H.

21. Le composé de la revendication 1 où R⁹ est H.

22. Le composé de la revendication 1 où R¹⁰ est H.

23. Le composé de la revendication 1, ou le sel pharmaceutiquement acceptable de celui-ci, sélectionné dans le groupe constitué per :
N-(3-(N-(tert-butyl)sulfamoyl)phényl)-4-((3-méthyloxétan-3-yl)sulfonyl)-2-(6-azaspiro[2.5]octan-6-yl)benzamide ;
*N*-(3-(*N*-(*tert*-butyl)sulfamoyl)phényl)-4-(méthylsulfonyl)-2-(6-azaspiro[2.5]octan-6-yl)benzamide ;
*N*-(3-isopropylphényl)-4-(méthylsulfonyl)-2-(6-azaspiro[2.5]octan-6-yl)benzamide ;
*N*-(3-cyclopropylphényl)-4-(méthylsulfonyl)-2-(6-azaspiro[2.5]octan-6-yl)benzamide ;
*N*-(3-(*tert*-butyl)phényl)-4-(méthylsulfonyl)-2-(6-azaspiro[2.5]octan-6-yl)benzamide ;
4-(méthylsulfonyl)-*N*-(quinolin-8-yl)-2-(6-azaspiro[2.5]octan-6-yl)benzamide ;
*N*-(4-méthylquinolin-8-yl)-4-(méthylsulfonyl)-2-(6-azaspiro[2.5]octan-6-yl)benzamide ;
*N*-(chroman-8-yl)-4-(méthylsulfonyl)-2-(6-azaspiro[2.5]octan-6-yl)benzamide ;
*N*-(benzofuran-7-yl)-4-(méthylsulfonyl)-2-(6-azaspiro[2.5]octan-6-yl)benzamide ;
*N*-(benzo[b]thiophén-7-yl)-4-(méthylsulfonyl)-2-(6-azaspiro[2.5]octan-6-yl)benzamide ;
4-(méthylsulfonyl)-*N*-(3-morpholinophényl)-2-(6-azaspiro[2.5]octan-6-yl)benzamide ;
*N*-(3-(*N*-(*tert*-butyl)sulfamoyl)phényl)-4-((méthylsulfonyl)méthyl)-2-(6-azaspiro[2.5]octan-6-yl)benzamide ;
*N*-(3-(*N*-(*tert*-butyl)sulfamoyl)phényl)-4-(((2-hydroxyéthyl)sulfonyl)méthyl)-2-(6-azaspiro[2.5]octan-6-yl)benzamide ;
4-(*N-*(*tert-*butyl)sulfamoyl)-N-(3-(*N-*(*tert*-butyl)sulfamoyl)phényl)-2-(6-azaspiro[2.5]octan-6-yl)benzamide ;
*N*-(3-(*N*-(*tert*-butyl)sulfamoyl)phényl)-4-(3-méthyloxétan-3-yl)-2-(6-azaspiro[2.5]octan-6-yl)benzamide ;
*N*-(3-(*N*-(*tert*-butyl)sulfamoyl)phényl)-4-(3-hydroxyoxétan-3-yl)-2-(6-azaspiro[2.5]octan-6-yl)benzamide ;
N-(3-((1*-*hydroxy-2-méthylpropan-2-yl)amino)phényl)-4-(N-(3-méthyloxétan-3-yl)sulfamoyl)-2-(6-azaspiro[2.5]octan-6-yl)benzamide ;
*N*-(2-fluoro-3-((1-hydroxy-2-méthylpropan-2-yl)amino)phényl)-4-(*N*-(3-méthyloxétan-3-yl)sulfamoyl)-2-(6-azaspiro[2.5]octan-6-yl)benzamide ;
*N*-(2-fluoro-3-((1-hydroxy-2-méthylpropan-2-yl)amino)phényl)-4-((1-méthylcyclopropane)-1-sulfonamido)-2-(6-azaspiro[2.5]octan-6-yl)benzamide ;
*N*-(3-((1-hydroxy-2-méthylpropan-2-yl)amino)phényl)-4-((1-méthylcyclopropane)-1-sulfonamido)-2-(6-azaspiro[2.5]octan-6-yl)benzamide ;
N-(3-(N-(tert-Butyl)sulfamoyl)phényl)-4-((1-méthylcyclopropane)-1-sulfonamido)-2-(6-azaspiro[2.5]octan-6-yl)benzamide ;
*N*-(3-(*N*-(*tert*-butyl)sulfamoyl)phényl)-4-(méthylsulfonamido)-2-(6-azaspiro[2.5]octan-6-yl)benzamide ;
*N*-(3-(*N*-(*tert*-butyl)sulfamoyl)phényl)-4-(éthylsulfonamido)-2-(6-azaspiro[2.5]octan-6-yl)benzamide ;
*N*-(3-(*N*-(*tert*-butyl)sulfamoyl)phényl)-4-((1-méthyléthyl)sulfonamido)-2-(6-azaspiro[2.5]octan-6-yl)benzamide ;
*N*-(3-(*N*-(*tert*-butyl)sulfamoyl)phényl)-4-(cyclopropanesulfonamido)-2-(6-azaspiro[2.5]octan-6-yl)benzamide ;
*N*-(3-(*N*-(*tert*-butyl)sulfamoyl)phényl)-4-((1,1-diméthyléthyl)sulfonamido)-2-(6-azaspiro[2.5]octan-6-yl)benzamide ;
*N*-(3-(*N*-(*tert*-butyl)sulfamoyl)phényl)-4-(1,1-dioxidoisothiazolidin-2-yl)-2-(6-azaspiro[2.5]octan-6-yl)benzamide ;
N-(3-(N-(tert-butyl)sulfamoyl)phényl)-4-((2-hydroxyéthyl)sulfonamido)-2-(6-azaspiro[2.5]octan-6-yl)benzamide ;
(*R*)-4-((2-hydroxyéthyl)sulfonamido)-*N*-(3-(2-méthylmorpholino)phényl)-2-(6-azaspiro[2.5]octan-6-yl)benzamide ;
(*R*)-*N*-(2-fluoro-3-(2-méthylmorpholino)phényl)-4-((2-hydroxyéthyl)sulfonamido)-2-(6-azaspiro[2.5]octan-6-yl)benzamide ;
(*R*)-*N*-(3-fluoro-5-(2-méthylmorpholino)phényl)-4-((2-hydroxyéthyl)sulfonamido)-2-(6-azaspiro[2.5]octan-6-yl)benzamide ;
(*R*)-*N*-(4-fluoro-3-(2-méthylmorpholino)phényl)-4-((2-hydroxyéthyl)sulfonamido)-2-(6-azaspiro[2.5]octan-6-yl)benzamide ;
*N*-(3-(4,4-difluoropipéridin-1-yl)-5-méthylphényl)-4-((2-hydroxyéthyl)sulfonamido)-2-(6-azaspiro[2.5]octan-6-yl)benzamide ;
*N*-(3-(4,4-difluoropipéridin-1-yl)phényl)-4-((2-hydroxyéthyl)sulfonamido)-2-(6-azaspiro[2.5]octan-6-yl)benzamide ;
*N*-(3-(4,4-difluoropipéridin-1-yl)-2-fluorophényl)-4-((2-hydroxyéthyl)sulfonamido)-2-(6-azaspiro[2.5]octan-6-yl)benzamide ;
(*S*)-*N*-(3-(*N*-(*tert*-butyl)sulfamoyl)phényl)-4-((2-hydroxy-1-méthyléthyl)sulfonamido)-2-(6-azaspiro[2.5]octan-6-yl)benzamide ;
(*R*)-*N*-(3-(*N*-(*tert*-butyl)sulfamoyl)phényl)-4-((2-hydroxy-1-méthyléthyl)sulfonamido)-2-(6-azaspiro[2.5]octan-6-yl)benzamide ;
*N*-(3-(2-Hydroxy-2-méthylpropoxy)phényl)-4-((2-hydroxyéthyl)sulfonamido)-2-(6-azaspiro[2.5]octan-6-yl)benzamide ;
4-(Azétidin-1-ylsulfonyl)-*N*-(3-(*N*-(*tert*-butyl)sulfamoyl)phényl)-2-(6-azaspiro[2.5]octan-6-yl)benzamide ;
*N*-(3-(*N*-(*tert*-butyl)sulfamoyl)phényl)-4-(*N*-(1-hydroxy-2-méthylpropan-2-yl)sulfamoyl)-2-(6-azaspiro[2.5]octan-6-yl)benzamide ;
*N*-(3-(*N*-(*tert*-butyl)sulfamoyl)phényl)-4-(*N*-(2-hydroxyéthyl)sulfamoyl)-2-(6-azaspiro[2.5]octan-6-yl)benzamide ;
*N*-(3-(*N*-(*tert*-butyl)sulfamoyl)phényl)-4-(*N*,*N*-diméthylsulfamoyl)-2-(6-azaspiro[2.5]octan-6-yl)benzamide ;
*N*-(3-(*N*-(*tert*-butyl)sulfamoyl)phényl)-4-(*N*-méthylsulfamoyl)-2-(6-azaspiro[2.5]octan-6-yl)benzamide ;
*N*-(3-(*N*-(*tert*-butyl)sulfamoyl)phényl)-4-(*N*-(oxétan-3-yl)sulfamoyl)-2-(6-azaspiro[2.5]octan-6-yl)benzamide ;
*N-(3-(N-(tert-butyl)sulfamoyl)phényl)-4-(N-cyclopropylsulfamoyl)-2-(6-*azaspiro[2.5]octan-6-yl)benzamide ;
*N*-(3-(*N*-(*tert*-butyl)sulfamoyl)phényl)-4-(*N*-(1-méthylcyclopropyl)sulfamoyl)-2-(6-azaspiro[2.5]octan-6-yl)benzamide ;
*N*-(3-(*N*-(*tert*-butyl)sulfamoyl)phényl)-2-(6-azaspiro[2.5]octan-6-yl)-4-sulfamoylbenzamide ;
(*R*)-*N*-(3-(*N*-(*tert*-butyl)sulfamoyl)phényl)-4-(1,2-dihydroxypropan-2-yl)-2-(6-azaspiro[2.5]octan-6-yl)benzamide ;
(*S*)-*N*-(3-(*N*-(*tert*-butyl)sulfamoyl)phényl)-4-(1,2-dihydroxypropan-2-yl)-2-(6-azaspiro[2.5]octan-6-yl)benzamide ;
*N*-(3-(*N*-(*tert*-butyl)sulfamoyl)phényl)-4-(1-méthyl-1H-imidazol-2-yl)-2-(6-azaspiro[2.5]octan-6-yl)benzamide ;
*N*-(3-(*N*-(*tert*-butyl)sulfamoyl)phényl)-4-(1-méthyl-1H-pyrazol-5-yl)-2-(6-azaspiro[2.5]octan-6-yl)benzamide ;
*N*-(3-(*N*-(*tert*-butyl)sulfamoyl)phényl)-4-(1-méthyl-1H-pyrazol-4-yl)-2-(6-azaspiro[2.5]octan-6-yl)benzamide ;
*N*-(3-(*N*-(*tert*-butyl)sulfamoyl)phényl)-4-(oxazol-2-yl)-2-(6-azaspiro[2.5]octan-6-yl)benzamide ;
*N*-(3-(*N*-(*tert*-Butyl)sulfamoyl)phényl)-4-((1-hydroxy-2-méthylpropan-2-yl)amino)-2-(6-azaspiro[2.5]octan-6-yl)benzamide ;
*N*-(3-(*N*-(*tert*-butyl)sulfamoyl)phényl)-4-((2-hydroxyéthyl)amino)-2-(6-azaspiro[2.5]octan-6-yl)benzamide ;
*N*¹-(3-(*N*-(*tert*-Butyl)sulfamoyl)phényl)-*N*⁴-(2-hydroxyéthyl)-2-(6-azaspiro[2.5]octan-6-yl)téréphthalamide ;
*N¹*-(3-(*N*-(*tert*-butyl)sulfamoyl)phényl)-*N⁴*-méthyl-2-(6-azaspiro[2.5]octan-6-yl)téréphthalamide ;
*N*¹-(3-(*N*-(*tert*-butyl)sulfamoyl)phényl)-*N*⁴-(2-hydroxyéthyl)-*N*⁴-méthyl-2-(6-azaspiro[2.5]octan-6-yl)téréphthalamide ;
*N*¹-(3-(*N*-(*tert*-butyl)sulfamoyl)phényl)-*N*⁴-(1-hydroxy-2-méthylpropan-2-yl)-2-(6-azaspiro[2.5]octan-6-yl)téréphthalamide ;
*N*¹-(3-(cyclopentylsulfonyl)phényl)-*N*⁴-(2-hydroxyéthyl)-2-(6-azaspiro[2.5]octan-6-yl)téréphthalamide ;
(*R*)-*N*-(3-(Azétidine-1-sulfonimidoyl)phényl)-4-((2-hydroxyéthyl)sulfonamido)-2-(6-azaspiro[2.5]octan-6-yl)benzamide ;
(*S*)-*N*-(3-(Azétidine-1-sulfonimidoyl)phényl)-4-((2-hydroxyéthyl)sulfonamido)-2-(6-azaspiro[2.5]octan-6-yl)benzamide ;
(*R*)-4-((2-hydroxyéthyl)sulfonamido)-*N*-(3-(S-méthylsulfonimidoyl)phényl)-2-(6-azaspiro[2.5]octan-6-yl)benzamide ;
(*S*)-4-((2-hydroxyéthyl)sulfonamido)-*N*-(3-(S-méthylsulfonimidoyl)phényl)-2-(6-azaspiro[2.5]octan-6-yl)benzamide ;
(*R*)-4-((2-hydroxyéthyl)sulfonamido)-*N*-(3-(2-méthylpropan-2-ylsulfonimidoyl)phényl)-2-(6-azaspiro[2.5]octan-6-yl)benzamide ;
(*S*)-4-((2-hydroxyéthyl)sulfonamido)-*N*-(3-(2-méthylpropan-2-ylsulfonimidoyl)phényl)-2-(6-azaspiro[2.5]octan-6-yl)benzamide ; ou
*N*-(4-((2-hydroxyéthyl)sulfonamido)-2-(6-azaspiro[2.5]octan-6-yl)phényl)-3-(pipéridin-1-yl)benzamide.

24. Le composé de n'importe laquelle des revendications précédentes, sélectionné dans le groupe constitué par :
| Exempl e n° | Structure chimique | Nom |
|---|---|---|
| 100 | 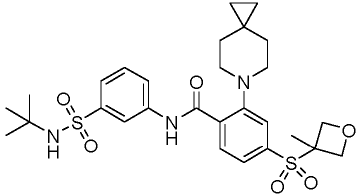 | N-(3-(N-(tert-Butyl)sulfamoyl)phényl)-4-((3-méthyloxétan-3-yl)sulfonyl)-2-(6-azaspiro[2.5]octan-6-yl)benzamide |
| 100-7 | 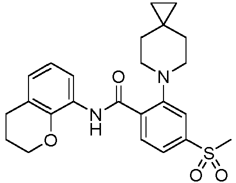 | *N*-(Chroman-8-yl)-4-(méthylsulfonyl)-2-(6-azaspiro[2.5]octan-6-yl)benzamide |
| 100-11 | 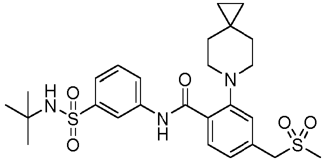 | *N-*(3-*(N-*(*tert-*Butyl)sulfamoyl)phényl)-4-((méthylsulfonyl)méthyl)-2-(6-azaspiro[2.5]octan-6-yl)benzamide |
| 100-13 | 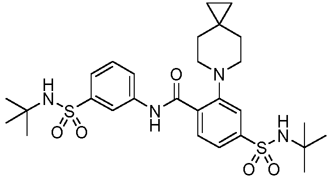 | 4-(*N*-(*tert*-Butyl)sulfamoyl)-N*-*(3-(*N-*(*tert-*butyl)sulfamoyl)phényl)-2-(6-azaspiro[2.5]octan-6-yl)benzamide |
| 101 | 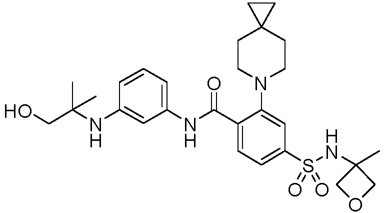 | N-(3-((1-Hydroxy-2-méthylpropan-2-yl)amino)phényl)-4-(N-(3-méthyloxétan-3-yl)sulfamoyl)-2-(6-azaspiro[2.5]octan-6-yl)benzamide |
| 101-1 | 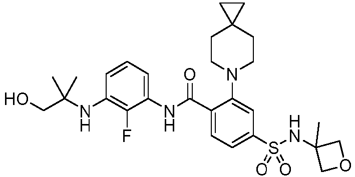 | *N*-(2-Fluoro-3-((1-hydroxy-2-méthylpropan-2-yl)amino)phényl)-4-(*N*-(3-méthyloxétan-3-yl)sulfamoyl)-2-(6-azaspiro[2.5]octan-6-yl)benzamide |
| 102 | 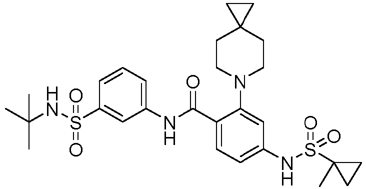 | N-(3-(N-(tert-Butyl)sulfamoyl)phényl)-4-((1-méthylcyclopropane)-1-sulfonamido)-2-(6-azaspiro[2.5]octan-6-yl)benzamide |
| 102-3 | 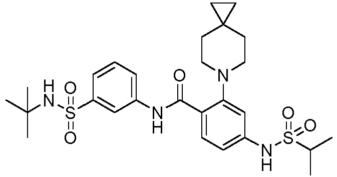 | *N-*(3-(*N-*(*tert-*Butyl)sulfamoyl)phényl)-4-((1-méthyléthyl)sulfonamido)-2-(6-azaspiro[2.5]octan-6-yl)benzamide |
| 102-4 | 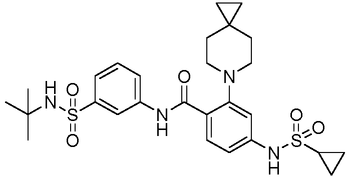 | *N-*(3*-*(*N-*(*tert-*Butyl)sulfamoyl)phényl)-4-(cyclopropanesulfonamido )-2-(6-azaspiro[2.5]octan-6-yl)benzamide |
| 103 | 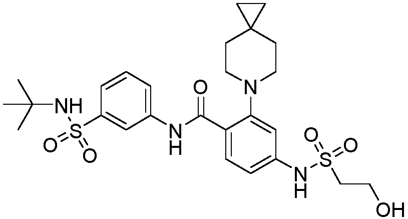 | N-(3-(N-(tert-Butyl)sulfamoyl)phényl)-4-((2-hydroxyéthyl)sulfonamido) -2-(6-azaspiro[2.5]octan-6-yl)benzamide |
| 104 | 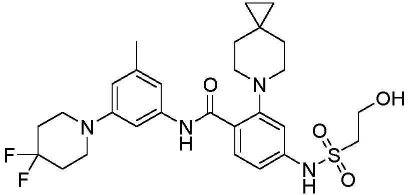 | *N*-(3-(4,4-Difluoropipéridin-1-yl)-5-méthylphényl)-4-((2-hydroxyéthyl)sulfonamido) -2-(6-azaspiro[2.5]octan-6-yl)benzamide |
| 104-1 | 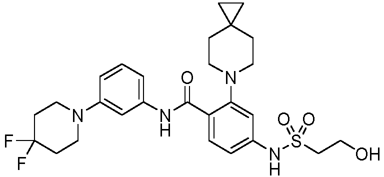 | *N*-(3-(4,4-Difluoropipéridin-1-yl)phényl)-4-((2-hydroxyéthyl)sulfonamido) -2-(6-azaspiro[2.5]octan-6-yl)benzamide |
| 104-2 | 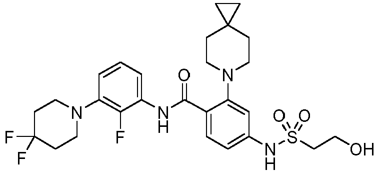 | *N*-(3-(4,4-Difluoropipéridin-1-yl)-2-fluorophényl)-4-((2-hydroxyéthyl)sulfonamido) -2-(6-azaspiro[2.5]octan-6-yl)benzamide |
| 105-1 | 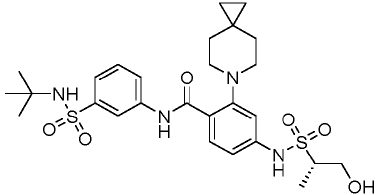 | (*S*)*-N-*(3-(*N-*(*tert-*Butyl)sulfamoyl)phényl)-4-((2-hydroxy-1-méthyléthyl)sulfonamido)-2-(6-azaspiro[2.5]octan-6-yl)benzamide |
| 105-2 | 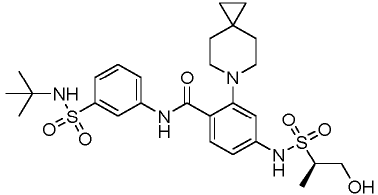 | (*R*)*-N-*(3-(*N-*(*tert-*Butyl)sulfamoyl)phényl)-4-((2-hydroxy-1-méthyléthyl)sulfonamido)-2-(6-azaspiro[2.5]octan-6-yl)benzamide |
| 106 | 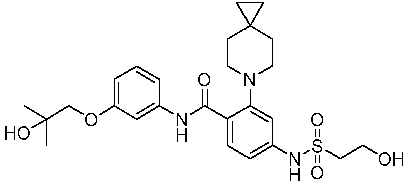 | *N*-(3-(2-Hydroxy-2-méthylpropoxy)phényl)-4-((2-hydroxyéthyl)sulfonamido) -2-(6-azaspiro[2.5]octan-6-yl)benzamide |
| 107 | 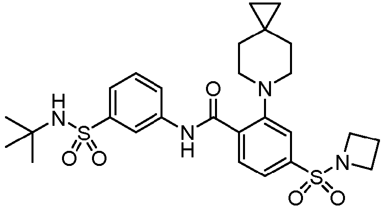 | 4-(Azétidin-1-ylsulfonyl)-*N-*(3-(*N-*(*tert-*butyl)sulfamoyl)phényl)-2-(6-azaspiro[2.5]octan-6-yl)benzamide |
| 108-1 | 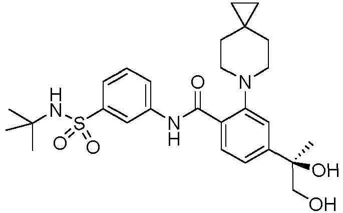 | (*R*)*-N-*(3-(*N-*(*tert-*Butyl)sulfamoyl)phényl)-4-(1,2-dihydroxypropan-2-yl)-2-(6-azaspiro[2.5]octan-6-yl)benzamide |
| 108-2 | 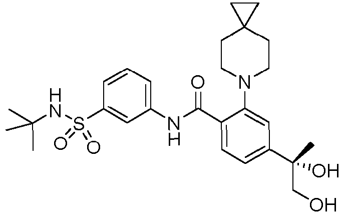 | (*S*)*-N-*(3*-*(*N-*(*tert-*Butyl)sulfamoyl)phényl)-4-(1,2-dihydroxypropan-2-yl)-2-(6-azaspiro[2.5]octan-6-yl)benzamide |
| 109 | 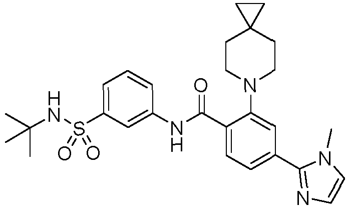 | *N-*(3-(*N-*(*tert-*Butyl)sulfamoyl)phényl)-4-(1-méthyl-1H-imidazol-2-yl)-2-(6-azaspiro[2.5]octan-6-yl)benzamide |
| 110 | 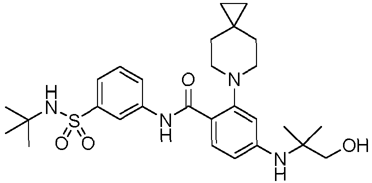 | *N-*(3-(*N-*(*tert-*Butyl)sulfamoyl)phényl)-4-((1-hydroxy-2-méthylpropan-2-yl)amino)-2-(6-azaspiro[2.5]octan-6-yl)benzamide |
| 111 | 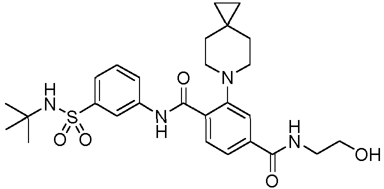 | *N*¹*-*(3-(*N-*(*tert-*Butyl)sulfamoyl)phényl)-*N*⁴-(2-hydroxyéthyl)-2-(6-azaspiro[2.5]octan-6-yl)téréphthalamide |
| 112-1 | 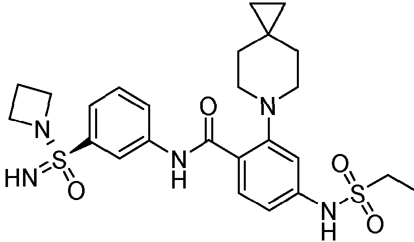 | (*R*)-*N*-(3-(Azétidine-1-sulfonimidoyl)phényl)-4-((2-hydroxyéthyl)sulfonamido) -2-(6-azaspiro[2.5]octan-6-yl)benzamide |
| 112-2 | 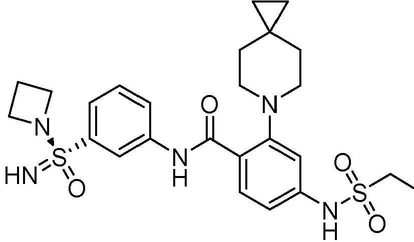 | (*S*)-*N*-(3-(Azétidine-1-sulfonimidoyl)phényl)-4-((2-hydroxyéthyl)sulfonamido) -2-(6-azaspiro[2.5]octan-6-yl)benzamide |
| 115 | 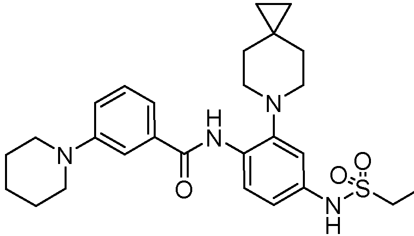 | *N*-(4-((2-Hydroxyéthyl)sulfonamido) -2-(6-azaspiro[2.5]octan-6-yl)phényl)-3-(pipéridin-1-yl)benzamide |
; ou n'importe quel sel pharmaceutiquement acceptable de celui-ci.

25. Une composition pharmaceutique comprenant le composé selon n'importe laquelle des revendications précédentes ou le sel pharmaceutiquement acceptable de celui-ci, et un diluant ou véhicule pharmaceutiquement acceptable.

26. Un composé selon les revendications 1 à 24 ou le sel pharmaceutiquement acceptable de celui-ci, ou une composition pharmaceutique selon la revendication 25 pour son utilisation comme médicament.

27. Un composé selon les revendications 1 à 24 ou le sel pharmaceutiquement acceptable de celui-ci, ou une composition pharmaceutique selon la revendication 25 pour son utilisation dans une méthode de traitement d'une affection pouvant être traitée par des inhibiteurs de KIF18a, la méthode comprenant le fait d'administrer à un patient qui en a besoin une quantité thérapeutiquement efficace du composé conformément à n'importe laquelle des revendications 1 à 24, ou de la composition selon la revendication 25, où ladite affection est un trouble prolifératif sélectionné parmi un cancer, un psoriasis, la dermatite atopique, un trouble auto-immun, ou une maladie inflammatoire de l'intestin ; où ledit cancer peut être un mélanome, un cancer de la prostate, un cancer du col de l'utérus, un cancer du sein, un cancer du côlon, un sarcome, ou une leucémie ; où ledit trouble auto-immun peut être la polyarthrite rhumatoïde, le lupus érythémateux systémique, le syndrome de Sjögren, la sclérodermie, la connectivite mixte, la dermatomyosite, la polymyosite, le syndrome de Reiter, le syndrome lymphoprolifératif auto-immun (SLAI), également appelé syndrome de Canale-Smith, ou une maladie auto-immune du système nerveux central, comme la sclérose en plaques, la myasthénie grave, et l'encéphalomyélite ; et où la maladie inflammatoire de l'intestin peut être la rectocolite hémorragique ou la maladie de Crohn.

28. Un composé selon les revendications 1 à 24 ou le sel pharmaceutiquement acceptable de celui-ci, ou une composition pharmaceutique selon la revendication 25 pour son utilisation dans une méthode de réduction de la taille d'une tumeur solide chez un sujet, la méthode comprenant le fait d'administrer au sujet qui en a besoin une quantité thérapeutiquement efficace du composé conformément à n'importe laquelle des revendications 1 à 24, ou de la composition selon la revendication 25.

29. Un composé selon les revendications 1 à 24 ou le sel pharmaceutiquement acceptable de celui-ci, ou une composition pharmaceutique selon la revendication 25 pour son utilisation dans une méthode de traitement d'un trouble de la prolifération cellulaire chez un sujet, la méthode comprenant le fait d'administrer au sujet qui en a besoin une quantité thérapeutiquement efficace du composé conformément à n'importe laquelle des revendications 1 à 24, ou de la composition selon la revendication 25.

30. Un composé selon les revendications 1 à 24 ou le sel pharmaceutiquement acceptable de celui-ci, ou une composition pharmaceutique selon la revendication 25 pour son utilisation dans une méthode « *ex vivo* » d'inhibition de KIF18A dans une cellule, comprenant le fait de mettre en contact la cellule avec un composé, ou des sels pharmaceutiquement acceptables de celui-ci, conformément à n'importe laquelle des revendications 1 à 24, ou la composition selon la revendication 25.
